# EUROPEAN PATENT APPLICATION

(11) **EP 2 239 012 A2**
(43) Date of publication of application: **13.10.2010**
(21) Application number: 10164336.9
(22) Date of filing: 06.04.2004
(51) Int. Cl.: A61P 3/04, A61K 31/16, A61K 31/415

(54) **Pharmaceutical use of substituted amides**

(30) Priority: 11.04.2003 DK 200300565; 02.05.2003 US 467800 P; 27.06.2003 DK 200300972; 30.06.2003 DK 200300988; 30.06.2003 DK 200300989; 30.06.2003 DK 200300990; 02.07.2003 DK 200300998; 10.07.2003 US 486095 P; 10.07.2003 US 486097 P; 10.07.2003 US 486094 P; 10.07.2003 US 486078 P; 10.07.2003 US 486098 P; 22.12.2003 DK 200301910; 06.01.2004 DK 200400009; 16.01.2004 US 537099 P
(62) Divisional of application: 04725891.8
(71) Applicant: High Point Pharmaceuticals, LLC, High Point, NC 27265 (US)
(72) Inventor: Andersen, Henrik Sune, 2800/DK, Lyngby (DK); Kampen, Gita Camilla Tejlgaard, 2850/DK, Naerum (DK); Christensen, Inge Thoger, 2800/DK, Lyngby (DK); Mogensen, John Patrick, 2730/DK, Herlev (DK); Larsen, Annette Rosendal, 2800/DK, Lyngby (DK); Kilburn, John Paul, 4690/DK, Haslev (DK)
(74) Representative: Russell, Lindsey

(57) **Abstract**

The use of substituted amides for modulating the activity of 11β-hydroxysteroid dehydrogenase type 1 (11βHSD1) and the use of these compounds as pharmaceutical compositions, are described. Also a novel class of substituted amides, their use in therapy, pharmaceutical compositions comprising the compounds, as well as their use in the manufacture of medicaments are described. The present compounds are modulators and more specifically inhibitors of the activity of 11βHSD1 and may be useful in the treatment, prevention and/or prophylaxis of a range of medical disorders where a decreased intracellular concentration of active glucocorticoid is desirable.

## Description

### FIELD OF INVENTION

The present invention relates to use of substituted amides and pharmaceutical compositions comprising the compounds for treating disorders where it is desirable to modulate the activity of 11β-hydrooysteroid dehydrogenase type 1 (11βHSD1).

The present invention also relates to novel substituted amides, to their use in therapy, to pharmaceutical compositions comprising the compounds, to the use of said compounds in the manufacture of medicaments, and to therapeutic methods comprising the administration of said compounds. The present compounds modulate the activity of 11β-hydroxysteroid dehydrogenase type 1 (11βHSD1) and are accordingly useful in the treatment of diseases in which such a modulation is beneficial, such as the metabolic syndrome.

### BACKGROUND OF THE INVENTION

The metabolic syndrome is a major global health problem. In the US, the prevalence in the adult population is currently estimated to be approximately 25%, and it continues to increase both in the US and worldwide. The metabolic syndrome is characterised by a combination of insulin resistance, dyslipidemia, obesity and hypertension leading to increased morbidity and mortality of cardiovascular diseases. People with the metabolic syndrome are at increased risk of developing frank type 2 diabetes, the prevalence of which is equally escalating.

In type 2 diabetes, obesity and dyslipidemia are also highly prevalent and around 70% of people with type 2 diabetes additionally have hypertension once again leading to increased mortality of cardiovascular diseases.

In the clinical setting, it has long been known that glucocorticoids are able to induce all of the cardinal features of the metabolic syndrome and type 2 diabetes.

11β-hydroxysteroid dehydrogenase type 1 (11βHSD1) catalyses the local generation of active glucocorticoid in several tissues and organs including predominantly the liver and adipose tissue, but also e.g. skeletal muscle, bone, pancreas, endothelium, ocular tissue and certain parts of the central nervous system. Thus, 11βHSD1 serves as a local regulator of glucocorticoid actions in the tissues and organs where it is expressed (Tannin et al., J. Biol. Chem., 266, 16653 (1991); Bujalska et al., Endocrinology, 140, 3188 (1999); Whorwood et al., J Clin Endocrinol Metab., 86, 2296 (2001); Cooper et al., Bone, 27, 375 (2000); Davani et al., J. Biol. Chem., 275, 34841 (2000); Brem et al., Hypertension, 31, 459 (1998); Rauz et al., Invest. Ophthalmol. Vis. Sci., 42, 2037 (2001); Moisan et al., Endocrinology, 127, 1450 (1990)).

The role of 11βHSD1 in the metabolic syndrome and type 2 diabetes is supported by several lines of evidence. In humans, treatment with the non-specific 11βHSD1 inhibitor carbenoxolone improves insulin sensitivity in lean healthy volunteers and people with type 2 diabetes. Likewise, 11βHSD1 knock-out mice are resistant to insulin resistance induced by obesity and stress. Additionally, the knock-out mice present with an anti-atherogenic lipid profile of decreased VLDL triglycerides and increased HDL-cholesterol. Conversely, mice that overexpress 11βHSD1 in adipocytes develop insulin resistance, hyperlipidemia and visceral obesity, a phenotype that resembles the human metabolic syndrome (Andrews et al., J. Clin. Endocrinol. Metab., 88, 285 (2003); Walker et al., J. Clin. Endocrinol. Metab., 80, 3155 (1995); Morton et al., J. Biol. Chem., 276, 41293 (2001); Kotelevtsev et al., Proc. Natl. Acad. Sci. USA, 94, 14924 (1997); Masuzaki et al., Science, 294, 2166 (2001)).

The more mechanistic aspects of 11βHSD1 modulation and thereby modulation of intracellular levels of active glucocorticoid have been investigated in several rodent models and different cellular systems. 11βHSD1 promotes the features of the metabolic syndrome by increasing hepatic expression of the rate-limiting enzymes in gluconeogenesis, namely phosphoenolpyuvate carboxykinase and glucose-6-phosphatase, promoting the differentiation of preadipocytes into adipocytes thus facilitating obesity, directly and indirectly stimulating hepatic VLDL secretion, decreasing hepatic LDL uptake and increasing vessel contractility (Kotelevtsev et al., Proc. Natl. Acad. Sci. USA, 94, 14924(1997); Morton et al., J. Biol. Chem. 276, 41293 (2001); Bujaiska et al., Endocrinology, 140, 3188 (1999); Souness et al., Steroids, 67, 195 (2002), Brindley & Salter, Prog. Lipid Res., 30, 349 (1991)).

WO 01/90090, WO 01/90091, WO 01/90092, WO 01/90093 and WO 01/90094 discloses various thiazol-sulfonamides as inhibitors of the human 11β-hydroxysteroid dehydrogenase type 1 enzyme, and further states that said compounds may be useful in treating diabetes, obesity, glaucoma, osteoporosis, cognitive disorders, immune disorders and depression.

We have now found substituted amides that modulate the activity of 11βHSD1 leading to altered intracellular concentrations of active glucocorticoid. More specifically, the present compounds inhibit the activity of 11βHSD1 leading to decreased intracellular concentrations of active glucocorticoid. Thus, the present compounds can be used to treat disorders where a decreased level of active intracellular glucocorticoid is desirable, such as e.g. the metabolic syndrome, type 2 diabetes, impaired glucose tolerance (IGT), impaired fasting glucose (IFG), dyslipidemia, obesity, hypertension, diabetic late complications, cardiovascular diseases, arteriosclerosis, atherosclerosis, myopathy, muscle wasting, osteoporosis, neurodegenerative and psychiatric disorders, and adverse effects of treatment or therapy with glucocorticoid receptor agonists.

One object of the present invention is to provide compounds, pharmaceutical compositions and use of compounds that modulate the activity of 11βHSD1.

### DEFINITIONS

In the following structural formulas and throughout the present specification, the following terms have the indicated meaning:
The term "halo" includes fluorine, chlorine, bromine, and iodine.
The term "trihalomethyl" includes trifluoromethyl, trichloromethyl, tribromomethyl, and triiodomethyl.
The term "trihalomethoxy" includes trifluorometoxy, trichlorometoxy, tribromometoxy, and triiodometoxy.
The term "alkyl" includes C₁-C₈ straight chain saturated and methylene aliphatic hydrocarbon groups, C₃-C₈ branched saturated hydrocarbon groups having the specified number of carbon atoms. For example, this definition shall include but is not limited to methyl (Me), ethyl (Et), propyl (Pr), butyl (Bu), pentyl, hexyl, isopropyl (i-Pr), isobutyl (i-Bu), *tert*-butyl (*t*-Bu), sec-butyl (*s*-Bu), isopentyl, neopentyl, and the like.
The term "alkenyl" includes C₂-C₈ straight chain unsaturated aliphatic hydrocarbon groups and branched C₃-C₈ unsaturated aliphatic hydrocarbon groups having the specified number of carbon atoms. For example, this definition shall include but is not limited to ethenyl, propenyl, butenyl, pentenyl, hexenyl, methylpropenyl, methylbutenyl and the like.
The term "alkynyl" includes C₂-C₆ straight chain unsaturated aliphatic hydrocarbon groups and C₄-C₆ branched unsaturated aliphatic hydrocarbon groups having the specified number of carbon atoms. For example, this definition shall include but is not limited to ethynyl, propynyl, butynyl, pentynyl, hexynyl, methylbutynyl, and the like.
The term "saturated or partially saturated cyclic, bicyclic or tricyclic ring system" represents but are not limit to aziridinyl, azepanyl, azocanyl, pyrrolinyl, pyrrolidinyl, 2-imidazolinyl, imidazolidinyl, 2-pyrazolinyl, morpholinyl, piperidinyl, thiomorpholinyl, piperazinyl, phthalimide, 1,2,3,4-tetrahydro-quinolinyl, 1,2,3,4-tetrahydro-isoquinolinyl, 1,2,3,4-tetrahydro-quinoxalinyl, indolinyl, 1, 6-aza-bicyclo[3.2.1]octane, 2-aza-bicyclo[4.1.1]octane. 2-aza-bicyclo[3.2.1]octanyl, 7-aza-bicyclo[4.1.1]octanyl, 9-aza-bicyclo[3.3.2]decanyl, 4-aza-tricyclo[4.3.1.1^{3,8}]undecanyl, 9-aza-tricyclo[3.3.2.0^{3,7}]decanyl.
The term "saturated or partially saturated cyclic ring system" represents but are not limited to cyclopropyl, cyclobutyl, cyclopentyl, cyclohexyl, cyclobutenyl, cyclopentenyl, cyclohexenyl, cycloheptenyl, cyclooctenyl, cyclononenyl, cyclodecenyl, tetrahydrofuranyl or tetrahydropyranyl.
The term "saturated or partially saturated aromatic ring system" represents but are not limited to cyclopentyl, cyclohexyl, cyclobutenyl, cyclopentenyl, cyclohexenyl, cycloheptenyl, cyclooctenyl, cyclononenyl, cyclodecenyl, tetrahydrofuranyl, tetrahydropyranyl, phenyl, pyridyl or pyrimidinyl.
The term "cycloalkyl" (e.g. cyclopropyl, cyclobutyl, cyclopentyl, cyclohexyl, cycloheptyl, cyclooctyl, cyclononyl, cyclodecyl, bicyclo[3.2.1]octyl, spiro[4.5]decyl, norpinyl, norbonyl, norcaryl, adamantyl and the like) represents a saturated, mono-, bi-, tri- or spirocarbocyclic group having the specified number of carbon atoms.
The term "cycloalkylalkyl" (e.g. cyclopropylmethyl, cyclobutylethyl, adamantylmethyl and the like) represents a cycloalkyl group as defined above attached through an alkyl group having the indicated number of carbon atoms or substituted alkyl group as defined above.
The term "cycloalkenyl" (e.g. cyclobutenyl, cyclopentenyl, cyclohexenyl, cycloheptenyl, cyclooctenyl, cyclononenyl, cyclodecenyl and the like) represents a partially saturated, mono-, bi-, tri- or spirocarbocyclic group having the specified number of carbon atoms.
The term "cycloalkylcarbonyl" (e.g. cyclopropylcarbonyl, cyclohexylcarbonyl) represents an cycloalkyl group as defined above having the indicated number of carbon atoms attached through a carbonyl group.
The term "hetcycloalkylcarbonyl" (e.g. 1-piperidin-4-yl-carbonyl, 1-(1,2,3,4-tetrahydro-isoquinolin-6-yl)carbonyl) represents an hetcycloalkyl, group as defined above having the indicated number of carbon atoms attached through a carbonyl group.
The term "cycloalkylalkylcarbonyl" (e.g. cyclohexylmethylcarbonyl, cycloheptylethylcarbonyl and the like) represents a cycloalkyl group as defined above attached through an alkyl group having the indicated number of carbon atoms or substituted alkyl group as defined above.
The term "hetcycloalkyl" (tetrahydrofuranyl, tetrahydropyranyl, tertahydrothiopyranyl, piperidine, pyridzine and the like) represents a saturated mono-, bi-, tri- or spirocarbocyclic group having the specified number of carbon atoms and one or two additional heteroatoms or groups selected from nitrogen, oxygen, sulphur, SO or SO₂.
The term "hetcycloalkylalkyl" (e.g. tetrahydrofuranylmethyl, tetrahydropyranylethyl, tertahydrothlopyranylmethyl, and the like) represents a hetcycloalkyl group as defined above attached through an alkyl group having the indicated number of carbon atoms or substituted alkyl group as defined above.
The term "alkyloxy" (e.g. methoxy, ethoxy, propyloxy, allyloxy, cyclohexyloxy) represents an alkyl group as defined above having the indicated number of carbon atoms attached through an oxygen bridge.
The term "alkyloxyalkyl" (e.g. methyloxymethyl and the like) represents an alkyloxy group as defined above attached through an "alkyl" group.
The term "aryloxyhetaryl" (e.g. 2-phenoxy-pyridyl and the like) represents an aryloxy group as defined below attached through a"hetaryl" group.
The term "aryloxy" (e.g. phenoxy, naphthyloxy and the like) represents an aryl group as defined below attached through an oxygen bridge.
The term "hetaryloxy" (e.g. 2-pyridyloxy and the like) represents a hetaryl group as defined below attached through an oxygen bridge.
The term "arylalkyloxy" (e.g. phenethyloxy, naphthylmethyloxy and the like) represents an arylalkyl group as defined below attached through an oxygen bridge.
The term "hetarylalkyloxy" (e.g. 2-pyridylmethyloxy and the like) represents a hetarylalkyl group as defined below attached through an oxygen bridge.
The term "alkyloxycarbonyl" (e.g. methylformiat, ethylformiat and the like) represents an alkyloxy group as defined above attached through a carbonyl group.
The term "aryloxycarbonyl" (e.g. phenylformiat, 2-thiazolylformiat and the like) represents an aryloxy group as defined above attached through a carbonyl group.
The term "arylalkyloxycarbonyl" (e.g. benzylformiat, phenyletylformiat and the like) represents an "arylalkyloxy" group as defined above attached through a carbonyl group.
The term "alkylthio" (e.g. methylthio, ethylthio and the like) represents an alkyl group as defined above attached through a sulphur bridge.
The term "arylthio" (e.g. benzenthiol, naphthylthiol and the like) represents an aryl group as defined below attached through a sulphur bridge.
The term "hetarylthio" (e.g. pyridine-2-thiol, thiazole-2-thiol and the like) represents an hetaryl group as defined below attached through a sulphur bridge.
The term "arylthioalkyl" (e.g. methylsulfanyl benzene, ethylsulfanyl naphthalene and the like) represents an arylthio group as defined below attached through an alkyl group having the indicated number of carbon atoms.
The term "hetarylthioalkyl" (e.g. 2-methylsulfanyl-pyridine, 1-ethylsulfanyl-isoquinoline and the like) represents a hetarylthio group as defined below attached through an alkyl group having the indicated number of carbon atoms.
The term "hetaryloxyaryl" (e.g. 1-phenoxy-isoquinolyl, 2-phenoxypyridyl and the like) represents a hetaryloxy group as defined above attached through an "aryl" group as defined below.
The term "hetaryloxyhetaryl" (e.g. 1-(2-pyridyloxy-isoquinoline), 2-(imidazol-2-yloxy-pyridine) and the like) represents a hetaryloxy group as defined above attached through a "hetaryl" group as defined below.
The term "aryloxyalkyl" (e.g. phenoxymethyl, naphthyloxyethyl and the like) represents an aryloxy group as defined above attached through an "alkyl" group having the indicated number of carbon atoms.
The term "aryloxaryl" (e.g. 1-phenoxy-naphthalene, phenyloxyphenyl and the like) represents an aryloxy group as defined above attached through an "aryl" group as defined below.
The term "arylalkyloxyalkyl" (e.g. ethoxymethyl-benzene, 2-methoxymethyl-naphthalene and the like) represents an arylalkyloxy group as defined above attached through an "alkyl" group having the indicated number of carbon atoms.
The term "hetaryloxyalkyl" (e.g. 2-pyridyloxymethyl, 2-quinolyloxyethyl and the like) represents a hetaryloxy group as defined above attached through an "alkyl" group having the indicated number of carbon atoms.
The term "hetarylalkyloxyalkyl" (e.g. 4-methoxymethyl-pyrimidine, 2-methoxymethyl-quinoline and the like) represents a hetarylalkyloxy group as defined above attached through an "alkyl" group having the indicated number of carbon atoms.
The term "arylalkyl" (e.g. benzyl, phenylethyl, 3-phenylpropyl, 1-naphtylmethyl, 2-(1-naphtyl)ethyl and the like ) represents an aryl group as defined below attached through an alkyl having the indicated number of carbon atoms or substituted alkyl group as defined above.
The term "hetarylalkyl" or "hetaralkyl" (e.g. (2-furyl)methyl, (3-furyl)methyl, (2-thienyl)methyl, (3-thienyl)methyl, (2-pyridyl)methyl, 1-methy[-1-(2-pyrimidyl)ethyl and the like) represents a hetaryl group as defined below attached through an alkyl having the indicated number of carbon atoms or substituted alkyl group as defined above.
The term "alkylcarbonyl" (e.g. octylcarbonyl, pentylcarbonyl, 3-hexenylcarbonyl) represents an alkyl group as defined above having the indicated number of carbon atoms attached through a carbonyl group.
The term "arylcarbonyl" (e.g. benzoyl) represents an aryl group as defined below attached through a carbonyl group.
The term "hetarylcarbonyl" (e.g. 2-thiophenylcarbonyl, 3-methoxy-anthrylcarbonyl, oxazolylcarbonyl and the like) represents a hetaryl group as defined below attached through a carbonyl group.
The term "carbonylalkyl" (e.g. acetyl and the like) represents a carbonyl group attached through alkyl group as defined above having the indicated number of carbon atom.
The term "alkylcarbonylalkyl" (e.g. propan-2-one, 4,4-dimethyl-pentan-2-one and the like) represents an alkylcarbonyl group as defined above attached through an alkyl group as defined above having the indicated number of carbon atoms.
The term "arylcarbonylalkyl" (e.g. 1-phenyl-propan-1-one, 1-(3-chloro-phenyl)-2-methyl-butan-1-one and the like) represents a arylcarbonyl group as defined above attached through an alkyl group as defined above having the indicated number of carbon atoms.
The term "hetarylcarbonylalkyl" (e.g. 1-pyridin-2-yl-propan-1-one, 1-(1-*H*-imidazol-2-yl)-propan-1-one and the like) represents a hetarylcarbonyl group as defined above attached through an alkyl group as defined above having the indicated number of carbon atoms.
The term "arylalkylcarbonyl" (e.g. phenylpropylcarbonyl, phenylethylcarbonyl and the like) represents an arylalkyl group as defined above having the indicated number of carbon atoms attached through a carbonyl group.
The term "hetarylalkylcarbonyl" (e.g. imidazolylpentylcarbonyl and the like) represents a hetarylalkyl group as defined above wherein the alkyl group is in turn attached through a carbonyl.
The term "alkylcarbonylamino" (e.g. methylcarbonylamino, cyclopentylcarbonylaminomethyl, methylcarbonylaminophenyl) represents an "alkylcarbonyl" group as defined above wherein the carbonyl is in turn attached through the nitrogen atom of an amino group. The nitrogen atom may itself be substituted with an alkyl or aryl group.
The term "alkylcarbonylaminoalkyl" (e.g.N-prapyl-acetamide, N-butyl-propionamide and the like) represents an "alkylcarbonylamino" group attached through an alkyl group as defined above having the indicated number of carbon atoms.
The term "arylalkylcarbonylamino" (e.g. phenylacetamide, 3phenyl-propionamide and the like) represents an "arylalkylcarbonyl" group as defined above attached through an amino group.
The term "arylalkylcarbonylaminoalkyl" (e.g. N-ethyl-phenylacetamide, N-butyl-3-phenyl-propionamide and the like) represents an "arylalkylcarbonylamino" group attached through an alkyl group as defined above having the indicated number of carbon atoms.
The term "arylcarbonylamino" (e.g. benzamide, naphthalene-1-carboxylic acid amide and the like) represents an "arylcarbonyl" group as defined above attached through an amino group.
The term "arylcarbonylaminoalkyl" (e.g. N-propyl-benzamide, N-Butyl-naphthalene-1-carboxylic acid amide and the like) represents an "arylcarbonylamino" group attached through an alkyl group as defined above having the indicated number of carbon atoms.
The term "alkylcarboxy" (e.g. heptylcarboxy, cyclopropylcarboxy, 3-pentenylcarboxy) represents an alkylcarbonyl group as defined above wherein the carbonyl is in turn attached through an oxygen bridge.
The term "arylcarboxy" (e.g. benzoic acid and the like) represents an arylcarbonyl group as defined above wherein the carbonyl is in turn attached through an oxygen bridge.
The term "alkylcarboxyalkyl" (e.g. heptylcarboxymethyl, propylcarboxy *tert*-butyl, 3-pentylcarboxyethyl) represents an
The term "arylalkylcarboxy" (e.g. benzylcarboxy, phenylpropylcarboxy and the like) represents an arylalkylcarbonyl group as defined above wherein the carbonyl is in turn attached through an oxygen bridge.
The term "arylalkylcarboxyalkyl" (e.g. benzylcarboxymethyl, phenylpropylcarboxypropyl and the like) represents an arylalkylcarboxy group as defined above wherein the carboxy group is in turn attached through an alkyl group as defined above having the indicated number of carbon atoms.
The term "hetarylcarboxy" (e.g. pyridine-2-carboxylic acid and the like) represents a hetarylcarbonyl group as defined above wherein the carbonyl is in turn attached through an oxygen bridge.
The term "hetarylalkylcarboxy" (e.g. (1-*H*-imidazol-2-yl)-acetic acid, 3-pyrimidin-2-yl-propionic acid and the like) represents a hetarylalkylcarbonyl group as defined above wherein the carbonyl is in turn attached through an oxygen bridge.
The term "aryl" includes but is not limited to a carbocyclic aromatic ring system being either monocyclic, bicyclic, or polycyclic, such as phenyl, biphenyl, naphthyl, anthracenyl, phenanthrenyl, fluorenyl, indenyl, pentalenyl, azulenyl, biphenylenyl and the like. Aryl is also intended to include the partially hydrogenated derivatives of the carbocyclic aromatic systems enumerated above. Non-limiting examples of such partially hydrogenated derivatives are 1,2,3,4-tetrahydronaphthyl, 1,4-dihydronaphthyl and the like.
The term "aryl1" includes phenyl, biphenyl, naphthyl, anthracenyl, phenanthrenyl, and fluorenyl.
The term "aryl2" includes phenyl, biphenyl, and naphthyl.
The term "hetaryl" includes but is not limited to pyrrolyl (2-pyrrolyl), pyrazolyl (3-pyrazolyl), imidazolyl (1-imidazolyl, 2-imidazolyl, 4-imidazolyl, 5-imidazolyl), triazolyl (1,2,3-triazol-1-yl, 1,2,3-triazol-2-yl 1,2,3-triazol-4-yl, 1,2,4-triazol-3-yl), oxazolyl (2-oxazolyl, 4-oxazolyl, 5-oxazolyl), isoxazolyl (3-isoxazolyl, 4-isoxazolyl, 5-isoxazolyl), thiazolyl (2-thiazolyl, 4-thiazolyl, 5-thiazolyl), thiophenyl (2-thiophenyl, 3-thiophenyl, 4-thiophenyl, 5-thiophenyl), furanyl (2-furanyl, 3-furanyl, 4-furanyl, 5-furanyl), pyridyl (2-pyridyl, 3-pyridyl, 4-pyridyl, 5-pyridyl), 5-tetrazolyl, pyrimidinyl (2-pyrimidinyl, 4-pyrimidinyl, 5-pyrimidinyl, 6-pyrimidinyl), pyrazinyl, pyridazinyl (3-pyridazinyl, 4-pyridazinyl, 5-pyridazinyl), quinolyl (2-quinolyl, 3-quinolyl, 4-quinolyl, 5-quinolyl, 6-quinolyl, 7-quinolyl, 8-quinolyl), isoquinolyl (1-isoquinolyl, 3-isoquinolyl, 4-isoquinolyl, 5-isoquinolyl, 6-isoquinolyl, 7-isoquinolyl, 8-isoquinolyl), benzo[b]furanyl (2-benzo[b]furanyl, 3-benzo[b]furanyl, 4-benzo[b]furanyl, 5-benzo[b]furanyl, 6-benzo[b]furanyl, 7-benzo[b]furanyl), 2,3-dihydro-benzo[b]furanyl (2-(2,3-dihydro-benzo[b]furanyl), 3-(2,3-dihydro-benzo[b]furanyl), 4-(2,3-dihydro-benzo[b]furanyl),5-(2,3-dihydro-benzo-[b]furanyl), 6-(2,3-dihydro-benzo-[b]furanyl), 7-(2,3-dihydro-benzo[b]furanyl)), benzo[b]thiophenyl (2-benzo[b]thiophenyl, 3-benzo[b]thiophenyl, 4-benzo[b]thiophenyl, 5-benzo[b]thiophenyl, 6-benzo[b]thiophenyl, 7-benzo[b]thiophenyl), 2,3-dihydro-benzo[b]thiophenyl (2-(2,3-dihydro-benzo[b]thiophenyl), 3-(2,3-dihydro-benzo[b]thiophenyl), 4-(2,3-dihydrobenzo[b]thiophenyl), 5-(2,3-dihydro-benzo[b]thiophenyl), 6-(2,3-dihydro-benzo[b]thiophenyl), 7-(2,3-dihydro-benzo[b]thiophenyl)), 4,5,6,7-tetrahydro-benzo[b]thiophenyl (2-(4,5,6,7-tetrahydro-benzo[b]thiophenyl), 3-(4,5,6,7-tetrahydro-benzo[b]thiophenyl), 4-(4,5,6,7-tetrahydro-benzo[b]thiophenyl), 5-(4,5,6,7-tetrahydro-benzo[b]thiophenyl), 6-(4;5,6,7-tetrahydro-benzo[b]thiophenyl), 7-(4,5,6,7-tetrahydro-benzo[b]thiophenyl)), thieno[2,3-b]thiophenyl, 4,5,6,7-tetrahydro-thieno[2,3-c]pyridyl (4-(4,5,6,7-tetrahydro-thieno[2,3-c]pyridyl), 5-4,5,6,7-tetrahydro-thieno[2,3-c]pyridyl), 6-(4,5,6,7-tetrahydro-thieno[2,3-c]pyridyl), 7-(4,5,6,7-tetrahydro-thieno[2,3-c]pyridyl)), indolyl (1-indolyl, 2-indolyl, 3-ihdolyl, 4-indolyl, 5-indolyl, 6-indolyl, 7-indolyl), isoindolyl (1-isoindolyl, 2-isoindolyl, 3-isoindolyl, 4-isoindolyl, 5-isoindolyl, 6-isoindolyl, 7-isoindolyl), 1,3-dihydro-isoindolyl (1-(1,3-dihydro-isoindolyl), 2-(1,3-dihydro-isoindolyl), 3-(1,3-dihydro-isoindolyl), 4-(1,3-dihydro-isoindolyl), 5-(1,3-dihydro-isoindolyl), 6-(1,3-dihydro-isoindolyl), 7-(1,3-dihydro-isoindolyl)), indazole (1-indazolyl, 3-indazolyl, 4-indazolyl, 5-indazolyl, 6-indazolyl, 7-indazolyl), benzimidazolyl (1-benzimidazolyl, 2-benzimidazolyl, 4-benzimidazolyl, 5-benzimidazolyl, 6-benzimidazolyl, 7-benzimidazolyl, 8-benzimidazolyl), benzoxazolyl (1-benz-oxazolyl, 2-benzoxazolyl), benzothiazolyl (1-benzothiazolyl, 2-benzothiazolyl, 4-benzothiazolyl, 5-benzothiazolyl, 6-benzothiazolyl, 7-benzothiazolyl), benzo-[1,2,5]oxadiazolyl, (4-benzo[1,2,5]oxadiazole, 5-benzo[1,2,5]oxadiazole), carbazolyl (1-carbazolyl, 2-carbazolyl, 3-carbazolyl, 4-carbazolyl), piperidinyl (2-piperidinyl, 3-piperidinyl, 4-piperidinyl), pyrrolidinyl (1-pyrrolidinyl, 2-pyrrolidinyl, 3-pyrrolidinyl).
The term "alkylSOₘ" (e.g. ethylsulfonyl, ethylsulfinyl and the like) represents an alkyl group as defined above, wherein the alkyl group is in turn attached through a sulphur bridge wherein the sulphur is substituted with m oxygen atoms.
The term "arylSOₘ" (e.g. phenylsulfinyl, naphthyl-2-sulfonyl and the like) represents an aryl group as defined above, wherein the aryl group is in turn attached through a sulphur bridge wherein the sulphur is substituted with m oxygen atoms.
The term "hetarylSOₘ" (e.g. thiazol-2-sulfinyl, pyridine-2-sulfonyl and the like) represents a hetaryl group as defined above, wherein the hetaryl group is in turn attached through a sulphur bridge wherein the sulphur is substituted with m oxygen atoms.

With respect to formula I and II, the term "NR⁴R⁵carbonylalkyl" (e.g. *N,N-*dimethyl-propionamide, *N*-isopropyl-*N*-methyl-propionamide and the like) represents NR⁴R⁵ substituted by a carbonylalkyl group as defined above.

With respect to formula I and II, the term "alkylR⁶alkyl" (e.g. 2-ethoxymethyl, N-ethyl-N-methy amine, methyl-propyl-amide, ethanesulfonic acid methylamide and the like) represents an alkyl group as defined above, substituted by R⁶, which is substituted by an alkyl group as defined above.

With respect to formula I and II, the term"arylR⁶alkyl" (e.g. ethoxy-benzene, N-ethyl-N-methyl-phenyl-amine, *N*-ethyl-benzamide, *N*-isobutyl-benzenesulfonamide and the like) represents an aryl group as defined above, substituted by R⁶, which is substituted by an alkyl group as defined above.

With respect to formula I and II, the term "arylalkylR⁶alkyl" (e.g. benzyloxymethyl, N-ethyl-N-methyl-benzyl-amine, *N*-ethyl-benzylamide and the like) represents an arylalkyl group as defined above, substituted by R⁶, which is substituted by an alkyl group as defined above.

With respect to formula I and II, the term "hetarylR⁶alkyl" (e.g. 2-ethoxy-1*H*-imidazol, ethyl-quinolin-2-yl-amine, thiazole-2-carboxylic acid, methyl-propyl-amide, pyridine-3-sulfonic acid isobutyl-amide and the like) represents a hetaryl group as defined above, substituted by R⁶, which is substituted by an alkyl group as defined above.

With respect to formula I and II, the term "arylcarbonylNR¹⁵" (e.g. *N*-benzyl-*N-*methyl-benzamide and the like) represents an arylcarbonyl group as defined above, substituted by NR¹⁵.

With respect to formula I and II, the term "arylSOₘNR⁸" (e.g. N-methyl-benzenesulfonamide and the like) represents an aryl group as defined above, wherein the aryl group is in turn attached through a SOₘNR⁸ group wherein the sulphur is substituted with m oxygen atoms and the nitrogen atom substituted by R⁸.

With respect to formula III, the term "alkylNR⁵alkyl" (e.g. N-ethyl-N-isobutyl-amine, N,N-dimethylamine and the like wherein the amino group (N) is substituted with R⁵ as defined below) represents an alkylNR⁵ group as defined above attached through an "alkyl" group.

With respect to formula III, the term "arylalkylNR⁵alkyl" (e.g. N-benzyl-N-methyl-amine, N-phenethyl-N-ethyl-amine and the like wherein the amino group (N) is substituted with R⁵ as defined below) represents an arylalkylNR⁵ group as defined above attached through an "alkyl" group.

Certain of the above defined terms may occur more than once in the structural formulae, and upon such occurrence each term shall be defined independently of the other.

The term "optionally substituted" as used herein means that the groups in question are either unsubstituted or substituted with one or more of the substituents specified. When the groups in question are substituted with more than one substituent, the substituents may be the same or different.

The term "treatment" is defined as the management and care of a patient for the purpose of combating or alleviating the disease, condition or disorder, and the term includes the administration of the active compound to prevent the onset of the symptoms or complications, or alleviating the symptoms or complications, or eliminating the disease, condition, or disorder.

The term "pharmaceutically acceptable" is defined as being suitable for administration to humans without adverse events.

The term "prodrug" is defined as a chemically modified form of the active drug, said prodrug being administered to the patient and subsequently being converted to the active, drug. Techniques for development of prodrugs are well known in the art.

### DETAILED DESCRIPTION OF THE INVENTION

In one aspect, the present invention provides the use of a substituted amide, a prodrug thereof, or a salt thereof with a pharmaceutically acceptable acid or base, or any optical isomer or mixture of optical isomers, including a racemic mixture, or any tautomeric forms for
a) modulation of the activity of 11βHSD1;or
b) inhibition of 11βHSD1,
   in a patient in need thereof.
In another aspect, the present invention provides the use of a substituted amide, a prodrug thereof, or a salt thereof with a pharmaceutically acceptable acid or base, or any optical isomer or mixture of optical isomers, including a racemic mixture, or any tautomeric forms for the preparation of a pharmaceutical composition for the treatment, prevention and/or prophylaxis of any disorder and disease where it is desirable to
a) modulate the activity of 11βHSD1; or
b) inhibit 11βHSD1,
   in a patient in need thereof.

In another embodiment, the invention provides the present use of substituted amides, or a prodrug thereof of the general formula (I) 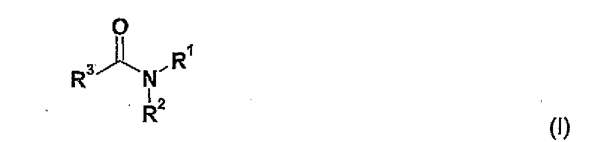 wherein
R¹ is C₃-C₁₀cycloalkyl, C₃-C₁₀hetcycloalkyl, C₁-C₈alkyl, aryl, hetaryl, arylC₁-C₆alkyl or hetarylC₁-C₆alkyl, wherein the cycloalkyl, hetcycloalkyl, alkyl, arylalkyl and hetarylalkyl groups independently are optionally substituted with one or more of R⁴.
R² is hydrogen, C₁-C₈alkyl, aryl, hetaryl, arylC₁-C₆alkyl, C₃-C₁₀cycloalkylC₁-C₆alkyl, C₁-C₆alkyl-carboxyC₁-C₆alkyl wherein the alkyl, aryl and cycloalkyl groups independently are optionally substituted with one or more of R⁵; or
R¹ and R² together with the nitrogen to which they are attached, are forming a saturated or partially saturated cyclic, bicyclic or tricyclic ring system containing from 4 to 10 carbon atoms and from 0 to 2 additional heteroatoms selected from nitrogen, oxygen or sulphur, the ring system optionally being substituted with at least one of C₁-C₈alkyl, aryl, hetaryl, arylC₁-C₆alkyl, hetarylC₁-C₆alkyl, hydroxy, oxo, cyano, C₁-C₆alkyloxy, arylC₁-C₆alkyloxy, hetarylC₁-C₆alkyloxy, C₁-C₆alkyloxyC₁-C₆alkyl, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy or arylC₁-C₆alkylcarboxy wherein the alkyl and aryl groups independently are optionally substituted with one ore more of R¹⁴;
R³ is C₁-C₈alkyl, C₁-C₆alkenyl, C₁-C₆alkynyl, C₃-C₁₀cycloalkyl, C₃-C₁₀hetcycloalkyl, aryl, hetaryl, arylC₁-C₆alkyl, C₁-C₆alkyloxyC₁C₆alkyl, hetarylC₁-C₆alkyl, aryl-R⁶-C₁-C₆alkyl, hetaryl-R⁶-C₁C₆alkyl or arylC₁-C₆alkyl-R⁶-C₁-C₆alkyl wherein the alkyl, cycloalkyl, hetcycloalkyl, alkenyl, alkynyl, aryl and hetaryl groups independently are optionally substituted with one or more of R⁷;
R⁴ and R⁵ independently are hydrogen, hydroxy, oxo, cyano, halo, methylendioxo, NR⁸R⁹, C₁-C₈alkyl, C₁-C₆alkyloxy, trihalomethyl, trihalomethyloxy, C₃-C₁₀cycloalkyl, C₃-C₁₀hetcycloalkyl, C₃-C₁₀cycloalkenyl, aryl, hetaryl, hetarylSOₙ, arylC₁-C₆alkyloxy, hetarylC₁-C₆alkyloxy, C₁-C₆alkyl-R⁶-C₁-C₆alkyl, arylC₁-C₆alkyl-R⁶-C₁-C₆alkyl, C₁-C₆alkylcarbonyl, arylcarbonyl, arylC₁-C₆alkylcarbonyl, hetarylcarbonyl, hetarylC₁-C₆alkyl-carbonyl, C₁-C₆alkylSOₙ, C₁-C₆-alkyl-carboxy, arylcarboxy, hetarylcarboxy, arylC₁-C₆alkylcarboxy or hetarylC₁-C₆alkylcarboxy wherein the alkyl, cycloalkyl, hetcycloalkyl, aryl and hetaryl groups independently are optionally substituted with one ore more of R¹⁵;
R⁶ is oxygen, sulphur, SOₙ or NR¹⁶;
R⁷ is hydrogen, halo, hydroxy, cyano, nitro, COOR¹⁷, C₁-C₈alkyl, C₃-C₁₀cycloalkyl, C₃-C₁₀hetcycloalkyl, methylendioxo, trihalomethyl, trihalomethyloxy, aryl, arylC₁-C₆alkyl, C₁-C₆alkyloxy, C₁-C₆alkyloxyC₁-C₆alkyl, aryloxy, arylC₁-C₆alkyloxy, aryloxyC₁-C₆alkyl, arylC₁-C₆alkyloxyC₁-C₆alkyl, hetaryl, hetarylC₁-C₆alkyl, hetaryloxy, hetarylC₁-C₆alkyloxy, hetaryloxyC₁-C₆alkyl, hetarylC₁-C₆alkyl-oxyC₁-C₆alkyl, NR⁸R⁹, SO₂NR⁸R⁹, NR⁴R⁵carbonylC₁-C₆alkyl, arylthio, hetarylthio, R¹⁶carbonylNR⁸, arylSOₙ, hetarylSOₙ, R¹⁹SOₘNR⁸, arylthioC₁-C₆alkyl, hetarylthioC₁-C₆alkyl or arylC₁₋C₆alkylR⁶C₁-C₆alkyl; wherein the aryl and hetaryl groups independently are optionally substituted with one or more R¹⁰;
R⁸ and R⁹ independently are hydrogen, C₁-C₈alkyl, aryl, hetaryl, arylC₁-C₆alkyl or hetarylC₁-C₆alkyl wherein the alkyl, aryl and hetaryl groups independently are optionally substituted with one or more of R¹¹; or
R⁸ and R⁹ together with the nitrogen to which they are attached, are forming a saturated or partially saturated cyclic, bicyclic or tricyclic ring system containing from 4 to 10 carbon atoms and from 0 to 2 additional heteroatoms selected from nitrogen, oxygen or sulfur, the ring system optionally being substituted with at least one halo, cyano, C₁-C₈alkyl, aryl, hetaryl, arylC₁-C₆alkyl, hetarylC₁-C₆alkyl, hydroxy, oxo, C₁-C₆alkyloxy, arylC₁-C₆alkyloxy, hetarylC₁-C₆alkyloxy, C₁-C₆alkyloxyC₁-C₆alkyl, C₁-C₆alkyl-carbonyl, arylcarbonyl, hetarylcarbonyl, arylC₁-C₆alkylcarbonyl, hetarylC₁-C₆alkylcarbonyl, C₁-C₆alkylcarboxy, arylcarboxy, hetarylcarboxy, arylC₁-C₆alkylcarboxy or hetarylC₁-C₆alkylcarboxy;
R¹⁰ and R¹¹ independently are hydrogen, hydroxy, oxo, halo, cyano, nitro, C₁-C₆alkyl, C₁-C₆alkyloxy, NR¹²R¹³, methylendioxo, trihalomethyl or trihalomethyloxy;
R¹² and R¹³ independently are hydrogen, C₁-C₈alkyl or arylC₁-C₆alkyl;
R¹⁴ is hydrogen, halo, hydroxy, oxo, nitro, cyano, C₁-C₈alkyl, C₁-C₆alkyloxy or aryloxy;
R¹⁵ is hydrogen, halo, hydroxy, oxo, nitro, cyano, CONR⁸R⁹ or COOR¹⁷;
R¹⁶ is hydrogen, C₁-C₈alkyl, C₃-C₁₀cycloalkyl, C₃-C₁₀hetcycloalkyl, aryl, arylC₁-C₆alkyl, hetaryl, hetarylC₁-C₆alkyl, alkylcarbonyl, arylcarbonyl, arylC₁-C₆alkylcarbonyl, aryloxyC₁-C₆alkyl, hetaryloxyC₁-C₆alkyl, arylthioC₁-C₆alkyl or hetarylthioC₁-C₆alkyl; wherein the alkyl, cycloalkyl, hetcycloalkyl, aryl and hetaryl groups independently are optionally substituted with one or more of R¹⁰;
R¹⁷ is hydrogen, C₁-C₈alkyl, aryl or arylC₁-C₆alkyl;
R¹⁸ is C₁-C₆alkyl, C₂-C₆alkenyl, aryl, arylC₁-C₆alkyl, hetaryl, hetarylC₁-C₆alkyl, C₃-C₁₀cycloalkyl, C₃-C₁₀hetcycloalkyl, C₁C₆alkyloxy, aryloxy, arylC₁-C₆alkyloxy, arylC₁-C₆alkyloxyC₁-C₆alkyl, hetaryloxy, hetarylC₁-C₆alkyloxy, hetarylC₁-C₆alkyloxyC₁-C₆alkyl or R⁸R⁹NC₁-C₆alkyl wherein the alkyl, alkenyl, cycloalkyl, hetcycloalkyl, aryl and hetaryl groups are optionally substituted with R¹⁵;
R¹⁹ is C₁-C₆alkyl, C₃-C₁₀cycloalkyl, C₃-C₁₀hetcycloalkyl, aryl, arylC₁-C₆alkyl, hetaryl, hetaryl-C₁-C₆alkyl;
m is 1 or 2;
n is 0, 1 or 2; or
a salt thereof with a pharmaceutically acceptable acid or base, or any optical isomer or mixture of optical isomers, including a racemic mixture, or any tautomeric forms.

In another embodiment, the invention provides the present use of a substituted amide, or a prodrug thereof of the above general formula (I) wherein
R¹ is C₃-C₁₀cycloalkyl, C₃-C₁₀hetcycloalkyl, C₁-C₈alkyl, aryl, hetaryl-, arylC₁-C₆alkyl or hetaryl,-C₁-C₆alkyl, wherein the cycloalkyl, hetcycloalkyl, alkyl, arylalkyl and hetarylalkyl groups independently are optionally substituted with one or more of R⁴;
R² is hydrogen, C₁-C₈alkyl, aryl, hetaryl, arylC₁-C₆alkyl, C₃-C₁₀cycloalkylC₁-C₆alkyl, C₁-C₆-alkylcarboxyC₁-C₆alkyl wherein the alkyl, aryl and cycloalkyl groups independently are optionally substituted with one or more of R⁵; or
R¹ and R² are together with the nitrogen to which they are attached, are forming a saturated or partially saturated cyclic, bicyclic or tricyclic ring system containing from 4 to 10 carbon atoms and from 0 to 2 additional heteroatoms selected from nitrogen, oxygen or sulphur, the ring system optionally being substituted with at least one of C₁-C₈alkyl, aryl, hetaryl, arylC₁-C₆alkyl, hetarylC₁-C₆alkyl, hydroxy, oxo, cyano, C₁-C₆alkyloxy, arylC₁-C₆alkyloxy, hetarylC₁-C₆alkyloxy, C₁-C₆alkyloxyC₁-C₆alkyl, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy or arylC₁-₆alkylcarboxy wherein the alkyl and aryl groups independently are optionally substituted with one ore more of R¹⁴;
R³ is C₁-C₈alkyl, C₁-C₆alkenyl, C₁-C₆alkynyl, C₃-C₁₀cycloalkyl, C₃-C₁₀hetcycloalkyl, aryl, hetaryl, arylC₁-C₆alkyl, C₁-C₆alkyloxyC₁-C₆alkyl, hetarylC₁-C₆alkyl, aryl-R⁶-C₁-C₆alkyl, hetaryl-R⁶-C₁-C₆alkyl or arylC₁-C₆alkyl-R⁶-C₁₋C₆alkyl wherein the alkyl, cycloalkyl, hetcycloalkyl, alkenyl, alkynyl, aryl and hetaryl groups independently are optionally substituted with one or more of R⁷;
R⁴ and R⁵ independently are hydrogen, hydroxy, oxo, cyano, halo, methylendioxo, NR⁸R⁹, C₁-C₈alkyl, C₁-C₆alkyloxy, trihalomethyl, trihalomethyloxy, C₃-C₁₀cycloalkyl, C₃-C₁₀hetcycloalkyl, C₃-C₁₀cycloalkenyl, aryl, hetaryl, hetarylSOₙ, arylC₁-C₆alkyloxy, hetarylC₁-C₆alkyloxy, C₁₋C₆alkyl-R⁶-C₁-C₆alkyl, arylC₁-C₆alkyl-R⁶-C₁-C₆alkyl, C₁-C₆alkylcarbonyl, arylcarbonyl, arylC₁-C₆alkylcarbonyl, hetarylcarbonyl, hetarylC₁-C₆alkyl-carbonyl, G₁-C₆alkylSOₙ, C₁-C₆alkyl-carboxy, arylcarboxy, hetarylcarboxy, arylC₁-C₆alkylcarboxy or hetarylC₁-C₆alkylcarboxy wherein the alkyl, cycloalkyl, hetcycloalkyl, aryl and hetaryl groups independently are optionally substituted with one ore more of R¹⁵;
R⁶ is oxygen, sulphur, SOₙ, NR¹⁶;
R⁷ is hydrogen, halo, hydroxyl, cyano, nitro, COOR¹⁷, C₁-C₈alkyl, C₃-C₁₀cycloalkyl, C₃-C₁₀hetcycloalkyl, methylendioxo, trihalomethyl, trihalomethyloxy, aryl, arylC₁-C₆alkyl, C₁-C₆alkyloxy, C₁-C₆alkyloxyC₁-C₆alkyl, aryloxy, aryloxyC₁-C₆alkyl, arylC₁-C₆alkyloxyC₁-C₆alkyl, hetaryl, hetarylC₁-C₆alkyl, hetaryloxy, hetarylC₁-C₆alkyloxy, hetaryloxyC₁-C₆alkyl, hetarylC₁-C₆alkyloxyC₁-C₆alkyl, NR⁸R⁹, SO₂NR⁸R⁹, NR⁴R⁵carbonylalkyl, arylcarbonylNR⁸, arylthio, hetarylthio; arylSOₙ, hetarylSOₙ, arylSOₘNR⁸, arylthioC₁-C₆alkyl, hetarylthioC₁-C₆alkyl or arylC₁-C₆alkylR⁶C₁-C₆alkyl; wherein the aryl and hetaryl groups independently are optionally substituted with one or more R¹⁰;
R⁸ and R⁹ independently are hydrogen, C₁-C₈alkyl, aryl, hetaryl-, arylC₁-C₆alkyl or hetarylC₁-C₆alkyl wherein the alkyl, aryl and hetaryl groups independently are optionally substituted with one or more of R¹¹; or
R⁸ and R⁹ together with the nitrogen to which they are attached, are forming a saturated or partially saturated cyclic, bicyclic or tricyclic ring system containing from 4 to 10 carbon atoms and from 0 to 2 additional heteroatoms selected from nitrogen, oxygen or sulfur, the ring system optionally being substituted with at least oneC₁-C₈alkyl, aryl, hetaryl, arylC₁-C₆alkyl, hetarylC₁-C₆alkyl, hydroxy, oxo, C₁-C₆alkyloxy, arylC₁-C₆alkyloxy, hetarylC₁-C₆alkyloxy, C₁-C₆alkyloxyC₁-C₆alkyl, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy, hetarylcarboxy, arylC₁-C₆alkyl-carboxy or hetarylC₁-C₆alkylcarboxy;
R¹⁰ and R¹¹ independently are hydrogen, hydroxy, oxo, halo, cyano, nitro, C₁-C₆alkyl, C₁-C₆-alkyloxy, NR¹²R¹³, methylendioxo, trihalomethyl or trihalomethyloxy;
R¹² and R¹³ independently are hydrogen, C₁-C₈alkyl or arylC₁-C₆alkyl;
R¹⁴ is hydrogen, halo, hydroxy, oxo, nitro, cyano, C₁-C₈alkyl, C₁-C₆alkyloxy or aryloxy;
R¹⁵ is hydrogen, halo, hydroxy, oxo, nitro, cyano or COOR¹⁷;
R¹⁶ is hydrogen, C₁-C₈alkyl, C₃-C₁₀cycloalkyl, C₃-C₁₀hetcycloalkyl, aryl, arylC₁-C₆alkyl, hetaryl, hetarylC₁-C₆alkyl, alkylcarbonyl, arylcarbonyl, arylC₁-C₆alkylcarbonyl, aryloxyC₁-C₆alkyl, hetaryloxyC₁-C₆alkyl, arylthioC₁-C₆alkyl or hetarylthioC₁-C₆alkyl; wherein the alkyl, cycloalkyl, hetcycloalkyl, aryl and hetaryl groups independently are optionally substituted with one or more of R¹⁰;
R¹⁷ is hydrogen, C₁-C₈alkyl, aryl or arylC₁-C₆alkyl;
m is 1 or 2;
n is 0, 1 or 2; or
a salt thereof with a pharmaceutically acceptable acid or base, or any optical isomer or mixture of optical isomer, including a racemic mixture, or any tautomeric forms.

In another embodiment, the invention provides the present use of a substituted amide, or a prodrug thereof of the general formula (I) wherein R¹ is C₃-C₁₀cycloalkyl or C₃-C₁₀hetcycloalkyl wherein the cycloalkyl and hetcycloalkyl groups independently are optionally substituted with one or more of R⁴ as defined above.

In another embodiment, the invention provides the present use of a substituted amide, or a prodrug thereof of the general formula (I) wherein R¹ is C₃-C₁₀cydoalkyl optionally substituted with one or more of R⁴ as defined above.

In another embodiment, the invention provides the present use of a substituted amide, or a prodrug thereof of the general formula (I) wherein R² is hydrogen or C₁-C₈alkyl, wherein the the alkyl group is optionally substituted with one or more of R⁵ as defined above.

In another embodiment, the invention provides the present use of a substituted amide, or a prodrug thereof of the general formula (I) wherein R² is C₁-C₈alkyl optionally substituted with one or more of R⁵ as defined above.

In another embodiment, the invention provides the present use of a substituted amide, or a prodrug thereof of the general formula (I) wherein R³ is C₃-C₁₀cycloalkyl, C₃-C₁₀hetcycloalkyl, aryl, hetaryl, arylC₁-C₆alkyl, hetarylC₁-C₆alkyl, aryl-R⁶-C₁-C₆alkyl, hetaryl-R⁶-C₁-C₆alkyl or arylC₁-C₆alkyl-R⁶-C₁-C₆alkyl wherein the alkyl, cycloalkyl, hetcycloalkyl, aryl and hetaryl, groups independently are optionally substituted with one or more of R⁷.

In another embodiment, the invention provides the present use of a substituted amide, or a prodrug thereof of the general formula (I) wherein R³ is aryl or hetaryl, wherein the aryl and hetaryl groups are optionally substituted with one or more of R⁷ as defined above.

In another embodiment, the invention provides the present use of a substituted amide, or a prodrug thereof of the general formula (I) wherein R³ is is phenyl optionally substituted with one or more of R⁷ as defined above.

In another embodiment, the invention provides the present use of a substituted amide, or a prodrug thereof of the general formula (I) wherein R³ is phenyl optionally substituted independently in position 2(ortho) or 4(para) with one or more of R⁷ as defined above.

In another embodiment, the invention provides the present use of a substituted amide, or a prodrug thereof of the general formula (I) wherein R⁴ and R⁵ independently are hydrogen, hydroxy, oxo, halo, C₁-C₈alkyl, wherein the alkyl group is optionally substituted with one ore more of R¹⁵.

In another embodiment, the invention provides the present use of a substituted amide, or a prodrug thereof of the general formula (I) wherein R⁶ is oxygen.

In another embodiment, the invention provides the present use of a substituted amide, or a prodrug thereof of the general formula (I) wherein R⁷ is hydrogen, halo, hydroxy, cyano, C₁-C₈alkyl, C₃-C₁₀cycloalkyl, C₃-C₁₀het-cycloalkyl, trihalomethyl, aryl, arylC₁-C₆alkyl, C₁-C₆alkyloxy, C₁-C₆alkyloxyC₁-C₆alkyl, aryloxy, arylC₁-C₆alkyloxy, aryloxyC₁-C₆alkyl, arylC₁-C₆alkyloxyC₁-C₆aikyl, hetaryl, hetarylC₁-C₆alkyl, hetaryloxy, hetarylC₁-C₆alkyloxy, hetaryloxyC₁-C₆alkyl, hetarylC₁-C₆alkyl-oxyC₁-C₆alkyl, NR⁸R⁹, NR⁴R⁵carbonylC₁-C₆alkyl, R¹⁸carbonylNR⁸, R¹⁹SOₘNR⁸, wherein the aryl and hetaryl groups independently are optionally substituted with one or more R¹⁰.

In another embodiment, the invention provides the present use of a substituted amide, or a prodrug thereof of the general formula (I) wherein R⁸ and R⁹ together with the nitrogen to which they are attached, are forming a saturated or partially saturated cyclic, bicyclic or tricyclic ring system containing from 4 to 10 carbon atoms and from 0 to 2 additional heteroatoms selected from nitrogen, oxygen or sulfur, the ring system optionally being substituted with at least one halo, cyano, C₁-C₈alkyl, aryl, hetaryl, arylC₁-C₆alkyl, hetarylC₁-C₆alkyl, hydroxy, oxo, C₁-C₆alkyloxy, arylC₁-C₆alkyloxy, hetarylC₁-C₆alkyloxy, C₁-C₆alkyloxyC₁-C₆alkyl, C₁-C₆alkyl-carbonyl, arylcarbonyl, hetarylcarbonyl, arylC₁-C₆alkylcarbonyl, hetarylC₁-C₆alkylcarbonyl, C₁-C₆alkylcarboxy, arylcarboxy, hetarylcarboxy, arylC₁-C₆alkylcarboxy or hetarylC₁-C₆alkylcarboxy.

In another embodiment, the invention provides the present use of a substituted amide, or a prodrug thereof of the general formula (I) wherein R¹⁵ is CONR⁸R⁹.

In another embodiment, the invention provides the present use of a substituted amide, or a prodrug thereof of the general formula (I) wherein R¹⁸ is C₁-C₆alkyl optionally substituted with R¹⁵;

In another embodiment, the invention provides the present use of a substituted amide, or a prodrug thereof of general formula (I), selected from.the group consisting of:
3-(10,11-Dihydro-dibenzo[b,f]azepin-5-yl)-1-[4-(1H-imidazol-4-yl)-piperidin-1-yl]-propan-1-one;
4-(10,11-Dihydro-dibenzo[b,f]azepin-5-yl)-1-[4-(3H-imidazol-4-yl)-piperidin-1-yl]-butan-1-one; 2,4-Bis-benzyloxy-benzamide;
(1H-Indol-4-yl)-piperidin-1-yl-methanone;
N-(1,4-Dioxo-1,4-dihydro-naphthalen-2-yl)-benzamide;
N-(2,3-Dihydroxy-propyl)-2-(2-phenyl-adamantan-2-yl)-acetamide;
(6-Fluoro-2-methyl-3,4-dihydro-2H-quinolin-1-yl)-phenyl-methanone;
(2-Chloro-phenyl)-(6-fluoro-2-methyl-3,4-dihydro-2H-quinolin-1-yl)-methanone;
3-Cyclopentyl-1-(6-fluoro-2-methyl-3,4-dihydro-2H-quinolin-1-yl)-propan-1-one;
(3-Chloro-thieno[2,3-b]thiophen-2-yl)-thiomorpholin-4-yl-methanone;
2-[2-(4-Chloro-phenyl)-adamantan-2-yl]-1-[4-(4-methoxy-phenyl)-piperazin-1-yl]-ethanone;
1-(4-Benzyl-piperazin-1-yl)-2-[2-(4-chloro-phenyl)-adamantan-2-yl]-ethanone;
2-[2-(4-Chloro-phenyl)-adamantan-2-yl]-1-(4-methyl-piperazin-1-yl)-ethanone;
1-[4-(6-Chloro-pyridin-2-yl)-piperazin-2-yl]-2-(2-phenyl-adamantan-2-yl)-ethanone;
4-Chloro-N-(1,7,7-trimethyl-bicyclo[2.2.1]hept-2-yl)-benzamide;
3-Chloro-benzo[b]thiophene-2-carboxylic acid (2-cyano-ethyl)-cyclohexyl-amide;
2-[2-(Bicyclo[2.2.1]hept-5-en-2-ylamino)-4-oxo-4,5-dihydro-thiazol-5-yl]-N-(2-chloro-phenyl)-acetamide;
[3-(4-sec-Butyl-phenoxy)-phenyl]-piperidin-1-yl-methanone;
3-(6-Chloro-pyridin-2-yloxy)-N-ethyl-benzamide;
N-Benzyl-2,4-dichloro-N-pyridin-2-yl-benzamide;
2-[Benzoyl-(3-chloro-4-fluoro-phenyl)-amino]-propionic acid butyl ester;
2-[Benzoyl-(3-chloro-4-fluoro-phenyl)-amino]-propionic acid pentyl ester;
3-(4-Fluoro-phenyl)-1-(4-phenyl-piperidin-1-yl)-but-2-en-1-one;
N-(1,7,7-Trimethyl-bicyclo[2.2.1]hept-2-yl)-benzamide;
1-(3-Cyclopentyl-propionyl)-piperidine-3-carboxylic acid ethyl ester;
4-Phenyl-1-phenylacetyl-piperidine-4-carbonitrile;
1-Octanoyl-4-phenyl-piperidine-4-carbonitrile;
2,2-Dimethyl-1-(1,3,3-trimethyl-6-aza-bicyclo[3.2.1]oct-6-yl)-propan-1-one;
(4-Chloro-phenyl)-(1,3,3-trimethyl-6-aza-bicyclo[3.2.1]oct-6-yl)-methanone;
N-[1-(4-Methanesulfonyl-phenyl)-ethyl]-N-(tetrahydro-furan-2-ylmethyl)-benzamide;
2-(2-Amino-phenylsulfanyl)-1-(5-fluoro-2,3-dihydro-indol-1-yl)-ethanone;
N-(1-Methyl-2,3-dihydro-1H-indol-5-ylmethyl)-N-(tetrahydro-furan-2-ylmethyl)-benzamide;
1-Benzoyl-piperidine-2-carboxylic acid ethyl ester;
N-(2-Chloro-phenyl)-2-(1,2,3,4-tetrahydro-isoquinolin-1-yl)-acetamide;
(Decahydro-naphthalen-1-yl)-(4-methyl-piperazin-1-yl)-methanone;
(4-Methyl-plperazin-1-yl)-(2-p-tolylsulfanyl-phenyl)-methanone;
Adamantane-1-carboxylic acid (3-benzyloxy-2-ethyl-6-methyl-pyridin-4-yl)-amide; (6-Fluoro-2-methyl-3,4-dihydro-2H-quinolin-1-yl)-(3,4,5-trimethoxy-phenyl)-methanone; N-Bicyclo[2.2.1]hept-2-yl-3-cyclopentyl-proplonamide;

(2-Benzo[1,2,5]oxadiazol-5-yl-thiazol-4-yl)-piperidin-1-yl-methanone; Thiophene-2-carboxylic acid [4-(4-fluoro-phenyl)-4-hydroxy-butyl]-isopropyl-amide; N-Cyclohexyl-3-(1,3-dioxo-1,3-dihydro-isoindol-2-y1)-propionamide; 2-[(Adamantane-l-carbonyl)-amino]-3-(1H-indol-3-yl)-propionic acid methyl ester; Adamantane-1-carboxylic acid [3-(1H-benzoimidazol-2-ylsulfanyl)-5-nitro-phenyl]-amide; N-Benzyl-N-(1-cyclopropyl-ethyl)-4-fluoro-benzamide;
Thiophene-2-carboxylic acid 2-[2-(2-phenyl-adamantan-2-yl)-acetylamino]-ethyl ester; N-(4-Acetyl-phenyl)-2-(2-phenyl-adamantan-2-yl)-acetamide;
2-[2-(4-Chloro-phenyl)-adamantan-2-yl]-N-(2-hydroxy-ethyl)-acetamide; (4-Benzoyl-piperidin-1-yl)-thiophen-2-yl-methanone;
N-(2-Benzoyl-4-methyl-phenyl)-3-phenyl-acrylamide;
N-(2-Benzoyl-4-methyl-phenyl)-2-fluoro-benzamide;
Adamantane-1-carboxylic acid (4-ethoxy-benzothiazol-2-yl)-amide; Adamantane-1-carboxylic acid (5-benzoyl-4-phenyl-thiazol-2-yl)-amide; 3-(2-Hydroxy-phenyl)-1,3-di-piperidin-1-yl-propan-1-one;
N-(1-Methyl-2-phenyl-ethyl)-3-phenyl-propionamide;
4-Oxo-4-piperidin-1-yl-butyric acid 4-tert-butyl-cyclohexyl ester;
N-tert-Butyl-N-(4-methoxy-benzyl)-4-nitro-benzamide;
{4-[(Adamantane-1-carbonyl)-amino]-phenoxy}-acetic acid;
2-(4-Isobutyl-phenyl)-N-(1-phenyl-ethyl)-propionamide;
Adamantane-1-carboxylic acid 4-[(adamantane-1-carbonyl)-amino]-2,6-dimethyl-pyridin-3-yl ester;

2-Phenyl-1-(3-phenyl-pyrrolidin-1-yl)-ethanone;
Adamantane-1-carboxylic acid 4-[(adamantane-1-carbonyl)-amino]-2-ethyl-6-methyl-pyridin-3-yl ester;
N-(1,3-Dioxo-1,3-dihydro-isoindol-2-ylmethyl)-N-(4-hydroxy-phenyl)-benzamide; Biphenyl-4-yl-piperidin-1-yl-methanone;
Azepan-1-yl-(3,5-dichloro-phenyl)-methanone;
Azepan-1-yl-biphenyl-4-yl-methanone;
Azepan-1-yl-(4-chloro-phenyl)-methanone;
3-Heptylcarbamoyl-bicyclo[2.2.1]hept-5-ene-2-carboxylic acid; Adamantan-1-yl-azepan-1-yl-methanone;
N,N-Dibenzyl-3,4-dimethoxy-benzamide;
N-Benzyl-N-isopropyl-4-nitro-benzamide;
N-[2-(1H-indol-3-yl)-1-methyl-ethyl]-2-(4-isobutyl-phenyl)-propionamide; N-tert-Butyl-2-(4-isobutyl-phenyl)-propionamide;
Adamantane-1-carboxylic acid (2-acetyl-phenyl)-amide; N-(1,3-Dioxo-1,3-dihydro-isoindol-2-ylmethyl)-N-(4-fluoro-phenyl)-benzamide; (Octahydro-quinolin-1-yl)-phenyl-methanone;
(7-Hydroxy-octahydro-quinolin-1-yl)-phenyl-methanone; N-(1,3-Dioxo-1,3-dihydro-isoindol-2-ylmethyl)-N-p-tolyl-benzamide; N,N-Dibenzyl-4-methyl-benzamide;
(2-Chloro-phenyl)-(2-methyl-piperidin-1-yl)-methanone; [4-Bromo-3-(pipendine-1-sulfonyl)-phenyl]-piperidin-1-yl-methanone; 2-Chloro-N-(3,4-dimethyl-phenyl)-benzamide;
1-Azepan-1-yl-2-(3,3-dimethyl-3,4-dihydro-2H-isoquinolin-1-ylidene)-ethanone; N-Cyclohexyl-4-(2,4-dichioro-phenoxy)-butyramide;
N-Benzo[1,3]dioxol-5-yl-2-chloro-benzamide;
(4-Benzyl-piperldin-1-yl)-(2-chloro-phenyl)-methanone; 2-(Benzothiazol-2-ylsulfanyl)-N-cyclohexyl-acetamide;
Cyclohexanecarboxylic acid (7,7-dimethyl-5-oxo-5,6,7,8-tetrahydro-quinazolin-2-yl)-amide; 2,4-Dichloro-N-ethyl-N-o-tolyl-benzamide;
(4-Benzyl-piperidin-1-yl)-(4-fluoro-phenyl)-methanone; N-Cyclohexyl-4-(2,4-dichloro-phenoxy)-N-methyl-butyramide; 3-[2-(1,3-Dioxo-1,3-dihydro-isoindol-2-yl)-ethyl]-adamantane-1-carboxylic acid; Morpholin-4-yl-(3-p-tolyl-adamantan-1-yl)-methanone;

N-Benzyl-2,4-dichloro-N-methyl-benzamide;
Thiophene-2-carboxylic acid dibenzylamide;
Azepan-1-yl-(2-bromo-phenyl)-methanone;
(3,4-Dichloro-phenyl)-(4-methyl-piperidin-1-yl)-methanone;
N,N-Dibenzyl-3,4-dichloro-benzamide;
4-(2,4-Dichloro-phenoxy)-1-piperidin-1-yl-butan-1-one;
N,N-Dibenzyl-2-fluoro-benzamide;
(2-Chloro-phenyl)-piperidin-1-yl-methanone;
2-Chloro-N-(3-trifluoromethyl-phenyl)-benzamide;
N-Benzyl-N-ethyl-2-phenyl-acetamide;
(3,4-Dihydro-2H-quinolin-1-yl)-p-tolyl-methanone;
Thiophene-2-carboxylic acid benzyl-ethyl-amide;
N-Adamantan-1-yl-2-dibenzylamino-acetamide;
N-Cyclohexyl-2-(1,3-dioxo-1,3-dihydro-isoindol-2-yl)-3-phenyl-propionamide; Thiophene-2-carboxylic acid cycloheptylamide;
N-Cyclohexyl-3-diethylsulfamoyl-4-methyl-benzamide;
4-Benzoyl-N-methyl-N-phenyl-benzamide;
N-Benzyl-2-bromo-N-methyl-benzamide;
2-Chloro-N-methyl-N-phenyl-benzamide;
4-Chloro-N-ethyl-N-o-tolyi-benzamide;
N-Benzyl-4,N-dimethyl-benzamide;
2-(4-Chloro-3,5-dimethyl-phenoxy)-N-cyclohexyl-N-methyl-acetamide; N-Benzyl-2-bromo-N-isopropyl-benzamide;
3-(2-Chloro-phenyl)-N-cyclohexyl-N-methyl-acrylamide;
N-Phenyl-N-(2,2,5-trimethyl-hex-4-enyl)-acetamide;
N-m-Tolyl-N-(2,2,5-trimethyl-hex-4-enyl)-acetamide;
(3-Chloro-benzo[b]thiophen-2-yl)-(4-methyl-piperazin-1-yl)-methanone; Adamantane-1-carboxylic acid (5-methyl-pyridin-2-yl)-amide;
3-Bromo-N-[2-methyl-1-(1,3,3-trimethyl-6-aza-bicyclo[3.2.1]octane-6-carbonyl)-butyl]-benzamide;

4-Chloro-N-[2-methyl-l-(1,3,3-trimethyl-6-aza-bicyclo[3.2.1]octane-6-carbonyl)-butyl]-benzamide;
4-Methyl-N-[2-methyl-1-(1,3,3-trimethyl-6-aza-bicyclo[3.2.1]octane-6-carbonyl)-butyl]-benzamide;
Cyclohexanecarboxylic acid [2-methyl-1-(1,3,3-trimethyl-6-aza-bicyclo[3.2.1]octane-6-carbonyl)-butyl]-amide;
3-Cyclopentyl-N-[2-methyl-1-(1,3,3-trimethyl-6-aza-bicyclo[3.2.1]octane-6-carbonyl)-butyl]-propionamide;
2-Chloro-N-[2-(4-ethyl-benzoylamino)-ethyl]-N-(4-fluoro-phenyl)-benzamide; N-{1-Benzyl-2-[4-(3-cyclopentyl-propionyl)-piperazin-1-yl]-2-oxo-ethyl}-3-cyclopentyl-propionamide;
N-Bicyclo[2.2.1]hept-5-en-2-ylmethyl-3-cyclopentyl-N-[2-(1H-indol-3-yl)-ethyl]-propionamide; N-Bicyclo[2.2.1]hept-5-en-2-ylmethyl-2,4-dichloro-N-[2-(1H-indol-3-yl)-ethyl]-benzamide; Naphthalene-2-carboxylic acid (2-oxo-azepan-3-yl)-thiophen-3-ylmethyl-amide; 3,4,5-Trimethoxy-N-(4-methyl-benzyl)-N-[6-(pyridin-2-ylamino)-hexyl]-benzamide; 3-Cyclopentyl-N-(4-methyl-benzyl)-N-[6-(pyridin-2-ylamino)-hexyl]-propionamide; N-(3,4-Dimethoxy-benzyl)-3-methoxy-N-[6-(pyridin-2-ylamino)-hexyl]-benzamide; N,N-Dimethyl-2-[3-(4-nitro-phenyl)-adamantan-1-yl]-acetamide;
Adamantane-1-carboxylic acid [4-(1,3-dioxo-1,3-dihydro-isoindol-2-yl)-butyl]-p-tolyl-amide; 2-(1,3-Dioxo-1,3-dihydro-isoindol-2-yl)-3-methyl-N-(2-trifluoromethyl-phenyl)-butyramide; 2-(4-Chloro-2-methyl-phenoxy)-N-(2-trifluoromethyl-phenyl)-propionamide; 4-(2,4-Dichloro-phenoxy)-1-[4-(4-fluoro-phenyl)-piperazin-1-yl]-butan-1-one; (3,4-Dihydro-2H-quinolin-1-yl)-[3-(piperidine-1-sulfonyl)-phenyl]-methanone;
Acetic acid 4-(azepane-1-carbonyl)-phenyl ester;
N-Adamantan-1-ylmethyl-benzamide;
[3-(4-Nitro-phenyl)-adamantan-1-yl]-piperidin-1-yl-methanone;
N-(1,1-Dimethyl-hexyl)-2-morpholin-4-yl-acetamide;
Adamantyl-1-carboxylic acid (2-methoxy-ethyl)-amide;
N-(4-Adamantan-1-yl-2-methyl-phenyl)-acetamide;
3-p-Tolyl-adamantane-1-carboxylic acid (2,5-dichloro-phenyl)-amide; (3-Chloro-adamantan-1-yl)-pyrrolidin-1-yl-methanone;
2-Amino-5-cyclohexylcarbamoyl-4-methyl-thiophene-3-carboxylic acid ethyl ester; N-(2-Chloro-phenyl)-2-{3-[(2-chloro-phenylcarbamoyl)-methyl]-adamantan-9-yl}-acetamide; 3-p-Tolyl-adamantane-1-carboxylic acid (2,4-difluoro-phenyl)-amide; Adamantyl-1-carboxylic acid tert-butylamide;
2-Adamantan-1-yl-N-tert-butyl-acetamide;
N-Methyl-N-phenyl-4-(pyrrolidine-1-sulfonyl)-benzamide;
N-(1-Adamantan-1-yl-ethyl)-2-fluoro-benzamide;
Adamantane-1-carboxylic acid [2-(3,4-dimethoxy-phenyl)-ethyl]-amide; Adamantane-1-carboxylic acid dimethylamide;
N-Benzyl-4-chloro-N-(1-cyclopropyl-vinyl)-benzamide;
3,5-Dimethyl-adamantane-1-carboxylic acid benzylamide;
2,4-Dichloro-N-cyclohexyl-N-(2-hydroxy-ethyl)-benzamide;
N-Adamantan-1-yl-2,4-dichloro-N-ethyl-benzamide;
2-[(3-p-Tolyl-adamantane-1-carbonyl)-amino]-propionic acid methyl ester;
N-Adamantan-1-yl-3-morpholin-4-yl-propionamide;
3-p-Tolyl-adamantane-l-carboxylic acid isopropylamide;
N-Adamantan-1-yl-2-benzylamino-acetamide;
N-Benzyl-2,4-dichloro-N-(1-cyclopropyl-ethyl)-5-methyl-benzamide;
2-[(Adamantane-1-carbonyl)-amino]-benzoic acid ethyl ester;
N-Benzyl-N-isopropyl-4-methyl-3-nitro-benzamide;
(3,4-Dihydro-2H-quinolin-1-yl)-(2-fluoro-phenyl)-methanone;
N-Ethyl-2-fluoro-N-phenyl-benzamide;
2-Phenethyl-N-(2-tifluoromethyl-phenyl)-benzamide;
1-(3,4-Dihydro-2H-quinolin-1-yl)-2-o-tolyloxy-ethanone;
2-(1-Benzyl-1H-imidazol-2-ylsulfanyl)-N-cyclohexyl-acetamide;
Cyclohexanecarboxylic acid (2-propoxy-phenyl)-amide;
2-{3-[4-(2-Chloro-phenyl)-piperazin-1-yl]-3-oxo-propyl}-isoindole-1,3-dione;
N-Cyclopentyl-2-(2,4-dichloro-phenoxy)-propionamide;
Adamantane-1-carboxylic acid (2-trifluoromethyl-phenyl)-amide;
(4-Chloro-3-nitro-phenyl)-(2,6-dimethyl-piperidin-1-yl)-methanone;
4-(2-Ethyl-phenyl)-4-aza-tricyclo[5.2.2.0^{2.6}]undec-8-ene-3,5-dione;
2-Phenyl-N-(2-trifluoromethyl-phenyl)-butyramide;
N-Adamantan-1-yl-4-chloro-2-nitro-benzamide;
3-p-Tolyl-adamantane-1-carboxylic acid (2,3-dimethyl-phenyl)-amide;
N-Benzyl-3-methyl-4-p-tolyl-butyramide;
N-(2-Cyclohex-1-enyl-ethyl)-2-(1,3-dioxo-1,3-dihydro-isoindol-2-yl)-propionamide;

(4-tert-Butyl-phenyl)-(3,4-dihydro-1H-isoquinolin-2-yl)-methanone;
2-[1-(4-Chloro-benzenesulfonyl)-1H-benzoimidazol-2-ylsulfanyl]-N-thiophen-2-ylmethyl-acetamide;
2-Phenoxy-1-[4-(2-trifluoromethyl-benzyl)-piperazin-1-yl]-ethanone;
Cyclohexanecarboxylic acid [5-(2-fluoro-benzylsulfanylmethyl)-[1,3,4]thiadiazol-2-yl]-amide; 4-Methyl-2-phenyl-thiazole-5-carboxylic acid naphthalen-1-ylamide;
4-Fluoro-N-[4-(1,3,3-trimethyl-6-aza-bicyclo[3.2.1]octane-6-carbonyl)-phenyl]-benzenesulfonamide;
(3-Methoxy-phenyl)-(4-o-tolyl-piperazin-1-yl)-methanone;
N-Adamantan-1-yl-3-(1,3-dioxo-1,3-dihydro-isoindol-2-yl)-propionamide;
N-Cyclooctyl-2-methoxy-3-methyl-benzamide;
2-[4-(2,3,3-Trimethyl-6-aza-bicyclo[3.2.1]octane-6-carbonyl)-phenyl]-isoindole-1,3-dione;
(2,3-Diphenyl-quinoxalin-6-yl)-(2,3,3-trimethyl-6-aza-bicyclo[3.2.1]oct-6-yl)-methanone;
Adamantan-1-yl-(1,3,4,5-tetrahydro-pyrido[4,3-b]indol-2-yl)-methanone;
N-{4-[1-(Naphthalene-2-sulfonyl)-piperidin-3-yl]-butyl}-N-p-tolyl-oxalamide;
N-Benzyl-N-(2-oxo-2-pyrrolidin-1-yl-ethyl)-benzenesulfonamide;
(4-Amino-phenyl)-(1,3,3-trimethyl-6-aza-bicyclo[3.2.1]oct-6-yl)-methanone;
1-[4-(4-Fluoro-phenyl)-piperazin-1-yl]-2-(2-isopropyl-5-methyl-phenoxy)-ethanone;
Adamantane-1-carboxylic acid benzyl-pyridin-2-yl-amide;
Adamantan-1-yl-piperidin-1-yl-methanone;
Adamantan-1-yl-pyrrolidin-1-yl-methanone;
(3,4-Dihydro-2H-quinolin-1-yl)-o-tolyl-methanone;
Adamantyl-1-carboxylic acid benzylamide;
Pyridine-2-carboxylic acid adamantan-2-ylamide;
(3-Chloro-adamantan-1-yl)-piperidin-1-yl-methanone;
Adamantan-1-yl-(4-methyl-piperidin-1-yl)-methanone;
2-[3-(Azepane-1-carbonyl)-phenyl]-isoindole-1,3-dione;
2-[3-(Piperidine-1-carbonyl)-phenyl]-isoindole-1,3-dione;
4-(Benzyl-methanesulfonyl-amino)-N-furan-2-ylmethyl-benzamide;
(4-Nitro-phenyl)-(1,3,3-trimethyl-6-aza-bicyclo[3.2.1]oct-6-yl)-methanone;
1-Cyclohexyl-5-oxo-pyrrolidine-3-carboxylic acid (4-chloro-3-nitro-phenyl)-amide;
N-(2-Chloro-phenyl)-2-(2-oxo-2-phenyl-ethylsulfanyl)-acetamide;
3-(4-Hydroxy-phenyl)-N-isochroman-1-ylmethyl-3-phenyl-propionamide;
(4-Ethoxy-phenyl)-(2-methyl-piperidin-1-yl)-methanone;
1-Cyclohexyl-5-axo-pyrrolidine-3-carboxylic acid (3-chloro-phenyl)-amide; N-[4-(Benzyl-isopropyl-sulfamoyl)-phenyl]-acetamide;
N-(3,4-Dimethyl-phenyl)-N-[4-(piperidine-1-carbonyl)-benzyl]-methanesulfonamide;

2-(5-Phenyl-1H-imidazol-2-ylsulfanyl)-N-(1,1,3,3-tetramethyl-butyl)-acetamide;
2-(Benzothiazol-2-ylsulfanyl)-N-(1,1,3,3-tetramethyl-butyl)-acetamide;
2-(Benzooxazol-2-ylsulfanyl)-N-(1,1,3,3-tetramethyl-butyl)-acetamide;
2-(Naphthalen-2-ylcarbamoylmethylsulfanyl)-N-(1,1,3,3-tetramethyl-butyl)-acetamide;
Acetic acid 4-(3,5-dimethyl-piperidine-1-carbonyl)-phenyl ester;
[1-(4-Chloro-benzenesulfonyl)-piperidin-3-yl]-(octahydro-quinolin-1-yl)-methanone;
(4-Fluoro-phenyl)-(3,4,4a,8a-tetrahydro-2H-quinolin-1-yl)-methanone;
N-Adamantan-1-yl-2-ethoxy-acetamide;
2-(2-Oxo-2-phenothiazin-10-yl-ethyl)-hexahydro-isoindole-1,3-dione;
Adamantane-1-carboxylic acid (tetrahydro-furan-2-ylmethyl)-amide;
2-Bromo-N-cycloheptyl-benzamide;
Bicyclo[2.2.1]hept-2-yl-[4-(2-ethoxy-phenyl)-piperazin-1-yl]-methanone; N-Furan-2-ylmethyl-2-phenyl-2-phenylsulfanyl-acetamide;
Adamantane-1-carboxylic acid benzyl-methyl-amide;
1-(3,4-Dihydro-2H-quinolin-1-yl)-3-(4-fluoro-phenyl)-propenone; Adamantan-1-yl-(2,6-dimethyl-piperidin-1-yl)-methanone;
4-Methyl-N-homoadamantyl-3-yl-benzamide;
(3,5-Dimethyl-piperidin-1-yl)-(3-methyl-4-nitro-phenyl)-methanone; Quinoline-2-carboxylic acid cyclooctylamide;
Adamantane-1-carboxylic acid [2-(2,4-dimethoxy-phenyl)-ethyl]-amide;
(3,4-Dihydro-1H-isoquinolin-2-yl)-o-tolyl-methanone;
(3,6-Dichloro-benzo[b]thiophen-2-yl)-(4-methyl-piperazin-1-yl)-methanone; 3-(1-Benzyl-1H-imidazol-2-ylsulfanyl)-N-cyclohexyl-proplonamide;
Propionic acid 2-amino-4-methyl-5-p-tolylcarbamoyl-thiophen-3-yl ester; 2-Cyclohexyl-N-(2,6-dimethyl-phenyl)-N-furan-2-ylmethyl-acetamide;
(3-Methoxy-phenyl)-(2,2,4.trimethyl-4-phenyl-3,4-dihydro-2H-quinolin-1-yl)-methanone;
1-[4-(2,3,3-Trimethyl-6-aza-bicyclo[3.2,1]octane-6-carbonyl)-phenyl]-pyrrolidine-2,5-dione;
1-(3,4-Dihydro-2H-quinolin-1-yl)-2-(1-naphthalen-1-yl-1H-tetrazol-5-ylsulfanyl)-ethanone;
[4-(2,3-Dimethyl-phenyl)-piperazin-1-yl]-o-tolyl-methanone;
(4-Benzyl-piperidin-1-yl)-(4-methyl-3-nitro-phenyl)-methanone;
N-(2-Cyano-phenyl)-2-(9-ethyl-9H-1,3,4,9-tetraaza-fluoren-2-ylsulfanyl)-acetamide;
N-(2-Cyano-phenyl)-2-(9-methyl-9H-1,3,4,9-tetraaza-fluoren-2-ylsulfanyl)-acetamide;

1-(Thiophene-2-carbonyl)-2,3-dihydro-1H-quinolin-4-one;
(3-Chloro-6-nitro-benzo[b]thiophen-2-yl)-piperidin-1-yl-methanone; (4-Bromo-phenyl)-(3,5-dimethyl-piperidin-1-yl)-methanone;
2-Morpholin-4-yl-N-(1-phenyl-cyclopentylmethyl)-acetamide;
9-Oxo-9H-fluorene-1-carboxylic acid (3-methyl-butyl)-amide;
[4-(2,5-Dimethyl-pyrrol-1-yl)-phenyl]-(4-methyl-piperidin-1-yl)-methanone;
N-Cycloheptyl-3-diethylsulfamoy-benzamide;
(4-Methoxy-phenyl)-(3-phenyl-piperidin-1-yl)-methanone;
3-Amino-N-cyclohexyl-N-methyl-benzamide;
N-Ethyl-3,4-dimethyl-N-phenyl-benzamide;
N-Benzyl-3,4,N-trimethyl-benzamide;
(4-Fluoro-phenyl)-(3-phenyl-piperidin-1-yl)-methanone;
[4-(2,3-Dimethyl-phenyl)-piperazin-1-yl]-(3-methoxy-phenyl)-methanone;
Furan-2-carboxylic acid [4-(4-methyl-pipendine-1-sulfonyl)-phenyl]-amide;
N-(2-Cyclohex-1-enyl-ethyl)-2-o-tolyloxy-acetamide;
5-(2-Chloro-phenoxymethyl)-furan-2-carboxylic acid (1-bicyclo[2,2.1]hept-2-y-ethyl)-amide;
3-(2-Chloro-phenyl)-1-[4-(2,3-dimethyl-phenyl)-piperazin-1-yl]-propenone;
N-[3-(Azepane-1-carbonyl)-phenyl]-benzamide;
[3-(Piperidine-1-carbonyl)-pyrazol-1-yl]-o-tolyl-methanone;
N-(1-Phenyl-cyclopentylmethyl)-2-piperidin-1-yl-proplonamide;
2-Morpholin-4-yl-N-(1-phenyl-cyclopentylmethyl)-propionamide;
N-[4-(Azepane-1-sulfonyl)-phenyl]-2,2,2-trifluoro-acetamide;
2,3-Dihydro-benzo[1,4]dioxine-6-carboxylic acid (1-adamantan-1-yl-ethyl)-amide;
N-Adamantan-1-yl-2-(3-methoxy-phenoxy)-acetamide;
3-Chloro-benzo[b]thiophene-2-carboxylic acid (2-cyano-ethyl)-phenyl-amide;
[4-(4-Nitro-benzyl)-piperidin-1-yl]-phenyl-methanone;
[4-(2-Nitro-benzyl)-piperldin-1-yl]-phenyl-methanone;
3-[5-(4-Fluoro-phenyl)-furan-2-yl]-1-(1,3,3-trimethyl-6-aza-bicyclo[3,2.1]oct-6-yl)-propenone;
2-(3-Fluoro-benzoylamino)-4-methyl-5-(piperidine-1-carbonyl)-thiophene-3-carboxylic acid methyl ester;
N-(2-Ethyl-phenyl)-2-(3-methyl-piperidin-1-yl)-acetamide;
2-(2-Methoxy-benzoylamino)-4-methyl-5-(piperidine-1-carbonyl)-thiophene-3-carboxylic acid methyl ester;
1-Phenyl-cyclopentanecarboxylic acid (4-phenyl-tetrahydro-pyran-4-ylmethyl)-amide;
4-(2,4-Dichloro-phenoxy)-1-(4-phenyl-piperazin-1 yl)-butan-1-one;
Naphthalene-1-carboxylic acid cycloheptylamide;
N-indan-5-yl-2-methyl-3-nitro-benzamide;
N-Cyclohexyl-3-(2,2,2-trifluoro-ethoxymethyl)-benzamide;
2-Methoxy-N-(1-phenyl-cyclopentylmethyl)-benzamide;
[5-(2,5-Dichloro-phenoxymethyl)-furan-2-yl]-(2,6-dimethyl-morpholin-4-yl)-methanone;

[5-(2-Bromo-phenoxymethyl)-furan-2-yl]-(2-methyl-piperidin-1-yl)-methanone;
5-(2-Methoxy-phenoxymethyl)-furan-2-carboxylic acid cycloheptylamide;
(3,4-Dihydro-1H-isoquinolin-2-yl)-[1-(2-nitro-benzenesulfonyli)-piperidin-3-yl]-methanone;
N-Cyclooctyl-2-(4-methoxy-phenoxy)-acetamide;
N-(2,3-Dimethyl-phenyl)-4-[methyl-(toluene-4-sulfonyl)-amino]-butyramide;
N-Phenyl-N-[4-(piperidine-1-carbonyl)-benzyl]-benzenesulfonamide;
N-[4-(3,4-Dihydro-1H-isoquinoline-2-carbonyl)-benzyl]-N-(3,4-dimethyl-phenyl)-methanesulfonamide;
2,3-Dihydro-benzo[1,4]dioxine-2-carboxylic acid bicyclo[2.2.1]hept-2-ylamide; 4,5,6,7-Tetrahydro-benzo[b]thiophene-3-carboxylic acid cycloheptylamide;
N-(2-Azepan-1-yl-2-oxo-ethyl)-N-benzyl-4-fluoro-benzenesulfonamide;
1-(2,6-Dimethyl-morpholin-4-yl)-3,3-diphenyl-propan-1-one;
N-Bicyclo[2.2.1]hept-2-yl-4-morpholin-4-ylmethyl-benzamide;
[3-(2-Chloro-6-nitro-phenoxy)-phenyl]-piperidin-1-yl-methanone;
N-Adamantan-1-yl-2-(4-methyl-quinolin-2-ylsulfanyl)-acetamide;
Cyclohexanecarboxylic acid (2-phenylsulfanyl-phenyl)-amide;
(4-Hydroxy-4-phenyl-octahydro-quinolin-1-yl)-phenyl-methanone;
3-Cyclohexyl-N-(3-phenyl-propyl)-propionamide;
2-[1-(2,5-Dimethyl-phenyl)-1H-tetrazol-5-ylsulfanyl]-N-isopropyl-N-phenyl-acetamide
N-{2-[4-(3,4-Dihydro-1H-isoquinoline-2-sulfonyl)-phenyl]-ethyl}-acetamide;
N-Benzyl-N-[2-oxo-2-(4-phenyl-piperazin-1-yl)-ethyl]-benzenesulfonamide;
[4-(2-Chloro-6-nitro-phenoxy)-phenyl]-piperidin-1-yl-methanone;
N-Cycloheptyl-3-phenyl-propionamide;
(3-Chloro-6-methyl-benzo[b]thiophen-2-yl)-piperidin-1-yl-methanone;
N-Cycloheptyl-2,4-dimethoxy-benzamide;
N-(3-Chloro-phenyl)-2-(8,11,11-trimethyl-3,4,6-triaza-tricyclo[6.2.1.0^{2.7}]undeca-2(7),3,5-trien-5-ylsulfanyl)-acetamide;
N-(2-Nitro-phenyl)-2-(8,11,11-trimethyl-3.4,6-triaza-tricyclo[6.2.1.0^{2,7}]undeca-2(7),3,5-trien-5-ylsulfanyl)-acetamide;
N-Phenyl-2-(8,11,11-trimethyl-3,4,6-triaza-tricyclo[6.2.1.0^{2.7}]undeca-2(7),3,5-trien-5-ylsulfanyl)-acetamide;
N-Ethyl-3-methyl-N-o-tolyl-benzamide;
N-[5-(2,4-Dichloro-benzylsulfanyl)-[1,3,4]thiadiazol-2-yl]-2,2-dimethyl-propionamide;
4-Bromo-1-ethyl-1H-pyrazole-3-carboxylic acid (2-methylsulfanyl-phenyl)-amide;
5-Benzoyl-furan-2-carboxylic acid diisopropylamide;
N-{2-[2-(4-Bromo-phenyl)-2-oxo-ethylsulfanyl]-benzothiazol-6-yl}-acetamide;
2-(6-Amino-benzothiazol-2-ylsulfanyl)-N-cyclohexyl-acetamide;
N-(2-Cyclohexylcarbamoylmethylsulfanyl-benzothiazol-6-yl)-2-methoxy-benzamide;
Benzofuran-2-yl-(4-phenyl-piperidin-1-yl)-methanone;
1-(2-Nitro-phenyl)-piperidine-3-carboxylic acid diethylamide;
1-(4-Nitro-phenyl)-piperidine-3-carboxylic acid diethylamide;
5-Bromo-furan-2-carboxylic acid adamantan-2-ylamide;
3,3-Dimethyl-pentanedioic acid bis-[(2,4-difluoro-phenyl)-amide];
2-(3-Bromo-benzylsulfanyl)-1-(4-phenyl-piperazin-1-yl)-ethanone;
N-(2-Azepan-1-yl-2-oxo-ethyl)-N-benzyl-4-bromo-benzenesulfonamide;
1-(2,3-Dihydro-indol-1-yl)-2-p-tolylsulfanyl-ethanone;
[4-(4-Bromo-benzenesulfonyl)-piperazin-1-yl]-furan-2-yl-methanone;
[5-(2-Bromo-phenoxymethyl)-furan-2-yl]-(2,6-dimethyl-morpholin-4-yl)-methanone;

5-(2-Chloro-phenoxymethyl)-furan-2-carboxylic acid diethylamide;
5-(2-Bromo-phenoxymethyl)-furan-2-carboxylic acid diethylamide;
5-(2-Chloro-phenoxymethyl)-furan-2-carboxylic acid methyl-phenyl-amide;
[5-(2-Chloro-phenoxymethyl)-furan-2-yl]-(4-methyl-piperidin-1-yl)-methanone;
[3-(2,5-Dichloro-phenoxymethyl)-phenyl]-pyrrolidin-1-yl-methanone;
[5-(4-Ethoxy-phenoxymethyl)-furan-2-yl]-(4-methyl-piperidin-1-yl)-methanone;
3-(1,3-Dioxo-1,3-dihydro-isoindol-2-yl)-N-(2-methyl-cyclohexyl)-propionamide;
3-(3,4-Dihydro-2H-quinoline-1-carbonyl)-N-phenyl-benzenesulfonamide;
[3-(2,3-Dihydro-indole-1-sulfonyl)-phenyl]-(3,4-dihydro-2H-quinolin-1-yl)-methanone;
[3-(2,5-Dimethyl-pyrrol-1-yl)-phenyl]-(4-methyl-piperidin-1-yl)-methanone;
N-Cyclohexyl-3-(2-hydroxy-4-methyl-phenyl)-3-phenyl-propionamide;
2-Diethylamino-N-(1-phenyl-cyclopentylmethyl)-propionamide;
(6-Fluoro-2-methyl-3,4-dihydro-2H-quinolin-1-yl)-(3-trifluoromethyl-phenyl)-methanone;
(2,6-Dimethyl-morpholin-4-yl)-[4-(naphthalen-1-yloxymethyl)-phenyl]-methanone;
N-Benzyl-4-bromo-N-ethyl-benzamide;
(3-Methyl-piperidin-1-yl)-[4-(naphthalen-1-yloxymethyl)-phenyl]-methanone;
Azepan-1-yl-[5-(2-chloro-phenoxymethyl)-furan-2-yl]-methanone;
(4-Methyl-piperidin-1-yl)-[4-(naphthalen-1-yloxymethyl)-phenyl]-methanone;
Azepan-1-yl-[5-(2,4-dichloro-phenoxymethyl)-furan-2-yl]-methanone;
N-Cycloheptyl-4-(4-methoxy-2-methyl-phenyl)-butyramide;
2-(4-Benzothiazol-2-yl-piperazin-1-yl)-N-cyclohexyl-acetamide;
N-Cycloheptyl-2-(2,6-dimethyl-phenoxy)-acetamide;
(3,4-Dihydro-2H-quinolin-1-yl)-(3-iodo-phenyl)-methanone;
N-Cycloheptyl-3-(2,2,2-trifluoro-ethoxymethyl)-benzamide;
Azepan-1-yl-[4-(2-chloro-phenoxymethyl)-phenyl]-methanone;
(2,6-Dimethyl-morpholin-4-yl)-[4-(naphthalen-2-yloxymethyl)-phenyl]-methanone;
Azepan-1-yl-[3-(4-ethoxy-phenoxymethyl)-phenyl]-methanone;
Benzo[b]thiophene-3-carboxylic acid (1,2,3,4-tetrahydro-naphthaien-1-yl)-amide;

2-(4-Chloro-2-methyl-phenoxy)-N-cycloheptyl-acetamide;
2,4-Dichloro-N-cyclohexyl-N-methyl-benzamide;
N-Cyclooctyl-2-p-tolyloxy-acetamide;
(3,5-Dimethyl-piperidin-1-yl)-(4-methyl-3-nitro-phenyl)-methanone;
Biphenyl-4-yl-(2,6-dimethyl-piperidin-1-yl)-methanone;
N-Cyclohexyl-4-fluoro-N-methyl-benzamide;
N-[4-(Azepane-1-carbonyl)-phenyl]-N-methyl-benzenesulfonamide;
N-Cycloheptyl-2-fluoro-benzamide;
N-Cycloheptyl-4-methyl-benzamide;
(3-Methyl-piperidin-1-yl)-p-tolyl-methanone;
[2-(3,4-Dimethoxy-phenylcarbamoyl)-piperidin-1-yl]-acetic acid benzyl ester;
N-[4-(2-Methyl-piperidine-1-sulfonyl)-phenyl]-acetamide;
2-(2,4-Dichloro-phenoxy)-N-(2-methyl-butyl)-propionamide;
[4-(2-Chloro-6-nitro-phenyl)-piperazin-1-yl]-(4-methoxy-phenyl)-methanone;
N-Cyclohexyl-4-(4-methoxy-3-methyl-phenyl)-butyramide;
(3-Chloro-6-methoxy-benzo[b]thiophen-2-yl)-(3,4-dihydro-1H-isoquinolin-2-yl)-methanone;
2-(4-Methyl-benzylsulfanyl)-1-piperidin-1-yl-ethanone;
N-Cyclohexyl-N-[(4-phenyl-thiazol-2-ylcarbamoyl)-methyl]-benzamide;
N-(2-Azepan-1-yl-2-oxo-ethyl)-N-(4-isopropyl-phenyl)-methanesulfonamide;
N-Adamantan-1-yl-3-p-tolylsufanyl-propionamide;
6-(2,4-Dichloro-phenylcarbamoyl)-3,4-dimethyl-cyclohex-3-enecarboxylic acid;
(4-Butyl-cyclohexyl)-morpholin-4-yl-methanone;
(3,4-Dichloro-phenyl)-(3,4-dihydro-2H-quinolin-1-yl)-methanone;
N-(2-Cyclohex-1-enyl-ethyl)-3-methoxy-benzamide;
N-Adamantan-2-yl-3-(1,5-dimethyl-1H-pyrazol-4-yl)-acrylamide;
N-Adamantan-1-yl-N-methyl-4-(4-nitro-pyrazol-1-ylmethyl)-benzamide;
5-(4-Chloro-3,5-dimethyl-pyrazol-1-ylmethyl)-furan-2-carboxylic acid adamantan-2-ylamide;
2-(4-Chloro-phenoxy)-N-(2-fluoro-5-methyl-phenyl)-2-methyl-propionamide;
N-Adamantan-1-yl-2-(4-chloro-3,5-dimethyl-phenoxy)-acetamide;
2-[(3-Carboxy-bicyclo[2.2.1]heptane-2-carbonyl)-amino]-5,6-dihydro-4H-cyclopenta[b]thiophene-3-carboxylic acid propyl ester;
2-Adamantan-1-yl-N-[1-(2,5-dimethyl-phenyl)-ethyl]-acetamide;
3-Methyl-thiophene-2-carboxylic acid cyclooctylamide;
N-p-Tolyl-2-(8,11,11-trimethyl-3,4,6-triaza-tricyclo[6.2.1.0^{2,7}]undeca-2,4,6-trien-5-ylsulfanyl)-propionamide;

Azepan-1-yl-[5-(4-chloro-5-methyl-3-nitro-pyrazol-1-ylmethyl)-furan-2-yl]-methanone;
N-Adamantan-2-yl-3-(4-bromo-3-nitro-pyrazol-1-ylmethyl)-benzamide;
N-Bicyclo[2.2.1]hept-2-yl-2-chloro-benzamide;
[5-(3-Chloro-phenoxymethyl)-furan-2-yl]-piperidin-1-yl-methanone;
1-(4-Ethyl-benzoyl)-6-methoxy-2-methyl-2,3-dihydro-1H-quinolin-4-one;
6-Fluoro-2-methyl-1-{3-[4-(propane-1-sulfonyl)-phenoxymethyl]-benzoyl}-2,3-dihydro-*1H-*puinolin-4-one;
N-Cycloheptyl-2-(naphthalen-1-yloxy)-acetamide;
N-Cyclohexyl-4-o-tolyloxy-butyramide;
(2-Benzylsulfanyl-phenyl)-morpholin-4-yl-methanone;
(2-Chloro-5,6-difluoro-3-methyl-phenyl)-(4-methyl-piperidin-1-yl)-methanone;
(3-Bromo-phenyl)-[4-(4-chloro-2-nitro-phenyl)-piperazin-1-yl]-methanone;
2-Bromo-N-(1,1,3,3-tetramethyl-butyl)-benzamide;
N-Adamantan-1-yl-2-(2-benzoyl-5-methoxy-phenoxy)-acetamide;
N-Cyclohexyl-3-methyl-4-p-tolyl-butyramide;
[5-(4-Methyl-2-nitro-phenoxymethyl)-furan-2-yl]-thiomorpholin-4-yl-methanone;
[5-(2,5-Dichloro-phenoxymethyl)-furan-2-yl]-thiomorpholin-4-yl-methanone;
5-(4-Chloro-2-nitro-phenoxymethyl)-furan-2-carboxylic acid adamantan-1-ylamide;
4,5,6,7-Tetrahydro-benzo[b]thiophene-3-carboxylic acid cyclohexylamide;
4-Chloro-1,5-dimethyl-1H-pyrazole-3-carboxylic acid adamantan-1-yl-methyl-amide;
4-(4-Methoxy-3-methyl-phenyl)-N-(2-methyl-cyclohexyl)-butyramide;
3-Benzo[1,3]dioxol-5-yl-1-(3,4-dihydro-1H-isoquinolin-2-yl)-propenone;
N-Bicyclo[2.2.1]hept-2-yl-3-phenylsulfanyl-propionamide;
Azepan-1-yl-[5-(2-nitro-phenoxymethyl)-furan-2-yl]-methanone;
N-Benzyl-2-(4-chloro-phenylsulfanyl)-N-methyl-acetamide;
1-(4-Benzyl-piperidin-1-yl)-2-benzylsulfanyl-ethanone;
2-(4-tert-Butyl-phenoxy)-1-(4-ethyl-piperazin-1-yl)-ethanone;
[4-(4-Ethoxy-phenoxymethyl)-phenyl]-(4-methyl-piperidin-1-yl)-methanone;
5-(4-Bromo-3,5-dimethyl-pyrazol-1-ylmethyl)-furan-2-carboxylic acid adamantan-2-ylamide;
1-Azepan-1-yl-3-(4-chloro-phenylsulfanyl)-propan-1-one;
N-Bicydo[2.2.1]hept-2-yl-2-(2-chloro-benzylsulfanyl)-acetamide;
2-(2-Methyl-benzylsulfanyl)-1-(4-phenyl-piperazin-1-yl)-ethanone;
N-[2-(1-Benzo[1,3]dioxol-5-yl-3-furan-2-yl-3-oxo-propylsulfanyl)-phenyl]-acetamide;
(3,5-Dimethyl-piperidin-1-yl)-(3-iodo-phenyl)-methanone;
[5-(2-Bromo-phenoxymethyl)-furan-2-yl]-(6-fluoro-2-methyl-3,4-dihydro-2H-quinolin-1-yl)-methanone;

N-Benzyl-N-cyclohex-1-enyl-isonicatinamide;
1-[4-(4-Fluoro-phenyl)-piperazin-1-yl]-2-(2-methyl-benzylsulfanyl)-ethanone;
2-(2-Bromo-4-methyl-phenoxy)-N-(2-cyclohex-1-enyl-ethyl)-acetamide;
2-[5-(2-Hydroxy-phenyl)-[1,3,4]oxadiazol-2-ylsulfanyl]-1-piperidin-1-yl-ethanone;
5-(4-Nitro-pyrazol-1 -ylmethyl)-furan-2-carboxylic acid adamantan-2-yiemide;
3-Benzo[1,3]dloxol-5-yl-3-(2-methoxy-phenyl)-1-pyrrolidin-1-yl-propan-1-one;
N-Adamantan-2-yl-3,4-dichloro-benzamide;
Benzo[b]thiophen-3-yl-(6-fluoro-2-methyl-3,4-dihydro-2H-quinolin-1-yl)-methanone;
2-Adamantan-1-yl-1-(3,4-dihydro-1H-isoquinolin-2-yl)-ethanone;
4,5,6,7-Tetrahydro-benzo[b]thiophene-3-carboxylic acid (2-cyclohex-1-enyl-enhyl)-amide;
Benzo[b]thiophene-3-carboxylic acid (3,3,5-trimethyl-cyclohexyl)-amide;
2-(2,6-Dimethyl-phenoxy)-N-(2-isopropyl-phenyl)-acetamide;
4-Bromo-N-[3-(piperidine-1-carbonyl)-phenyl]-benzamide;
N-Benzo[1,3]dioxol-5-ylmethyl-2-(2-cyano-phenylsulfanyl)-benzamide;
N-Adamantan-1-yl-2-(naphthalen-2-yloxy)-acetamide;
[4-(4-Chloro-phenylsulfanylmethyl)-phenyl]-morpholin-4-yl-methanone;
Thiophene-2-carboxylic acid (3,3,5-trimethyl-cyclohexyl)-amide;
Benzo[1,3]dioxol-5-yl-(3,4-dihydro-2H-quinolin-1-yl)-methanone;
3-Chloro-benzo[b]thiophene-2-carboxylic acid cyclooctylamide;
2-[2-Morpholin-4-yl-1-(4-nitro-benzyl)-2-oxo-ethyl]-isoindole-1,3-dione;
2-Hydroxy-4,4-dimethyl-6-oxo-cyclohex-1-enecarboxylic acid phenylamide;
(2,4-Dichloro-phenyl)-(2,6-dimethyl-piperidin-1-yl)-methanone;
Adamantane-1-carboxylic acid furan-2-ylmethyl-p-tolyl-amide;
Azocan-1-yl-(4-tert-butyl-plenyl)-methanone;
3-Chloro-benzo[b]thiophene-2-carboxylic acid benzyl-methyl-amide;
Adamantane-1-carboxylic acid (2-fluoro-phenyl)-amide;
2-(Piperidine-1-carbonyl)-5-piperidin-1-yl-oxazoe-4-carbonitrile
N-(4,6-Dimethyl-5-nitro-pyridin-3-yl)-benzamide;
Adamantan-1-yl-[4-(2-methoxy-phenyl)-piperazin-1-yl]-methanone;
(2-Methyl-piperidin-1-yl)-o-tolyl-methanone;
N-Benzyl-4-chloro-N-isopropyl-3-nitro-benzamide;
N-(3-Hexylsulfanyl-[1,2,4]thiadiazol-5-yl)-3-methyl-butyramide;
4,N-Dimethyl-N-[4-(piperidine-1-carbonyl)-phenyl]-benzenesulfonamide;
Azepan-1-yl-(5-tert-butyl-2H-pyrazol-3-yl)-methanone;
2-Amino-4-methyl-5-(piperidine-1-carbonyl)-thiophene-3-carboxylic acid ethyl ester;
5-Methyl-furan-2-carboxylic acid (1-adamantan-1-yl-ethyl)-amide;
(3-Chloro-6-methyl-benzo[b]thiophen-2-yl)-(3,4-dihydro-*1H*-isoquinolin-2-yl)-methanone;
N-Adamantan-1-yl-2-trifluoromethyl-benzamide;
(3-Bromo-phenyl)-(2,2,4-trimethyl-4-phenyl-3,4-dihydro-*2H*-quinolin-1-yl)-methanone;
Benzo[1,3]dioxole-5-carboxcylic acid dipropylamide;
N-(3,3-Diphenyl-propyl)-4-methoxy-benzamide;
[4-(2-Chloro-6-nitro-phenyl)-piperazin-1-yl]-p-tolyl-methanone;
Furan-2-yl-[4-(toluene-4-sulfonyl)-piperazin-1-yl]-methanone;
3-(2-Chloro-6-fluoro-phertyl)-1-(3,4-dihydro-2H-quinolin-1-yl)-propenone;
2-Chloro-N-cycloheptyl-benzamide;
1-[4-(4-Nitro-phenyl)-piperazin-1-yl]-3-phenyl-propan-1-one;
(3,4-Dihydro-1H-isoquinolin-2-yl)-(3,4-dimethyl-phenyl)-methanone;
(1-Adamantan-1-yl-4-bromo-1H-pyrazol-3-yl)-morpholin-4-yl-methanone;
2-Phenyl-cyclopropanecarboxylic acid cyclooctylamide;
3-[4-(2-Ethoxy-phenyl)-piperazine-1-carbonyl]-isochromen-1-one;
[3-(4-Bromo-pyrazol-1-ylmethyl)-phenyl]-(4-methyl-piperidin-1-yl)-methanone;
2-Azepan-1-yl-N-biphenyl-2-yl-acetamide;
N-[b-(3,4-Dimethoxy-benzyl)-[1,3,4]thiadiazol-2-yl]-3-methyl-butyramide;
Adamantan-1-yl-(4-phenyl-piperldin-1-yl)-methanone;

N-(2-Azepan-1-yl-2-oxo-ethyl)-N-(4-ethoxy-phenyl)-4-methylsulfanyl-benzenesulfonamide;
1-Adamantan-1-yl-4-bromo-1H-pyrazole-3-carboxylic acid diethylamide;
(6-Fluoro-2-methyl-3,4-dihydro-2H-quinolin-1-yl)-(2-fluoro-phenyl)-methanone;
3-[4-(2,3-Dimethyl-phenyl)-piperazine-1-carbonyl]-isochromen-1-one;
N-Cyclooctyl-2-(2-methoxy-phenoxy)-acetamide;
N-Cyclohexyl-N-methyl-2-nitro-benzamide;
Adamantane-1-carboxylic acid (1,1-dioxo-tetrahydro-thiophen-3-yl)-amide;
N-Adamantan-2-yl-2-(4-chloro-phenyl)-acetamide;
(2,4-Dichloro-phenyl)-(3-methyl-piperidin-1-yl)-methanone;
2-(4-tert-Butyl-phenoxy)-N-cyclooctyl-acetamide;
(10,11-Dihydro-dibenzo[b,f]azepin-5-yl)-(2-methoxy-phenyl)-methanone;
(3-Chloro-phenyl)-(2-methyl-piperidin-1-yl)-methanone;
(3-Chloro-6-nitro-benzo[b]thiophen-2-yl)-(3-methyl-piperidin-1-yl)-methanone;
(2,5-Dichloro-phenyl)-(4-methyl-piperidin-1-yl)-methanone;
N-[5-(3,4-Dichloro-benzyl)-[1,3,4]thiadiazol-2-yl]-2,2-dimethyl-propionamide;
4-(4-Chloro-2-methyl-phenoxy)-1-(3,4-dihydro-2H-quinolin-1-yl)-butan-1-one;
(3,4-Dichloro-phenyl)-[4-(2,3-dimethyl-phenyl)-piperazin-1-yl]-methanone;
Cyclooctanecarboxylic acid [1-(naphthalene-2-sulfonyl)-pyrrolidin-2-yl]-amide;
1-Butyl-pyrrolidine-2-carboxylic acid benzo[1,3]dioxol-4-ylamide;
5-Methyl-furan-2-carboxylic acid dibenzylamide;
(3,4-Dihydro-2H-quinolin-1-yl)-[3-(4-phenyl-piperazine-1-sulfonyl)-phenyl]-methanone;
Bicyclo[2.2.1]hept-2-yl-[4-(2,3-dimethyl-phenyl)-piperazin-1-yl]-methanone;
N-Adamantan-1-yl-2-benzoyl-benzamide;
[5-(2-Chloro-phenoxymethyl)-furan-2-yl]-(3-methyl-piperldin-1-yl)-methanone;
(3,5-Dimethyl-piperidin-1-yl)-(2-iodo-phenyl)-methanone;
1-Benzenesulfonyl-pyrrolidine-2-carboxylic acid cyclooctylamide;
(3,4-Dimethoxy-phenyl)-(6-fluoro-2-methyl-3,4-dihydro-2H-quinolin-1-yl)-methanone;
3-(2,6-Dichloro-phenyl)-1-(2-ethyl-piperidin-1-yl)-propenone;
N-(3,4-Difluoro-phenyl)-2,6-difluoro-benzamide;
2,6-Difluoro-N-naphthalen-1-yl-benzamide;
(4-Chloro-phenyl)-(3,5-dimethyl-piperidin-1-yl)-methanone;
N-[4-(2,6-Dimethyl-piperidine-1-carbonyl)-phenyl]-2-(naphthalen-2-yloxy)-acetamide;

(2-Chloro-phenyl)-(3-methyl-piperidin-1-yl)-methanone;
N-{2-[4-(Piperidine-1-sulfonyl)-phenyl]-ethyl}-acetamide;
N-Biphenyl-2-yl-2-(pyridin-2-ylsulfanyl)-acetamide;
Azepan-1-yl-[5-(4-chloro-3,5-dimethyl-pyrazol-1-ylmethyl)-furan-2-yl]-methanone;
Acetic acid 4-(4-methyl-piperidine-1-carbonyl)-phenyl ester;
Acetic acid 4-(4-benzyl-piperidine-1-carbonyl)-phenyl ester;
Benzo[1,3]dioxole-5-carboxylic acid cyclohoptylamide;
2-(2,4-Dimethyl-phenoxy)-1-(6-fluoro-2-methyl-3,4-dihydro-2H-quinolin-1-yl)-ethanone;
Acetic acid 4-(3,4-dihydro-2H-quinoline-1-carbonyl)-phenyl ester;
Azepan-1-yl-(3,5-dibromo-phenyl)-methanone;
(3,5-Dibromo-phenyl)-[4-(2-methoxy-phenyl)-piperazin-1-yl]-methanone;
N-Cyclooctyl-4-isopropyl-benzamide;
N-Cyclooctyl-2-(4-methoxy-phenyl)-acetamide;
(4-tert-Butyl-piperidin-1-yl)-phenyl-methanone;
N-(4-tert-Butyl-3-nitro-phenyl)-acetamide;
(2,6-Dimethyl-piperidin-1-yl)-[5-(2,3,5,6-tetrafluoro-phenoxymethyl)-furan-2-yl]-methanone;
N-Cyclohexyl-3-(1,3-dioxo-1,3-dihydro-isoindol-2-yl)-N-methyl-propionamide;
2-(4-Chloro-3-methyl-phenoxy)-N-cyclohexyl-N-methyl-acetamide;
N-Cyclopentyl-3-(3,4-dihydro-2H-quinoline-1-carbonyl)-benzenesulfonamide;
(3,4-Dihydro-1H-isoquinolin-2-yl)-(3-dimethylamino-phenyl)-methanone;
3-Cyclohexylcarbamoyl-bicyclo[2.2.1]hept-5-ene-2-carboxylic acid isopropyl ester;
1-(3,4-Dihydro-1H-isoquinolin-2-yl)-2-(2-methoxy-phenyl)-ethanone;
N-Benzyl-N-cyclohex-1-enyl-benzamide;
[1-(Thiophene-2-sulfonyl)-piperidin-4-yl]-(1,3,3-trimethyl-6-aza-bicyclo[3.2.1]oct-6-yl)-methenone;
N-Adamantan-1-yl-2-(1-phenyl-1H-tetrazol-5-ylsulfanyl)-acetamide;
(3,4-Dihydro-2H-quinolin-1-yl)-[4-(morpholine-4-sulfonyl)-phenyl]-methanone;
(3,4-Dihydro-2H-quinolin-1-yl)-[4-(pyrrolidine-1-sulfonyl)-phenyl]-methanone;
(3,4-Dihydro-2H-quinolin-1-yl)-[1-(thiophene-2-sulfonyl)-pipefidin-4-yl]-methanone;
(1-Benzenesulfonyl-piperidin-3-yl)-(3,4-dihydro-1H-isoquinolin-2-yl)-methanone;
6,7-Dimethyl-4-oxa-tricyclo[4.3.0.0^{3,7}]nonane-3-carboxylic acid cyclohexylamide;
6,7-Dimethyl-4-oxa-tricyclo[4.3.0.0^{3,7}]nonane-3-carboxylic acid (2-chloro-phenyl)-amide;
(6,7-Dimethyl-4-oxa-tricyclo[4.3.0.0^{3,7}]non-3-yl)-piperidin-1-yl-methanone;
2-(5,6-Dimethyl-4-oxo-3,4-dihydro-thieno[2,3-d]pyrimidin-2-ylsulfanyl)-N-furan-2-ylmethyl-acetamide;
N-Allyl-2-(5,6-dimethyl-4-oxo-3,4-dihydro-thieno[2,3-d]pyrimidin-2-ylsulfanyl)-acetamide;
N-Adamantan-1-yl-2-(5,6,7,8-tetrahydro-benzo[4,5]thieno[2,3-d]pyrimidin-4-ylsulfanyl)-acetamide;
1-(3,4-Dihydro-2H-quinoline-1-carbonyl)-4,7,7-trimethyl-2-oxa-bicyclo[2.2.1]heptan-3-one;
1-(3,4-Dihydro-1H-isoquinoline-2-carbonyl)-4,7,7-trimethyl-2-oxa-bicyclo[2.2.1]heptan-3-one;
Azepan-1-yl-(6,7-dimethyl-4-oxa-tricyclo[4.3.0.0^{3,7}]non-3-yl)-methanone;
2,5-Dimethyl-furan-3-carboxylic acid (1-adamantan-1-yl-ethyl)-amide;
1-Cyclohexyl-5-oxo-pyrrolidine-3-carboxylic acid (3-cyano-4,5,6,7-tetrahydro-benzo[b]thiophen-2-yl)-amide;
2-(2-Cyano-phenylsulfanyl)-N-cyclopentyl-benzamide;
[5-(2-Methoxy-4-propyl-phenoxymethyl)-furan-2-yl]-(3-methyl-piperidin-1-yl)-methanone;
(4-tert-Butyl-phenyl)-(3,5-dimethyl-piperldin-1-yl)-methanone;
[4-(2-Methoxy-naphthalen-1-ylmethyl)-piperazin-1-yl]-(4-methoxy-phenyl)-methanone;

(3,4-Dichloro-phenyl)-(3,5-dimethyl-piperidin-1-yl)-methanone;
(4-Ethoxy-phenyl)-(4-methyl-piperidin-1-yl)-methanone;
2-Phenethyl-N-(tetrahydro-furan-2-ylmethyl)-benzamide;
N-Cycloheptyl-2-phenoxy-benzamide;
Adamantane-1-carboxylic acid (2-ethoxy-phenyl)-amide;
N-Adamantan-2-yl-2-o-tolyloxy-acetamide;
(2-Chloro-phenyl)-(3,5-dimethyl-piperidin-1-yl)-methanone;
1-Morpholin-4-yl-2-[3-(4-nitro-phenyl)-adamantan-1-yl]-ethanone;
2-Dimethylamino-N-(2-nitro-phenyl)-benzamide;
N-Benzyl-2-(4,4,6-trimethyl-1-p-tolyl-1,4-dihydro-pyrimidin-2-ylsulfanyl)-acetamide;
[4-(3,5-Dinitro-phenoxy)-phenyl]-(2-ethyl-piperidin-1-yl)-methanone;
1-(4-Chloro-benzoyl)-2,3-dihydro-1H-benzo[g]quinolin-4-one;
2-[(Adamantane-1-carbonyl)-amina]-3-phenyl-propionic acid methyl ester;
[Benzyl-(4-nitro-benzoyl)-amino]-acetic acid ethyl ester;
9-Oxo-9H-fluorene-3-carboxylic acid methyl-(4-nitro-phenyl)-amide;
Adamantane-1-carboxylic acid [2-(4-methoxy-phenyl)-ethyl]-amide;
(10,11-Dihydro-dibenzo[b,f]azepin-5-yl)-(4-fluoro-phenyl)-methanone;
2-Benzylsulfanyl-N-[2-(2-methoxy-phenoxy)-ethyl]-acetamide;
N-Adamantan-1-yl-2-(2-oxo-4-phenyl-pyrrolidin-1-yl)-acetamide;
2-Bromo-N-tricyclo[3.2.1.0^{2,4}]oct-6-ylmethyl-benzamide;
Adamantane-1-carboxylic acid (2,6-dimethoxy-pyrimidin-4-yl)-amide;
Hexanedioic acid (2,7,7-trimethyl-bicyclo[2.2.1]hept-1-yl)-amide (1,7,7-trimethyl-bicyclo[2.2.1]hept-2-yl)-amide;
2-Chloro-N-(2-cyclohexyl-ethyl)-benzamide;
2-[3-(2-Ethyl-piperidin-1-yl)-3-oxo-propyl]-isoindole-1,3-dione;
N-Adamantan-1-yl-2-hydroxy-2,2-diphenyl-acetamide;
Adamantane-1-carboxylic acid (naphthalen-1-ylmethyl)-amide;
Adamantane-1-carboxylic acid (benzo[1,3]dioxol-5-ylmethyl)-amide;
1-(Azepane-1-carbonyl)-fluoren-9-one;
2-(Quinolin-2-ylsulfanyl)-N-p-tolyl-acetamide;
2,4-Dichloro-N-[3-(piperidine-1-carbonyl)-phenyl]-benzamide;
2-Chloro-4,5-difluoro-N-(3,3,5-trimethyl-cyclohexyl)-benzamide;
2-(2-Chloro-benzylsulfanyl)-N-p-tolyl-acetamide;
[4-(4-Chloro-phenylsulfanylmethyl)-phenyl]-pyrrolidin-1-yl-methanone;
N-Adamantan-1-yl-N-methyl-isonicotinamide;
Azepan-1-yl-[4-(4-chloro-phenylsulfanylmethyl)-phenyl]-methanone;
(2-Chloro-phenyl)-(1,5,7-trimethyl-3,7-diaza-bicyclo[3.3.1]non-3-yl)-methanone;
(3-Chloro-benzo[b]thiophen-2-yl)-(4-methyl-piperidin-1-yl)-methanone;
Benzoic acid 1-benzoyl-decahydro-quinolin-4-yl ester;
2-(3-Bromo-benzylsulfanyl)-1-[4-(2-methoxy-phenyl)-piperazin-1-yl]-ethanone;
4-Methyl-N-[2-(phenoxazine-10-carbonyl)-phenyl]-benzenesulfonamide;
2-[1-(Azepane-1-carbonyl)-2-methyl-propyl]-isoindole-1,3-dione;
2-(3-Bromo-benzylsulfanyl)-1-pipendin-1-yl-ethanone;
1-[3-(4-Bromo-phenyl)-1-furan-2-yl-3-oxo-propyl]-pyrrolidin-2-one;
2-Chloro-N-cyclooctyl-4,5-difluoro-benzamide;
2,4-Dichloro-N-(2-furan-2-ylmethyl-cyclohexyl)-benzamide;
N-(4-Benzoyl-furazan-3-yl)-2-fluoro-benzamide;
N-Adamantan-1-yl-2-(3-cyano-4-methoxymethyl-6-methyl-pyridin-2-ylsulfanyl)-acetamide;
4-tert-Butyl-N-cyclooctyl-benzamide;
N-Adamantan-1-yl-2-phenyl-butyramide;
(3-Chloro-6-methoxy-benzo[b]thlophen-2-yl)-piperidin-1-yl-methanone;
(3,7-Dichloro-6-methoxy-benzo[b]thiophen-2-yl)-piperidin-1-yl-methanone;
Acetic acid 1-benzoyl-decahydro-quinolin-4-yl ester;
2-Bromo-N-methyl-N-phenyl-benzamide;
N-Benzo[1,3]dioxol-5-yl-2,4-dichloro-benzamide;
(3-Chloro-6-fluoro-benzo[b]thiophen-2-yl)-piperidin-1-yl-methanone;
N-(1,2,3,5,6,7-Hexahydro-s-indacen-1-yl)-2-piperidin-1-yl-acetamide;
2-[2-(1,3-Dioxo-1,3-dihydro-isoindol-2-yl)-3-phenyl-propionylamino]-3-methyl-butyric acid methyl ester;

2-(6-Oxo-6-piperidin-1-yl-hexyl)-isoindole-1,3-dione;
2-[2-(1,3-Dioxo-1,3-dihydro-isoindol-2-yl)-3-phenyl-propionylamino]-3-methyl-butyric acid methyl ester;
Adamantane-1-carboxylic acid (2,6-dihydroxy-pyrimidin-4-yl)-amide;
Adamantane-1-carboxylic acid methyl-phenyl-amide;
3-Chloro-benzo[b]thiophene-2-carboxylic acid dibenzylamide;
N-Adamantan-1-yl-2-(3-cyano-6-methyl-4-trifluoromethyl-pyridin-2-ylsulfanyl)-acetamide; 2-(3-Oxo-3-phenyl-propenyl)-soindole-1,3-dione;
N-[5-(5-Chloro-benzooxazol-2-yl)-2-methyl-phenyl]-2-methoxy-benzamide;
N-[2-(2-Bromo-phenyl)-benzooxazol-5-yl]-2-methoxy-benzamide;
2-(4-Chcloro-phenoxy)-N-(4-chloro-3-trifluoromethyl-phenyl)-acetamide;
2,2-Dimethyl-N-(5-propyl-[1,3,4]thiadiazol-2-yl)-propionamide;
2-[2-(2,6-Dimethyl-morpholin-4-yl)-1-methyl-2-oxo-ethyl]-isoindole-1,3-dione;
2-(2-Cyano-phenylsulfanyl)-N-(2-trifluoromethyl-phenyl)-benzamide;
Azepan-1-yl-(3,6-dichloro-benzo[b]thiophen-2-yl)-methanone;
Benzo[1,3]dioxol-5-yl-(4-benzyl-piperidin-1-yl)-methanone;
Azepan-1-yl-(3-chloro-6-methyl-benzo[b]thiophen-2-yl)-methanone;
N-(5-Hexyl-[1,3,4]thiadiazol-2-yl)-isobutyramide;
(3-Chloro-phenyl)-(10,11-dihydro-dibenzo[b,f]azepin-5-yl)-methanone;
(2-Chloro-phenyl)-(10,11-dihydro-dibenzo[b,f]azepin-5-yl)-methanone;
2-Amino-5-(azepane-1-carbonyl)-4-methyl-thiophene-3-carboxylic acid ethyl ester;
Adamantan-1-yl-(4-cyclopropyl-1,4,6,7-tetrahydro-imidazo[4,5-c]pyridin-5-yl)-methanone;
Adamantan-1-yl-(4-trifluoromethyl-1,4,6,7-tetrahydro-imidazo[4,5-c]pyridin-5-yl)-methanone;
Adamantan-1-yl-[4-(1H-benzoimidazol-2-ylsulfanyl)-piperidin-1-yl]-methanone;
Adamantan-1-yl-(1,4,6,7-tetrahydro-imidazo[4,5-c]pyridin-5-yl)-methanone;
[4-(1H-Imidazol-4-yl)-piperldin-1-yl]-(4-pentyl-phenyl)-methanone;
3-Cyclohexyl-1-[4-(1H-imidazol-4-yl)-piperidin-1-yl]-propan-1-one;
1-(4-Propyl-piperazin-1-yl)-3-(4-trifluoromethyl-phenyl)-propan-1-one;
N-(2-Hydroxy-benzyl)-3-thiophen-3-yl-N-(2-thiophen-2-yl-ethyl)-acrylamide;
N-(1,3-Dimethyl-pentyl)-2-(3-fluoro-phenyl)-N-(4-hydroxy-benzyl)-acetamide;
N-Cyclobutyl-2-(3-fluoro-phenyl)-N-(4-hydroxy-benzyl)-acetamide;
N-Cyclobutyl-N-(4-hydroxy-benzyl)-4-trifluoromethyl-benzamide;
N-(3-Hydroxy-benzyl)-2-methyl-3-nitro-N-(4-sulfamoyl-benzyl)-benzamide;
N-(4-Bromo-benzyl)-N-(4-hydroxy-benzyl)-2-naphthalen-1-yl-acetamide;
6-(2-Bromo-phenylsulfanyl)-hexanoic acid (3-amino-2,2-dimethyl-propyl)-amide;
N-(3-Amino-2,2-dimethyl-propyl)-4-[2-(2-isopropyl-phenylsulfanyl)-ethyl]-benzamide;
N-(3-Amino-2,2-dimethyl-propyl)-4-[4-(4-chloro-phenyl)-pyrimidin-2-ylsulfanylmethyl]-3-nitro-benzamide;

4-(4-Bromo-phenyl)-N-(2-hydroxy-benzyl)-4-oxo-N-thiophen-2-ylmethyl-butyramide;
N-[2-(2,4-Dichloro-phenyl)-ethyl]-N-(4-hydroxy-benzyl)-2-thlophen-3-yl-acetamide;
N-(2-Chloro-benzyl)-N-(4-hydroxy-benzyl)-2-thiophen-2-yl-acetamide;
Heptanoic acid benzyl-(4-hydroxy-benzyl)-amide;
N-(4-Fluoro-benzyl)-N-(4-hydroxy-benzyl)-2-thiophen-3-yl-acetamide;
4-Methyl-pentanoic acid (4-fluoro-benzyl)-(4-hydroxy-benzyl)-amide;
N-Allyl-2-(4-chloro-phenyl)-N-(4-hydroxy-benzyl)-acetamide;
N-Allyl-2-benzo[b]thlophen-3-yl-N-(4-hydroxy-benzyl)-acetamide;
Heptanoic acid (3-ethoxy-propyl)-(4-hydroxy-benzyl)-amide;
Dec-3-enoic acid (4-hydroxy-benzyl)-(4-trifluoromethyl-benzyl)-amide;
6-Oxo-6-phenyl-hexanoic acid (4-hydroxy-benzyl)-(4-trifluoromethyl-benzyl)-amide;
2-(3,4-Difluoro-phenyl)-N-(4-hydroxy-benzyl)-N-thiophen-2-ylmethyl-acetamide;
2-Methyl-pent-4-enoic acid (3-hydroxy-benzyl)-[2-(2-methoxy-phenyl)-ethyl]-amide;
Heptanoic acid (3-hydroxy-benzyl)-(4-isopropyl-benzyl)-amide;
5-(2,6-Dichloro-phenylsulfanyl)-pentanoic acid (naphthalen-1-ylmethyl)-amide;
N-(6,6-Dimethyl-bicyclo[3.1.1]hept-2-ylmethyl)-4-[2-(5-methyl-1H-benzoimidazol-2-ylsulfanyl)-ethyl]-benzamide;
N-(6,6-Dimethyl-bicyclo[3.1.1]hept-2-ylmethyl)-4-[2-(4-phenoxy-pyrimidin-2-ylsulfanyl)-ethyl]-benzamide;
N-(6,6-Dimethyl-bicyclo[3.1.1]hept-2-ylmethyl)-4-[2-(4-fluoro-phenylsulfanyl)-ethyl]-benzamide;
4-(2,6-Dichloro-phenylsulfanyl)-N-(6,6-dimethyl-bicyclo[3.1.1]hept-2-ylmethyl)-butyramide;
5-(3-Methylsulfanyl-[1,2,4]thiadiazol-5-ylsulfanyl)-pentanoic acid (6,6-dimethyl-bicyclo[3.1.1]hept-2-ylmethyl)-amide;
5-(2,6-Dichloro-phenylsulfanyl)-pentanoic acid [2-(3-trifluoromethyl-phenyl)-ethyl]-amide;
4-[2-(2,6-Dichloro-phenylsulfanyl)-ethyl]-N-[2-(2-fluoro-phenyl)-ethyl]-benzamide;
2-Cyclohexylamino-thiazole-4-carboxylic acid (1,2,3,4-tetrahydro-naphthalen-1-yl)-amide;
2-Cyclohexylamino-thiazole-4-carboxylic acid (3-chloro-4-hydroxy-phenyl)-amide;
2-Cyclohexylamino-thiazole-4-carboxylic acid (1,2-dimethyl-propyl)-amide;
2-Cyclohexylamino-thiazole-4-carboxylic acid (1-ethyl-propyl)-amide;
2-Cyclohexylamino-thiazole-4-carboxylic acid [3-(1-hydroxy-ethyl)-phenyl]-amide;
2-Cyclohexylamino-thiazole-4-carboxylic acid (1-ethynyl-cyclohexyl)-amide;
2-Cyclohexylamino-thiazole-4-carboxylic acid (2-methoxy-dibenzofuran-3-yl)-amide;
2-Cyclohexylamino-thiazole-4-carboxylic acid [2-(4-hydroxy-phenyl)-ethyl]-amide;
2-Cyclohexylamino-thiazole-4-carboxylic acid (4-hydroxy-cyclohexyl)-amide;
2-(2,6-Difluoro-benzylamino)-N-[2-(3-trifluoromethyl-phenyl)-ethyl]-acetamide;
4-{4-[2-(4-Dimethylamino-phenyl)-acetyl]-piperazin-1-yl}-3-(2-phenyl-propylamino)-benzamide;
2-(2-Ethyl-phenylsulfanyl)-3-[methyl-(2-pyridin-4-yl-ethyl)-amino]-N-prop-2-ynyl-propionamide;

4-Methyl-cyclohexanecarboxylic acid {[2-(2-chloro-6-fluoro-benzylsulfanyl)-ethylcarbamoyl]-methyl}-prop-2-ynyl-amide;
2-Benzylsulfanyl-N-{[2-(2-chloro-6-fluoro-benzylsulfanyl)-ethylcarbamoyl]-methyl}-N-(2-methoxy-ethyl)-acetamide;
4-[2-(5-Cyclopropylmethylsulfanyl-[1,3,4]thiadiazol-2-ylsulfanyl)-ethyl]-N-(6,6-dimethyl-bicyclo[3.1.1]hept-2-ylmethyl)-benzamide;
N-(6,6-Dimethyl-bicyclo[3.1.1]hept-2-ylmethyl)-2-p-tolyloxy-acetamide;
Bicyclo[2.2.1]hept-5-ene-2-carboxylic acid [4-(2,5-difluoro-phenoxy)-butyl]-amide;
4-Trifluoromethyl-cyclohexanecarboxylic acid [6-(2,6-difluoro-phenoxy)-hexyl]-amide;
N-Cyclopropyl-3-methoxy-N-(2-piperidin-4-yl-ethyl)-5-(pyridine-2-carbonyl)-benzamide;
3-Methoxy-N-(2-methoxy-ethyl)-N-(2-piperidin-4-yl-ethyl)-5-(pyridine-2-carbonyl)-benzamide;
3-Methoxy-N-(2-piperidin-4-yl-ethyl)-5-(pyridine-2-carbonyl)-N-(tetrahydro-furan-2-ylmethyl)-benzamide;
3-Methoxy-N-(2-oxo-azepan-3-yl)-N-(2-piperidin-4-yl-ethyl)-5-(pyridine-2-carbonyl)-benzamide;
3-Methoxy-N-[3-(2-oxo-pyrrolidin-1-yl)-propyl]-N-(2-piperidin-4-yl-ethyl)-5-(pyridine-2-carbonyl)-benzamide;
3-Methoxy-N-methyl-N-(2-piperldin-4-yl-ethyl)-5-(pyridine-2-carbonyl)-benzamide; [2-({Cyclopropyl-[3-methoxy-5-(pyridine-2-carbonyl)-benzoyl]-amino}-methyl)-phenoxy]-acetic acid;
(2-{[[3-Methoxy-5-(pyridine-2-carbonyl)-benzoyl]-(3-methyl-butyl)-amino]-methyl}-phenoxy)-acetic acid;
[2-({Cyclopentyl-[3-methoxy-5-(pyridine-2-carbonyl)-benzoyl]-amino}-methyl)-phenoxy]-acetic acid;
[2-({(2-Methoxy-ethyl)-[3-methoxy-5-(pyridine-2-carbonyl)-benzoyl]-amino}-methyl)-phenoxy]-acetic acid;
[2-({Carbamoylmethyl-[3-methoxy-5-(pyridine-2-carbonyl)-benzoyl]-amino}-methyl)-phenoxy]-acetic acid;
[2-({[3-Methoxy-5-(pyridine-2-carbonyl)-benzoyl]-pyridin-4-yl-amino}-methyl)-phenoxy]-acetic acid;
[2-({Cyclopropylmethyl-[3-methoxy-5-(pyridine-2-carbonyl)-benzoyl]-amino}-methyl)-phenoxy]-acetic acid;
[2-({[3-Methoxy-5-(pyridine-2-carbonyl)-benzoyl]-methyl-amino}-methyl)-phenoxy]-acetic acid;
[4-(4-Hydroxy-benzyl)-piperazin-1-yl]-[3-methoxy-5-(pyridine-2-carbonyl)-phenyl]-methanone;
{Carbamoylmethyl-[3-methoxy-5-(pyridine-2-carbonyl)-benzoyl]-amino}-acetic acid;
{(3-imidazol-1-yl-propyl)-[3-methoxy-5-(pyridine-2-carbonyl)-benzoyl]-amino}-acetic acid;
{[3-Methoxy-5-(pyridine-2-carbonyl)-benzoyl]-pyridin-4-yl-amino}-acetic acid;
[[3-Methoxy-5-(pyridine-2-carbonyl)-benzoyl]-(2-oxo-azepan-3-yl)-amino]-acetic acid;
3-Methoxy-N-(2-methoxy-ethyl)-N-piperidin-3-ylmethyl-5-(pyridine-2-carbonyl)-benzamide;
4-[3-Methoxy-5-(pyridine-2-carbonyl)-benzoylamino]-piperidine-1-carboxylic acid ethyl ester;
3-Methoxy-N-[3-(2-oxo-pyrrolidin-1-yl)-propyl]-5-(pyridine-2-carbonyl)-benzamide;
3-({Carbamoylmethyl-[3-methoxy-5-(pyridine-2-carbonyl)-benzoyl]-amino}-methyl)-benzoic acid;
3-({(3-Imidazol-1-yl-propyl)-[3-methoxy-5-(pyridine-2-carbonyl)-benzoyl]-amino}-methyl)-benzoic acid;
4-Amino-N-(3-hydroxy-benzyl)-N-indan-2-yl-2-propionylamino-butyramide;
5-Amino-2-propionylamino-pentanoic acid (3-hydroxy-benzyl)-indan-2-yl-amide;
N-Ethyl-2-hexylamino-N-(4-hydroxy-benzyl)-acetamide;
2-Hexylamino-N-(4-hydroxy-benzyl)-N-methyl-acetamide;
1-[1-(6-Phenyl-hexanoyl)-piperidin-4-yl]-1,3-dihydro-benzoimidazol-2-one;
1-[1-(3-Cyclohexyl-propionyl)-piperidin-4-yl]-1,3-dihydro-benzoimidazol-2-one;
N-(2-Hydroxy-benzyl)-N-isobutyl-benzamide;
N-(2-Hydroxy-benzyl)-2-(4-hydroxy-phenyl)-N-isobutyl-acetamide;
N-(2-Hydroxy-benzyl)-N-(3-methyl-butyl)-benzamide;
N-(4-Hydroxy-benzyl)-N-isobutyl-benzamide;
4-Hydroxy-N-(4-hydroxy-benzyl)-N-isobutyl-benzamide;
N-(4-Hydroxy-benzyl)-2-(4-hydroxy-phenyl)-N-isobutyl-acetamide;
N-(4-Hydroxy-benzyl)-N-(3-methyl-butyl)-benzamide;
N-(2-Ethoxcy-ethyl)-4-hydroxy-N-(4-hydroxy-benzyl)-benzamide;
N-(4-Hydroxy-benzyl)-N-(3-isopropoxy-propyl)-benzamide;
N-(3-Hydroxy-benzyl)-N-(4-methyl-pentyl)-benzamide;
N-(3-Hydroxy-benzyl)-2-(4-hydroxy-phenyl)-N-(4-methyl-pentyl)-acetamide; N-(3-Hydroxy-benzyl)-N-(3-isopropoxy-propyl)-benzamide;
N-(2-Hydroxy-benzyl)-N-(3-methyl-butyl)-4-propyl-benzamide;
N-(4-Hydroxy-benzyl)-N-(6-hydroxy-hexyl)-4-propyl-benzamide;
N-(4-Hydroxy-benzyl)-N-(3-methyl-butyl)-4-propyl-benzamide;
N-[2-(4-Fluoro-benzylamino)-thiazol-4-ylmethyl]-N-phenethyl-butyramide; or
   a salt thereof with a pharmaceutically acceptable acid or base, or any optical isomer or mixture of optical isomers, including a racemic mixture, or any tautomeric forms.

In another embodiment, the present invention is concerned the substituted amides or prodrugs thereof of the general formula (II) 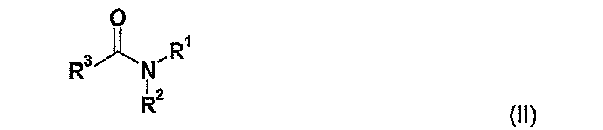 wherein

R¹ is C₃-C₁₀cycloalkyl or C₃-C₁₀hetcycloalkyl, wherein the cycloalkyl and hetcycloalkyl groups independently are optionally substituted with one or more of R⁴;

R² is hydrogen, C₁-C₈alkyl, arylC₁-C₆alkyl, wherein the alkyl and aryl groups independently are optionally substituted with one or more of R⁵; or

R¹ and R² together with the nitrogen to which they are attached, are forming a saturated or partially saturated cyclic, bicyclic or tricyclic ring system containing from 4 to 10 carbon atoms and from 0 to 2 additional heteroatoms selected from nitrogen, oxygen or sulphur, the ring system optionally being substituted with at least one of C₁-C₈alkyl, aryl, hetaryl, arylC₁-C₆alkyl, hetarylC₁-C₆alkyl, hydroxy, oxo, cyano, C₁-C₆alkyloxy, arylC₁-C₆alkyloxy, hetarylC₁-C₆alkyloxy, C₁-C₆alkyloxyC₁-C₆alkyl, C₁-C₆alkylcarbonyl, arylcarbonyl, hetarylcarbonyl, arylC₁-C₆aJkylcarbonyl, hetarylC₁-C₆alkylcarbonyl, C₁-C₆alkylcarboxy, arylcarboxy or arylC₁-C₆alkylcarboxy wherein the alkyl and aryl groups independently are optionally substituted with one ore more of R¹⁴;

R³ is C₃-C₁₀cycloalkyl, C₃-C₁₀hetcycloalkyl, aryl or hetaryl, wherein the alkyl, cycloalkyl, hetcycloalkyl, aryl and hetaryl groups independently are optionally substituted with one or more of R⁷;

R⁴ and R⁵ independently are hydrogen, hydroxy, oxo, cyano, halo, methylendioxo, NR⁸R⁹, C₁-C₈alkyl, C₁-C₆alkyloxy, trihalomethyl, trihalomethyloxy, C₃-C₁₀cycloalkyl, C₃-C₁₀hetcycloalkyl, C₃-C₁₀cycloalkenyl, aryl, hetaryl, heterylSOₙ, arylC₁-C₆alkyloxy, hetarylC₁-C₆alkyloxy, C₁-C₆alkyl-R⁶-C₁-C₆alkyl, arylC₁-C₆alkyl-R⁶-C₁-C₆alkyl, C₁-C₆alkylcarbonyl, arylcarbonyl, arylC₁-C₆alkylcarbonyl, hetarylcarbonyl, hetarylC₁-C₆alkylcarbonyl, C₁-C₆alkylSOₙ, C₁-C₆-alkylcarboxy, arylcarboxy, hetarylcarboxy, arylC₁-C₆alkylcarboxy or hetarylC₁-C₆alkylcarboxy wherein the alkyl, cycloalkyl, hetcycloalkyl, aryl and hetaryl groups independently are optionally substituted with one ore more of R¹⁵;

R⁶ is oxygen, sulphur, SOₙ or NR¹⁶;

R⁷ is hydrogen, halo, hydroxy, cyano, nitro, COOR¹⁷, C₁-C₈alkyl, C₃-C₁₀cycloalkyl, C₃-C₁₀hetcycloalkyl, methylendioxo, trihalomethyl, trihalomethyloxy, aryl, arylC₁-C₆alkyl, C₁-C₆alkyloxy, C₁-C₆alkyloxyC₁-C₆alkyl, aryloxy, arylC₁-C₆alkyloxy, aryloxyC₁-C₆alkyl, arylC₁-C₆alkyloxyC₁-C₆alkyl, hetaryl, hetarylC₁-C₆alkyl, hetaryloxy, hetarylC₁-C₆alkyloxy, hetaryloxyC₁-C₆alkyl, hetarylC₁-C₆alkyl-oxyC₁-C₆alkyl, NR⁸R⁹, SOₘNR⁸R⁹, NR⁴R⁵carbonylC₁-C₆alkyl, arylthio, hetarylthio, R¹⁸carbonylNR⁸, arylSOₙ, hetarylSOₙ, R¹⁹SOₘNR⁸, arylthioC₁-C₆alkyl, hetarylthioC₁-C₆alkyl or arylC₁-C₆alkylR⁶C₁-C₆alkyl; wherein the aryl and hetaryl groups independently are optionally substituted with one or more R¹⁰;

R⁸ and R⁹ independently are hydrogen, C₁-C₈alkyl, aryl, hetaryl, C₁-C₆alkylSOₘ, arylSOₘ, arylC₁C₆alkylSOm, arylC₁-C₆alkyl or hetarylC₁-C₆alkyl wherein the alkyl, aryl and hetaryl groups independently are optionally substituted with one or more of R¹¹; or

R⁸ and R⁹ together with the nitrogen to which they are attached, are forming a saturated or partially saturated cyclic, bicyclic or tricyclic ring system containing from 4 to 10 carbon atoms and from 0 to 2 additional heteroatoms selected from nitrogen, oxygen or sulfur, the ring system optionally being substituted with at least one halo, cyano, C₁-C₈alkyl, aryl, hetaryl, arylC₁-C₆alkyl, hetarylC₁-C₆alkyl, hydroxy, oxo, C₁-C₆alkyloxy, arylC₁-C₆alkyloxy, hetarylC₁-C₆alkyloxy, C₁-C₆alkyloxyC₁-C₆alkyl, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy, hetarylcarboxy, arylC₁-C₆alkylcarboxy or hetarylC₁-C₆alkylcarboxy;

R¹⁰ and R¹¹ independently are hydrogen, hydroxy, oxo, halo, cyano, nitro, C₁-C₈alkyl, C₁-C₆alkyl-oxy, NR¹²R¹³, methylendioxo, trihalomethyl or trihalomethyloxy;

R¹² and R¹³ independently are hydrogen, C₁-C₈alkyl or arylC₁-C₆alkyl;

R¹⁴ is hydrogen, halo, hydroxy, oxo, nitro, cyano, C₁-C₈alkyl, C₁-C₆alkyloxy or aryloxy;

R¹⁵ is hydrogen, halo, hydroxy, oxo, nitro, cyano, CONR⁸R⁹ or COOR¹⁷;

R¹⁶ is hydrogen, C₁-C₈alkyl, C₃-C₁₀cycloalkyl, C₃-C₁₀hetcycloalkyl, aryl, arylC₁-C₆alkyl, hetaryl, hetarylC₁-C₆alkyl, alkylcarbonyl, arylcarbonyl, arylC₁-C₆alkylcarbonyl, aryloxyC₁-C₆alkyl, hetaryloxyC₁-C₆alkyl, arylthioC₁-C₆alkyl or hetarylthioC₁-C₆alkyl; wherein the alkyl, cycloalkyl, hetcycloalkyl, aryl and hetaryl groups independently are optionally substituted with one or more of R¹⁰;

R¹⁷ is hydrogen, C₁-C₈alkyl, aryl or arylC₁-C₆alkyl;

R¹⁸ is C₁-C₆alkyl, C₂-C₆alkenyl, aryl, arylC₁-C₆alkyl, hetaryl, hetarylC₁-C₆alkyl, C₃-C₁₀cycloalkyl, C₃-C₁₀hetcycloalkyl, C₁-C₆alkyloxy, aryloxy, arylC₁-C₆alkyloxy, arylC₁-C₆alkyloxyC₁-C₆alkyl, hetarylC₁-C₆alkyloxyC₁-C₆alkyl, hetaryloxy, hetarylC₁-C₆alkyloxy or R⁸R⁹NC₁-C₆alkyl wherein the alkyl, alkenyl, cycloalkyl, hetcycloalkyl, aryl and hetaryl groups are optionally substituted with R¹⁵;

R¹⁹ is C₁-C₆alkyl, C₃-C₁₀cycloalkyl, C₃-C₁₀hetcycloalkyl, aryl, arylC₁-C₆alkyl, hetaryl, hetarylC₁-C₆alkyl;
m is 1 or 2;
n is 0, 1 or 2; or
a salt thereof with a pharmaceutically acceptable acid or base, or any optical isomer or mixture of optical isomers, including a racemic mixture, or any tautomeric forms.

In another embodiment of the present invention, in formula (II) R¹ is C₃-C₁₀cycloalkyl or C₃-C₁₀hetcycloalkyl, wherein the cycloalkyl and hetcycloalkyl groups independently are optionally substituted with one or more of R⁴ as defined above.

In another embodiment of the present invention, in formula (II) R² is hydrogen or C₁-C₈alkyl, wherein the alkyl group is optionally substituted with one or more of R⁵ as defined above.

In another embodiment of the present invention, in formula (II) R¹ and R² together with the nitrogen to which they are attached, are forming a saturated or partially saturated cyclic, bicyclic or tricyclic ring system containing from 4 to 10 carbon atoms and from 0 to 2 additional heteroatoms selected from nitrogen, oxygen or sulphur, the ring system optionally being substituted with at least one of C₁-C₈alkyl, aryl, hetaryl, arylC₁-C₆alkyl, hetarylC₁-C₆-alkyl, hydroxy, oxo, cyano, C₁-C₆alkyloxy, arylC₁-C₆alkyloxy, hetarylC₁-C₆alkyloxy, C₁-C₆alkyloxyC₁-C₆alkyl, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy or arylC₁-C₆alkylcarboxy wherein the alkyl and aryl groups independently are optionally substituted with one ore more of R¹⁴;as defined above.

In another embodiment of the present invention, in formula (II) R¹ and R² together with the nitrogen to which they are attached are 6-aza-bicyclo[3.2.1]octane.

In another embodiment of the present invention, in formula (II) R³ is aryl or hetaryl, wherein the aryl and hetaryl groups independently are optionally substituted with one or more of R⁷ as defined above.

In another embodiment of the present invention, in formula (II) R³ is C₃-C₁₀cydoalkyl or C₃-C₁₀hetcycloalkyl, optionally substituted with one or more of R⁷ as defined above.

In another embodiment of the present invention, in formula (II) R⁴ and R⁵ independently are hydrogen, hydroxy, oxo, halo, C₁-C₈alkyl, wherein the alkyl group is optionally substituted with one ore more of R¹⁵ as defined above.

In another embodiment of the present invention, in formula (II) R⁷ is hydrogen, halo, hydroxy, cyano, G₁-C₈alkyl, C₃-C₁₀cycloalkyl, C₃-C₁₀het-cycloalkyl, trihalomethyl, aryl, arylC₁-C₆alkyl, C₁-C₆alkyloxy, C₁-C₆alkyloxyC₁-C₆alkyl, aryloxy, arylC₁-C₆alkyloxy, aryloxyC₄-C₆alkyl, arylC₁-C₆alkyloxyC₁-C₆alkyl, hetaryl, hetarylC₁-C₆alkyl, hetaryloxy, hetarylC₁-C₆alkyloxy, hetaryloxyC₁-C₆alkyl, hetarylC₁-C₆alkyl-oxyC₁-C₆alkyl, NR⁸R⁹, R¹⁸carbonylNR⁸, or R¹⁹SOₘNR⁸, wherein the aryl and hetaryl groups independently are optionally substituted with one or more R¹⁰ as defined above.

In another embodiment of the present invention, in formula (II) R⁷ is halo, cyano, C₁-C₈alkyl, C₃-C₁₀hetcycloalkyl, trihalomethyl, aryl, C₁-C₆alkyloxy, aryloxy, arylC₁-C₆alkyloxy, aryloxyC₁-C₆alkyl, arylC₁-C₆alkyloxyC₁-C₆alkyl, hetaryl, hetaryloxy, hetarylC₁-C₆alkyloxy, hetaryloxyC₁-C₆alkyl, hetarylC₁-C₆alkyloxyC₁-C₆alkyl, NR⁸R⁹, R¹⁸carbonylNR⁸, or R¹⁹SOₘNR⁸, wherein the aryl and hetaryl groups are independently optionally substituted with one or more R¹⁰ as defined above.

In another embodiment of the present invention, in formula (II) R⁷ is R¹⁸carbonylNR⁸ or R¹⁹SOₘNR⁸; wherein m, R⁸, R¹⁸ and R¹⁹ are defined as above.

In another embodiment of the present invention, in formula (II) R⁸ is C₁-C₆alkyl.

In another embodiment of the present invention, in formula (II) R⁸ and R⁹ together with the nitrogen to which they are attached, are forming a saturated or partially saturated cyclic, bicyclic or tricyclic ring system containing from 4 to 10 carbon atoms and from 0 to 2 additional heteroatoms selected from nitrogen, oxygen or sulfur, the ring system optionally being substituted with at least one halo, cyano, C₁-C₈alkyl, aryl, hetaryl, arylC₁-C₆alkyl, hetarylC₁-C₆alkyl, hydroxy, oxo, C₁-C₆alkyloxy, arylC₁-C₆alkyloxy, hetarylC₁-C₆alkyloxy, C₁-C₆alkyloxyC₁-C₆alkyl, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy, hetarylcarboxy, arylC₁-C₆alkylcarboxy or hetarylC₁-C₆alkylcarboxy.

In another embodiment of the present invention, in formula (II) R¹⁵ is CONR⁸R⁹.

In another embodiment of the present invention, in formula (II) R¹⁸ is C₁-C₆alkyl, optionally substituted with R¹⁵ as defined above.

In another embodiment of the present invention, in formula (II) R¹⁸ is aryl or hetaryl.

In another embodiment of the present invention, in formula (II) R¹⁸ is arylC₁-C₆alkyloxyC₁-C₆alkyl or hetarylC₁-C₆alkyloxyC₁-C₆alkyl.

In another embodiment of the present invention, in formula (II) R¹⁸ is R⁸R⁹NC₁-C₆-alkyl; wherein R⁸ and R⁹ are defined as above.

In another embodiment of the present invention, in formula (II) R¹⁹ is aryl or hetaryl

In another embodiment of the present invention, in formula (II) the aryl group is phenyl or pyridyl.

In another embodiment of the present invention, in formula (II) the hetaryl group is thienyl, imidazolyl, oxazolyl, thiazolyl, or indolyl.

In another embodiment of the present invention, the compounds or prodrugs thereof of the general formula (II) are selected from the group consisting of the compounds of examples 4 through 8 as described under EXAMPLES, COMPOUNDS OF GENERAL FORMULAS (I) AND (II).

In another embodiment, the present invention is concerned the substituted amides or prodrugs thereof of the general formula (III) 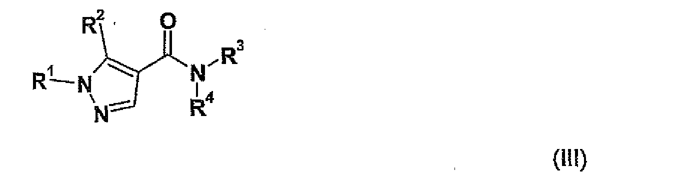 wherein
R¹ is aryl, arylC₁-C₆alkyl, hetaryl or hetarylC₁-C₆alkyl optionally substituted with one or more
of R⁶ independently;

R² is halo, C₁-C₆alkyl, C₂-C₆alkenyl, C₂-C₆alkynyl, C₃-C₁₀cycloalkyl, C₃-C₁₀cycloalkylC₁-C₆-alkyl, trihalomethyl, aryl, arylC₁-C₆alkyl, C₁-C₆alkyloxyC₁-C₆alkyl, arylC₁-C₆alkyloxyC₁-Caalkyl, C₁-C₆alkylNR⁵C₁-C₆alkyl, arylC₁-C₆alkylNR⁵C₁-C₆alkyl, hetaryl, or hetarylC₁-C₆alkyl wherein the alkyl, alkenyl, alkynyl, cycloalkyl and aryl groups independently are optionally substituted with one or more R⁷;

R³ is C₁-C₆alkyl optionally substituted with one or more of R⁸;

R⁴ is C₆-C₁₀cycloalkyl; C₆-C₁₀hetcycloalkyl, C₆-C₁₀cycloalkylC₁-C₆alkyl or C₆-C₁₀hetcycloalkylC₁-C₆alkyl wherein the alkyl, cycloalkyl and hetcycloalkyl groups independently are optionally substituted with one or more of R⁸; or

R³ and R⁴ together with the nitrogen to which they are attached, are forming a saturated or partially saturated bicyclic/bridge ring system containing from 7 to 12 carbon atoms and from 0 to 2 additional heteroatoms selected from nitrogen, oxygen or sulphur, the ring system optionally being substituted with at least one of C₁-C₆alkyl, aryl, hetaryl, arylC₁-C₆alkyl, hetaryl-C₁-C₆alkyl, hydroxy, oxo, C₁-C₆alkyloxy, arylC₁-C₆alkyloxy or hetarylC₁-C₆alkyloxy, wherein the alkyl and aryl groups independently are optionally substituted with one ore more of R⁹;

R⁵ is C₁-C₆alkyl, C₂-C₆alkenyl, C₂-C₆alkynyl, C₃-C₁₀cycloalkyl, C₃-C₁₀hetcycloalkyl, C₃-C₁₀-cycloalkylC₁-C₆alkyl, C₃-C₁₀hetcycloalkylC₁-C₆alkyl, aryl, hetaryl, arylC₁-C₆alkyl or hetarylC₁-C₆alkyl wherein the alkyl, alkenyl, alkynyl, aryl, hetaryl, cycloalkyl and hetcycloalkyl groups independently are optionally substituted with one or more of R⁹;

R⁶ and R⁷ independently are hydrogen; hydroxy, oxo, halo, nitro, cyano, C₁-C₆alkyl, C₁-C₆-alkyloxy, trihalomethyl, trihalomethoxy, NR¹⁰R¹¹, arylC₁-C₆alkyloxy, hetarylC₁-C₆alkyloxy, C₁-C₆alkylcarbonyl, arylcarbonyl, hetarylcarbonyl, arylC₁-C₆alkylcarbonyl, C₁-C₆alkyloxycarbonyl, aryloxycarbonyl, arylC₁-C₆alkyloxycarbonyl, C₁-C₆alkylcarboxy, arylcarboxy or arylC₁-C₆alkylcarboxy;

R⁸ and R⁹ independently are hydrogen, C₁-C₆alkyl, aryl, hetaryl, arylC₁-C₆alkyl, hetarylC₁-C₆-alkyl, hydroxy, oxo, cyano, NR¹⁰R¹¹, C₁-C₆alkyloxy, aryloxy, arylC₁-C₆alkyloxy; hetaryloxy, hetarylC₁-C₆alkyloxy, C₁-C₆alkyloxyC₁-C₆alkyl, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy or arylC₁-C₆alkylcarboxy;

R¹⁰ and R¹¹ independently are hydrogen, C₁-C₈alkyl, aryl, hetaryl, arylC₁-C₆alkyl, C₃-C₁₀-cycloalkyl, C₃-C₁₀hetcycloalkyl, C₃-C₁₀cycloalkylC₁-C₆alkyl, C₁-C₆alkylcarbonyl, C₁-C₆alkylcarboxyC₁-C₆alkyl; or

R¹⁰ and R¹¹ together with the nitrogen to which they are attached, are forming a saturated or partially saturated cyclic, bicyclic or tricyclic ring system containing from 4 to 10 carbon atoms and from 0 to 2 additional heteroatoms selected from nitrogen, oxygen or sulphur, the ring system optionally being substituted with at least one of C₁-C₈alkyl, aryl, hetaryl, arylC₁-C₆alkyl, hetarylC₁-C₆alkyl, hydroxy, oxo, cyano, C₁-C₆alkyloxy, arylC₁-C₆alkyloxy, hetarylC₁-C₆alkyloxy, C₁-C₆alkyloxyC₁-C₆alkyl, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy or arylC₁₋₆-alkylcarboxy; or
a salt thereof with a pharmaceutically acceptable acid or base, or any optical isomer or mixture of optical isomers, including a racemic mixture, or any tautomeric forms.

In another embodiment, the present invention is concerned with compounds or prodrugs thereof of the general formula (III) wherein:
R¹ is aryl or hetaryl optionally substituted with one or more R⁶ independently;

R² is halo, C₁-C₆alkyl, C₂-C₆alkenyl, C₂-C₆alkynyl, C₃-C₁₀cycloalkyl, C₃-C₁₀cycloalkylC₁-C₆-alkyl, aryl, arylC₁-C₆alkyl, C₁-C₆alkyloxyC₁-C₆alkyl, arylC₁-C₆alkyloxyC₁-C₆alkyl, C₁-C₆-alkylNR⁵C₁-C₆alkyl, arylC₁-C₆alkylNR⁵C₁-C₆alkyl, hetaryl or hetarylC₁-C₆alkyl wherein the alkyl, alkenyl, alkynyl, cycloalkyl and aryl groups independently are optionally substituted with one or more R⁷;

R³ is C₁-C₆alkyl optionally substituted with one or more of R⁸;

R⁴ is C₆-C₁₀cycloalkyl, C₆-C₁₀hetcycloalkyl, C₆-C₁₀cycloalkylC₁-C₆alkyl or C₆-C₁₀hetcycloalkylC₁-C₆alkyl wherein the alkyl, cycloalkyl and hetcycloalkyl groups independently are optionally substituted with one or more of R⁸;

R⁵ is C₁-C₆alkyl, C₂-C₆alkenyl, C₂-C₆alkynyl, C₃-C₁₀cycloalkyl, C₃-C₁₀hetcycloalkyl, C₃-C₁₀-cycloalkylC₁-C₆alkyl, C₃-C₁₀hetcycloalkylC₁-C₆alkyl, aryl, hetaryl, arylC₁-C₆alkyl or hetarylC₁-C₆alkyl wherein the alkyl, alkenyl, alkynyl, aryl, hetaryl, cycloalkyl and hetcycloalkyl groups independently are optionally substituted with one or more of R⁹;

R⁶ and R⁷ independently are hydrogen, hydroxy, oxo, halo, nitro, cyano, C₁-C₆alkyl, C₁-C₆-alkyloxy, trihalomethyl, trihalomethoxy, NR¹⁰R¹¹, arylC₁-C₆alkyloxy, hetarylC₁-C₆alkyloxy, C₁-C₆alkylcarbonyl, arylcarbonyl, hetarylcarbonyl, arylC₁-C₆alkylcarbonyl, C₁-C₆alkyloxycarbonyl, aryloxycarbonyl, arylC₁-C₆alkyloxycarbonyl, C₁-C₆alkylcarboxy, arylcarboxy or arylC₁-C₆alkylcarboxy;

R⁸ and R⁹ independently are hydrogen, C₁-C₆alkyl, aryl, hetaryl, arylC₁-C₆alkyl, hetarylC₁-C₆-alkyl, hydroxy, oxo, cyano, NR¹⁰R¹¹, C₁-C₆alkyloxy, aryloxy, arylC₁-C₆alkyloxy, hetaryloxy, hetarylC₁-C₆alkyloxy, C₁-C₆alkyloxyC₁-C₆alkyl, C₁-C₆alkyl-carbonyl, arylcarbonyl, hetarylcarbonyl, arylC₁-C₆alkylcarbonyl, hetarylC₁-C₆alkyl-carbonyl, C₁-C₆alkylcarboxy, arylcarboxy or arylC₁-C₆alkylcarboxy;

R¹⁰ and R¹¹ independently are hydrogen, C₁-C₈alkyl, aryl, hetaryl, arylC₁-C₆alkyl, C₃-C₁₀-cycloalkyl, C₃-C₁₀hetcycloalkyl, C₃-C₁₀cycloalkylC₁-C₆alkyl, C₁-C₆alkylcarboxyC₁-C₆alkyl; or

R¹⁰ and R¹¹ together with the nitrogen to which they are attached, are forming a saturated or partially saturated cyclic, bicyclic or tricyclic ring system containing from 4 to 10 carbon atoms and from 0 to 2 additional heteroatoms selected from nitrogen, oxygen or sulphur, the ring system optionally being substituted with at least one of C₁-C₈alkyl, aryl, hetaryl, arylC₁-C₆alkyl, hetarylG₁-C₆alkyl, hydroxy, oxo, cyano, C₁-C₆alkyloxy, arylC₁-C₆alkyloxy, hetaryl C₁-C₆alkyloxy, C₁-C₆alkyloxyC₁-C₆alkyl, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy or arylC₁₋₆-alkylcarboxy; or
a salt thereof with a pharmaceutically acceptable acid or base, or any optical isomer or mixture of optical isomers, including a racemic mixture, a prodrug thereof, or any tautomeric forms.

In another embodiment of the present invention, in formula (III) R¹ is aryl, arylC₁-C₆alkyl or hetaryl optionally substituted with one or more of R⁶.

In another embodiment of the present invention, in formula (III) R¹ is aryl optionally substituted with one or more of R⁶.

In another embodiment of the present invention, in formula (III) R¹ is arylC₁-C₆alkyl optionally substituted with one or more of R⁶.

In another embodiment of the present invention, in formula (III) R¹ is hetaryl optionally substituted with one or more of R⁶.

In another embodiment of the present invention, in formula (III) R² is C₁-C₆alkyl, C₃-C₁₀cycloalkyl, C₃-C₁₀cycloalkylC₁-C₆alkyl, trihalomethyl, arylC₁-C₆alkyl, or hetarylC₁-C₆alkyl wherein the alkyl, cycloalkyl and aryl groups independently are optionally substituted with one or more R⁷.

In another embodiment of the present invention, in formula (III) R² is C₁-C₆alkyl optionally substituted with one or more R⁷.

In another embodiment of the present invention, in formula (III) R² is trihalomethyl.

In another embodiment of the present invention, in formula (III) R³ is C₁-C₆alkyl optionally substituted with one or more of R⁸.

In another embodiment of the present invention, in formula (III) R⁴ is C₆-C₁₀cycloalkyl, or C₆-C₁₀hetcycloalkyl, wherein the cycloalkyl and hetcycloalkyl, groups independently are optionally substituted with one or more of R⁸.

In another embodiment of the present invention, in formula (III) R⁴ is C₆-C₁₀cycloalkyl optionally substituted with one or more of R⁸.

In another embodiment of the present invention, in formula (III) R⁴ is C₆ C₆-C₁₀hetcycloalkyl optionally substituted with one or more of R⁸.

In another embodiment of the invention, in formula (III) R³ and R⁴ together with the nitrogen to which they are attached, are forming a saturated or partially saturated bicyclic/bridge ring system containing from 7 to 12 carbon atoms and from 0 to 2 additional heteroatoms selected from nitrogen, oxygen or sulphur, the ring system optionally being substituted with at least one of C₁-C₆alkyl, aryl, hetaryl, arylC₁-C₆alkyl, hetarylC₁-C₆alkyl, hydroxy, oxo, C₁-C₆alkyloxy, arylC₁-C₆alkyloxy or hetarylC₁-C₆alkyloxy, wherein the alkyl and aryl groups independently are optionally substituted with one ore more of R⁹.

In another embodiment of the present invention, in formula (III) the saturated or partially saturated bicyclic/bridge ring system is 6-aza-bicycio[3.2.1]octane.

In another embodiment of the present invention, in formula (III) R⁶ and R⁷ independently are hydrogen, hydroxy, oxo, halo, cyano, C₁-C₆alkyl, C₁-C₆alkyloxy, trihalomethyl, NR¹⁰R¹¹, arylC₁-C₆alkyloxy, hetarylC₁-C₁₀alkyloxy, C₁-C₆alkylcarbonyl, arylcarbonyl, hetarylcarbonyl, arylC₁-C₆alkylcarbonyl, C₁-C₆alkyloxycarbonyl, aryloxycarbonyl or arylC₁-C₆alkyloxycarbonyl.

In another embodiment of the present invention, in formula (III) R⁸ and R⁹ independently are hydrogen, C₁-C₆alkyl, hydroxy, oxo, C₁-C₆alkyloxy or arylC₁-C₆alkyloxy.

In another embodiment of the present invention, in formula (III) R¹⁰ and R¹¹ independently are hydrogen or C₁-C₈alkyl.

In another embodiment of the present invention the compound of the general formula (III) or a prodrug thereof is 1-(4-Chloro-phenyl)-5-propyl-1*H*-pyrazole-4-carboxylic acid cyclohexy-methyl-amide or a salt thereof with a pharmaceutically acceptable acid or base, or any optical isomer or mixture of optical isomers, including a racemic mixture, or any tautomeric forms.

In another embodiment of the present invention the compounds of the general formula (III) or a prodrug thereof is selected from the group consisting of:
1-(4-Chloro-phenyl)-5-trifluoromethyl-1H-pyrazole-4-carboxylic acid cyclohexyl-methylamide;
[1-(4-Methoxy-phenyl)-5-methyl-1*H*-pyrazol-4-yl]-(1,3,3-trimethyl-6-aza-bicyclo[3.2.1]oct-6-yl)-methanone; 1 [1-(4-Chloro-phenyl)-5-propy)-1*H*-pyrazol-4-yl]-(1,3,3-trimethyl-6-aza-bicyclo[3.2.1]oct-6-yl)-methanone;
[1-(3,5-Dichloro-phenyl)-5-propyl-1*H*-pyrazol-4-yl]-(1,3,3-trimethyl-6-aza-bicyclo[3.2.1]oct-6-yl)-methanone; or
   a salt thereof with a pharmaceutically acceptable acid or base, or any optical isomer or mixture of optical isomers, including a racemic mixture, or any tautomeric forms.

In yet another embodiment of the present invention the compounds of the general formula (III) or a prodrug thereof is selected from the group consisting of: 1-{Phenyl)-5-methyl-1*H*-pyrazole-4-carboxylic acid cyclohexyl-methyl-amide; 1-(4-Fluoro-phenyl)-5-methyl-1*H*-pyrazole-4-carboxylic acid cyclohexyl-methyl-amide; 1-(4-Methoxy-phenyl)-5-methyl-1*H*-pyrazole-4-carboxylic acid cyclohexyl-methyl-amlde; 1-(4-Chloro-phenyl)-5-methyl-1*H*-pyrazole-4-carboxylic acid cyclohexyl-methyl-amide; 1-(2-Methyl-phenyl)-5-methyl-1*H*-pyrazole-4-carboxylic acid cyclohexyl-methyl-amide; 1-(4-Amino-phenyl)-5-methyl-1*H*-pyrazole-4-carboxylic acid cyclohexyl-methyl-amide; 1-(2-Pyridyl)-5-methyl-1*H*-pyrazole-4-carboxylic acid cyclohexyl-methyl-amide; 1-(2-Pyridyl)-5-propyl-1*H*-pyrazole-4-carboxylic acid cyclohexyl-methyl-amide; or a salt thereof with a pharmaceutically acceptable acid or base, or any optical isomer or mixture of optical isomers, including a racemic mixture, or any tautomeric forms.

In another embodiment, the present invention is concerned with compounds or prodrugs thereof of the general formula (IV) 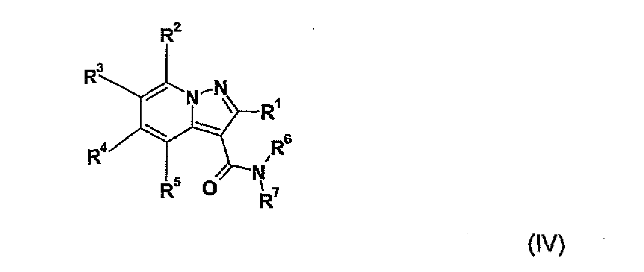 wherein
R¹ is hydrogen, trihalomethyl, C₁-C₆alkyl, C₁-C₆alkyloxy, C₁-C₆alkylthio, aryl, arylC₁-C₆alkyl, hetaryl or hetaralkyl, wherein the alkyl, aryl and hetaryl groups independently are optionally substituted with one or more of R⁸;

R², R³, R⁴ and R⁵ independently are hydrogen, halo, nitro, cyano, hydroxy, NR⁹R¹⁰, trihalomethyl, C₁-C₆alkyl, C₁-C₆alkyloxy, C₁-C₆alkylthio, aryl, arylC₁-C₆alkyl, hetaryl or hetaralkyl, wherein the alkyl, aryl and hetaryl groups independently are optionally substituted with one or more of R⁸; or

R² together with R³ are forming a saturated or partially saturated cyclic ring system containing from 3 to 6 carbon atoms and from 0 to 2 additional heteroatoms selected from nitrogen, oxygen or sulphur, the ring system optionally being substituted with at least one of C₁-C₆-alkyl, aryl, hetaryl, arylC₁-C₆alkyl, hetarylC₁-C₆alkyl, hydroxy, oxo, C₁-C₆alkyloxy, aryloxy, arylC₁-C₆alkyloxy or hetarylC₁-C₆alkyloxy; or

R³ together with R⁴ are forming a saturated or partially saturated cyclic ring system containing from 3 to 6 carbon atoms and from 0 to 2 additional heteroatoms selected from hitrogen, oxygen or sulphur, the ring system optionally being substituted with at least one of C₁-C₆-alkyl, aryl, hetaryl, arylC₁-C₆alkyl, hetarylC₁-G₆alkyl, hydroxy, oxo, C₁-C₆alkyloxy, aryloxy, arylC₁-C₆alkyloxy or hetarylC₁-C₆alkyloxy; or

R⁴ together with R⁵ are forming a saturated or partially saturated cyclic ring system containing from 3 to 6 carbon atoms and from 0 to 2 additional heteroatoms selected from nitrogen, oxygen or sulphur, the ring system optionally being substituted with at least one of C₁-C₆-alkyl, aryl, hetaryl, arylC₁-C₆alkyl, hetarylC₁-C₆alkyl, hydroxy, oxo, C₁-C₆alkyloxy, aryloxy, arylC₁-C₆alkyloxy or hetarylC₁-C₆alkyloxy;

R⁶ is aryl, hetaryl, arylC₁-C₆alkyl, C₃-C₁₀cycloalkyl, C₃-C₁₀hetcycloalkyl, C₃-C₁₀cycloalkylC₁-C₆alkyl, C₁-C₆alkylcarboxyC₁-C₆alkyl, wherein the alkyl, aryl and cycloalkyl groups independently are optionally substituted with one or more of R¹¹;

R⁷ is C₁-C₈alkyl, aryl, hetaryl, arylC₁-C₆alkyl, C₃-C₁₀cycloalkyl, C₃-C₁₀hetcycloalkyl, C₃-G₁₀cycloalkylC₁-C₆alkyl, C₁-C₆alkylcarboxyC₁-C₆alkyl, wherein the alkyl, aryl and cycloalkyl groups independently are optionally substituted with one or more of R¹¹; or

R⁶ and R⁷, together with the nitrogen to which they are attached, are forming a saturated or partially saturated cyclic, bicyclic or tricyclic ring system containing from 6 to 10 carbon atoms and from 0 to 2 additional heteroatoms selected from nitrogen, oxygen or sulphur, the ring system optionally being substituted with at least one of C₁-C₈alkyl, aryl, hetaryl, arylC₁-C₆alkyl, hetarylC₁-C₆alkyl, hydroxy, oxo, cyano, C₁-C₆alkyloxy, arylC₁-C₆alkyloxy, hetarylC₁-C₆alkyloxy, C₁-C₆alkyloxyC₁-C₆alkyl, C₁-C₆alkylcarbonyl, arylcarbonyl, hetarylcarbonyl, aryl-C₁-C₆alkylcarbonyl, hetarylC₁-C₆alkylcarbonyl, G₁-C₆alkylcarboxy, arylcarboxy or arylC₁-C₆-alkylcarboxy wherein the alkyl and aryl groups independently are optionally substituted with one ore more of R⁸;

R⁹ and R¹⁰ independently are hydrogen, C₁-C₈alkyl, aryl, hetaryl, arylC₁-C₆alkyl, C₃-C₁₀-cycloalkyl, C₃-C₁₀hetcycloalkyl, C₃-C₁₀cycloalkylC₁-C₆alkyl, C₁-C₆alkylcarboxyC₁-C₆alkyl, wherein the alkyl, aryl and cycloalkyl groups independently are optionally substituted with one or more of R¹¹; or

R⁹ and R¹⁰, together with the nitrogen to which they are attached, are forming a saturated or partially saturated cyclic, bicyclic or tricyclic ring system containing from 4 to 10 carbon atoms and from 0 to 2 additional heteroatoms selected from nitrogen, oxygen or sulphur, the ring system optionally being substituted with at least one of C₁-C₈alkyl, aryl, hetaryl, arylC₁-C₈alkyl, hetarylC₁-C₆alkyl, hydroxy, oxo, cyano, C₁-C₆alkyloxy, arylC₁-C₆alkyloxy, hetarylC₁-C₆alkyloxy, C₁-C₆alkyloxyC₃-C₆alkyl, C₁-C₆alkylcarbonyl, arylcarbonyl, hetarylcarbonyl, aryl-C₁-C₆alkylcarbonyl, hetarylC₁-C₆alkylcarbonyl, C₁-C₆alkylcarboxy, arylcarboxy or arylC₁-C₆-alkylcarboxy wherein the alkyl and aryl groups independently are optionally substituted with one ore more of R⁸;

R⁸ and R¹¹ independently are hydrogen, halo, hydroxy, oxo, nitro, cyano, C₁-C₈alkyl, C₁-C₆-alkyloxy or aryloxy; or
a salt thereof with a pharmaceutically acceptable acid or base, or any optical isomer or mixture of optical isomers, including a racemic mixture, or any tautomeric forms.

In one embodiment of the present invention, in formula (IV) R¹ is hydrogen or C₁-C₆alkyl, wherein the alkyl group is optionally substituted with one or more of R⁸.

In another embodiment of the present invention, in formula (IV) R¹ is hydrogen.

In another embodiment of the present invention, in formula (IV) R², R³, R⁴ and R⁵ are hydrogen.

In another embodiment of the present invention, in formula (IV) R³ together with R⁴ are forming a saturated or partially saturated cyclic ring system containing from 3 to 6 carbon atoms and from 0 to 2 additional heteroatoms selected from nitrogen or oxygen, the ring system optionally being substituted with at least one of C₁-C₆alkyl, aryl, hetaryl, arylC₁-C₆alkyl, hetarylC₁-C₆alkyl, hydroxy, oxo, C₁-C₆alkyloxy, aryloxy, arylC₁-C₆alkyloxy or hetarylC₁-C₆-alkyloxy.

In another embodiment of the present invention, in formula (IV) R⁴ together with R⁵ are forming a saturated or partially saturated cyclic ring system containing from 3 to 6 carbon atoms and from 0 to 2 additional heteroatoms selected from nitrogen or oxygen, the ring system optionally being substituted with at least one of C₁-C₆alkyl, aryl, hetaryl, arylC₁-C₆alkyl, hetarylC₁-C₆alkyl, hydroxy, oxo, C₁-C₆alkyloxy, aryloxy, arylC₁-C₆alkyloxy or hetarylC₁-C₆-alkyloxy.

In another embodiment of the present invention, in formula (iV) R⁶ and R⁷, together with the nitrogen to which they are attached, are forming a saturated or partially saturated cyclic, bicyclic or tricyclic ring system containing from 6 to 10 carbon atoms and from 0 to 2 additional heteroatoms selected from nitrogen or oxygen, the ring system optionally being substituted with at least one of C₁-C₈alkyl, aryl, hetaryl, arylC₁-C₆alkyl, hetarylC₁-C₆alkyl, hydroxy, oxo, cyano, C₁-C₆alkyloxy, arylC₁-C₆alkyloxy, hetarylC₁-C₆alkyloxy, C₁-C₆alkyloxyC₁-C₆alkyl, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy or arylC₁-C₆alkylcarboxy wherein the alkyl and aryl groups independently are optionally substituted with one ore more of R⁸;

In another embodiment of the present invention, in formula (IV) R⁶ and R⁷; together with the nitrogen to which they are attached, are forming a saturated or partially saturated bicyclic or tricyclic ring system containing from 6 to 10 carbon atoms and from 0 to 2 additional heteroatoms selected from nitrogen or oxygen, the ring system optionally being substituted with at least one of C₁-C₈alkyl, aryl, hetaryl, arylC₁-C₆alkyl, hetarylC₁-C₆alkyl, hydroxy, oxo, cyano, C₁-C₆alkyloxy, arylC₁-C₆alkyloxy, hetarylC₁-C₆alkyloxy, C₁-C₆alkyloxyC₁-C₆alkyl, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy or arylC₁-C₆alkylcarboxy wherein the alkyl and aryl groups independently are optionally substituted with one ore more of R⁸.

In another embodiment of the present invention, in formula (IV) R⁹ and R¹⁰, together with the nitrogen to which they are attached, are forming a saturated or partially saturated cyclic, bicyclic or tricyclic ring system containing from 4 to 10 carbon atoms and from 0 to 2 additional heteroatoms selected from nitrogen or oxygen, the ring system optionally being substituted with at least one of C₁-C₈alkyl, aryl, hetaryl, arylC₁-C₆alkyl, hetarylC₁-C₆alkyl, hydroxy, oxo, cyano, C₁-C₆alkyloxy, arylC₁-C₆alkyloxy, hetarylC₁-C₆alkyloxy, C₁-C₆alkyloxyC₁-C₆alkyl, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy or arylC₁-C₆alkylcarboxy wherein the alkyl and aryl groups independently are optionally substituted with one ore more of R⁸.

In another embodiment of the present invention a compound of the general formula (IV) or a prodrug thereof is pyrazolo[1,5-a]pyridin-3-yl-(1,3,3-trimethyl-6-aza-bicyclo[3.2.1]oct-6-yl)-methanone; or a salt thereof with a pharmaceutically acceptable acid or base, or any optical isomer or mixture of optical isomers, including a racemic mixture, or any tautomeric forms.

In another embodiment of the present invention the compounds of the general formula (IV) or a prodrug thereof are:
(2-Methyl-pyrazolo[1,5-a]pyridin-3-yl)-(1,3,3-trimethyl-6-aza-bicyclo[3.2.1]oct-6-yl)-methanone;
Pyrazolo[1,5-a]pyridine-3-carboxylic acid cyclohexyl-methyl-amide; or
   a salt thereof with a pharmaceutically acceptable acid or base, or any optical isomer or mixture of optical isomers, including a racemic mixture, or any tautomeric forms.

In another embodiment, the present invention is concerned with compounds or prodrugs thereof of the general formula (V)

Accordingly, the present invention is concerned with compounds or prodrugs thereof of the general formula (V): 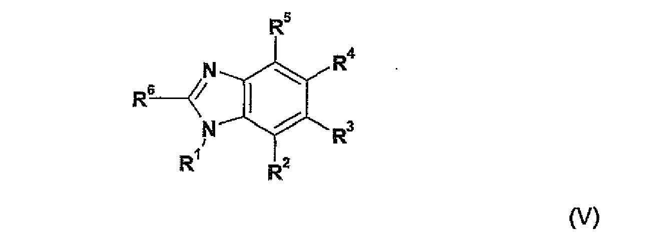 wherein
R¹ is hydrogen, C₁-C₈alkyl, C₁-C₆alkytoxyC₁-C₆alkyl, aryl, hetaryl, arylC₁-C₆alkyl, hetarylC₁-C₆alkyl, C₁-C₆SO₂, arylSO₂, hetarylSO₂, arylC₁-C₆alkylSO₂ or hetarylC₁-C₆alkylSO₂ all of which is optionally substituted with one or more R⁸;

R² and R⁵ independently are hydrogen, halo, nitro, cyano, trihalomethyl, C₁-C₆alkyl, aryl, arylC₁-C₆alkyl, hetaryl or hetarylC₁-C₆alkyl wherein the alkyl, aryl, arylalkyl, hetaryl and hetarylalkyl groups independently are substituted with one or more R⁹; and either R³ is hydrogen; and R⁴ is C(O)NR⁷R⁸; or R³ is C(Q)NR⁷R⁸; and R⁴ is hydrogen; and R⁶ is hydrogen, halo, cyano, trihalomethyl, NR¹²R¹³, C₁-C₆alkyl, aryl, arylC₁-C₆alkyl, hetaryl or hetarylC₁-C₆alkyl wherein the alkyl, aryl, arylalkyl, hetaryl and hetarylalkyl groups independently are substituted with one or more R⁹; and

R⁷ and R⁸ independently are C₁-C₈alkyl, C₃-C₁₀cycloalkyl, C₃-C₁₀hetcycloalkyl, C₃-C₁₀cycloalkylC₁-C₆alkyl, wherein the alkyl, cycloalkyl and hetcycloalkyl groups independently are optionally substituted with one or more of R¹⁰; or

R⁷ and R⁸ together with the nitrogen to which they are attached, are forming a saturated or partially saturated bicyclic or tricyclic ring system containing from 6 to 10 carbon atoms and from 0 to 2 additional heteroatoms selected from nitrogen or oxygen, the ring system optionally being substituted with at least one of C₁-C₈alkyl, arylC₁-C₆alkyl, hetarylC₁-C₆alkyl, hydroxy, cyano, C₁-C₆alkyloxy, arylC₁-C₆alkyloxy, hetarylC₁-C₆alkyloxy, C₁-C₆alkyloxyC₁-C₆alkyl, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy, or arylC₁-C₆alkylcarboxy wherein the alkyl and aryl groups independently are optionally substituted with one ore more of R¹¹;

R⁹ is hydrogen, hydroxy, oxo, halo, nitro, cyano, C₁-C₆alkyl, C₁-C₆alkyloxy, trihalomethyl, trihalomethoxy, NR¹²R¹³, C(O)NR¹²R¹³, arylC₁-C₆alkyloxy, C₁-C₆alkylearbonyl, arylcarbonyl, arylC₁-C₆alkylcarbonyl, C₁-C₆alkylcarboxy, arylcarboxy, or arylC₁-C₆alkylcarboxy;

R¹⁰ and R¹¹ independently are hydrogen, halo, oxo, hydroxy, C₁-C₆alkyl, aryl, arylC₁-C₆alkyl, hetaryl or hetarylalkyl;

R¹² and R¹³ independently are hydrogen, C₁-C₈alkyl, aryl, hetaryl, arylC₁-C₆alkyl, C₃-C₁₀-cycloalkyl, C₃-C₁₀hetcycloalkyl, C₃-C₁₀cycloalkylC₁-C₆alkyl, C₁-C₆alkylcarbonyl, arylcarbonyl, arylC₁-C₈alkylcarbonyl, hetarylcarbonyl, hetarylC₁-C₆alkylcarbonyl, C₁-C₆alkylcarboxyC₁-C₆alkyl; or

R¹² and R¹³ together with the nitrogen to which they are attached, are forming a saturated or partially saturated cyclic, bicyclic or tricyclic ring system containing from 4 to 10 carbon atoms and from 0 to 2 additional heteroatoms selected from nitrogen, oxygen or sulphur, the ring system optionally being substituted with at least one of C₁-C₈alkyl, aryl, hetaryl, arylC₁-C₆alkyl, hetarylC₁-C₆alkyl, hydroxy, oxo, cyano, C₁-C₆alkyloxy, arylC₁-C₆alkyloxy, hetarylC₁-C₆alkyloxy, C₁-C₆alkyl-oxyC₁-C₆alkyl, C₁-C₆alkylcarbonyl, arylcarbonyl, hetarylcarbonyl, arylC₁-G₆alkyl-carbonyl, hetarylC₁-C₆alkylcarbonyl, C₁-C₆alkylcarboxy, arylcarboxy or arylC₁-C₆alkylcarboxy; or
a salt thereof with a pharmaceutical acceptable acid or base, or any optical isomer or mixture of optical isomers, including a racemic mixture, a prodrug thereof, or any tautomeric forms.

In another embodiment, the present invention is concerned with compounds or prodrugs thereof of the general formula (Va) 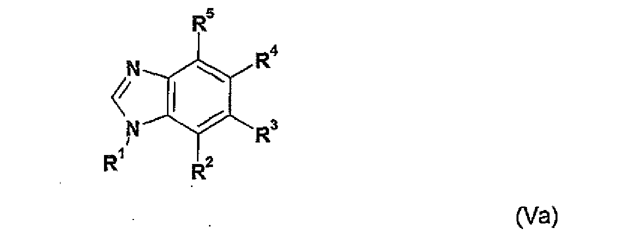 wherein
R¹ is hydrogen, C₁-C₈alkyl, aryl, hetaryl, arylC₁-C₆alkyl or hetarylC₁-C₆alkyl optionally substituted with one or more R⁸;

R² and R⁵ independently are hydrogen, halo, nitro, cyano, trihalomethyl, C₁-C₆alkyl, aryl, arylC₁-C₆alkyl, hetaryl or hetarylC₁-C₆alkyl wherein the alkyl, aryl, arylalkyl, hetaryl and hetarylalkyl groups independently are substituted with one or more R⁸; and either R³ is hydrogen; and R⁴ is C(O)NR⁶R⁷; or R³ is C(O)NR⁶R⁷; and R⁴ is hydrogen;

R⁶ and R⁷ independently are C₁-C₈alkyl, C₃-C₁₀cycloalkyl, C₃-C₁₀hetcycloalkyl, C₃-C₁₀cycloalkylC₁-C₆alkyl, wherein the alkyl, cycloalkyl and hetcycloalkyl groups independently are optionally substituted with one or more of R⁹; or

R⁶ and R⁷ together with the nitrogen to which they are attached, are forming a saturated or partially saturated bicyclic or tricyclic ring system containing from 6 to 10 carbon atoms and from 0 to 2 additional heteroatoms selected from nitrogen or oxygen, the ring system optionally being substituted with at least one of C₁-C₆alkyl, arylC₁-C₆alkyl, hetarylC₁-C₆alkyl, hydroxy, cyano, C₁-C₆alkyloxy, arylC₁-C₆alkyloxy, hetarylC₁-C₆alkyloxy, C₁-C₆alkyloxyC₁-C₆alkyl, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy or arylC₁-C₆alkylcarboxy wherein the alkyl and aryl groups independently are optionally substituted with one ore more of R¹⁰;

R⁸ is hydrogen, hydroxy, oxo, halo, nitro, cyano, C₁-C₆alkyl, C₁-C₆alkyloxy; trihalomethyl, trihalomethoxy, NR¹¹R¹², arylC₁-C₆alkyloxy, C₁-C₆alkylcarbonyl, arylcarbonyl, arylC₁-C₆alkylcarbonyl, C₁-C₆alkylcarboxy, arylcarboxy or arylC₁-C₆alkylcarboxy;

R⁹ and R¹⁰ independently are hydrogen, halo, oxo, hydroxy, C₁-C₆alkyl, aryl, arylC₁-C₆alkyl, hetaryl or hetarylalkyl;

R¹¹ and R¹² independently are hydrogen, C₁-C₈alkyl, aryl, hetaryl, arylC₁-C₆alkyl, C₃-C₁₀-cycloalkyl, C₃-C₁₀hetcycloalkyl, C₃-C₁₀cycloalkylC₁-C₆alkyl, C₁-C₆alkyl-carboxyC₁-C₆alkyl; or

R¹¹ and R¹² together with the nitrogen to which they are attached, are forming a saturated or partially saturated cyclic, bicyclic or tricyclic ring system containing from 4 to 10 carbon atoms and from 0 to 2 additional heteroatoms selected from nitrogen, oxygen or sulphur, the ring system optionally being substituted with at least one of C₁-C₈alkyl, aryl, hetaryl, arylC₁-C₆alkyl, hetarylC₁-C₆alkyl, hydroxy, oxo, cyano, C₁-C₆alkyloxy, arylC₁-C₆alkyloxy, hetarylC₁-C₆alkyloxy, C₁-C₆alkyl-oxyC₁-C₆alkyl, C₁-C₆alkylcarbonyl, arylcarbonyl, hetarylcarbonyl, arylC₁-C₆alkyl-carbonyl, hetarylC₁-C₆alkylcarbonyl, C₁-C₆alkylcarboxy, arylcarboxy or arylC₁-C₆alkylcarboxy; or
a salt thereof with a pharmaceutically acceptable acid or base, or any optical isomer or mixture of optical isomers, including a racemic mixture, or any tautomeric forms.

In another embodiment of the present invention, in formula (V) and (Va) R¹ is hydrogen, C₁-C₈alkyl, arylC₁-C₆alkyl, hetarylC₁-C₆alkyl, arylSO₂, hotarylSO₂, arylC₁-C₆alkylSO₂ or hetarylC₁-C₆alkylSO₂ all of which is optionally substituted with one or more R⁸.

In another embodiment of the present invention, in formula (V) and (Va) R¹ is hydrogen, C₁-C₈alkyl, arylC₁-C₆alkyl, hetarylC₁-C₆alkyl all of which is optionally substituted with one or more R⁸.

In another embodiment of the present invention, in formula (V) and (Va) R¹ is arylSO₂, hetarylSO₂, arylC₁-C₆alkylSO₂ or hetarylC₁-C₆alkylSO₂ all of which is optionally substituted with one or more R⁸.

In another embodiment of the present invention, in formula (V) and (Va) R² is hydrogen.

In another embodiment of the present invention, in formula (V) and (Va) R³ is hydrogen and R⁴ is C(O)NR⁷R⁸.

In another embodiment of the present invention, in formula (V) and (Va) R³ is C(O)NR⁷R⁸ and R⁴ is hydrogen.

In another embodiment of the present invention, in formula (V) and (Va) R⁵ is hydrogen.

In another embodiment of the present invention, in formula (V) R⁶ is hydrogen, NR¹²R¹³, C₁-C₆alkyl, aryl or hetaryl wherein the alkyl, aryl and hetaryl independently are substituted with one or more R⁹.

In another embodiment of the present invention, in formula (V) and (Va) R⁷ and R⁸ independently are C₁-C₈alkyl or C₃-C₁₀cycloalkyl, wherein the alkyl and cycloalkyl groups independently are optionally substituted with one or more of R¹⁰.

In another embodiment of the present invention, in formula (V) and (Va) R⁷ and R⁸ together with the nitrogen to which they are attached, are forming a saturated or partially saturated bicyclic or tricyclic ring system containing from 6 to 10 carbon atoms and from 0 to 2 additional heteroatoms selected from nitrogen or oxygen, the ring system optionally being substituted with at least one of C₁-C₈alkyl, arylC₁-C₆alkyl, hetarylC₁-C₆alkyl, hydroxy, cyano, C₁-C₆alkyloxy, arylC₁-C₆alkyloxy, hetarylC₁-C₆alkyloxy, C₁-C₆alkyloxyC₁-C₆alkyl, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy or arylC₁-C₆alkylcarboxy wherein the alkyl and aryl groups independently are optionally substituted with one ore more of R¹¹.

In another embodiment of the present invention, in formula (V) and (Va) R⁹ is hydrogen, hydroxy, oxo, halo, nitro, cyano, C₁-C₆alkyl, C₁-C₆alkyloxy, trihalomethyl, trihalomethoxy, NR¹²R¹³, SC(O)NR¹²R¹³, arylC₁-C₆alkyloxy, C₁-C₆alkylcarbonyl, arylcarbonyl, arylC₁-C₆alkylcarbonyl.

In another embodiment of the present invention, in formula (V) and (Va) R¹⁰ and R¹¹ independently are hydrogen, halo, oxo, hydroxy, C₁-C₆alkyl, aryl, arylC₁-C₆alkyl, hetaryl or hetarylalkyl.

In another embodiment of the present invention, in formula (V) and (Va) R¹¹ and R¹² together with the nitrogen to which they are attached, are forming a saturated or partially saturated cyclic, bicyclic or tricyclic ring system containing from 4 to 10 carbon atoms and from 0 to 2 additional heteroatoms selected from nitrogen or oxygen, the ring system optionally being substituted with at least one of C₁-C₈alkyl, aryl, hetaryl, arylC₁-C₆alkyl, hetarylC₁-C₆alkyl, hydroxy, oxo, cyano, C₁-C₆alkyloxy, arylC₁-C₆alkyloxy, hetarylC₁-C₆alkyloxy, C₁-C₆alkyl-oxyC₁-C₆alkyl, C₁-C₆alkylcarbonyl, arylcarbonyl, hetarylcarbonyl, arylC₁-C₆alkyl-carbonyl, hetarylC₁-C₆alkylarbonyl, C₁-C₆alkylcarboxy, arylcarboxy or arylC₁-C₆alkylcarboxy.

In another embodiment of the present invention the compound of the general formulas (V) and (Va), or a prodrug thereof is 1*H*-Benzoimidazole-5-carboxylic acid cyclohexyl-methyl-amide; or a salt thereof with a pharmaceutical acceptable acid or base, or any optical isomer or mixture of optical isomers, including a racemic mixture, or any tautomeric forms.

In yet another embodiment of the present invention the compounds of the general formulas (V) and (Va), or a prodrug thereof is selected from the group consisting of: 1-Benzyl-1*H*-benzoimidazole-5-carboxylic acid cyclohexyl-methyl-amide; (1*H*-Benzoimidazol-5-yl)-(1,3,3-trimethyl-6-aza-bicyclo[3.2.1]oct-6-yl)-methanone; or a salt thereof with a pharmaceutically acceptable acid or base, or any optical isomer or mixture of optical isomers, inclucting a racemic mixture, or any tautomeric forms.

In another embodiment of the present invention, the compounds of general formulas (V) and (Va), or a prodrug thereof is sleceted from the group consisting of:
lsopropyl-2-trifluoromethyl-1*H*-benzolmidazole-5-carboxylic acid cyclohexyl-methyl-amide;
1-Benzyl-1*H*-benzoimidazole-5-carboxylic acid cyclohexyl-methyl-amide;
2-Methyl-1*H*-benzoimidazole-5-carboxylic acid cyclohexyl-methyl-amide;
2-Hydroxymethyl-1*H*-benzoimidazole-5-carboxylic acid cyclohexyl-methyl-amide;
2-(4-Amino-phenyl)-1*H*-benzoimidazole-5-carboxylic acid cyclohexyl-methyl-amide;
(1*H*-Benzoimidazol-5-yl)-(1,3,3-trimethyl-6-aza-bicyclo[3.2.1]oct-6-yl)-methanone;
(2-Methyl-1*H*-benzoimidazol-5-yl)-(1,3,3-trimethyl-6-aza-bicyclo[3.2.1]oct-6-yl)-methanone;
(2-Amino-1*H*-benzoimidazol-5-yl)-(1,3,3-trimethyl-6-aza-bicyclo[3.2.1]oct-6-yl)-methanone;
(2-Benzo[1,3]dioxof-5-yl-*1H*-benzoimidazol-5-yl)-(1,3,3-trimethyl-6-aza-bicyclo[3.2.1]oct-6-yl)-methanone;
3-[5-(1,3,3-Trimethyl-6-aza-bicyclo[3.2.1]octane-6-carbonyl)-*1H*-benzoimidazol-2-yl]-benzoic acid methyl ester;
(2-Thiophen-2-yl-*1H*-benzoimidazo)-5-yl)-(1,3,3-trimethyl-6-aza-bicyclo[3.2.1]oct-6-yl)-methanone;
[2-(2-Nitro-phenyl)-*1H*-benzoimidazol-5-yl]-(1,3,3-trimethyl-6-aza-bicyclo[3.2.1]oct-6-yl)-methanon; or
   a salt thereof with a pharmaceutically acceptable acid or base, or any optical isomer or mixture of optical isomers, including a racemic mixture, a prodrug thereof, or any tautomeric forms.

In another embodiment, the present invention is concerned with compounds or prodrugs thereof of the general formula (VI) 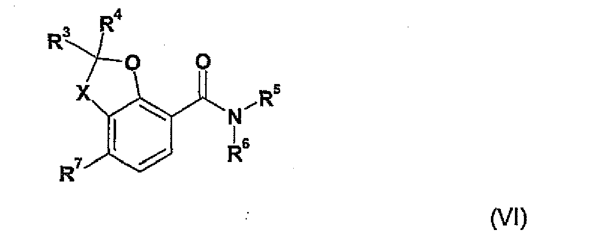 wherein
X is oxygen or (CR¹R²)ₙ;

R¹, R², R³, and R⁴ independently are hydrogen, C₁-C₆alkyl, aryl, arylC₁-C₆alkyl, hetaryl or hetarylC₁-C₆alkyl optionally substituted with one or more R⁸ independently; or

R¹ and either R³ or R⁴ together are forming a saturated or partially saturated ring system containing from 4 to 8 carbon atoms, the ring system optionally being substituted with at least one of C₁-C₆alkyl, hydroxy, oxo, aryl, hetaryl, arylC₁-C₆alkyl or hetarylC₁-C₆alkyl; or

R¹ and either R³ or R⁴ together with the single bond are forming a carbon-carbon double bond;

R⁵ is C₁-C₈alkyl optionally substituted with one or more of R⁹;

R⁶ is C₃-C₁₀cycloalkyl, C₃-C₁₀hetcycloalkyl, C₃-C₁₀cycloalkylC₁-C₆alkyl, wherein the alkyl, cycloalkyl and hetcycloalkyl, groups independently are optionally substituted with one or more of R⁹; or

R⁵ and R⁶ together with the nitrogen to which they are attached, are forming a saturated or partially saturated cyclic, bicyclic or tricyclic ring system containing from 6 to 10 carbon atoms and from 0 to 2 additional heteroatoms selected from nitrogen, oxygen or sulphur, the ring system optionally being substituted with at least one of C₁-C₆alkyl, aryl, hetaryl, arylC₁-C₆alkyl, hetarylC₁-C₆alkyl, hydroxy, oxo, cyano, C₁-C₆alkyloxy, arylC₁-C₆alkyloxy, hetarylC₁-C₆alkyloxy, C₁-C₆alkyloxyC₁-C₆alkyl, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy, or arylC₁-C₆alkylcarboxy wherein the alkyl and aryl groups independently are optionally substituted with one ore more of R¹⁰;

R⁷ is hydrogen, halo, nitro, NR¹²R¹³, cyano, trihalomethyl, C₁-C₆alkyl, aryl, arylC₁-C₆alkyl, C₁-C₆alkyloxy, aryloxy, arylC₁-C₆alkyloxy, hetaryl, hetarylC₁-C₆alkyl, hetaryloxy or hetarylC₁-C₆-alkyloxy optionally substituted with one or more R¹¹ independently;

R⁸ and R⁹ independently are hydrogen, hydroxy, oxo, halo, nitro, cyano, C₁-C₆alkyl, C₁-C₆-alkyloxy, trihalomethyl, trihalomethoxy, NR¹²R¹³. arylC₁-C₆alkyloxy, C₁-C₆alkylcarbonyl, arylcarbonyl, arylC₁-C₆alkylcarbonyl, C₁-C₆alkylcarboxy, arylcarboxy or arylC₁-C₆alkylcarboxy;

R¹⁰ is hydrogen, C₁-C₈alkyl, arylC₁-C₆alkyl, hetarylC₁-C₆alkyl, C₁-C₆alkyloxy, arylC₁-C₆alkyloxy, hetarylC₁-C₆alkyloxy;

R¹¹ is hydrogen, halo, hydroxy, oxo, nitro, cyano, C₁-C₈alkyl, C₁-C₆alkyloxy, aryloxy or hetaryloxy;

R¹² and R¹³ independently are hydrogen, C₁-C₈alkyl, aryl, hetaryl, arylC₁-C₆alkyl, C₃-C₁₀cycloalkyl, C₃-C₁₀hetcycloalkyl, C₁-C₆alkylcarbonyl, arylC₁-C₆alkylcarbonyl, C₃-C₁₀cycloalkylC₁-C₆-alkyl, C₁-C₆alkyloxycarbonyl; or

R¹² and R¹³ are together with the nitrogen to which they are attached, are forming a saturated or partially saturated cyclic, bicyclic or tricyclic ring system containing from 4 to 10 carbon atoms and from 0 to 2 additional heteroatoms selected from nitrogen, oxygen or sulphur, the ring system optionally being substituted with at least one of C₁-C₆alkyl, aryl, hetaryl, arylC₁-C₆alkyl, hetarylC₁-C₆alkyl, hydroxy, oxo, cyano, C₁-C₆alkyloxy, arylC₁-C₆alkyloxy, hetarylC₁-C₆alkyloxy, C₁-C₆alkyloxyC₁-C₆alkyl, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy or arylC₁-₆alkylcarboxy;
n is 1 or 2; or
a salt thereof with a pharmaceutical acceptable add or base, or any optical isomer or mixture of optical isomers, including a racemic mixture, a prodrug thereof, or any tautomeric forms.

In one embodiment of the present invention, in formula (VI) X is (CR¹R²)ₙ, wherein R¹, R² and n are as defined above.

In another embodiment of the present invention, in formula (VI) n is 1.

In another embodiment of the present invention, in formula (VI) X is oxygen.

In another embodiment of the present invention, in formula (VI) R¹, R², and R⁴ independently are hydrogen, C₁-C₆alkyl or arylC₁-C₆alkyl, optionally substituted with one or more R⁸.

In another embodiment of the present invention, in formula (VI) R¹ and either R³ or R⁴ together with the single bond are forming a carbon-carbon double bond.

In another embodiment of the present invention, in formula (VI) R⁵ is C₁-C₈alkyl optionally substituted with one or more of R⁹.

In another embodiment of the present invention, in formula (VI) R⁶ is C₃-C₁₀cycloalkyl or C₃-C₁₀hetcycloalkyl each of which is optionally substituted with one or more of R⁹.

In another embodiment of the present invention, in formula (VI) R⁶ is C₃-C₁₀cycloalkyl optionally substituted with one or more of R⁹.

In another embodiment of the present invention, in formula (VI) R⁵ and R⁶ together with the nitrogen to which they are attached, are forming a saturated or partially saturated cyclic, bicyclic or tricyclic ring system containing from 6 to 10 carbon atoms and from 0 to 2 additional heteroatoms selected from nitrogen, oxygen or sulphur, the ring system optionally being substituted with at least one of C₁-C₆alkyl, aryl, hetaryl, arylC₁-C₆alkyl, hetarylC₁-C₆-alkyl, hydroxy, oxo, cyano, C₁-C₆alkyloxy, arylC₁-C₆alkyloxy, hetarylC₁-C₆alkyloxy, C₁-C₆alkyloxyC₁-C₆alkyl, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy or arylC₁-C₆alkylcarboxy wherein the alkyl and aryl groups independently are optionally substituted with one ore more of R¹⁰.

In another embodiment of the present invention, in formula (VI) R⁷ is hydrogen, halo, NR¹²R¹³, trihalomethyl, C₁-C₆alkyloxy, aryloxy, arylC₁-C₆alkyloxy or hetaryloxy optionally substituted with one or more R¹¹ independently.

In another embodiment of the present invention, in formula (VI) R⁸ and R⁹ independently are hydrogen, hydroxy, oxo, halo, nitro, cyano, C₁-C₆alkyl, C₁-C₆alkyloxy, trihatomethyl, or NR¹²R¹³_{.}

In another embodiment of the present invention, in formula (VI) R¹⁰ is hydrogen or C₁-C₈alkyl.

In yet another embodiment of the present invention, in formula (VI) the bicyclic ring system is 6-aza-bicyclo[3.2.1]octane optionally substituted with one or more of C₁-C₆alkyl.

In yet another embodiment of the present invention, in formula (VI) the bicyclic ring system is 1,3,3-trimethyl-6-aza-bicycio[3.2.1]octane.

In yet another embodiment of the present invention a compound of the general formula (VI) or a prodrug thereof is 2,3-Dimethyl-2,3-dihydro-benzofuran-7-carboxylic acid cyclohexyl-methyl-amide or a salt thereof with a pharmaceutically acceptable acid or base, or any optical isomer or mixture of optical isomers, including a racemic mixture, or any tautomeric forms.

In another embodiment of the present invention the compounds of the general formula (VI) or a prodrug thereof is selected from the group consisting of:
2,5-Dimethyl-3-phenyl-benzofuran-7-carboxylic acid cyclohexyl-methyl-amide;
2,2-Dimethyl-2,3-dihydro-benzofuran-7-carboxylic acid cyclohexyl-methyl-amide;
2-Methyl-2,3-dihydro-benzofuran-7-carboxylic acid cyclohexyl-methyl-amide;
(2,3-Dimethyl-2,3-dihydro-benzofuran-7-yl)-(2,4,4-trimethyl-6-aza-bicyclo[3.2.1]oct-6-yl)-methanone;
4-Methoxy-2-methyl-2,3-dihydro-benzofuran-7-carboxylic acid cyclohexyl-methyl-amide;
2-Methyl-benzofuran-7-carboxylic acid cyclohexyl-m6thyi-amide;
(2-Methyl-2,3-dihydro-benzofuran-7-yl)-(1,3,3-trimethyl-6-aza-bicyclo[3.2.1]oct-6-yl)-methanone;
Benzofuran-7-carboxylic acid cyclohexyl-methyl-amide;
2,3-Dihydro-benzofuran-7-carboxylic acid cyclohexyl-methyl-amide;
3,3-Dimethyl-2,3-dihydro-benzofuran-7-carboxylic acid cyclohexyl-methyl-amide;
Chroman-8-carboxylic acid cyclohexyl-methyl-amide; or
   a salt thereof with a pharmaceutically acceptable acid or base, or any optical isomer or mixture of optical isomers, including a racemic mixture, or any tautomeric forms.

In another embodiment of the present invention the compounds of the general formula (VI) or a prodrug thereof is selected from the group consisting of:
2,3-Dihydro-benzofuran-7-carboxylic acid cyclohexyl-methyl-amide;
Benzofuran-7-carboxylic acid cyclohexyl-methyl-amide;
2-Methyl-2,3-dihydro-benzofuran-7-carboxylic acid cyclohexyl-methyl-amide;
2-Methyl-benzofuran-7-carboxylic acid cycloheoyl-methyl-amide;
3,3-Dimethyl-2,3-dihydro-benzofuran-7-carboxylic acid cyclohexyl-methyl-amide;
(2,3-Dimethyl-2,3-dihydro-benzofuran-7-yl)-(2,4,4-trimethyl-6-aza-bicyclo[3.2.1]oct-6-yl)-methanone;
4-Methoxy-2-methyl-2,3-dihydro-benzofuran-7-carboxylic acid cyclohexyl-methyl-amide;
2,2-Dimethyl-2,3-dihydro-benzofuran-7-carboxylic acid cyclohexyl-methyl-amide;
(2-Methyl-2,3-dihydro-benzofuran-7-yl)-(1,3,3-trimethyl-6-aza-bicyclo[3.2.1]oct-6-yl)-methanone;
Chroman-8-carboxylic acid cyclohexyl-methyl-amide; or
   a salt thereof with a pharmaceutically acceptable acid or base, or any optical isomer or mixture of optical isomers, including a racemic mixture, a prodrug thereof, or any tautomeric forms.

In another embodiment, the present invention is concerned with compounds or prodrugs thereof of the general formula (VII) 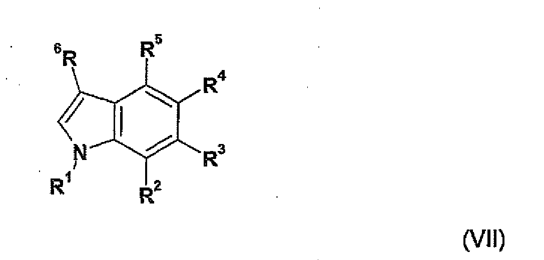 wherein
R¹ is hydrogen, C₁-C₈alkyl, hetaryl, arylC₁-C₆alkyl or hetarylC₁-C₆alkyl optionally substituted with one or more R⁹;

R², R³, R⁴, R⁵ and R⁶ independently are hydrogen, halo, nitro, cyano, trihalomethyl, carboxy, N(R¹²R¹³), C(O)NR⁷R⁸, C₁-C₈alkyl, C₃-C₁₀cycloalkyl, C₃-C₁₀hetcycloalkyl, N(R¹²R¹³)C₁-C₆alkyl, C₁-C₆alkyloxy, C₁-C₆alkyloxyC₁-C₆alkyl, aryl, arylC₁-C₆alkyl, aryloxy, aryloxyC₁-C₆alkyl, arylC₁-C₆alkyloxy, arylC₁-C₆alkyloxyC₁-C₆alkyl, C₁-C₆alkylcarboxy, arylcarboxy, arylC₁-C₆alkylcarboxy, hetaryl, hetarylC₁-C₆alkyl, hetarylC₁-C₆alkyloxy, hetaryloxyC₁-C₆alkyl or hetarylC₁-C₆alkyloxyC₁-C₆alkyl wherein wherein the alkyl, aryl, arylalkyl, hetaryl and hetarylalkyl groups independently are substituted with one or more R⁹;

R⁷ is hydrogen or C₁-C₈alkyl optionally substituted with one or more of R¹⁰;

R⁸ is C₃-C₁₀cycloalkyl, C₃-C₁₀hetcycloalkyl, C₃-C₁₀cycloalkylC₁-C₆alkyl, wherein the cycloalkyl and hetcycloalkyl groups independently are optionally substituted with one or more of R¹⁰; or

R⁷ and R⁸ together with the nitrogen to which they are attached, are forming a saturated or partially saturated cyclic, bicyclic or tricyclic ring system containing from 4 to 10 carbon atoms and from 0 to 2 additional heteroatoms selected from nitrogen or oxygen, the ring system optionally being substituted with at least one of C₁-C₈alkyl, aryl, hetaryl, arylC₁-C₆alkyl, hetarylC₁-C₆alkyl, hydroxy, oxo, cyano, C₁-C₆alkyloxy, arylC₁-C₆alkyloxy, hetarylC₁-C₆alkyloxy, C₁-C₆alkyloxyC₁-C₆alkyl, C₁-C₆alkylcarbonyl, arylcarbonyl, hetarylcarbonyl, arylC₁-C₆-alkylcarbonyl, hetarylC₁-C₆alkylcarbonyl, C₁-C₆alkylcarboxy, arylcarboxy or arylC₁-C₆alkylcarboxy wherein the alkyl and aryl groups independently are optionally substituted with one ore more of R¹¹;

R⁹ is hydrogen, hydroxy, oxo, halo, nitro, cyano, C₁-C₆alkyl, C₁-C₆alkyloxy, trihalomethyl, trihalomethoxy, NR¹²R¹³, arylC₁-C₆alkyloxy, C₁-C₆alkylcarbonyl, arylcarbonyl, arylC₁-C₆alkylcarbonyl, C₁-C₆alkylcarboxy, arylcarboxy or arylC₁-C₆alkylcarboxy;

R¹⁰ and R¹¹ independently are hydrogen, halo, oxo, hydroxy, C₁-C₆alkyl, aryl, arylC₁-C₆alkyl, hetaryl or hetarylalkyl;

R¹² and R¹³ independently are hydrogen, C₁-C₈alkyl, aryl, hetaryl, arylC₁-C₆alkyl, C₃-C₁₀-cycloalkyl, C₃-C₁₀hetcycloalkyl, C₃-C₁₀cycloalkylC₁-C₆alkyl, C₁-C₆alkylcarbonyl, arylcarbonyl, arylC₁-C₆alkylcarbonyl, C₃-C₁₀cycloalkylcarbonyl, C₃-C₁₀hetcycloalkylcarbonyl or C₃-C₁₀cycloalkylC₁-C₆alkylcarbonyl wherein the alkyl and aryl groups independently are optionally substituted with one or more of R¹¹, wherein R¹¹ is as defined above; or

R¹² and R¹³ together with the nitrogen to which they are attached, are forming a saturated or partially saturated cyclic, bicyclic or tricyclic ring system containing from 4 to 10 carbon atoms and from 0 to 2 additional heteroatoms selected from nitrogen, oxygen or sulphur, the ring system optionally being substituted with at least one of C₁-C₈alkyl, aryl, hetaryl, arylC₁-C₆alkyl, hetarylC₁-C₆alkyl, hydroxy, oxo, cyano, C₁-C₆alkyloxy, arylC₁-C₆alkyloxy, hetarylC₁-C₆alkyloxy, C₁-C₆alkyl-oxyC₁-C₆alkyl, C₁-C₆alkylcarbonyl, arylcarbonyl, hetarylcarbonyl, arylC₁-C₆alkyl-carbonyl, hetarylC₁-C₆alkylcarbonyl, C₁-C₆alkylcarboxy, arylcarboxy or arylC₁-C₆alkylcarboxy; or
a salt thereof with a pharmaceutically acceptable acid or base, or any optical isomer or mixture of optical isomers, including a racemic mixture, or any tautomeric forms.

In another embodiment, the present invention is concerned with compounds or prodrugs thereof of the general formula (VII) wherein

R¹ is hydrogen, C₁-C₈alkyl, hetaryl, arylC₁-C₆alkyl or hetarylC₁-C₆alkyl optionally substituted with one or more R⁹;

R² and R⁵ independently are hydrogen, halo, nitro, cyano, trihalomethyl, G₁-C₆alkyl, C₁-C₆-alkyloxy, C₁-C₆alkyloxyC₁-C₆alkyl, aryl, arylC₁-C₆alkyl, aryloxy, aryloxyC₁-C₆alkyl, arylC₁-C₆-alkyloxy, arylC₁-C₆alkyloxyC₁-C₆alkyl, hetaryl or hetarylC₁-C₆alkyl wherein the alkyl, aryl, arylalkyl, hetaryl and hetarylalkyl groups independently are substituted with one or more R⁹; and either R³ is C(C)NR⁷R⁸, an R⁴ is hydrogen; or R³ is hydrogen, and R⁴ is C(O)NR⁷R⁸;

R⁶ is C₁-C₈alkyl, C₃-C₁₀cycloalkyl, C₃-C₁₀hetcycloalkyl, N(R¹²R¹³)C₁-C₆alkyl, C₁-C₆alkyloxyC₁-C₆alkyl, aryloxyC₁-C₆alkyl, arylC₁-C₆alkyloxy or arylC₁-C₆alkyloxyC₁-C₆alkyl;

R⁷ is C₁-C₈alkyl optionally substituted with one or more of R¹⁰;

R⁸ is C₃-C₁₀cycloalkyl, C₃-C₁₀hetcycloalkyl, C₃-C₁₀cycloalkylC₁-C₆alkyl, wherein the cycloalkyl and hetcycloalkyl groups independently are optionally substituted with one or more of R¹⁰; or

R⁷ and R⁸ together with the nitrogen to which they are attached, are forming a saturated or partially saturated cyclic, bicyclic or tricyclic ring system containing from 6 to 10 carbon atoms and from 0 to 2 additional heteroatoms selected from nitrogen or oxygen, the ring system optionally being substituted with at least one of C₁-C₈alkyl, aryl, hetaryl, arylC₁-C₆alkyl, hetarylC₁-C₆alkyl, hydroxy, oxo, cyano, C₁-C₆alkyloxy, arylC₁-C₆alkyloxy, hetarylC₁-C₆alkyloxy, C₁-C₆alkyloxyC₁-C₆alkyl, C₁-C₆alkylcarbonyl, arylcarbonyl, hetarylcarbonyl, arylC₁-C₆-alkylcarbonyl, hetarylC₁-C₆alkylcarbonyl, C₁-C₆alkylcarboxy, arylcarboxy or arylC₁-C₆alkylcarboxy wherein the alkyl and aryl groups independently are optionally substituted with one ore more of R¹¹;

R⁹ is hydrogen, hydroxy, oxo, halo, nitro, cyano, C₁-C₆alkyl, C₁-C₆alkyloxy, trihalomethyl, trihalomethoxy, NR¹²R¹³, arylC₁-C₆alkyloxy, C₁-C₆alkylcarbonyl, arylcarbonyl, arylC₁-C₆alkylcarbonyl, C₁-C₆alkylcarboxy, arylcarboxy or arylC₁-C₆alkylcarboxy;

R¹⁰ and R¹¹ independently are hydrogen, halo, oxo, hydroxy, C₁-C₆alkyl, aryl, arylC₁-C₆alkyl, hetaryl or hetarylalkyl;

R¹² and R¹³ independently are hydrogen, C₁-C₈alkyl, aryl, hetaryl, arylC₁-C₆alkyl, C₃-C₁₀-cycloalkyl, C₃-C₁₀hetcycloalkyl, C₃-C₁₀cycloalkylC₁-C₆alkyl, C₁-C₆alkylcarbonyl, arylcarbonyl, arylC₁-C₆alkylcarbonyl, C₃-C₁₀cycloalkylcarbonyl, C₃-C₁₀hetcycloalkylcarbonyl or C₃-C₁₀cycloalkylC₁-C₆alkylcarbonyl; or

R¹² and R¹³ together with the nitrogen to which they are attached, are forming a saturated or partially saturated cyclic, bicyclic or tricyclic ring system containing from 4. to 10 carbon atoms and from 0 to 2 additional heteroatoms selected from nitrogen, oxygen or sulphur, the ring system optionally being substituted with at least one of C₁-C₈alkyl, aryl, hetaryl, arylC₁-C₆alkyl, hetarylC₁-C₆alkyl, hydroxy, oxo, cyano, C₁-C₆alkyloxy, arylC₁-C₆alkyloxy, hetarylC₁-C₆alkyloxy, C₁-C₆alkyl-oxyC₁-C₆alkyl, C₁-C₆alkylcarbonyl, arylcarbonyl, hetarylcarbonyl, arylC₁-C₆alkyl-carbonyl, hetarylC₁-C₆alkylcarbonyl, C₁-C₆alkylcarboxy, arylcarboxy or arylC₁-C₆alkylcarboxy; or
a salt thereof with a pharmaceutically acceptable acid or base, or any optical isomer or mixture of optical isomers, including a racemic mixture, or any tautomeric forms.

In another embodiment, the present invention, in formula (VII) R² is C(O)NR⁷R⁸ and R³ R⁴ and R⁵ are hydrogen, wherein R⁷ and R⁸ are as defined above.

In another embodiment, the present invention, in formula (VII) R³ is C(O)NR⁷R⁸ and R² R⁴ and R⁵ are hydrogen, wherein R⁷ and R⁸ are as defined above.

In another embodiment, the present invention, in formula (VII) R⁴ is C(Q)NR⁷R⁸ and R² R³ and R⁵ are hydrogen, wherein R⁷ and R⁸ are as defined above.

In another embodiment, the present invention, in formula (VII) R⁵ is C(O)NR⁷R⁸ and R² R³ and R⁴ ara hydrogen, wherein R⁷ and R⁸ are as defined above.

In another embodiment, the present invention, in formula (VII) R⁶ is C(O)NR⁷R⁸, wherein R⁷ and R⁸ are as defined above.

In another embodiment, the present invention, in formula (VII) R³ is C(O)NR⁷R⁸ and

R⁴ is hydrogen, wherein R⁷ and R⁸ are as defined above.

In another embodiment, the present invention, in formula (VII) R³ is hydrogen and R⁴ is C(O)NR⁷R⁸, wherein R⁷ and R⁸ are as defined above.

In another embodiment, the present invention, in formula (VII) R⁸ is C₃-C₁₀cycloalkyl or C₃-C₁₀hetcycloalkyl, each of which is optionally substituted with one or more of R¹⁰,
wherein R¹⁰ is as defined above.

In another embodiment, the present invention, in formula (VII) R⁷ and R⁸ together with the nitrogen to which they are attached, are forming a saturated or partially saturated bicyclic or tricyclic ring system containing from 6 to 10 carbon atoms and from 0 to 2 additional heteroatoms selected from nitrogen or oxygen, the ring system optionally being substituted with at least one of C₁-C₈alkyl, aryl, hetaryl, arylC₁-C₆alkyl, hetarylC₁-C₆alkyl, hydroxy, oxo, cyano, C₁-C₆alkyloxy, arylC₁-C₆alkyloxy, hetarylC₁-C₆alkyl-oxy, C₁-C₆alkyloxyC₁-C₆alkyl, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy or arylC₁-C₆alkylcarboxy wherein the alkyl and aryl groups independently are optionally substituted with one ore more of R¹¹, wherein R¹¹ is as defined above.

In a further embodiment of the present invention, in formula (VII) the bicyclic ring system is 6-aza-bicyclo[3.2.1]octane optionally substituted with one or more C₁-C₆alkyl.

In yet a further embodiment of the present invention, in formula (VII) the bicyclic ring system is 1,3,3-trimethyl-6-aza-bicyclo[3.2.1]octane.

In another embodiment of the present invention the compounds of the general formula (VII) or a prodrug thereof is selected from the group consisting of:
(1*H*-Indol-5-yl)-(1,3,3-trimethyl-6-aza-bicyclo[3.2.1]oct-6-yl)-methanone;
1*H*-Indole-6-carboxylic acid cyclohexyl-methyl-amide; or
   a salt thereof with a pharmaceutically acceptable acid or base, or any optical isomer or mixture of optical isomers, including a racemic mixture, or any tautomeric forms.

In another embodiment of the present invention, the compounds or prodrugs thereof of the general formula (VII) are selected from the group consisting of the compounds of examples 3 through 20 as described under EXAMPLES, COMPOUNDS OF GENERAL FORMULA (VII).

In another embodiment, the present invention is concerned with compounds or prodrugs thereof of the general formula (VIII) 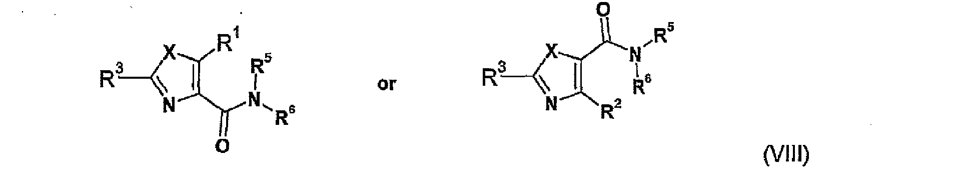 wherein
X is NR⁴, S or O;

R¹ and R² independently are hydrogen, halo, cyano, trihalomethyl, C₁-C₆alkyl or C₁-C₆-alkyloxy, wherein the alkyl groups independently are optionally substituted with one or more of R⁷;

R³ is hydrogen, C₁-C₆alkyl, C₃-C₁₀cycloalkyl, C₁-C₆alkyloxy, C₁-C₆alkylthio, aryl, arylC₁-C₆-alkyl, hetaryl or hetarylalkyl, wherein the alkyl, cycloalkyl, aryl, hetaryl and hetarylalkyl groups independently are optionally substituted with one or more of R⁷;

R⁴ is hydrogen, C₁-C₈alkyl, C₁-C₆alkyloxyC₁-C₆alkyl, aryl, hetaryl, hetarylC₁-C₆alkyl, arylC₁-C₆alkyl, arylC₁-C₆alkyloxyC₁-C₆alkyl, C₃-C₁₀cycloalkyl, C₃-C₁₀hetcycloalkyl, C₃-C₁₀cycloalkylC₁-C₆alkyl, C₁-C₆alkylcarboxyC₁-C₆alkyl wherein the alkyl, aryl, hetaryl, cycloalkyl and hetcycloalkyl groups independently are optionally substituted with one or more of R⁷;

R⁵ is hydrogen, and R⁶ is adamantyl optionally substituted with hydroxy, C₁-C₆alkyloxy, aryl, arylC₁-C₆alkyl, aryloxy, arylC₁-C₆alkyloxy, hetaryl, hetaryloxy or hetarylC₁-C₆alkyloxy wherein the alkyl, aryl and hetaryl groups independently are optionally substituted with one or more:of R⁷; or

R⁵ and R⁶ are together with the nitrogen to which they are attached, forming a saturated or partially saturated cyclic, bicyclic or tricyclic ring system containing from 6 to 10 carbon atoms and from 0 to 2 additional heteroatoms selected from nitrogen, oxygen or sulphur, the ring system optionally being substituted with at least one of C₁-C₆alkyl, aryl, hetaryl, arylC₁-C₆alkyl, hetarylalkyl, hydroxy, oxo, cyano, C₁-C₆alkyloxy, arylC₁-C₆alkyloxy, hetarylC₁-C₆alkyloxy, C₁-C₆alkyloxyC₁-C₆alkyl, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy or arylC₁-C₆alkylcarboxy wherein the alkyl and aryl groups independently are optionally substituted with one ore more of R⁷;

R⁷ are independently hydrogen, halo, hydroxy, oxo, nitro, NR⁹R¹⁰, cyano, COOR⁸, CONR⁹R¹⁰, C₁-C₈alkyl, C₁-C₆alkyloxy, aryloxy, arylC₁-C₆alkyloxy, hetaryloxy or hetarylC₁-C₆alkyloxy;

R⁸ is hydrogen, C₁-C₆alkyl, aryl, arylC₁-C₆alkyl, hetarylalkyl, wherein the alkyl, aryl and hetarylalkyl groups independently are optionally substituted with one or more of R⁷;

R⁹ and R¹⁰ independently are hydrogen, C₁-C₈alkyl, C₃-C₁₀cycloalkyl, C₃-C₁₀hetcycloalkyl, C₃-C₁₀cycloalkylC₁-C₆alkyl, wherein the alkyl, cycloalkyl and hetcycloalkyl groups independently are optionally substituted with one or more of R⁷; or

R⁹ and R¹⁰ together with the nitrogen to which they are attached, are forming a saturated or partially saturated bicyclic or tricyclic ring system containing from 4 to 10 carbon atoms and from 0 to 2 additional heteroatoms selected from nitrogen or oxygen, the ring system optionally being substituted with at least one of C₁-C₈alkyl, arylC₁-C₆alkyl, hetarylC₁-C₆alkyl, hydroxy, cyano, C₁-C₆alkyloxy, arylC₁-C₆alkyloxy, hetarylC₁-C₆alkyloxy, C₁-C₆alkyloxyC₁-C₆alkyl, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy or arylC₁-C₆alkylcarboxy wherein the alkyl and aryl groups independently are optionally substituted with one ore more of R¹¹;

R¹¹ is hydrogen, halo, oxo, hydroxy, C₁-C₆alkyl, aryl, arylC₁-C₆alkyl, hetaryl or hetarylalkyl;
a salt thereof with a pharmaceutically acceptable acid or base, or any optical isomer or mixture of optical isomers, including a racemic mixture, or any tautomeric forms.

In the definitions of R⁴, in the above formula (VIII), hetcycloalkyl cannot be 7-aza[2,2,1]bicycleheptane.

In another embodiment, the present invention is concerned with compounds or prodrugs thereof of the above general formula (VIII) wherein
X is NR⁴, S or O;

R¹ and R² independently are hydrogen, halo, cyano, trihalomethyl, C₁-C₆alkyl or C₁-C₆-alkyloxy, wherein the alkyl groups independently are optionally substituted with one or more of R⁷;

R³ is hydrogen, C₁-C₆alkyl, C₃-C₁₀cycloalkyl, C₁-C₆alkyloxy, C₁-C₆alkylthio, aryl, arylC₁-C₈-alkyl, hetaryl or hetarylalkyl, wherein the alkyl, cycloalkyl, aryl, hetaryl and hetarylalkyl groups independently are optionally substituted with one or more of R⁷;

R⁴ is hydrogen, C₁-C₈alkyl, C₁-C₆alkyloxyC₁-C₆alkyl, aryl, hetaryl, arylC₁-C₆alkyl, arylC₁-C₆alkyloxyC₁-C₆alkyl, C₃-C₁₀cycloalkyl, C₃-C₁₀hetcycloalkyl, C₃-C₁₀cycloalkylC₁-C₆alkyl, C₁-C₆alkylcarboxyC₁-C₆alkyl wherein the alkyl, aryl, hetaryl, cycloalkyl and hetcycloalkyl groups independently are optionally substituted with one or more of R⁷;

R⁵ is hydrogen, and R⁶ is adamantyl optionally substituted with hydroxy, C₁-C₆alkyloxy, aryl, arylC₁-C₆alkyl, aryloxy, arylC₁-C₆alkyloxy, hetaryl, hetaryloxy or hetarylC₁-C₆alkyloxy wherein the alkyl, aryl and hetaryl groups independently are optionally substituted with one or more of R⁷; or

R⁵ and R⁶ are together with the nitrogen to which they are attached, forming a saturated or partially saturated cyclic, bicyclic or tricyclic ring system containing from 5 to 10 carbon atoms and from 0 to 2 additional heteroatoms selected from nitrogen, oxygen or sulphur, the ring system optionally being substituted with at least one of C₁-C₆alkyl, aryl, hetaryl, arylC₁-C₆alkyl, hetarylalkyl, hydroxy, oxo, cyano, C₁-C₆alkyloxy, arylC₁-C₆alkyloxy, hetarylC₁-C₆alkyloxy, C₁-C₆alkyloxyC₁-C₆alkyl, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy or arylC₁-C₆alkylcarboxy wherein the alkyl and aryl groups independently are optionally substituted with one ore more of R⁷;

R⁷ are independently hydrogen, halo, hydroxy, oxo, nitro, NR⁵R⁶, cyano, COOR⁸, CONR⁶R⁵, C₁-C₈alkyl, C₁-C₆alkyloxy, aryloxy or hetaryloxy;

R⁸ is hydrogen, C₁-C₆alkyl, aryl, arylC₁-C₆alkyl, hetarylalkyl, wherein the alkyl, aryl and hetarylalkyl groups independently are optionally substituted with one or more of R⁷; or a salt thereof with a pharmaceutically acceptable acid or base, or any optical isomer or mixture of optical isomers, including a racemic mixture, or any tautomeric forms.

In one embodiment of the present invention, in formula (VIII) X is NR⁴ or S wherein R⁴ is defined as above.

In another embodiment of the present invention, in formula (VIII) X is O.

In another embodiment of the present invention, in formula (VIII) X is S.

In another embodiment of the present invention, in formula (VIII) is NR⁴ wherein R⁴ is defined as above.

In another embodiment of the present invention, in formula (VIII) R¹ and R² independently are hydrogen, halo, trihalomethyl or C₁-C₆alkyl, wherein the alkyl groups independently are optionally substituted with one or more of R⁷.

In another embodiment of the present invention, in formula (VIII) R³ is hydrogen, C₁-C₈alkyl, aryl, arylC₁-C₆alkyl, hetaryl or hetarylalkyl, wherein the alkyl, cycloalkyl, aryl, hetaryl and hetarylalkyl groups independently are optionally substituted with one or more of R⁷.

In another embodiment of the present invention, in formula (VIII) R⁴ is hydrogen, C₁-C₈alkyl, aryl, hetaryl, hetarylC₁-C₆alkyl, arylC₁-C₆alkyl, wherein the alkyl, aryl, hetaryl, groups independently are optionally substituted with one or more of R⁷.

In another embodiment of the present invention, in formula (VIII) R⁵ and R⁶ are together with the nitrogen to which they are attached, forming a saturated or partially saturated cyclic, bicyclic or tricyclic ring system containing from 6 to 10 carbon atoms and from 0 to 2 additional heteroatoms selected from nitrogen, oxygen or sulphur, the ring system optionally being substituted with at least one of C₁-C₆alkyl, aryl, hetaryl, arylC₁-C₆alkyl, hetarylalkyl, hydroxy, oxo, cyano, C₁-C₆alkyloxy, arylC₁-C₆alkyloxy, hetarylC₁-C₆alkyloxy, C₁-C₆alkyloxyC₁-C₆alkyl, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy or arylC₁-C₆alkylcarboxy wherein the alkyl and aryl groups independently are optionally substituted with one ore more of R⁷.

In yet another embodiment of the present invention, in formula (VIII) R⁵ and R⁶, to gether with the nitrogen to which they are attached, are azepane, azocane, 6-aza-bicyclo[3.2.1]octane, 8-aza-bicyclo[3.2.1]octane, 3-aza-bicyclo[3.2.1]octane, 2-aza-bicyclo[3.2.1]octane, 3-oxa-6-aza-bicyclo[3.2.1]octane, 6-aza-bicyclo[3.2.2]nonane, 3-aza-bicyclo[3.2.2]nonane, 4-aza-tricyclo[4.3.1.1^{3,8}]undecane.

In another embodiment of the present invention the compounds of the general formulas (VIII) or a prodrug thereof is selected from the group consisting of :
(4-Methyl-2-phenyl-thiazol-5-yl)-(1,3,3-trimethyl-6-aza-bicyclo[3.2.1]oct-6-yl)-methanone;
(2,4-Dimethyl-thiazol-5-yl)-(1,3,3-trimethyl-6-aza-bicyclo[3.2.1]oct-6-yl)-methanone;
(4-Methyl-2-pyrazin-2-yl-thiazol-5-yl)-(1,3,3-trimethyl-6-aza-bicyclo[3.2.1]oct-6-yl)-methanone;
[4-Methyl-2-(4-trifluoromethyl-phenyl)-thiazol-5-yl]-(1,3,3-trimethyl-6-aza-bicyclo[3.2.1]oct-6-yl)-methanone;
(3-Aza-bicyclo[3.2.2]non-3-yl)-(2,4-dimethyl-thiazol-5-yl)-methanone;
(1H-Imidazol-4-yl)-(1,3,3-trimethyl-6-aza-bicyclo[3.2.1]oct-6-yl)-methanone;
(3-Aza-bicyclo[3.2.2]non-3-yl)-(4-methyl-2-phenyl-thiazol-5-yl)-methanone;
2,4-Dimethyl-thiazole-5-carboxylic acid cycloheptylamide;
Azepan-1-y1-(2,4-dimethyl-thiazol-5-y1)-methanone;
2,4-Dimethyl-thiazole-5-carboxylic acid adamantan-1-ylamide;
(3-Aza-bicyclo[3.2.2]non-3-yl)-(1H-imidazol-4-yl)-methanone;
2,4-Dimethyl-thiazole-5-carboxylic acid (3-trydroxy-adamantan-1-yl)-amide; or
   a salt thereof with a pharmaceutically acceptable acid or base, or any optical isomer or mix ture of optical isomers, including a racemic mixture, or any tautomeric forms.

In another embodiment of the present invention the compounds of the general formulas (VIII) or a prodrug thereof is selected from the group consisting of :
(1-Methyl-1H-imidazol-4-yl)-(1,3,3-trimethyl-6-aza-bicyclo[3.2.1]oct-6-yl)-methanone;
[1-(6-Methyl-pyridin-2-yl)-1H-imidazol-4-yl]-(1,3,3-trimethyl-6-aza-bicyclo[3.2.1]oct-6-yl)-methanone;
[1-(4-Chloro-benzyl)-5-methyl-1H-imidazol-4-yl]-(1,3,3-trimethyl-6-aza-bicyclo[3.2.1]oct-6-yl)-methanone; or
   a salt thereof with a pharmaceutically acceptable acid or base, or any optical isomer or mixture of optical isomers, including a racemic mixture, or any tautomeric forms.

The compounds of the present invention have asymmetric centers and may occur as racemates, racemic mixtures, and as individual enantiomers or diastereoisomers, with all isomeric forms being included in the present invention as well as mixtures thereof.

The present invention also encompasses pharmaceutically acceptable salts of the present compounds. Such salts include pharmaceutically acceptable acid addition salts, pharmaceutically acceptable base addition salts, pharmaceutically acceptable metal salts, ammonium and alkylated ammonium salts. Acid addition salts include salts of inorganic acids as well as organic acids. Representative examples of suitable inorganic acids include hydrochloric, hydrobromic, hydroiodic, phosphoric, sulfuric, nitric acids and the like. Representative examples of suitable organic acids include formic, acetic, trichloroacetic, trifluoroacetic, propionic, benzoic, cinnamic, citric, fumaric, glycolic, lactic, maleic, malic, malonic, mandelic, oxalic, picric, pyruvic, salicylic, succinic, methanesulfonic, ethanesulfonic, tartaric, ascorbic, pamoic, bismethylene salicylic, ethanedisuifonic, gluconic, citraconic, aspartic, stearic, palmitic, EDTA, glycolic, p-aminobenzoic, glutamic, benzenesulfonic, p-toluenesulfonic acids, sulphates, nitrates, phosphates, perchlorates, borates, acetates, benzoates, hydroxynaphthoates, glycerophosphates, ketoglutarates and the like. Further examples of pharmaceutically acceptable inorganic or organic acid addition salts include the pharmaceutical acceptable salts listed in J. Pharm. Sci., 66, 2 (1977), which is incorporated herein by reference. Examples of metal salts include lithium, sodium, potassium, barium, calcium, magnesium, zinc, calcium salts and the like. Examples of amines and organic amines include ammonium, methylamine, dimethylamine, trimethylamine, ethylamine, diethylamine, propylamine, butylamine, tetramethylamine, ethanolamine, diethanolamine, triethanolamine, meglumine, ethylenediamine, choline, N,N'-dibenzylethylenediamine, N-benzylphenylethylamine, N-methyl-D-glucamine, guanidine and the like. Examples of cationic amino acids include lysine, arginine, histidine and the like.

Further, some of the compounds of the present invention may form solvates with water or common organic solvents. Such solvates are encompassed within the scope of the invention.

The pharmaceutically acceptable salts are prepared by reacting a compound of the present invention with 1 to 4 equivalents of a base such as sodium hydroxide, sodium methoxide, sodium hydride, potassium *tert*-butoxide, calcium hydroxide, magnesium hydroxide and the like, in solvents like ether, THF, methanol, *tert*-butanol, dioxane, isopropanol, ethanol etc. Mixtures of solvents may be used. Organic bases like lysine, arginine, diethanolamine, choline, guandine and their derivatives etc. may also be used. Alternatively, acid addition salts wherever applicable are prepared by treatment with acids such as hydrochloric acid, hydrobromic acid, nitric acid, sulfuric acid, phosphoric acid, p-toluenesulphonic acid, methanesulfonic acid, acetic acid, citric acid, maleic acid salicylic acid, hydroxynaphthoic acid, ascorbic acid, palmitic acid, succinic acid, benzoic acid, benzenesulfonic acid, tartaric acid and the like in solvents like ethyl acetate, ether, alcohols, acetone, THF, dioxane etc. Mixture of solvents may also be used.

The stereoisomers of the compounds forming part of this invention may be prepared by using reactants in their single enantiomeric form in the process wherever possible or by conducting the reaction in the presence of reagents or catalysts in their single enantiomer form or by resolving the mixture of stereoisomers by conventional methods. Some of the preferred methods include use of microbial resolution, enzymatic resolution, resolving the diastereomeric salts formed with chiral acids such as mandelic acid, camphorsulfonic acid, tartaric acid, lactic acid, and the like wherever applicable or chiral bases such as brucine, (R)-or (S)-phenylethylamine, cinchona alkaloids and their derivatives and the like. Commonly used methods are compiled by Jaques et al. in "Enantiomers, Racemates and Resolution" (Wiley Interscience, 1981). More specifically the compound of the present invention may be converted to a 1:1 mixture of diastereomeric amides by treating with chiral amines, aminoacids, aminoalcohols derived from aminoacids; conventional reaction conditions may be employed to convert acid into an amide; the diastereomers may be separated either by fractional crystallization or chromatography and the stereoisomers of compound of formula I may be prepared by hydrolysing the pure diastereomeric amide.

Various polymorphs of the compounds forming part of this invention may be prepared by crystallization of said compounds under different conditions. For example, using different solvents commonly used or their mixtures for recrystallization; crystallizations at different temperatures; various modes of cooling, ranging from very fast to very slow cooling during crystallizations. Polymorphs may also be obtained by heating or melting the compound followed by gradual or fast cooling. The presence of polymorphs may be determined by solid probe nmr spectroscopy, ir spectroscopy, differential scanning calorimetry, powder X-ray diffraction or such other techniques.

The invention also encompasses prodrugs of the present compounds, which on administration undergo chemical conversion by metabolic processes before becoming active pharmacological substances. In general such prodrugs will be functional derivatives of the present compounds, which are readily convertible *in vivo* into the required compound of the present invention. Conventional procedures for the selection and preparation of suitable prodrug derivatives are described, for example, in "Design of Prodrugs", ed. H. Bundgaard, Elsevier, 1985.

It is a well known problem in drug discovery that compounds, such as enzyme inhibitors, may be very potent and selective in biochemical assays, yet be inactive *in vivo.* This lack of so-called bioavailability may be ascribed to a number of different factors such as lack of or poor absorption in the gut, first pass metabolism in the liver and/or poor uptake in cells. Although the factors determining bioavailability are not completely understood, there are many examples in the scientific literature - well known to those skilled in the art - of how to modify compounds, which are potent and selective in biochemical assays but show low or no activity *in vivo,* into drugs that are biologically active.

It is within the scope of the invention to modify the compounds of the present invention, termed the 'original compound', by attaching chemical groups that will improve the bioavailability of said compounds in such a way that the uptake in cells or mammals is facilitated.

Examples of said modifications, which are not intended in any way to limit the scope of the invention, include changing of one or more carboxy groups to esters (for instance methyl esters, ethyl esters, *tert*-butyl, acetoxymethyl, pivaloyloxymethyl esters or other acyloxymethyl esters). Compounds of the invention, original compounds, such modified by attaching chemical groups are termed 'modified compounds'.

The invention also encompasses active metabolites of the present compounds.

The compounds according to the invention alter, and more specifically, reduce the level of active intracellular glucocorticoid and are accordingly useful for the treatment, prevention and/or prophylaxis of disorders and diseases in which such a modulation or reduction is beneficial.

Accordingly, the present compounds may be applicable for the treatment, prevention and/or prophylaxis of the metabolic syndrome, insulin resistance, dyslipidemia, hypertension, obesity, type 2 diabetes, impaired glucose tolerance (IGT), impaired fasting glucose (IFG), Latent Autoimmune Diabetes in the Adult (LADA), type 1 diabetes, diabetic late complications including cardiovascular diseases, cardiovascular disorders, disorders of lipid metabolism, neurodegenerative and psychiatric disorders, dysregulation of intraocular pressure including glaucoma, immune disorders, inappropriate immune responses, musculo-skeletal disorders, gastrointestinal disorders, polycystic ovarie syndrome (PCOS), reduced hair growth or other diseases, disorders or conditions that are influenced by intracellular glucocorticoid levels, adverse effects of increased blood levels of active endogenous or exogenous glucocorticoid, and any combination thereof, adverse effects of increased plasma levels of endogenous active glucocorticoid, Cushing's disease, Cushing's syndrome, adverse effects of glucocorticoid receptor agonist treatment of autoimmune diseases, adverse effects of glucocorticoid receptor agonist treatment of inflammatory diseases, adverse effects of glucocorticoid receptor agonist treatment of diseases with an inflammatory component, adverse effects of glucocorticoid receptor agonist treatment as a part of cancer chemotherapy, adverse effects of glucocorticoid receptor agonist treatment for surgical/post-surgical or other trauma, adverse effects of glucocorticoid, receptor agonist therapy in the context of organ or tissue transplantation or adverse effects of glucocorticoid receptor agonist treatment in other diseases, disorders or conditions where glucocorticoid receptor agonists provide clinically beneficial effects.

More specifically the present compounds may be applicable for the treatment, prevention and/or prophylaxis of the metabolic syndrome, type 2 diabetes, diabetes as a consequence of obesity, insulin resistance, hyperglycemia, prandial hyperglycemia, hyperinsulinemia, inappropriately low insulin secretion, impaired glucose tolerance (IGT), impaired fasting glucose (IFG), increased hepatic glucose production, type 1 diabetes, LADA, pediatric diabetes, dyslipidemia, diabetic dyslipidemia, hyperlipidemia, hypertriglyceridemia, hyperlipoproteinemia, hypercholesterolemia, decreased HDL cholesterol, impaired LDL/HDL ratio, other disorders of lipid metabolism, obesity, visceral obesity, obesity as a consequence of diabetes, increased food intake, hypertension, diabetic late complications, micro-/macroalbuminuria, nephropathy, retinopathy, neuropathy, diabetic ulcers, cardiovascular diseases, arteriosclerosis, atherosclerosis, coronary artery disease, cardiac hypertrophy, myocardial ischemia, heart insufficiency, congestional heart failure, stroke, myocardial infarction, arrythmia, decreased blood flow, erectile dysfunction (male or female), myopathy, loss of muscle tissue, muscle wasting, muscle catabolism, osteoporosis, decreased linear growth, neurodegenerative and psychiatric disorders, Alzheimers disease, neuronal death, impaired cognitive function, depression, anxiety, eating disorders, appetite regulation, migraine, epilepsia, addiction to chemical substances, disorders of intraocular pressure, glaucoma, polycystic ovary syndrome (PCOS), inappropriate immune responses, inappropriate T helper-1/T helper-2 polarisation, bacterial infections, mycobacterial infections, fungal infections, viral infections, parasitic infestations, suboptimal responses to immunizations, immune dysfunction, partial or complete baldness, or other diseases, disorders or conditions that are influenced by intracellular glucocorticoid levels and any combination thereof, adverse effects of glucocorticoid receptor agonist treatment of allergic-inflammatory diseases such as asthma and atopic dermatitis, adverse effects of glucocorticoid receptor agonist treatment of disorders of the respiratory system e.g. asthma, cystic fibrosis, emphysema, bronchitis, hypersensitivity, pneumonitis, eosinophilic pneumonias, pulmonary fibrosis, adverse effects of glucocorticoid receptor agonist treatment of inflammatory bowel disease such as Crohn's disease and ulcerative colitis; adverse effects of glucocorticoid receptor agonist treatment of disorders of the immune system, connective tissue and joints e.g. reactive arthritis, rheumatoid arthritis, Sjögren's syndrome, systemic lupus erythematosus, lupus nephritis, Henoch-Schoniein purpura, Wegener's granulomatosis, temporal arteritis, systemic sclerosis, vasculitis, sarcoidosis, dermatomyositis-poiymyositis, pemphigus vulgaris; adverse effects of glucocorticoid receptor agonist treatment of endocrinological diseases such as hyperthyroidism, hypoaldosteronism, hypopituitarism; adverse effects of glucocorticoid receptor agonist treatment of hematological diseases e.g. hemolytic anemia, thrombocytopenia, paroxysmal nocturnal hemoglobinuria; adverse effects of glucocorticoid receptor agonist treatment of cancer such as spinal cord diseases, neoplastic compression of the spinal cord, brain tumours, acute lymphoblastic leukemia, Hodgkin's disease, chemotherapy-induced nausea, adverse effects of glucocorticoid receptor agonist treatment of diseases of muscle and at the neuro-muscular joint e.g. myasthenia gravis and heriditary myopathies (e.g. Duchenne muscular dystrophy), adverse effects of glucocorticoid receptor agonist treatment in the context of surgery & transplantation e.g. trauma, post-surgical stress, surgical stress, renal transplantation, liver transplantation, lung transplantation, pancreatic islet transplantation, blood stem cell transplantation, bone marrow transplantation, heart transplantation, adrenal gland transplantation, tracheal transplantation, intestinal transplantation, corneal transplantation, skin grafting, Keratoplasty, lens implantation and other procedures where immunosuppression with glucocorticoid receptor agonists is beneficial; adverse effects of glucocorticoid receptor agonist treatment of brain absess, nausea/vomiting, infections, hypercalcemia, adrenal hyperplasia, autoimmune hepatitis, spinal cord diseases, saccular aneurysms or adverse effects to glucocorticoid receptor agonist treatment in other diseases, disorders and conditions where glucocorticoid receptor agonists provide clinically beneficial effects.

Accordingly, in a further aspect the invention relates to a compound according to the invention for use as a pharmaceutical composition.

The invention also relates to pharmaceutical compositions comprising, as an active ingredient, at least one compound according to the invention together with one or more pharmaceutically acceptable carriers or diluents.

The pharmaceutical composition is preferably in unit dosage form, comprising from about 0.05 mg/day to about 2000 mg/day, preferably from about 1 mg/day to about 500 mg/day of a compound according to the invention.

In another embodiment, the patient is treated with a compound according to the invention for at least about 1 week, for at least about 2 weeks, for at least about 4 weeks, for at least about 2 months or for at least about 4 months.

In yet another embodiment, the pharmaceutical composition is for oral, nasal, transdermal, pulmonal or parenteral administration.

Furthermore, the invention relates to the use of a compound according to the invention for the preparation of a pharmaceutical composition for the treatment, prevention and/or prophylaxis of disorders and diseases wherein a modulation or an inhibition of the activity of 11βHSD1 is beneficial.

The invention also relates to a method for the treatment, prevention and/or prophylaxis of disorders and diseases wherein a modulation or an inhibition of the activity of 11βHSD1 is beneficial, the method comprising administering to a subject in need thereof an effective amount of a compound according to the invention.

In a preferred embodiment of the invention the present compounds are used for the preparation of a medicament for the treatment, prevention and/or prophylaxis of any diseases and conditions that are influenced by intracellular glucocorticoid levels as mentioned above.

Thus, in a preferred embodiment of the invention the present compounds are used for the preparation of a medicament for the treatment, prevention and/or prophylaxis of conditions and disorders where a decreased level of active intracellular glucocorticoid is desirable, such as the conditions and diseases mentioned above.

In yet a preferred embodiment of the invention the present compounds are used for the preparation of a medicament for the treatment, prevention and/or prophylaxis of the metabolic syndrome including insulin resistance, dyslipidemia, hypertension and obesity.

In yet another preferred embodiment of the invention the present compounds are used for the preparation of a medicament for the treatment, prevention and/or prophylaxis of type 2 diabetes, impaired glucose tolerance (IGT), impaired fasting glucose (IFG).

In yet another preferred embodiment of the invention the present compounds are used for the preparation of a pharmaceutical composition for the delaying or prevention of the progression from IGT to type 2 diabetes.

In yet another preferred embodiment of the invention the present compounds are used for the preparation of a pharmaceutical composition for the delaying or prevention of the progression of the metabolic syndrome into type 2 diabetes.

In still another preferred embodiment of the invention the present compounds are used for the preparation of a pharmaceutical composition for the treatment, prevention and/or prophylaxis of diabetic late complications including cardiovascular diseases; arteriosclerosis; atherosclerosis.

In a further preferred embodiment of the invention the present compounds are used for the preparation of a pharmaceutical composition for the treatment, prevention and/or prophylaxis of neurodegenerative and psychiatric disorders.

In yet a further preferred embodiment of the invention the present compounds are used for the preparation of a pharmaceutical composition for the treatment, prevention and/or prophylaxis of adverse effects of glucocorticoid receptor agonist treatment or therapy

In another embodiment of the present invention, the route of administration may be any route which effectively transports a compound according to the invention to the appropriate or desired site of action, such as oral, nasal, buccal, transdermal, pulmonal, or parenteral.

In still a further aspect of the invention the present compounds are administered in combination with one or more further active substances in any suitable ratios. Such further active substances may e.g. be selected from antiobesity agents, antidiabetics, agents modifying the lipid metabolism, antihypertensive agents, glucocorticoid receptor agonists, agents for the treatment and/or prevention of complications resulting from or associated with diabetes and agents for the treatment and/or prevention of complications and disorders resulting from or associated with obesity.

Thus, in a further aspect of the invention the present compounds may be administered in combination with one or more antiobesity agents or appetite regulating agents.

Such agents may be selected from the group consisting of CART (cocaine amphetamine regulated transcript) agonists, NPY (neuropeptide Y) antagonists, MC4 (melanocortin 4) agonists, orexin antagonists, TNF (tumor necrosis factor) agonists, CRF (corticotropin releasing factor) agonists, CRF BP (corticotropin releasing factor binding protein) antagonists, urocortin agonists, β3 agonists, MSH (melanocyte-stimulating hormone) agonists, MCH (melanocyte-concentrating hormone) antagonists, CCK (cholecystokinin) agonists, serotonin re-uptake inhibitors, serotonin and noradrenaline re-uptake inhibitors, mixed serotonin and noradrenergic compounds, 5HT (serotonin) agonists, bombesin agonists, galanin antagonists, growth hormone, growth hormone releasing compounds, TRH (thyreotropin releasing hormone) agonists, UCP 2 or 3 (uncoupling protein 2 or 3) modulators, leptin agonists, DA agonists (bromocriptin, doprexin), lipase/amylase inhibitors, PPAR (peroxisome proliferator-activated receptor) modulators, RXR (retinoid X receptor) modulators, TR β agonists, AGRP (Agouti related protein) inhibitors, H3 histamine antagonists, opioid antagonists (such as naltrexone), exendin-4, GLP-1 and ciliary neurotrophic factor.

In one embodiment of the invention the antiobesity agent is leptin; dexamphetamine or amphetamine; fenfluramine or dexfenfluramine; sibutramine; orlistat; mazindol or phentermine.

Suitable antidiabetic agents include insulin, insulin analogues and derivatives such as those disclosed in EP 792 290 (Novo Nordisk A/S), e.g. N^{εB29}-tetradecanoyl des (B30) human insulin, EP 214 826 and EP 705 275 (Novo Nordisk A/S), e.g. Asp^{B28} human insulin, US 5,504,188 (Eli Lily), e.g. Lys^{B2B} Pro^{B29} human insulin, EP 368 187 (Aventis), eg Lantus, which are all incorporated herein by reference, GLP-1 (glucagon like peptide-1) and GLP-1 derivatives such as those disclosed in WO 98/08871 to Novo Nordisk A/S, which is incorporated herein by reference as well as orally active hypoglycaemic agents.

The orally active hypoglycaemic agents preferably comprise sulphonylureas, biguanides, meglitinides, glucosidase inhibitors, glucagon antagonists such as those disclosed in WO 99/01423 to Novo Nordisk A/S and Agouron Pharmaceuticals, Inc., GLP-1 agonists, potassium channel openers such as those disclosed in WO 97/26265 and WO 99/03861 to Novo Nordisk A/S which are incorporated herein by reference, DPP-IV (dipeptidyl peptidase-IV) inhibitors; inhibitors of hepatic enzymes involved in stimulation of gluconeogenesis and/or glycogenolysis, glucose uptake modulators, compounds modifying the lipid metabolism such as antihypertipidemic agents and antilipidemic agents as PPARα modulators, PPARδ modulators, cholesterol absorption inhibitors, HSL (hormone-sensitive lipase) inhibitors and HMG CoA inhibitors (statins), nicotinic acid, fibrates, anion exchangers, compounds lowering food intake, bile acid resins, RXR agonists and agents acting on the ATP-dependent potassium channel of the β-cells.

In one embodiment, the present compounds are administered in combination with insulin or an insulin analogue or derivative, such as N^{εB29}-tetradecanoyl des (B30) human insulin, Asp^{B28} human insulin, Lys^{B28} Pro^{B29} human insulin, Lantus®, or a mix-preparation comprising one or more of these.

In a further embodiment the present compounds are administered in combination with a sulphonylurea e.g. tolbutamide, glibenclamide, glipizide or glicazide.

In another embodiment the present compounds are administered in combination with a biguanide e.g. metformin.

In yet another embodiment the present compounds are administered in combination with a meglitinide e.g. repaglinide or senaglinide.

In still another embodiment the present compounds are administered in combination with a thiazolidinedione e.g. troglitazone, ciglitazone, pioglitazone, rosiglitazone or compounds disclosed in WO 97/41097 such as 5-[[4-[3-Methyl-4-oxo-3,4-dihydro-2-quinazolinyl]methoxy]phenyl-methyl]thiazolidine-2,4-dione or a pharmaceutically acceptable salt thereof, preferably the potassium salt.

In yet another embodiment the present compounds may be administered in combination with the insulin sensitizers disclosed in WO 99/19313 such as (-) 3-[4-[2-Phenoxazin-10-yl)ethoxy]phenyl]-2-ethoxypropanoic acid or a pharmaceutically acceptable salts thereof, preferably the arginine salt.

In a further embodiment the present compounds are administered in combination with an α-glucosidase inhibitor e.g. miglitol or acarbose.

In another embodiment the present compounds are administered in combination with an, agent acting on the ATP-dependent potassium channel of the β-cells e.g. tolbutamide, glibenclamide, glipizide, glicazide or repaglinide.

Furthermore, the present compounds may be administered in combination with nateglinide.

In still another embodiment the present compounds are administered in combination with an antihyperlipidemic agent or antilipidemic agent e.g. cholestyramine, colestipol, clofibrate, gemfibrozil, fenofibrate, bezafibrate, tesaglitazar, EML-4156, LY-818, MK-767, atorvastatin, fluvastatin, lovastatin, pravastatin, simvastatin, acipimox, probucol, ezetimibe or dextrothyroxine.

In a further embodiment the present compounds are administered in combination with more than one of the above-mentioned compounds e.g. in combination with a sulphonylurea and metformin, a sulphonylurea and acarbose, repaglinide and metformin, insulin and a sulphonylurea, insulin and metformin, insulin, insulin and lovastatin, etc.

Further, the present compounds may be administered in combination with one or more antihypertensive agents. Examples of antihypertensive agents are β-blocker such as alprenolol, atenolol, timolol, pindolol, propranolol, metoprolol, bisoprololfumerate, esmolol, acebutelot, metoprolol, acebutolol, betaxolol, celiprolol, nebivolol, tertatolol, oxprenolol, amusolalul, carvedilol, labetalol, β2-receptor blockers e.g. S-atenolol, OPC-1085, ACE (angiotensin converting enzyme) inhibitors such as quinapril, lisinopril, enalapril, captopril, benazepril, perindopril, trandolapril, fosinopril, ramipril, cilazapril, delapril, imidapril, moexipril, spirapril, temocapril, zofenopril, S-5590, fasidotril, Hoechst-Marion Roussel: 100240 (EP 00481522), omapatrilat, gemopatrilat and GW-660511, calcium channel blockers such as nifedipine, felodipine, nicardipine, isradipine, nimodipine, diltiazem, amlodipine, nitrendipine, verapamil, lacidipine, lercanidipine, aranidipine, cilnidipine, clevidipine, azeinidipine, barnidipine, efonodipine, iasidipine, lemildipine, iercanidipine, manidipine, nilvadipine, pranidipine, fumidipine, α-blockers such as doxazosin, urapidil, prazosin, terazosin, bunazosin and OPC-28326, diuretics such as thiazides/sulphonamides (e.g. bendroflumetazide, chlorothalidone, hydrochlorothiazide and clopamide), loop-diuretics (e.g. bumetanide, furosemide and torasemide) and potassium sparing diuretics (e.g. amiloride, spironolactone), endothelin ET-A antagonists such as ABT-546, ambrisetan, atrasentan, SB-234551, Cl-1034, S-0139 and YM-598, endothelin antagonists e.g. bosentan and J-104133, renin inhibitors such as aliskiren, vasopressin V1 antagonists e.g. OPC-21288, vasopressin V2 antagonists such as tolvaptan, SR-121463 and OPC-31260, B-type natriuretic peptide agonists e.g. Nesiritide, angiotensin II antagonists such as irbesartan, candesartancilexetil, losartan, valsartan, telmisartan, eprosartan, candesartan, CL-329167, eprosartan, iosartan, olmesartan, pratosartan, TA-606, and YM-358, 5-HT2 agonists e.g. fenoldopam and ketanserin, adenosine A1 antagonists such as naftopidil, N-0861 and FK-352, thromboxane A2 antagonists such as KT2-962, endopeptidase inhibitors e.g. ecadotril, nitric oxide agonists such as LP-805, dopamine D1 antagonists e.g. MYD-37, dopamine D2 agonists such as nolomirole, n-3 fatty acids e.g. omacor, prostacyclin agonists such as treprostinil, beraprost, PGE1 agonists e.g. ecraprost, Na+/K+ ATPase modulators e.g. PST-2238, Potassium channel activators e.g. KR-30450, vaccines such as PMD-3117, Indapamides, CGRP-unigene, guanylate cyclase stimulators, hydralazines, methyldopa, docarpaimine, moxonidine, CoAprovel, MondoBiotech-811.

Further reference can be made to Remington: The Science and Practice of Pharmacy, 19th Edition, Gennaro, Ed., Mack Publishing Co., Easton, PA, 1995.

Furthermore, the present compounds may be administered in combination with one or more glucocorticoid receptor agonists. Examples of such glucocorticoid receptor agonists are betametasone, dexamethasone, hydrocortisone, methylprednisolone, prednisolone, prednisone, beclomethasone, butixicort, clobetasol, flunisolide, flucatisone (and analogues), momethasone, triamcinolonacetonide, triamcinolonhexacetonide GW-685698, NXG-1015, NXC-1020, NXC-1021, NS-126, P-4112, P-4114, RU-24858 and T-25 series.

It should be understood that any suitable combination of the compounds according to the invention with one or more of the above-mentioned compounds and optionally one or more further pharmacologically active substances are considered to be within the scope of the present invention.

### PHARMACEUTICAL COMPOSITIONS

The compounds of the present invention may be administered alone or in combination with pharmaceutically acceptable carriers or excipients, in either single or multiple doses. The pharmaceutical compositions according to the invention may be formulated with pharmaceutically acceptable carriers or diluents as well as any other known adjuvants and excipients in accordance with conventional techniques such as those disclosed in Remington: The Science and Practice of Pharmacy, 19th Edition, Gennaro, Ed., Mack Publishing Co., Easton, PA, 1995.

The pharmaceutical compositions may be specifically formulated for administration by any suitable route such as theoral, rectal, nasal, pulmonary, topical (including buccal and sublingual), transdermal, intracisternal, intraperitoneal, vaginal and parenteral (including subcutaneous, intramuscular, intrathecal, intravenous and intradermal) route, the oral route being preferred. It will be appreciated that the preferred route will depend on the general condition and age of the subject to be treated, the nature of the condition to be treated and the active ingredient chosen.

Pharmaceutical compositions for oral administration include solid dosage forms such as hard or soft capsules, tablets, troches, dragees, pills, lozenges, powders and granules. Where appropriate, they can be prepared with coatings such as enteric coatings or they can be formulated so as to provide controlled release of the active ingredient such as sustained or prolonged release according to methods well-known in the art.

Liquid dosage forms for oral administration include solutions, emulsions, suspensions, syrups and elixirs.

Pharmaceutical compositions for parenteral administration include sterile aqueous and non-aqueous injectable solutions, dispersions, suspensions or emulsions as well as sterile powders to be reconstituted in sterile injectable solutions or dispersions prior to use. Depot injectable formulations are also contemplated as being within the scope of the present invention.

Other suitable administration forms include suppositories, sprays, ointments, crèmes, gels, inhalants, dermal patches, implants etc.

A typical oral dosage is in the range of from about 0.001 to about 100 mg/kg body weight per day, preferably from about 0.01 to about 50 mg/kg body weight per day, and more preferred from about 0.05 to about 10 mg/kg body weight per day administered in one or more dosages such as 1 to 3 dosages. The exact dosage will depend upon the frequency and mode of administration, the sex, age, weight and general condition of the subject treated, the nature and severity of the condition treated and any concomitant diseases to be treated and other factors evident to those skilled in the art.

The formulations may conveniently be presented in unit dosage form by methods known to those skilled in the art. A typical unit dosage form for oral administration one or more times per day such as 1 to 3 times per day may contain from 0.05 to about 2000 mg, e.g. from about 0.1 to about 1000 mg, from about 0.5 mg to about 500 mg., from about 1 mg to about 200 mg, e.g. about 100 mg.

For parenteral routes, such as intravenous, intrathecal, intramuscular and similar administration, typically doses are in the order of about half the dose employed for oral administration.

The compounds of this invention are generally utilized as the free substance or as a pharmaceutically acceptable salt thereof. Examples are an acid addition salt of a compound having the utility of a free base and a base addition salt of a compound having the utility of a free acid. The term "pharmaceutically acceptable salts" refers to non-toxic salts of the compounds for use according to the present invention which are generally prepared by reacting the free base with a suitable organic or inorganic acid or by reacting the acid with a suitable organic or inorganic base. When a compound for use according to the present invention, contains a free base such salts are prepared in a conventional manner by treating a solution or suspension of the compound with a chemical equivalent of a pharmaceutically acceptable acid. When a compounds for use according to the present invention, contains a free acid such salts are prepared in a conventional manner by treating a solution or suspension of the compound with a chemical equivalent of a pharmaceutically acceptable base. Physiologically acceptable salts of a compound with a hydroxy group include the anion of said compound in combination with a suitable cation such as sodium or ammonium ion. Other salts which are not pharmaceutically acceptable may be useful in the preparation of compounds for use according to the present invention and these form a further aspect of the present invention.

For parenteral administration, solutions of the present compounds in sterile aqueous solution, aqueous propylene glycol or sesame or peanut oil may be employed. Such aqueous solutions should be suitable buffered if necessary and the liquid diluent first rendered isotonic with sufficient saline or glucose. The aqueous solutions are particularly suitable for intravenous, intramuscular, subcutaneous and intraperitoneal administration. The sterile aqueous media employed are all readily available by standard techniques known to those skilled in the art.

Suitable pharmaceutical carriers include inert solid diluents or fillers, sterile aqueous solution and various organic solvents. Examples of suitable carriers are water, salt solutions, alcohols, polyethylene glycols, polyhydroxyethoxylated castor oil, peanut oil, olive oil, syrup, phospholipids, gelatine, lactose, terra alba, sucrose, cyclodextrin, amylose, magnesium stearate, talc, gelatin, agar, pectin, acacia, stearic acid or lower alkyl ethers of cellulose, silicic acid, fatty acids, fatty acid amines, fatty acid monoglycerides and diglycerides, pentaerythritol fatty acid esters, polyoxyethylene, hydroxymethylcellulose and polyvinylpyrrolidone. Similarly the carrier or diluent may include any sustained release material known in the art, such as glyceryl monostearate or glyceryl distearate, alone or mixed with a wax. The formulations may also include wetting agents, emulsifying and suspending agents, preserving agents, sweetening agents or flavouring agents.

The pharmaceutical compositions formed by combining the compounds of the invention and the pharmaceutically acceptable carriers are then readily administered in a variety of dosage forms suitable for the disclosed routes of administration. The formulations may conveniently be presented in unit dosage form by methods known in the art of pharmacy.

Formulations of the present invention suitable for oral administration may be presented as discrete units such as capsules or tablets, each containing a predetermined amount of the active ingredient, and which may include a suitable excipient. These formulations may be in the form of powder or granules, as a solution or suspension in an aqueous or non-aqueous liquid, or as an oil-in-water or water-in-oil liquid emulsion.

Compositions intended for oral use may be prepared according to any known method, and such compositions may contain one or more agents selected from the group consisting of sweetening agents, flavouring agents, colouring agents, and preserving agents in order to provide pharmaceutically elegant and palatable preparations. Tablets may contain the active ingredient in admixture with non-toxic pharmaceutically acceptable excipients which are suitable for the manufacture of tablets. These excipients may be for example, inert diluents, such as calcium carbonate, sodium carbonate, lactose, calcium phosphate or sodium phosphate; granulating and disintegrating agents, for example corn starch or alginic acid; binding agents, for example, starch, gelatine or acacia; and lubricating agents, for example magnesium stearate, stearic acid or talc. The tablets may be uncoated or they may be coated by known techniques to delay disintegration and absorption in the gastrointestinal tract and thereby provide a sustained action over a longer period. For example, a time delay material such as glyceryl monostearate or glyceryl distearate may be employed. They may also be coated by the techniques described in U.S. Patent Nos. 4,356,108; 4,166,452; and 4,265,874, incorporated herein by reference, to form osmotic therapeutic tablets for controlled release.

Formulation for oral use may also be presented as hard gelatine capsules where the active ingredient is mixed with an inert solid diluent, for example, calcium carbonate, calcium phosphate or kaolin, or a soft gelatine capsule wherein the active ingredient is mixed with water ar an oil medium, for example peanut oil, liquid paraffin, or olive oil.

Aqueous suspensions may contain the active compounds in admixture with excipients suitable for the manufacture of aqueous suspensions. Such excipients are suspending agents, for example sodium carboxymethylcellulose, methylcellulose, hydroxypropylmethylcellulose, sodium alginate, polyvinylpyrrolidone, gum tragacanth and gum acacia; dispersing or wetting agents may be a naturally-occurring phosphatide such as lecithin, or condensation products of an alkylene oxide with fatty acids, for example polyoxyethylene stearate, or condensation products of ethylene oxide with long chain aliphatic alcohols, for example, heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more colouring agents, one or more flavouring agents, and one or more sweetening agents, such as sucrose or saccharin.

Oily suspensions may be formulated by suspending the active ingredient in a vegetable oil, for example arachis oil, olive oil, sesame oil or coconut oil, or in a mineral oil such as a liquid paraffin. The oily suspensions may contain a thickening agent, for example beeswax, hard paraffin or cetyl alcohol. Sweetening agents such as those set forth above, and flavouring agents may be added to provide a palatable oral preparation. These compositions may be preserved by the addition of an anti-oxidant such as ascorbic acid.

Dispersible powders and granules suitable for preparation of an aqueous suspension by the addition of water provide the active compound in admixture with a dispersing or wetting agent, suspending agent and one or more preservatives. Suitable dispersing or wetting agents and suspending agents are exemplified by those already mentioned above. Additional excipients, for example, sweetening, flavouring, and colouring agents may also be present.

The pharmaceutical compositions comprising a compound for use according to the present invention may also be in the form of oil-in-water emulsions. The oily phase may be a vegetable oil, for example, olive oil or arachis oil, or a mineral oil, for example a liquid paraffin, or a mixture thereof. Suitable emulsifying agents may be naturally-occurring gums, for example gum acacia or gum tragacanth, naturally-occurring phosphatides, for example soy bean, lecithin, and esters or partial esters derived from fatty acids and hexitol anhydrides, for example sorbitan monooleate, and condensation products of said partial esters with ethylene oxide, for example polyoxyethylene sorbitan monooleate. The emulsions may also contain sweetening and flavouring agents.

Syrups and elixirs may be formulated with sweetening agents, for example glycerol, propylene glycol, sorbitol or sucrose. Such formulations may also contain a demulcent, preservative and flavouring and colouring agent. The pharmaceutical compositions may be in the form of a sterile injectable aqueous or oleaginous suspension. This suspension may be formulated according to the known methods using suitable dispersing or wetting agents and suspending agents described above. The sterile injectable preparation may also be a sterile injectable solution or suspension in a non-toxic parenterally-acceptable diluent or solvent; for example as a solution in 1,3-butanediol. Among the acceptable vehicles and solvents that may be employed are water, Ringer's solution, and isotonic sodium chloride solution. In addition, sterile, fixed oils are conveniently employed as solvent or suspending medium. For this purpose, any bland fixed oil may be employed using synthetic mono- or diglycerides. In addition, fatty acids such as oleic acid find use in the preparation of injectables.

The compositions may also be in the form of suppositories for rectal administration of the compounds of the present invention. These compositions can be prepared by mixing the drug with a suitable non-irritating excipient which is solid at ordinary temperatures but liquid at the rectal temperature and will thus melt in the rectum to release the drug. Such materials include cocoa butter and polyethylene glycols, for example.

For topical use, creams, ointments, jellies, solutions of suspensions, etc., containing the compounds of the present invention are contemplated. For the purpose of this application, topical applications shall include mouth washes and gargles.

The compounds for use according to the present invention may also be administered in the form of liposome delivery systems, such as small unilamellar vesicles, large unilamellar vesicles, and multilamellar vesicles. Liposomes may be formed from a variety of phospholipids, such as cholesterol, stearylamine, or phosphatidylcholines.

In addition, some of the compounds for use according to the present invention may form solvates with water or common organic solvents. Such solvates are also encompassed within the scope of the present invention.

Thus, in a further embodiment, there is provided a pharmaceutical composition comprising a compound for use according to the present invention, or a pharmaceutically acceptable salt, solvate, or prodrug thereof, and one or more pharmaceutically acceptable carriers, excipients, or diluents.

If a solid carrier is used for oral administration, the preparation may be tabletted, placed in a hard gelatine capsule in powder or pellet form or it can be in the form of a troche or lozenge. The amount of solid carrier will vary widely but will usually be from about 25 mg to about 1 g. If a liquid carrier is used, the preparation may be in the form of a syrup, emulsion, soft gelatine capsule or sterile injectable liquid such as an aqueous or non-aqueous liquid suspension or solution.

A typical tablet which may be prepared by conventional tabletting techniques may contain:

| Core: | |
|---|---|
| Active compound (as free compound or salt thereof) | 5.0 mg |
| Lactosum Ph. Eur. | 67.8 mg |
| Cellulose, microcryst. (Avicel) | 31.4 mg |
| Amberlite®IRP88* | 1.0 mg |
| Magnesii stearas Ph. Eur. | q.s. |

| Coating: | |
|---|---|
| Hydroxypropyl methylcellulose | approx. 9 mg |
| Mywacett 9-40 T** | approx. 0.9 mg |

| | |
|---|---|
| * Polacrillin potassium NF, tablet disintegrant, Rohm and Haas. ** Acylated monoglyceride used as plasticizer for film coating. | |

The compounds of the invention may be administered to a patient which is a mammal, especially a human in need thereof. Such mammals include also animals, both domestic animals, e.g. household pets, and non-domestic animals such as wildlife.

Any novel feature or combination of features described herein is considered essential to this invention.

The present invention also relate to the below methods of preparing the compounds of the invention.

The present invention is further illustrated in the following representative examples which are, however, not intended to limit the scope of the invention in any way.

### EXAMPLES, COMPOUNDS OF GENERAL FORMULAS (I) AND (II)

The following examples and general procedures refer to intermediate compounds and final products for general formula (I) and (II) identified in the specification and in the synthesis schemes. The preparation of the compounds of general formula (I) and (II) of the present invention is described in detail using the following examples. Occasionally, the reaction may not be applicable as described to each compound included within the disclosed scope of the invention. The compounds for which this occurs will be readily recognised by those skilled in the art. In these cases the reactions can be successfully performed by conventional modifications known to those skilled in the art, which is, by appropriate protection of interfering groups, by changing to other conventional reagents, or by routine modification of reaction conditions. Alternatively, other reactions disclosed herein or otherwise conventional will be applicable to the preparation of the corresponding compounds of the invention. In all preparative methods, all starting materials are known or may easily be prepared from known starting materials. The structures of the compounds are confirmed by either elemental analysis or nuclear magnetic resonance (NMR), where peaks assigned to characteristic protons in the title compounds are presented where appropriate. ¹H NMR shifts (δ_{H}) are given in parts per million (ppm) down field from tetramethylsilane as internal reference standard. M.p.: is melting point and is given in °C and is not corrected. Column chromatography was carried out using the technique described by W.C. Still et al., J. Org. Chem. 43: 2923 (1978) on Merck silica gel 60 (Art. 9385). HPLC analyses are performed using 5µm C18 4 x 250 mm column eluted with various mixtures of water and acetonitrile, flow = 1 ml/min, as described in the experimental section.

**Microwave oven synthesis:** The reaction was heated by microwave irradiation in sealed microwave vessels in a single mode Emrys Optimizer EXP from PersonalChemistry®.

**Preparative HPLC: Column:** 1.9 x 15 cm Waters XTerra RP-18. Buffer: linear gradient 5 - 95 % in 15 min, MeCN, 0.1 % TFA, flow rate of 15 ml/min. The pooled fractions are either evaporated to dryness *in vacuo,* or evaporated *in vacuo* until the MeCN is removed, and then frozen and freeze dried.

The abbreviations as used in the examples have the following meaning:

| | |
|---|---|
| TLC: | Thin layer chromatography |
| CDCl₃: | Deuterio chloroform |
| CD₃OD: | Tetradeuterio methanol |
| DCM: | Dichloromethane |
| DMF: | N,N-dimethylformamide |
| DMSO-d₆: | Hexadeuterio dimethylsulfoxide |
| DMSO: | Dimethylsulfoxide |
| DIPEA: | Diisopropylethylamine |
| EDAC: | 1-(3-Dimethylaminopropyl)-3-ethylcarbodiimide hydrochloride |
| EtOAc: | Ethyl acetate |
| THF: | Tetrahydrofuran |
| DMF: | N,N-dimethylformamide |
| HOBT: | 1-Hydroxy-benzotriazole |
| MeCN: | Acetonitrile |
| NMP: | N-Methylpyrrolidinone |
| TFA: | Trifluoroacetic acid |
| EDAC: | 1-(3-Dimethylaminopropyl)-3-ethylcarbodiimide, hydrochloride |
| min: | minutes |
| hrs: | hours |

### General method A:

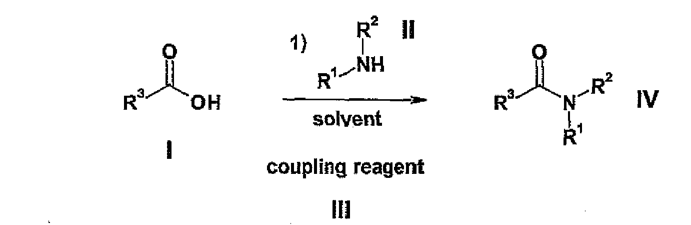

By allowing an acid (I) wherein R³ is defined as above to be coupled with an amine (II) wherein R¹ and R² are defined as above under standard amide forming conditions using a coupling reagent (III) (e.g. HOBT, EDAC and DIPEA in dry THF) affording amide (IV) wherein R¹, R² and R³ are defined as above.

### General method B:

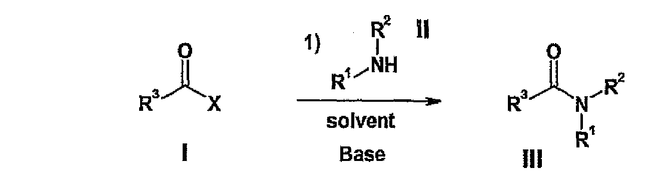

By allowing an acid derivative (I) wherein X is halo, R³(C=O)O-, C₁-C₆alkyloxy or arylC₁-C₆alkyloxy and R³ are defined as above to react with an amine (II) wherein R¹ and R² are defined as above under basic conditions (e.g. triethylamine, K₂CO₃, NaH and the like) in a solvent (e.g. THF, DCM, DMF, NMP and the like) affording amide (III); wherein R¹, R² and R³ are defined as above.

### General method C:

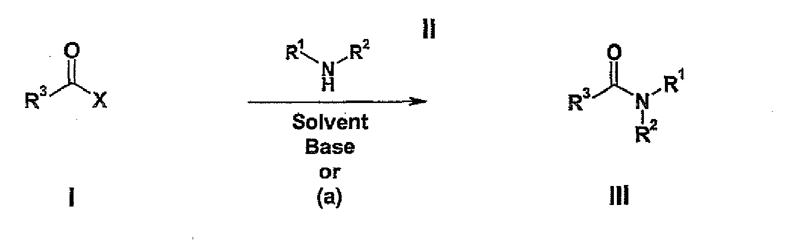

By allowing an acid derivative (I) wherein X is halo, R²⁰(C=O)O-, C₁-C₆alkyloxy or arylC₁-C₆alkyloxy, R²⁰ is C₁-C₆alkyl or arylC₁-C₆alkyl and R³ and X are defined as above to react with an amine **(II)** wherein R¹ and R² are defined as above under basic conditions (e.g. triethylamine, K₂CO₃, NaH and the like) in a solvent (e.g. THF, DCM, DMF, NMP and the like) affording amide **(III);** wherein R¹, R² and R³ are defined as above; or when X is hydroxy the acid derivative **(I)** wherein R³ is as defined above is coupled with an amine **(II)** wherein R¹ and R² are defined as above under standard amide forming conditions using a coupling reagent (a) (e.g. HOBT, EDAC and DIPEA in dry THF) affording amide **(III)** wherein R¹, R² and R³ are as defined above.

### Example 1 (General method (A))

### 4-(Benzo[1,3]dioxol-5-yloxy)-N-cyclohexyl-N-methyl-butyramide

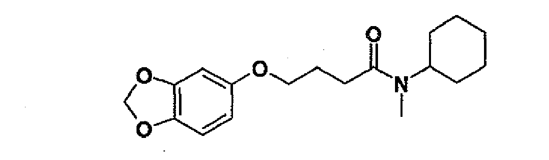

To a solution of 4-(benzo[1,3]dioxol-5-yloxy)-butyric acid (1.0 g, 4.46 mmol), HOBT (0.66 g, 4.91 mmol) in dry THF (75 ml) was added EDAC (0.94 g, 4.91 mmol) and the mixture was stirred for 20 minutes. Di-isopropyl ethyl amine (DIPEA) (860 µl, 4.91 mmol) and cyclohexyl-methyl-amine (555 mg, 4.91 mmol) was added and the resulting mixture was stirred for 16 hrs, at room temperature. The volatiles were evaporated *in vacuo* and the residue was purified by silicagel chromatography using a mixture of ethyl acetate/hexane as eluent. Pure fractions were collected and the solvent evaporated *in vacuo* to dryness. The oily residue crystallized on standing affording 1.1 g (77%) of the title compounds as a solid.
¹H NMR (300 MHz, CDCl₃) δ 1.07-1.85 (m, 11H), 2.10 (m, 2H), 2.48 (t, 1H), 2.53 (t, 1H), 2.81 and 2.83 (2x s, 3H, N-Me rotamers), 3.96 (t, 2H), 5.90 (s, 2H), 6.32 (dd, 1H), 6.49 (d, 1H), 6.69 (d,1H).
Calculated for C₁₈H₂₅NO₄; C, 67.69 %; H, 7.89 %; N, 4.39 %. Found:
C, 67.74 %; H, 7.99 %; N, 4.34 %.

### Example 2 (General method (A))

### N-Methyl-N-(1-methyl-piperidin-4-yl)-4-phenoxy-butyramide

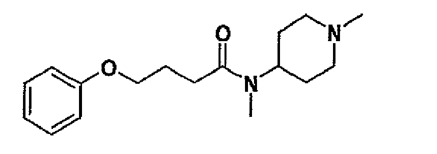

To a solution of 4-phenoxy-butyric acid (1.0 g, 5.55 mmol), HOBT (0.83 g, 6.1 mmol) in dry THF (75 ml) was added EDAC (1.17 g, 6.1 mmol) and the mixture was stirred for 20 minutes. Di-isopropyl ethyl amine (DIPEA) (1.06 ml, 6.1 mmol) and methyl-(1-methyl-piperidin-4-yl)-amine (783 mg, 6.1 mmol) was added and the resulting mixture was stirred for 16 hrs. at room temperature. The volatiles were evaporated *in vacuo* and the residue was purified by silicagel chromatography using a mixture of ethyl acetate/triethyf amine (1:25) as eluent. Pure fractions were collected and the solvent evaporated *in vacuo* to dryness affording 1.1 g (68%) of the title compounds as an oil.
¹H NMR (300 MHz, CDCl₃) δ 1.57 (m, 2H), 1.74 (dq, 1H), 1.86-2.18 (m, 5H), 2.28 (t, 3H), 2.52 (m, 2H), 2.82 and 2.85 (2x s, 3H, N-Me rotamers), 2.89 (bd, 2H), 3.60 and 4.51 (2x dt, 1 H), 4.04 (t, 2H), 6.91 (m, 3H), 7.28 (m, 2H).
Calculated for C₁₇H₂₆N₂O₂; C, 70.31 %; H, 9.02 %; N, 9.65 %. Found:
C, 69.72 %; H, 9.29 %; N,10.12%.

### Example 3 (General method (A))

### Azepan-1-yl-(3-chloro-phenyl)-methanone

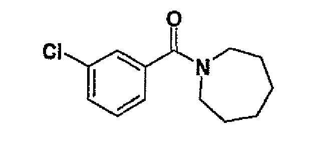

To a solution of 3-chloro benzoic acid (1.0 g, 6.39 mmol), HOBT (0.95 g, 7.03 mmol) in dry THF (50 ml) was added EDAC (1.35 g, 7.03 mmol) and the mixture was stirred for 20 minutes. Di-isopropyl ethyl amine (DIPEA) (1.22 ml, 7.03 mmol) and azepane (697 mg, 7.03 mmol) was added and the resulting mixture was stirred for 4 hrs. at room temperature. The volatiles were evaporated *in vacuo,* water (50 ml) was added, the resulting mixture extracted with diethyl ether (2 x 25 ml), dried (Na₂SO₄), filtered and evaporated *in vacuo.* The residue was purified by silicagel chromatography using a mixture of ethyl acetate/heptane (1:1) as eluent. Pure fractions were collected and the solvent evaporated *in vacuo* to dryness affording 0.9g (59%) of the title compounds as an oil,
¹H NMR (300 MHz, CDCl₃) δ 1.61 (m, 6H), 1.84 (m, 2H), 3.35 (t, 2H), 3.67 (t, 2H), 7.23-7.39 (m, 4H).

### Example 4 (General procedure C)

### (4-Tetrazol-1-yl-pheyl)-(1,3,3-trimethyl-6-aza-bicyclo[3.2.1]oct-6-yl)-methanone

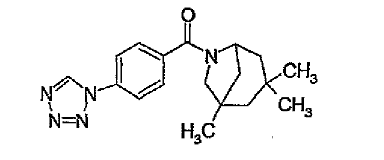

To a mixture of 4-(1*H*-tetrazol-5-yl)-benzoic acid (0.5 g, 2.63 mmol) and HOBT (0.39 g, 2.89 mmol) in dry THF (35 mL) was added EDAC (0.55 g, 2.89 mmol). The resulting mixture was stirred for 10 min. followed by addition of a mixture of DIPEA (0.50 ml, 2.89 mmol) and 1,3,3-trimethyl-6-aza-bicyclo[3.2.1]octane (0.49 ml, 2.89 mmol). The reaction mixture was stirred for an additional 6 hrs. and evaporated to dryness. To the residue was added way ter (10 ml) and the resulting mixture was extracted with diethyl ether (2x10 ml). The combined organic phases were dried (Na₂SO₄), filtered and evaporated in vacuo. The resulting residue was purified by column chromatography (silica gel) using a mixture of EtOAc-Heptane (1:2) as eluent. Pure fractions were collected and evaporated to dryness. To the residue was added diethyl ether (5 ml) and the precipitate was filtered off, washed with diethyl ether and dried in vacuo at 50 °C affording 220 mg (26 %) of the title compound as a solid.
TLC: EtOAc-Heptan (2:1), R_{f}: 0.18
¹H-NMR (300 MHz, CDCl₃) δ 0.95 -1.15 (m, 9H), 1.21 -1.64 (m, 5.5H), 2.25 (m, 0.5H), 3.17 - 3.32 (m, 1.5H), 3.63 (d, 0.5H), 3.98 and 4.60 (2xt, 1H), 7.68 (t, 2H), 7.78 (m, 2H), 9.06 (s, 1H).

The following compounds were made in a similar way as described in example 4 above:

| **No** | **Molecule** | **MW** | **IUPAC Name** |
|---|---|---|---|
| 4-1 | 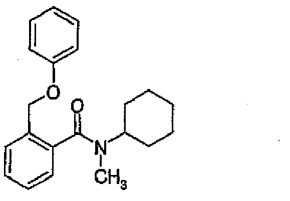 | 323.43 | N-Cyclohexyl-N-methyl-2-phenoxymethyl-benzamide |
| 4-2 | 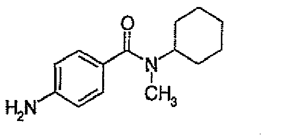 | 232.32 | 4-Amino-N-cyclohexyl-N-methyl-benzamide |
| 4-3 | 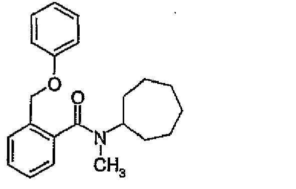 | 337.46 | N-Cycloheptyl-N-methyl-2-phenoxymethyl-benzamide |
| 4-4 | 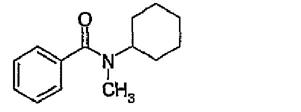 | 217.31 | N-Cyclohexyl-N-methyl-benzamide |
| 4-5 | 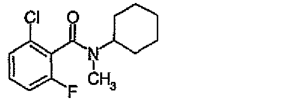 | 269.74 | 2-Chlorn-N-cyclohexyl-6-fluoro-N-methyl-benzamide |
| 4-6 | 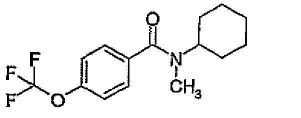 | 301.31 | N-Cyclohexyl-N-methyl-4-trifluoromethoxy-benzamide |
| 4-7 | 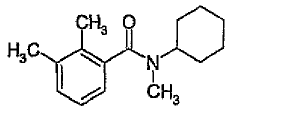 | 245.36 | N-Cyclohexyl-2,3,N-trimethyl-benzamide |
| 4-8 | 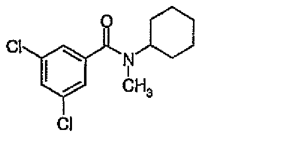 | 286.20 | 3,5-Dichloro-N-cyclohexyl-N-methyl-benzamide |
| 4-9 | 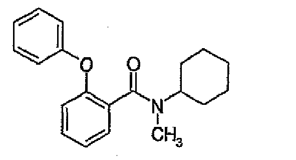 | 309.41 | N-Cyclohexyl-N-methyl-2-phenoxy-benzamide |
| 4-10 | 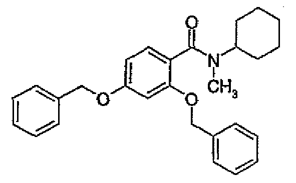 | 429.56 | 2,4-Bis-benzyloxy-N-cyclohexyl-N-methyl-benzamide |
| 4-11 | 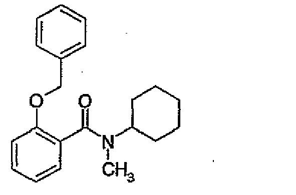 | 323.43 | 2-Benzyloxy-N-cyclohexyl-N-methyl-benzamide |
| 4-12 | 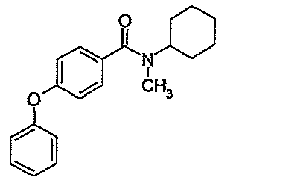 | 309.41 | N-Cyclohexyl-N-methyl4-phenoxy-benzamide |
| 4-13 | 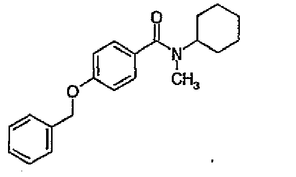 | 323.43 | 4-Benzyloxy-N-eyelohexyl-N-methyl-benzamide |
| 4-14 | 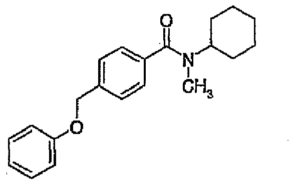 | 323.43 | N-Cyclohexyl-N-methyl-4-phenoxymethyl-benzamidebenzamide |
| 4-15 | 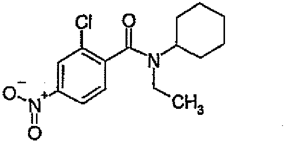 | 310.78 | 2-Chloro-N-cyclohexyl-N-ethyl-4-nitro-benzamide |
| 4-16 | 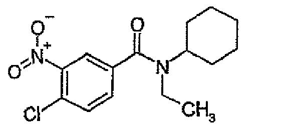 | 310.78 | 4-Chloro-N-cyclohexyl-N-ethyl-3-nitro-benzamide |
| 4-17 | 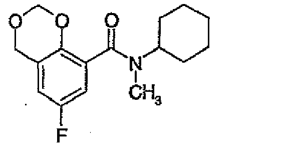 | 293.34 | 6-Fluoro-4H-benzo[1,3]dioxine-8-carboxylic acid cyclohexyl-methyl-amide |
| 4-18 | 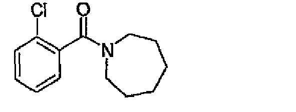 | 237.73 | Azepan-1-yl-(2-chloro-phenyl)-methanone |
| 4-19 | 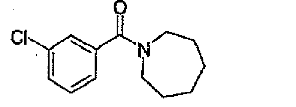 | 237.73 | Azepan-1-yl-(3-chloro-phenyl)-methanone |
| 4-20 | 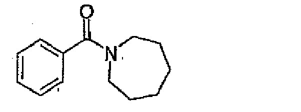 | 203.28 | Azepan-1-yl-phenyl-methanone |
| 4-21 | 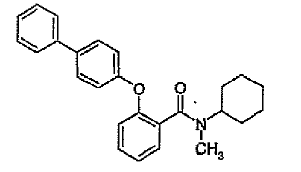 | 385.51 | 2-(Biphenyl-4-yloxy)-N-cyclohexyl-N-methyl- benzamide |
| 4-22 | 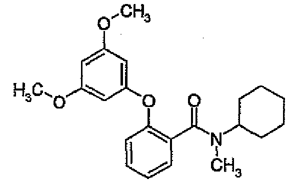 | 369.46 | N-Cyclohexyl-2-(3,5-dimethoxy-phenoxy)-N-methyl-benzamide |
| 4-23 | 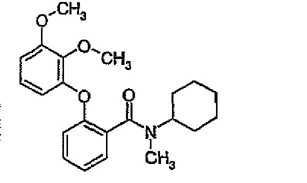 | 369.46 | N-Cyclohexyl-2-(2,3-dimethoxy-phenoxy)-N-methyl-benzamide |
| 4-24 | 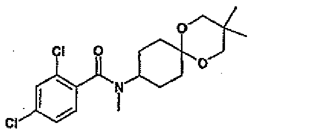 | 386.32 | 2,4-Dichloro-N-(3,3-dimethyl-1,5-dioxa-Spiro[5.5]undec-9-yl)-N-methyl-benzamide |
| 4-25 | 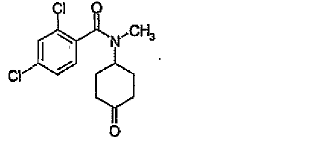 | 300.18 | 2,4-Dichloro-N-methyl-N-(4-oxo-cyclohexyl)-benzamide |
| 4-26 | 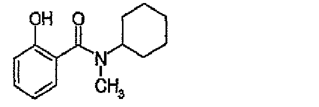 | 233.31 | N-Cyclohexyl-2-hydroxy-N-methyl-benzamide |
| 4-27 | 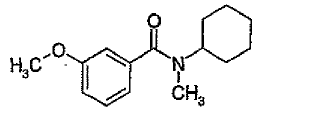 | 247.34 | N-Cyclohexyl-3-methoxy-N-methyl-benzamide |
| 4-28 | 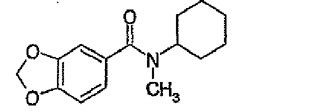 | 261.32 | Benzo[1,3]dioxole-5-carboxylicacid cyclohexyl-methyl-amide |
| 4-29 | 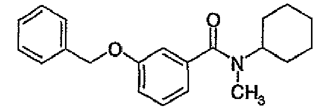 | 323.43 | 3-Benzyloxy-N-cyclohexyl-N-methyl-benzamide |
| 4-30 | 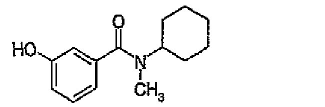 | 233.31 | N-Cyclohexyl-3-hydroxy-N-methyl-benzamide |
| 4-31 | 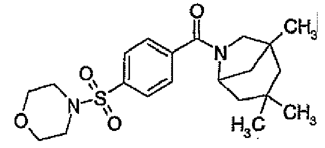 | 406.54 | [4-(Morpholine-4-sulfonyl)-phenyl]-(1,3,3-trimethyl-6-aza-bicyclo[3.2.1]oct-6-yl)-methanone |
| 4-32 | 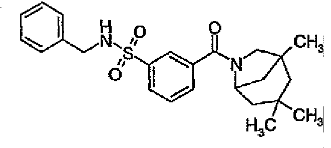 | 426.58 | N-Benzyl-3-(1,3,3-trimethyl-6-aza-bicyclo[3.2.1]octane-6-carbonyl)-benzenesulfonamide |
| 4-33 | 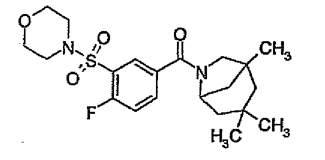 | 424.53 | [4-Fluoro-3-(morpholine-4-sulfonyl)-phenyl]-(1,3,3-trimethyl-6-aza-bicyclo[3.2.1]oct-6-yl)-methanone |
| 4-34 | 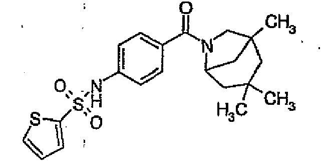 | 418.58 | Thiophene-2-sulfonic acid [4-(1,3,3-trimethyl-6-aza-bicyclo[3.2.1]octane-6-carbonyl)-phenyl]-amide |
| 4-35 | 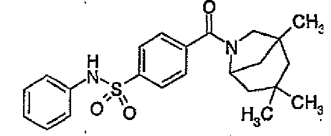 | 412.55 | N-Phenyl-4-(1,3,3-trimethyl-6-aza-bicyclo[3.2.1]octane-6-carbonyl)-benzenesulfonamide |
| 4-36 | 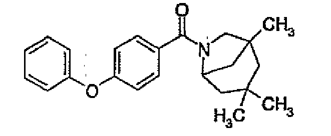 | 349.47 | (4-Phenoxy-phenyl)-(1,3,3-trimethyl-6-aza-bicyclo[3.2.1]oct-6-yl)-methanone |
| 4-37 | 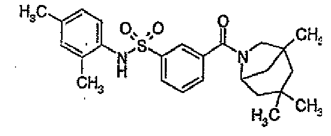 | 440.60 | N-(2,4-Dimethyl-phenyl)-3-(1,3,3-trimethyl-6-aza-bicyclo[3.2.1]octane-6-carbonyl)-benzenesulfonamide |
| 4-38 | 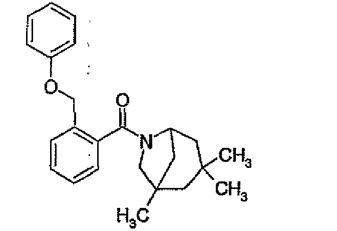 | 363.50 | (2-Phenoxymethyl-phenyl)-(1,3,3-trimethyl-6-aza-bicyclo[3.2.1]oct-6-yl)-methanone |
| 4-39 | 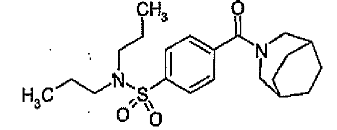 | 392.56 | 4-(3-Aza-bloyclo[3.2.2]nonane-3-carbonyl)-N,N-dipropyl-benzenesulfonamide |
| 4-40 | 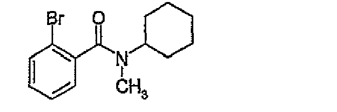 | 296.21 | 2-Bromo-N-cyclohexyl-N-methyl-benzamide |
| 4-41 | 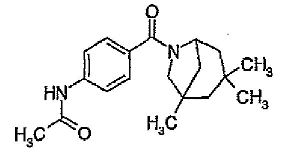 | 314.43 | N-[4-(1,3,3-Trimethy)-6-aza-bicyclo[3.2.1]octane-6-carbonyl)-phenyl]-acetamide |
| 4-42 | 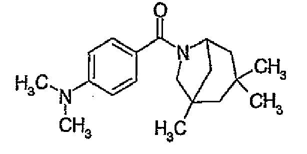 | 300.44 | (4-Dimethylamino-phenyl)-(1,3,3-trimethyl-6-aza-bicyclo[3.2.1]oct-6-yl)-methanone |
| 4-43 | 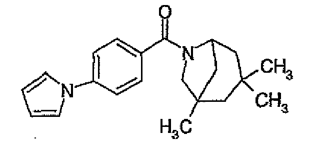 | 322.45 | (4-Pyrrol-1-yl-phenyl)-(1,3,3-trimethyl-6-aza-bicyclo[3.2.1]oct-6-yl)-methanone |
| 4-44 | 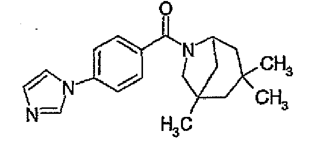 | 323.44 | (4-Imidazol-1-yl-phenyl)-(1,3,3-trimethyl-6-aza-bicyclo[3.2.1]oct-6-yl)-methanone |
| 4-45 | 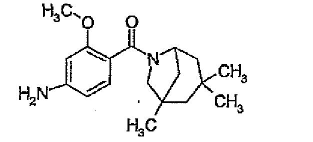 | 302.42 | (4-Amino-2-methoxy-phenyl)-(trimethyl-6-aza-bicyclo[3.2.1]oct-6-yl)-methanone |
| 4-46 | 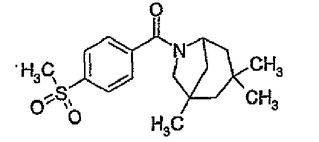 | 335.46 | (4-Methanesulfonyl-phenyl)-(1,3,3-trimethyl-6-aza-bicyclo[3.2.1]oct-6-yl)-methanone |
| 4-47 | 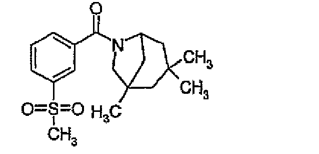 | 335.46 | (3-Methanesulfonyl-phenyl)-(1,3,3-trimethyl-6-aza-bicyclo[3.2.1]oct-6-yl)-methanone |
| | | | |
| 4-48 | 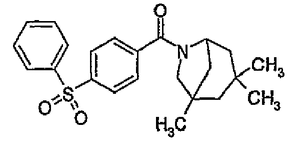 | 397.54 | (4-Benzenesulfonyl-phenyl)-(1,3,3-trimethyl-6-aza-bicyclo[3.2,1]oct-6-yl)-methanone |
| 4-49 | 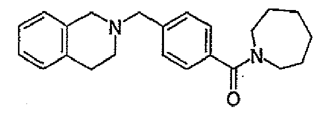 | 348.49 | Azepan-1-yl-[4-(3,4-dihydro-1H-isoquinolin-2-ylmethyl)-phenyl]-methanone |
| 4-50 | 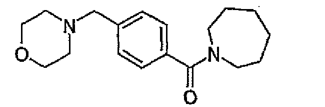 | 302.42 | Azepan-1-yl-(4-morpholin-4-ylmethyl-phenyl)-methanone |
| 4-51 | 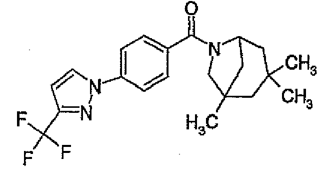 | 391.44 | [4-(3-Trifluoromethyl-pyrazol-1-yl)-phenyl]-(1,3,3-trimethyl-6-aza-bicyclo[3.2.1]oct-6-yl)-methanone |
| 4-52 | 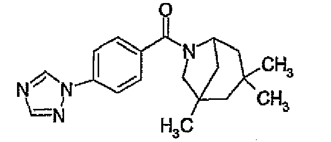 | 324.42 | (4-[1,2,4]Triazol-1-yl-phenyl)-(1,3,3-trimethyl-6-aza-bicyclo[3.2.1]oct-6-yl)-methanone |
| 4-53 | 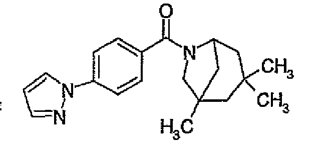 | 323.44 | (4-Pyrazol-1-yl-phenyl)-(1,3,3-trimethyl-6-aza-bicyclo[3.2.1]oct-6-yl)-methanone |
| 4-54 | 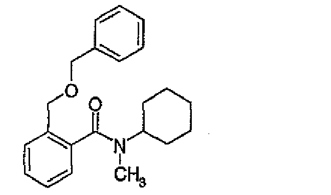 | 337.46 | 2-Benzyloxymethyl-N-cyclohexyl-N-methyl-benzamide |
| 4-55 | 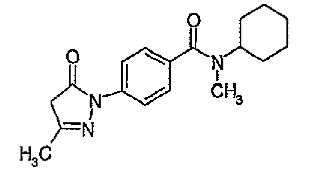 | 313.40 | N-Cyclohaxyl-N-methyl-4-(3-methyl-5-oxo-4,5-dihydro-pyrazo-1-yl)-benzamide |
| 4-56 | 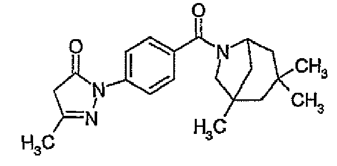 | 353.46 | 5-Methyl-2-[4-(1,3,3-trimethyl-6-aza-bicyclo[3.2.l]octane-6-carbonyl)-phenyl]-2,4-dihydro-pyrazol-3-one |
| 4-57 | 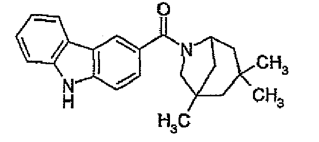 | 346.47 | (9H-Carbazol-3-yl)-(1,3,3-trimethyl-6-aza-bicyclo[3.2.1]oct-6-yl)-methanone |
| 4-58 | 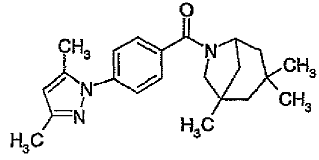 | 351.49 | [4-(3,5-Dimethyl-pyrazol-9-yl)-phenyl]-(1,3,3-trimethyl-6-aza-bicyclo[3.2.1]oct-6-yl)-methanone |
| 4-59 | 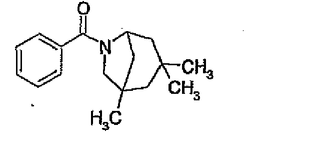 | 257.37 | Phenyl-(1,3,3-trimethyl-6-aza-bicyclo[3.2.1]oct-6-yl)-methanone |
| 4-60 | 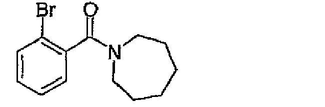 | 282.18 | Azepan-1-yl-(2-bromo-phenyl)-methanone |
| 4-61 | 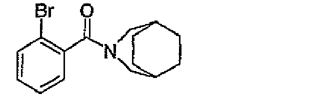 | 308.22 | (3-Aza-bicyclo[3.2.2]non-3-yl)-(2-bromo-phenyl)-methanone |
| 4-62 | 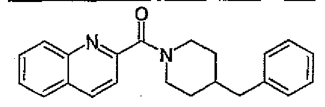 | 330.43 | (4-Benzyl-piperidin-1-yl)-quinolin-2-yl-methanone |
| 4-63 | 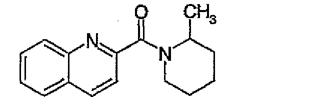 | 254.33 | (2-Methyl-piperidin-1-yl)-quinolin-2-yl-methanone |
| 4-64 | 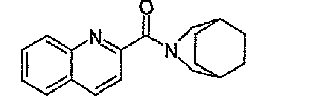 | 280.37 | (3-Aza-bicyclo[3.2.2]non-3-yl)-quinolin-2-yl-methanone |
| 4-65 | 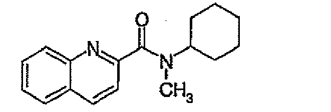 | 268.36 | Quinoline-2-carboxylic acid cyclohexyl-methyl-amide |
| 4-66 | 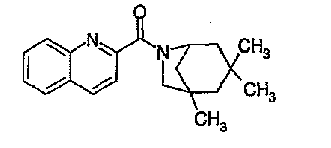 | 308.42 | Quinolin-2-yl-(1,3,3-trimethyl-6-aza-bicyclo[3.2.1]oct-6-yl)-methanone |
| 4-67 | 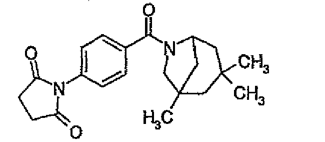 | 354.45 | 1-[4-(1,3,3-Trimethyl-6-aza-bicyclo[3.2.1]octane-6-carbonyl)-phenyl]-pyrrolidine-2,5-dione |
| 4-68 | 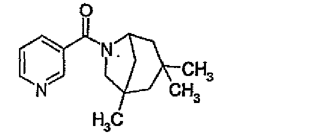 | 258.38 | Pyridin-3-yl-(1,3,3-trimethyl-6-aza-bicyclo[3.2.1]oct-6-yl)-methanone |
| 4-69 | 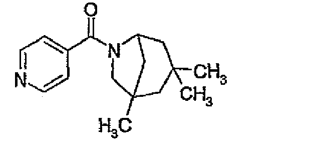 | 258.36 | Pyridin-4-yl-(1,3,3-trimethyl-6-aza-bicyclo[3.2.1]oct-6-yl)-methanone |
| 4-70 | 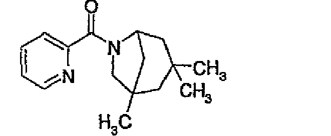 | 258.36 | Pyridin-2-yl-(1,3,3-trimethyl-6-aza-bicyclo[3.2.1]oct-6-yl)-methanone |
| 4-71 | 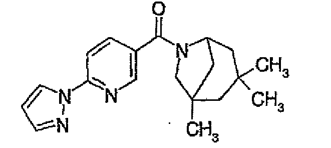 | 324.42 | (6-Pyrazol-1-yl-pyridin-3-yl)-(1,3,3-trimethyl-6-aza-bicyclo[3.2.1]oct-6-yl)-methanone |
| 4-72 | 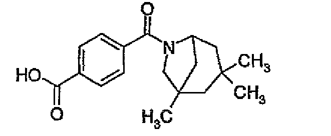 | 301.38 | 4-(1,3,3-Trimethyl-6-aza-bicyclo[3.2.1]octane-6-carbonyl)-benzoic acid |
| 4-73 | 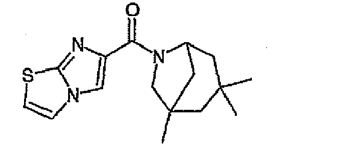 | 303.4 | Imidazo[2,1-b]thiazol-6-yl-(1,3,3-trimethyl-6-aza-bicyclo[3.2.1]oct-6-yl)-methanone |

### Example 5 (General procedure C)

### 8-(4-Dimethylamino-benzoyl)-8-aza-bicyclo[3.2.1]octan-3-one

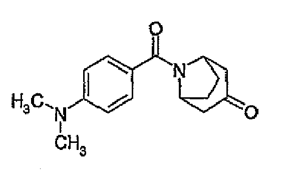

To a mixture of 8-aza-bicyclo[3.2.1]octane (1.8 g, 14.38 mmol), dry THF (75 ml) and TEA (4 ml, 28.76 mmol) was added dropwise a solution of 4-dimethylamino-benzoyl chloride (3.17 g, 17.26 mmol) in dry THF (75 ml). The resulting mixture was stirred for 1 hr. at room temperature followed by filtration and evaporation in vacuo. The residue was purified by column chromatography (silica gel) using a mixture of EtOAc-Heptane (1:2) as eluent. Pure fractions were collected and evaporated to dryness. The residue was crystallized from diethyl ether (25 ml), filtered off and dried in vacuo at 50°C affording 1.9 g (48 %) of the title compound as a solid.
TLC: EtOAc-Heptane (3:1), R_{f}: 0.4
¹H-NMR (300 MHz, CDCl₃) δ 1.74 (m, 2H), 2.15 (m, 2H), 2.39 (d, 2H), 2.27 (bs, 2H), 3.02 (s, 6H), 4.80 (bs, 2H), 6.68 (d, 2H), 7.50 (d, 2H).

### Example 6 (General procedure C)

### (4-Dimethylamino-phenyl)-(3-hydroxy-8-aza-bicyclo[3.2.1]oct-8-yl)-methanone

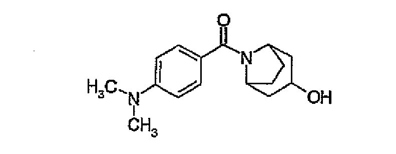

To a solution of 8-(4-dimethylamino-benzoyl)-8-aza-bicyclo[3.2.1]octan-3-one (2.0 g, 7.34 mmol) in MeOH (75 ml) was added NaBH₄ (0.45 g, 11.02 mmol, pellets). The resulting mixture was stirred for 18 hrs, evaporated and to the residue was added water (75 ml). The aqueous phase was acidified to pH 1 with conc. HCl and washed with diethyl ether (50 ml). The aqueous phase was neutralized to pH 7 with 1N NaOH. The precipitate was filtered off and washed with water, diethyl ether and dried in vacuo at 50°C affording 1.15 g (57 %) of the title compound as a solid.
¹H-NMR (400 MHz, CDCl₃) δ 1.66-1.95 (m, 6H), 2.22 (d, 2H), 2.99 (s, 6H), 4.17 (m, 1H), 4.22 (bs, 1H), 4.74 (bs, 1H), 6.66 (d, 2H), 7.41 (d, 2H).

### Example 7 (General procedure C)

### (4-Dimethylamino-phenyl)-(3-hydroxy-3-methyl-8-aza-bicyclo[3.2.1]oct-8-yl)-methanone

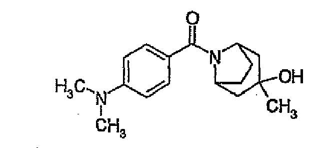

To a solution of 8-(4-dimethylamino-benzoyl)-8-aza-bicyclo[3.2.1]octan-3-one (200 mg, 0.734 mmol) in dry THF (40 ml) was added dropwise a solution of methylmagnesium bromide (0.74 ml, 2.20 mmol, 3M in diethyl ether). The resulting mixture was stirred for 36 hrs. at room temperature and quenched by addition of saturated aqueous ammonium chloride (50 ml). The aqueous phase was extracted with EtOAc (2 x 100 ml) and the combined organic phases were dried (Na₂SO₄), filtered and evaporated in vacuo. The residue was purified by column chromatography (silica gel) using a mixture of EtOAc-Heptane (5:1) as eluent. Pure fractions were collected and evaporated to dryness affording 45 mg (21 %) of the title compound as a solid.
TLC; EtOAc, R_{f}: 0.39
¹H-NMR (400 MHz, CDCl₃) δ 1.21 (s, 3H), 1.75 (d, 2H), 1.92 (m, 2H), 2.15 (bs, 1H), 2.22 (d, 2H), 3.0 (s, 6H), 4.26 (bs, 1H), 4.77 (bs, 1H), 6.66 (d, 2H), 7.42 (d, 2H).

### Example 8 (General procedure C)

### Trifluoro-acetic acid 8-(4-dimethylamino-benzoyl)-8-aza-bicyclo[3.2.1]oct-3-yl ester

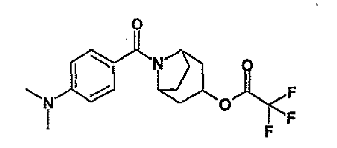

NaBH₄ (0.3 g, 2 pellets) was added to TFA (20 ml) at 0 °C and stirred for 30 min. To this mixture was added a solution of 8-(4-dimothylamino-benzoyl)-8-aza-bicyclo[3.2.1]octan-3-one (0.3 g, 1.102 mmol) in DCM (10 ml). The resulting mixture was stirred for 64 hrs. at room temperature, the volatiles evaporated and to the residue was added water (10 ml). The aqueous phase was neutralized to pH 7 with 1N NaOH and extracted with diethyl ether (2 x 25 ml). The combined organic phases were evaporated in vacuo and the residue purified by column chromatography (silica gel) using a mixture of EtOAc-Heptane (1:2) as eluent. Pure fractions were collected and evaporated to dryness affording 25 mg (9 %) of the title compound as a solid.
TLC: EtOAc-Heptane (2:1), R_{f}: 0.54
LC/MS: m/z: 371 H⁺
¹H-NMR (400 MHz, CDCl₃) δ 1.81 - 1.94 (m, 2H), 2.05 (s, 4H), 2.31 (bs, 2H), 3.01 (s, 6H), 4.59 (bs, 2H), 5.35 (t, 1H), 6.67 (d, 2H), 7.42 (d, 2H).

### EXAMPLES, COMPOUNDS OF GENERAL FORMULA (III)

The following examples and general procedures refer to intermediate compounds and final products for general formula (III) identified in the specification and in the synthesis schemes. The preparation of the compounds of general formula (III) of the present invention is described in detail using the following examples. Occasionally, the reaction may not be applicable as described to each compound included within the disclosed scope of the invention. The compounds for which this occurs will be readily recognised by those skilled in the art. In these cases the reactions can be successfully performed by conventional modifications known to those skilled in the art, that is, by appropriate protection of interfering groups, by changing to other conventional reagents, or by routine modification of reaction conditions. Alternatively, other reactions disclosed herein or otherwise conventional will be applicable to the preparation of the corresponding compounds of the invention. In all preparative methods, all starting materials are known or may easily be prepared from known starting materials. The structures of the compounds are confirmed by either elemental analysis or nuclear magnetic resonance (NMR), where peaks assigned to characteristic protons in the title compounds are presented where appropriate. ¹H NMR shifts (δ_{H}) are given in parts per million (ppm) down field from tetramethylsilane as internal reference standard. M.p.: is melting point and is given in °C and is not corrected. Column chromatography was carried out using the technique described by W.C. Still et al., J. Org. Chem. 43: 2923 (1978) on Merck silica gel 60 (Art. 9385). HPLC analyses are performed using 5µm C18 4 x 250 mm column eluted with various mixtures of water and acetonitrile, flow = 1 ml/min, as described in the experimental section.

The abbreviations as used in the examples have the following meaning:
- TLC:: thin layer chromatography
- CDCl₃:: deuterio chloroform
- CD₃OD:: tetradeuterio methanol
- DMSO-d₆:: hexadeuterio dimethylsulfoxide
- DMSO:: dimethylsulfoxide
- THF:: tetrahydrofuran
- DMF:: N,N-dimethylformamide
- HOBT:: 1-hydroxy-benzotriazole
- EDAC:: 1-(3-dimethylaminopropyl)-3-ethylcarbodilmide, hydrochloride
- min:: minutes
- hrs:: hours

The compounds of the invention are prepared as illustrated in the following reaction scheme 1:

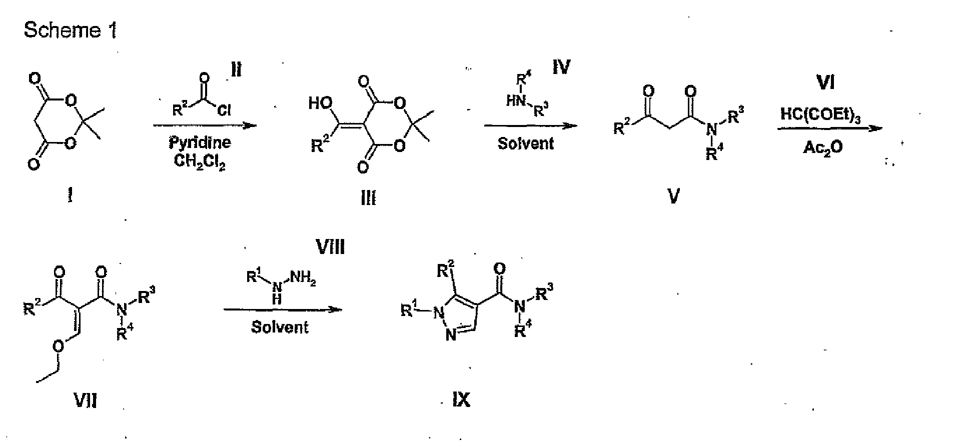

### General method:

By allowing 2,2-dimethyl-1,3-dioxane-4,6-dione (Meldrum's acid) (I) to react with an acid chloride (II), wherein R² is defined above, in a solvent mixture of pyridine and DCM and the like affording an acyl Meldrum's acid (III), wherein R² is as defined above. The acyl Meldrum's acid (III) is aminolysed with an substituted amine (IV), wherein R³ and R⁴ are defined above, in a solvent such as benzene, toluene, dioxane, and the like at a temperature interval from 50 °C up to reflux affording a *beta*-keto amide (V) wherein R², R³ and R⁴ are as defined above. The *beta*-keto amide (V) is treated with ortho-formiate (VI) in a solvent such as acetic acid anhydride and the like at a temperature interval from 50 °C up to reflux affording enol ether (VII) wherein R², R³, and R⁴ are as defined above. Condensation of the enol ether (VII) wherein R², R³, and R⁴ are as defined above with hydrazide (VIII) wherein R¹ is as defined above yields pyrazole (IX) wherein R¹, R², R³, and R⁴ are as defined above, in a solvent such as EtOH, i-PrOH, *tert*-BuOH and the like.

### Example 1

### 1-(4-Chloro-phenyl)-5-propyl-1H-prazole-4-carboxylic acid cyclohexyl-methyl-amide

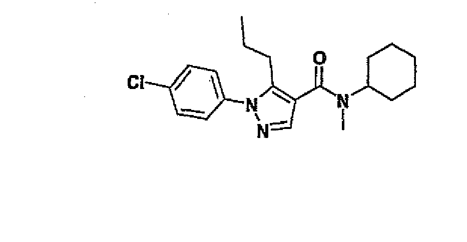

To a solution of 1-(4-chloro-phenyl)-5-propyl-1*H*-pyrazoie-4-carboxylic acid (1.0 g, 3.78 mmol), HOBT (0.56 g, 4.16 mmol) in dry THF (50 ml) was added EDAC (0.8 g, 4.16 mmol) and the mixture was stirred for 10 minutes. Di-isopropyl ethyl amine (DIPEA) (724 µl, 4.16 mmol) and cyclohexyl-methyl-amine (0.54 ml, 4.16 mmol) was added and the resulting mixture was stirred for 16 hrs. at room temperature. The volatiles were evaporated *in vacuo* and the residue was purified by silicagel chromatography using a mixture of ethyl acetate and heptane (1:4) as eluent. Pure fractions were collected and the solvent evaporated *in vacuo* affording 1.1 g (81%) of the title compounds as an oil. ¹H NMR (300 MHz, CDCl₃) δ 0.81 (t, 3H), 1.1 -1.86 (m, 13H), 2.81 (t, 2H), 2.98 (s, 3H), 3.89 and 4.50 (2 x bs, 1H), 7.37 (m, 2H), 7.47 (m, 2H), 7.58 (bs, 1H).
Calculated for C₂₀H₂₆ClN₃O, 0.4 H₂O; C, 65.44 %; H, 7.36 %; N, 11.45 %. Found:
C, 65.47 %; H, 7.89 %; N, 11.56 %.

The following compound was made in a similar way as described in example 1 above

### Example 2

### 1-(4-Chloro-phenyl)-5-trifluoromethyl-1H-pyrazole-4-carboxylic acid cyclohexyl-methyl-amide

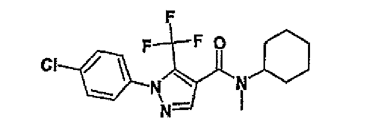

Calculated for C₁₈H₁₉ClF₃N₃O; C, 56.04 %;H, 4.96 %; N,10.89%. Found:
C, 56.02 %; H, 5.16%; N,10.76%.

### Example 3

### [1-(4-Methoxy-phenyl)-5-methyl-1H-pyrazol-4-yl]-(1,3.3-trimethyl-6-aza-bicyclo[3.2.1]oct-6-yl)-methanone

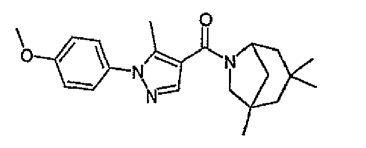

¹H NMR (400 MHz, CDCl₃₎ δ 0.97 (t, 5H), 1.11 (t, 4H), 1.25 -1.61 (m, 4H), 1.79 (m, 2H), 2.20 (d, 0.5H), 2.44 (d, 3H), 3.29 (d, 0.5H), 3.40 (d, 0.5H), 3.59 (m, 0.5H), 3.86 (s, 3H), 4.37 (m, 0.5H), 4.60 (m, 0.5H), 6.99 (d, 2H), 7.33 (d, 2H), 7.74 (s, 1H).

### EXAMPLES, COMPOUNDS OF GENERAL FORMULA (IV)

The following examples and general procedures refer to intermediate compounds and final products for general formula (IV) identified in the specification and in the synthesis schemes. The preparation of the compounds of general formula (IV) of the present invention is described in detail using the following examples. Occasionally, the reaction may not be applicable as described to each compound included within the disclosed scope of the invention. The compounds for which this occurs will be readily recognised by those skilled in the art. In these cases the reactions can be successfully performed by conventional modifications known to those skilled in the art, that is, by appropriate protection of interfering groups, by changing to other conventional reagents, or by routine modification of reaction conditions. Alternatively, other reactions disclosed herein or otherwise conventional will be applicable to the preparation of the corresponding compounds of the invention. In all preparative methods, all starting materials are known or may easily be prepared from known starting materials. The structures of the compounds are confirmed by either elemental analysis or nuclear magnetic resonance (NMR), where peaks assigned to characteristic protons in the title compounds are presented where appropriate. ¹H NMR shifts (δ_{H}) are given in parts per million (ppm) down field from tetramethylsilane as internal reference standard. M.p.: is melting point and is given in °C and is not corrected. Column chromatography was carried out using the technique described by W.C. Still et a/., J. Org. Chem. 43: 2923 (1978) on Merck silica gel 60 (Art. 9385). HPLC analyses are performed using 5µm C18 4 x 250 mm column eluted with various mixtures of water and acetonitrile, flow = 1 ml/min, as described in the experimental section.

The abbreviations as used in the examples have the following meaning:
- TLC:: Thin layer chromatography
- CDCl₃:: Deuterio chloroform
- CD₃OD:: Tetradeuterio methanol
- DMSO-d₆:: Hexadeuterio dimethylsulfoxide
- DMSO:: Dimethylsulfoxide
- THF:: Tetrahydrofuran
- DMF:: N,N-Dimethylformamide
- HOBT:: 1-Hydroxy-benzotriazole
- EDAC:: 1-(3-Dimethylaminopropyl)-3-ethylcarbodilmide, hydrochloride
- min:: minutes
- hrs:: hours

The compounds of the invention are prepared as illustrated in the following reaction scheme 1:

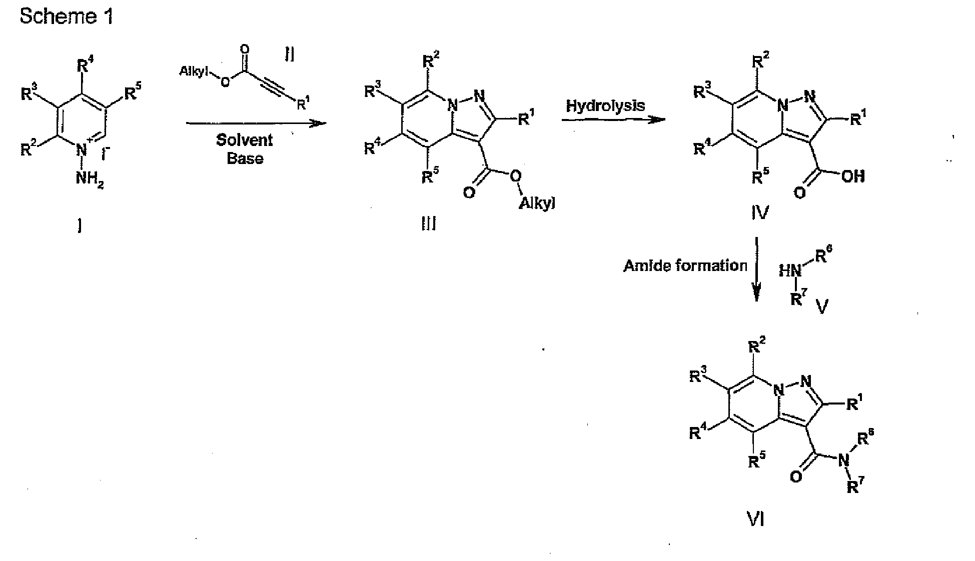

### General method:

By allowing a N-aminopyridinium iodide (I), wherein R², R³, R⁴ and R⁵ are as defined, above to react with a propiolate (II), wherein alkyl and R¹ are as defined above, in a solvent such as DMF and the like, with a suitable base, such as potassium carbonate and the like affording a pyrazolo[1,5-a]pyridine-3-carboxylic acid ester (III), wherein R¹, R², R³, R⁴, R⁵ and alkyl are as defined above. The pyrazolo[1,5-a]pyridine-3-carboxylic acid ester (III) is hydrolysed with a base affording a pyrazolo[1,5-a]pyridine-3-carboxylic acid (IV), wherein R¹, R², R³, R⁴ and R⁵ are as defined above. The pyrazolo[1,5-a]pyridine-3-carboxylic acid (IV) is coupled with an amine (V), wherein R⁶ and R⁷ are as defined above, under standard amide forming conditions (e.g. HOBT, EDAC and DIPEA in dry THF) affording pyrazolo[1,5-a]pyridine-3-amide (VI), wherein R¹, R², R³, R⁴, R⁵, R⁶ and R⁷ are as defined above.

### Example 1

### Pyrazolo[1,5-a]pyridin-3-yl-(1,3,3-trimethyl-6-aza-bicyclo[3.2.1]oct-6-yl)-methanone

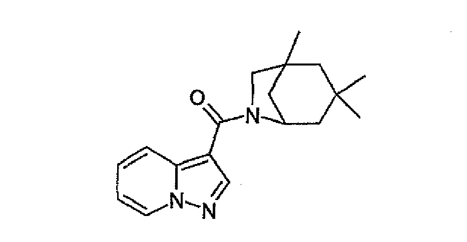

A solution of pyrazolo[1,5-A]pyridine-3-carboxylic acid (97 mg, 0.6 mmol), HOBT (89 mg, 0.66 mmol), EDAC (126 mg, 0.66 mmol) and di-isopropyl ethyl amine (DIPEA) (115 µl, 0.66 mmol) in dry THF (10 ml) was stirred for 1 hr. and 1,3,3 trimethyl-6-azabicyclo[3.2.1]-octane (101 mg, 0.66 mmol) was added. The mixture was stirred for 16 hrs. at room temperature. The mixture was quenched by addition of water (30 ml), extracted with EtOAc (2 x 50 ml), dried (MgSO₄), filtered and evaporated *in vacuo.* The crude product was stirred with water (10 ml) for 30 min., filtered off and dried *in vacuo* affording 130 mg (73 %) of the title compound as a white solid.
¹H NMR (400 MHz, CDCl₃) δ 0.95 (s, 3H), 1.05 (d, 3H), 1.15 (d, 3H), 1.36-1.88 (m, 5.5H), 2.23 (d, 0.5H), 3.37 (d, 0.5H), 3.51 (d, 0.5H), 3.61 (d, 0.5H), 3.73 (d, 0.5H), 4.56 (m, 0.5H), 4.71 (m, 0.5H), 6.92 (m, 1H), 7.33 (m, 1H), 8.19 (s, 1H), 8.47 (m, 2H

The following compounds were made in a similar way was described in example 1 above.

### Example 2

### (2-Methyl-pyrazolo[1,5-a]pyridin-3-yl-(1,3,3-trimethyl-6-aza-bicyclo[3.2.1]oct-6-yl)-methanone

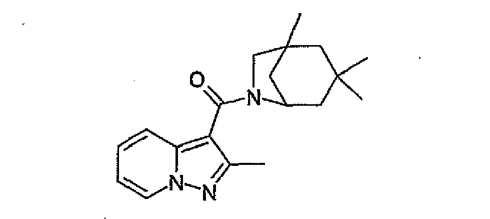

¹H NMR (400 MHz, CDCl₃) δ 0.88 (s, 3H), 1.00 (d, 3H), 1.14 (d, 3H), 1.22-1.45 (m, 3.5H), 1.58 (d, 0.5H), 1.80-1.90 (m, 1.5H), 2.40 (d, 0.5H), 2.50 (s, 3H), 3.20 (dd, 1.5H), 3.82 (d, 0.5H), 4.02 (m, 0.5H), 4.57 (m, 0.5H), 6.75 (t, 1H), 7.18 (t, 1H), 7.38 (t, 1H), 8.36 (d, 1H).

### Example 3

### Pyrazolo[1,5-a]pyridine-3-carboxylic acid cyclohexyl-methyl-amide

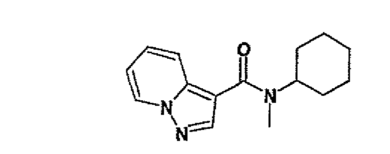

¹H NMR (400 MHz, CDCl₃) δ 1.12 (m, 1H), 1.39 (m, 2H), 1.57-1.70 (m, 3H), 1.83 (m, 4H), 3.07 (bs, 3H), 4.30 (bs, 1H), 6.89 (t, 1H), 7.30 (d, 1H), 8.08 (m, 2H), 8.48 (d, 1H).

### EXAMPLES, COMPOUNDS OF GENERAL FORMULA (V)

The following examples and general procedures refer to intermediate compounds and final products for general formulas (V) and (Va) identified in the specification and in the synthesis schemes. The preparation of the compounds of general formulas (V) and (Va) of the present invention is described in detail using the following examples.. Occasionally, the reaction may not be applicable as described to each compound included within the disclosed scope of the invention. The compounds for which this occurs will be readily recognised by those skilled in the art. In these cases the reactions can be successfully performed by conventional modifications known to those skilled in the art, that is, by appropriate protection of interfering groups, by changing to other conventional reagents, or by routine modification of reaction conditions. Alternatively, other reactions disclosed herein or otherwise conventional will be applicable to the preparation of the corresponding compounds of the invention. In all preparative methods, all starting materials are known or may easily be prepared from known starting materials. The structures of the compounds are confirmed by either elemental analysis or nuclear magnetic resonance (NMR), where peaks assigned to characteristic protons in the title compounds are presented where appropriate. ¹H NMR shifts (δ_{H}) are given in parts per million (ppm) down field from tetramethylsilane as internal reference standard. M.p.: is melting point and is given in °C and is not corrected. Column chromatography was carried out using the technique described by W.C. Still et al., J. Org. Chem. 43: 2923 (1978) on Merck silica gel 60 (Art. 9385). HPLC analyses are performed using 5µm C18 4 x 250 mm column eluted with various mixtures of water and acetonitrile, flow = 1 ml/min, as described in the experimental section.

The abbreviations as used in the examples have the following meaning:
- TLC:: Thin layer chromatography
- CDCl₃:: Deuterio chloroform
- CD₃OD:: Tetradeuterio methanol
- DIPEA:: Diisopropylethyl amine
- DMSO-d₆:: Hexadeuterio dimethylsulfoxide
- DMSO:: Dimethylsulfoxide
- THF:: Tetrahydrofuran
- DMF:: N,N-dimethylformamide
- HOBT:: 1-Hydroxy-benzotriazole
- EDAC:: 1-(3-Dimethylaminopropyl)-3-ethylcarbodiimide, hydrochloride
- min:: minutes
- hrs:: hours

The compounds of the invention are prepared as illustrated in the following reaction schemes:

### General method A

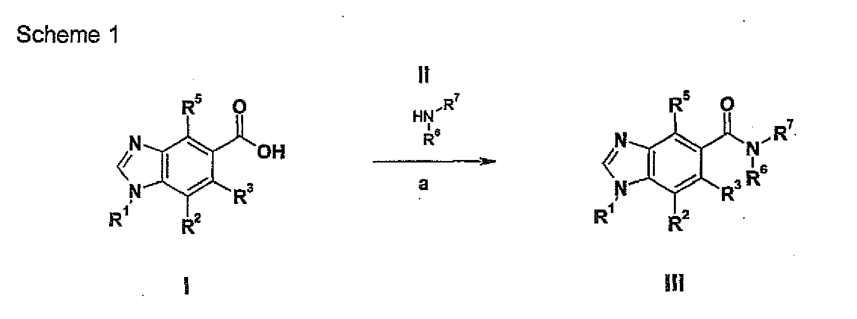

Benzimidazol carboxylic acids (I) wherein R¹, R², R³, R⁵, R⁶ and R⁷ are as defined above are generally prepared as described in the following literature references;
Sekikawa; Bull. Chem. Soc. Jpn. 31, (1958), 252,
Zehra; Chem. Ber. 23, (1890), 3629.
Palmer, B. D. et al.; J. Med. Chem. 41, (1998), 5457 - 5465.
Chi, Y.-C. and Sun, C.-M.; Syn. Lett. 5, (2000), 591 - 594.
Wu, Z. et al.; Tetrahedron Lett. 41, (2000), 9871 - 9874.

By allowing an acid (I) wherein R¹, R², R³, R⁵, R⁶ and R⁷ are as defined above to be coupled with an amine (II) wherein R¹ and R² are defined as above under standard amide forming conditions using a coupling reagent (a) (e.g. HOBT, EDAC and DIPEA in dry THF) affording amide (III) wherein R¹, R², R³, R⁴, R⁵, X, Y and Z are as defined above.

### General method B

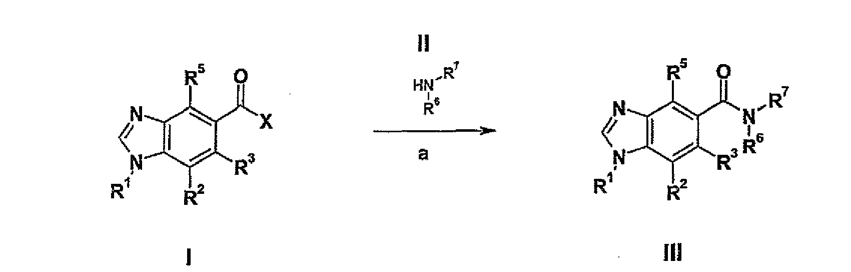

By allowing an acid derivative (I) wherein X is halo, R⁸(C=O)O-, C₁-C₆alkyloxy or arylC₁-C₆alkyloxy, R⁸ is C₁-C₆alkyl or arylC₁-C₆alkyl and R¹, R², R³, R⁵, R⁶ and R⁷ are defined as above to react with an amine (II) wherein R⁶ and R⁷ are defined as above under basic conditions (e.g. triethylamine, K₂CO₃, NaH and the like) in a solvent (e.g. THF, DCM, DMF, NMP and the like) affording amide (III); wherein R¹, R², R³, R⁴, R⁵, R⁶ and R⁷ are defined as above.

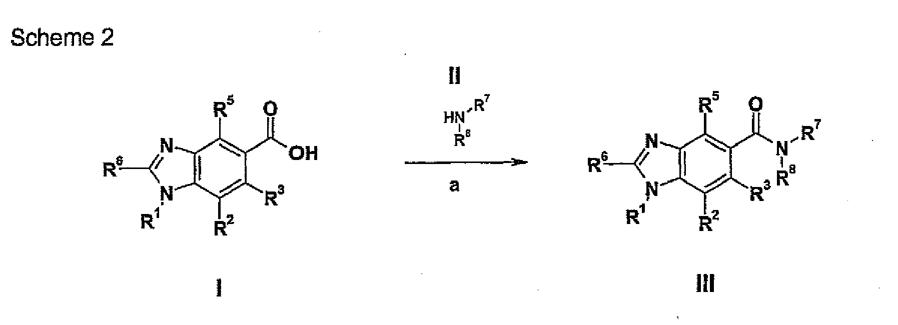

### General method A

Benzimidazol carboxylic acids (I) wherein R¹, R², R³, R⁵, and R⁶ are as defined above are generally prepared as described in the following literature references;
Sekikawa; Bull. Chem. Soc. Jpn. 31, (1958), 252.
Zehra; Chem. Ber. 23, (1890), 3629.
Palmer, B. D. et al.; J. Med. Chem. 41, (1998), 5457 - 5465.
Chi, Y.-C. and Sun, C.-M.; Syn. Lett. 5, (2000), 591 - 594.
Wu, Z. et al.; Tetrahedron Lett. 41, (2000), 9871 - 9874.

By allowing an acid (I) wherein R¹, R², R³, R⁵, and R⁶ are as defined above to be coupled with an amine (II) wherein R⁷ and R⁸ are defined as above under standard amide forming conditions using a coupling reagent (a) (e.g. HOBT, EDAC and DIPEA in dry THF) affording amide (III) wherein R¹, R², R³, R⁵, R⁵, R⁷ and R⁸ are as defined above.

### General method B

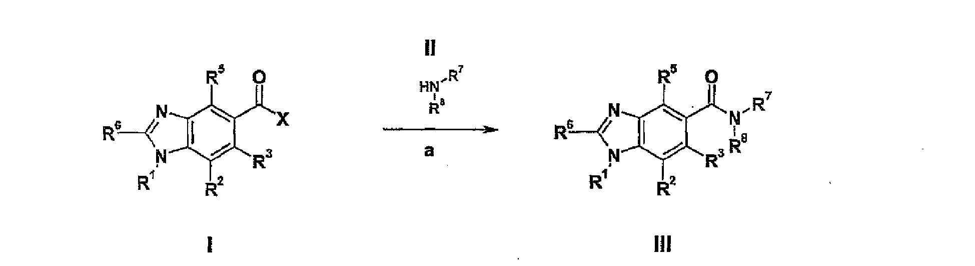

By allowing an acid derivative **(I)** wherein X is halo, R⁹(C=O)O-, C₁-C₆alkyloxy or arylC₁-C₆alkyloxy, R⁹ is C₁-C₆alkyl or arylC₁-C₆alkyl and R¹, R², R³, R⁵ and R⁶ are defined as above to react with an amine **(II)** wherein R⁷ and R⁸ are defined as above under basic conditions (e.g. triethylamine, K₂CO₃, NaH and the like) in a solvent (e.g. THF, DCM, DMF, NMP and the like) affording amide **(III);** wherein R¹, R², R³, R⁴, R⁵, R⁶, R⁷ and R⁸ are defined as above.

### Example 1

### 1H-Benzoimidazole-5-carboxylic acid cyclohexyl-methyl-amide

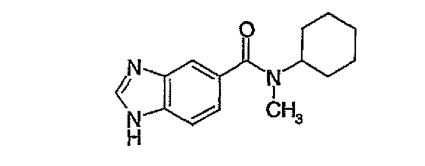

A solution of 1*H*-benzoimidazole-5-carboxylic add (10 g, 61.67 mmol) and HOBT (9.17 g, 67.84 mmol) in dry THF (250 ml) was stirred for 1 h. EDAC (13 g, 67.84 mmol) was added and the mixture was stirred for another 1 h. Di-isopropyl ethyl amine (DIPEA) (11.8 ml, 67.84 mmol) and cyclohexyl-methyl-amine (8.8 ml, 67.84 mmol) was added and the resulting mixture was stirred for 16 hrs. at room temperature. The precipitate was filtered off and the volatiles were evaporated *in vacuo.* To the residue was added water (150 ml) and diethyl ether (75 ml) and the resulting mixture was stirred for 15 minutes. The precipitate was filtered off and washed with water followed by diethyl ether and drying *in vacuo* at 50 °C which afforded 7.7 g (49%) of the title compounds as a solid.
¹H NMR (300 MHz, CDCl₃)δ1.03 (bs, 2H), 1.53 - 1.86 (m, 8H), 2.93 (bd, 3H), 3.57 and 4.56 (2 x bs, 1H), 7.19 (d, 1H), 7.44 (bs, 1H), 7.62 (s, 1H), 7.79 (s, 1H), 11.8 (bs, 1H, N*H*). Calculated for C₁₅H₁₉N₃O; C, 70.01 %; H, 7.44 %; N, 16.33 %. Found:
C, 69.63 %; H, 7.45 %; N, 16.17 %.

The following compounds were synthesised in a similar way as described in example 1

### Example 2

### Isopropyl-2-trifluoromethyl-1H-benzoimidazole-5-carboxylic acid cyclohexyl-methyl-amide

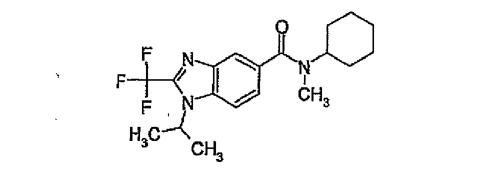

Calculated for C₁₉H₂₄F₃N₃O;
C 62.11 %; H 6.58 %; N 11.44 %. Found
C 62.10 %; H 6.69 %; N 11.66 %.

### Example 3

### 1-Benzyl-1H-benzoimidazole-5-carboxylic acid cyclohexyl-methyl-amide

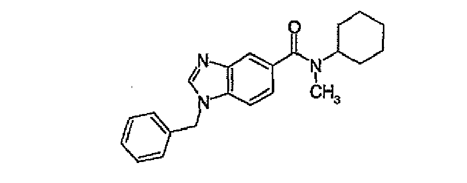

Calculated for C₂₂H₂₅N₃O;
C 76.05 %; H 7.25 %; N 12.09 %. Found
C 75.89 %; H 7.39 %; N 12.03 %.

### Example 4

### 2-Methyl-1H-benzoimidazole-5-carboxylic acid cyclohexyl-methyl-amide

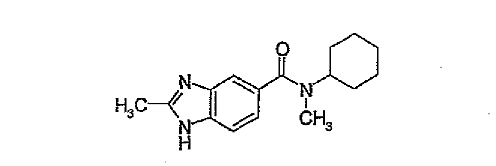

Calculated for C₁₆H₂₁N₃O; 0.1 x H₂O
C 70.35 %; H 7.82 %; N 15.38 %. Found
C 70.09 %; H 7.78 %; N 15.41 %.

### Example 5

### 2-Hydroxymethyl-1H-benzoimidazole-5-carboxylic acid cyclohexyl-methyl-amide

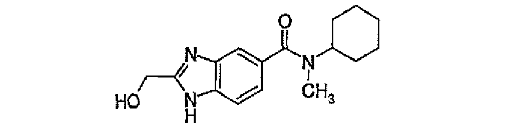

Calculated for C₁₆H₂₁N₃O₂;
C 66.88 %; H 7.37 %; N 14.62 %. Found
C 66.62 %; H 7.50%; N 14.43 %.

### Example 6

### 2-(4-Amino-phenyl)-1H-benzoimidazole-5-carboxylic acid cyclohexyl-methyl-amide

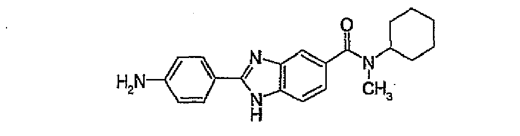

LC/MS m/z: 349 H⁺
¹H NMR (300 MHz, CDCl₃) δ 1.03 -1.3 (m, 4H), 1.45 - 1.80 (m, 6H), 2.84 (bd, 3H), 5.65 (bs, 2H), 6.66 (d, 2H), 7.12 (m, 1H), 7.48 (m, 2H), 7.84 (d, 2H), 12.6 (bs, 1H, N*H*).

### Example 7

### (1H-Benzoimidazol-5-yl)-(1,3,3-trimethyl-6-aza-bicyclo[3.2.1]oct-6-yl)-methanone

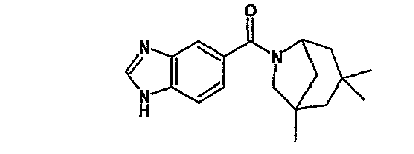

LC/MS m/z: 298 H^{+ 1}H NMR (400 MHz, CDCl₃) δ 0.95 (d, 3H), 1.04 (d, 3H), 1.15 (d, 3H), 1.19 -1.87 (m, 5.5H), 2.25 (m, 0.5H), 3.24 - 3.34 (m, 1.5H), 3.69 (d, 0.5H), 3.99 (m, 0.5H), 4.66 (m, 0.5H), 7.60 (t, ¹H), 7.84 (t, 1H), 7.94 (d, 1H), 9.08 (s, 1H).

### Example 8

### (2-Methyl-1H-benzoimidazol-5-yl)-(1,3,3-trimethyl-6-aza-bicyclo[3.2.1]oct-6-yl)-methanone

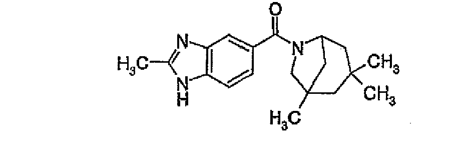

¹H NMR (300 MHz, CDCl₃) δ 0.93 (d, 3H), 1.02 (d, 3H), 1.15 (m, 3H), 1.19-1.85 (m, 5.5H), 2.27 (m, 0.5H), 2.50 (s, 3H), 3.22 - 3.35 (m, 1.5H), 3.64 (d, 0.5H), 4.06 (m, 0.5H), 4.64 (m, 0.5H), 7.22 (m, 2H), 7.58 (m,1H).

### Example 9

### (2-Amino-1H-benzoimidazol-5-yl)-(1.3.3-trimethyl-6-aza-bicyclo[3.2.1]oct-6-yl)-methanone

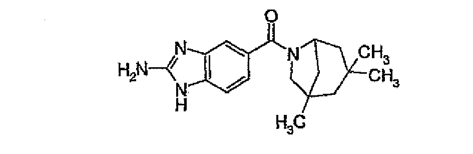

LC/MS m/z: 313 H⁺
¹H NMR (300 MHz, CDCl₃) δ 0.91 (d, 3H), 1.04 (d, 3H), 1.13 (s, 3H), 1.2-1.82 (m, 5.5H), 2.18 (m, 0.5H), 3.22-3.26 (m, 1.5H), 3.62 (d, 0.5H), 4.01 (m, 0.5H), 4.54 (m, 0.5H), 7.21 (m, 3H), 8.14 (m, 2H).

### EXAMPLES, COMPOUNDS OF GENERAL FORMULA (VI)

The following examples and general procedures refer to intermediate compounds and final products for general formula (VI) identified in the specification and in the synthesis schemes. The preparation of the compounds of general formula (VI) of the present invention is described in detail using the following examples. Occasionally, the reaction may not be applicable as described to each compound included within the disclosed scope of the invention. The compounds for which this occurs will be readily recognised by those skilled in the art. In these cases the reactions can be successfully performed by conventional modifications known to those skilled in the art, that is, by appropriate protection of interfering groups, by changing to other conventional reagents, or by routine modification of reaction conditions. Alternatively, other reactions disclosed herein or otherwise conventional will be applicable to the preparation of the corresponding compounds of the invention. In all preparative methods, all starting materials are known or may easily be prepared from known starting materials. The structures of the compounds are confirmed by either elemental analysis or nuclear magnetic resonance (NMR), where peaks assigned to characteristic protons in the title compounds are presented where appropriate. ¹H NMR shifts (δ_{H}) are given in parts per million (ppm) down field from tetramethylsilane as internal reference standard. M.p.: is melting point and is given in °C and is not corrected. Column chromatography was carried out using the technique described by W.C. Still ef al., J. Org. Chem. 43: 2923 (1978) on Merck silica gel 60 (Art. 9385). HPLC analyses are performed using 5µm C18 4 x 250 mm column eluted with various mixtures of water and acetonitrile, flow = 1 ml/min, as described in the experimental section.

The abbreviations as used in the examples have the following meaning:
- TLC:: thin layer chromatography
- CDCl₃:: deuterio chloroform
- CD₃OD: tetradeuterio methanol
- DMSO-d₆:: hexadeuterio dimethylsulfoxide
- DMSO:: dimethylsulfoxide
- THF:: tetrahydrofuran
- DMF:: N,N-dimethylformamide
- HOBT:: 1-hydroxy-benzotriazole
- EDAC:: 1-(3-dimethylaminopropyl)-3-ethylcarbodiimide, hydrochloride
- min:: minutes
- hrs:: hours

The compounds of the invention are prepared as illustrated in the following reaction scheme 1:

### Scheme 1

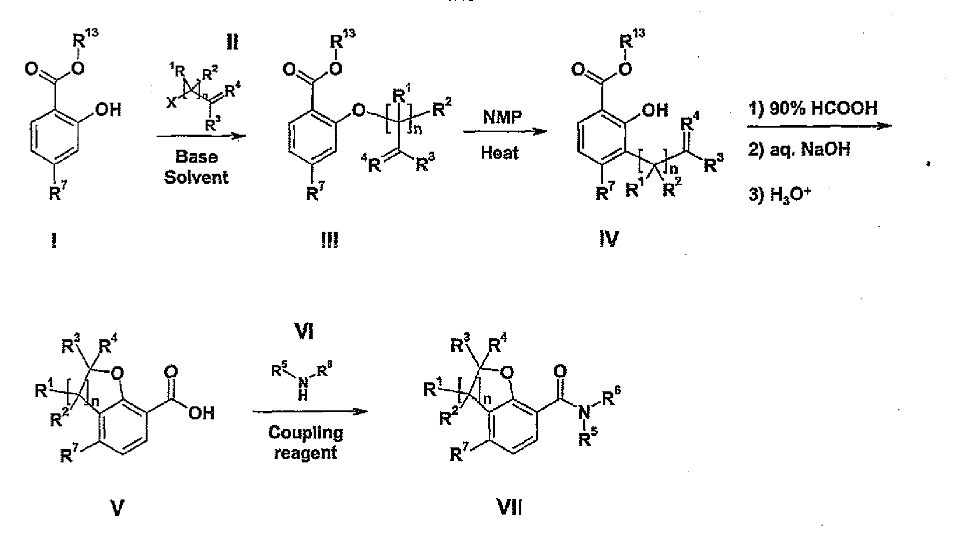

### General method A:

By allowing a carboxylic acid ester **(I)** wherein R¹³ is C₁-C₈alkyl or arylC₁-C₆alkyl and R⁷ is as defined above to react with an alkene **(II)** wherein X is halo and R¹, R², R³, R⁴, and n are as defined above under basic conditions using a base (K₂CO₃, TEA, DIPEA in dry acetone, MIBK and the like) affording aryl ether **(III)** wherein R¹, R², R³, R⁴, R⁷, R¹³ and n are as defined above. Aryl ether **(III)** wherein R¹, R², R³, R⁴, R⁷, R¹³ and n are as defined above is rearranged in e.g. NMP at reflux temperature into phenol **(IV)** wherein R¹, R², R³, R⁴,R⁷, R¹³ and n are as defined above. Treatment of phenol **(IV)** wherein R¹, R², R³, R⁴, R⁷, R¹³ and n are as defined above with 90% formic acid followed by basic hydrolysis and acidic work up affords dihydrobenzofurane **(V)** wherein R¹, R², R³, R⁴, R⁷ and n are as defined above. Dihydrobenzofurane **(V)** wherein R¹, R², R³, R⁴, R⁷ and n are as defined above is coupled with an amine **(VI)** wherein R⁵ and R⁶ are defined as above under standard amide forming conditions using a coupling reagent (a) (e.g. HOBT, EDAC and DIPEA in dry THF) affording amide **(VII)** wherein R¹ R², R³, R⁴, R⁵, R⁶, R⁷ and n are as defined above.

### General method B:

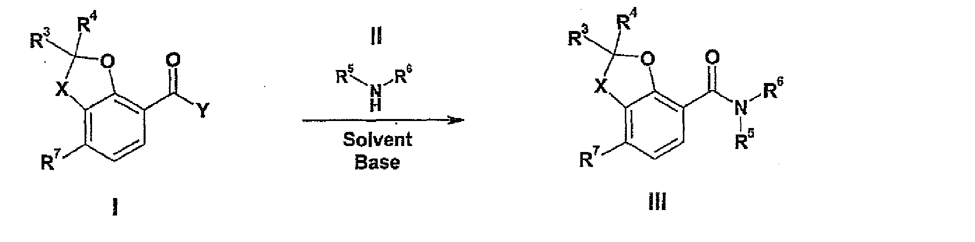

By allowing an acid derivative **(I)** wherein Y is halo, R13(C=O)O-, C₁-C₆alkyloxy or arylC₁-C₆alkyloxy R¹³ is C₁-C₆alkyl or arylc₁-c₆alkyl and R³, R⁴, R⁷ and X are defined as above to react with an amine **(II)** wherein R⁵ and R⁶ are defined as above under basic conditions (e.g. triethylamine, K₂CO₃, NaH and the like) in a solvent (e.g. THF, DCM, DMF, NMP and the like) affording amide **(III);** wherein R³, R⁴, R⁵, R⁶, R⁷ and X are defined as above.

In the following "general methods C to H" is provided guidelines for synthesis of substituted benzofuranes-, 2,3-dihydrobenzofuranes-7-carboxylic acids and croman-8-carboxylic acids (compound V in scheme 1) according to literature references;

### General method C:

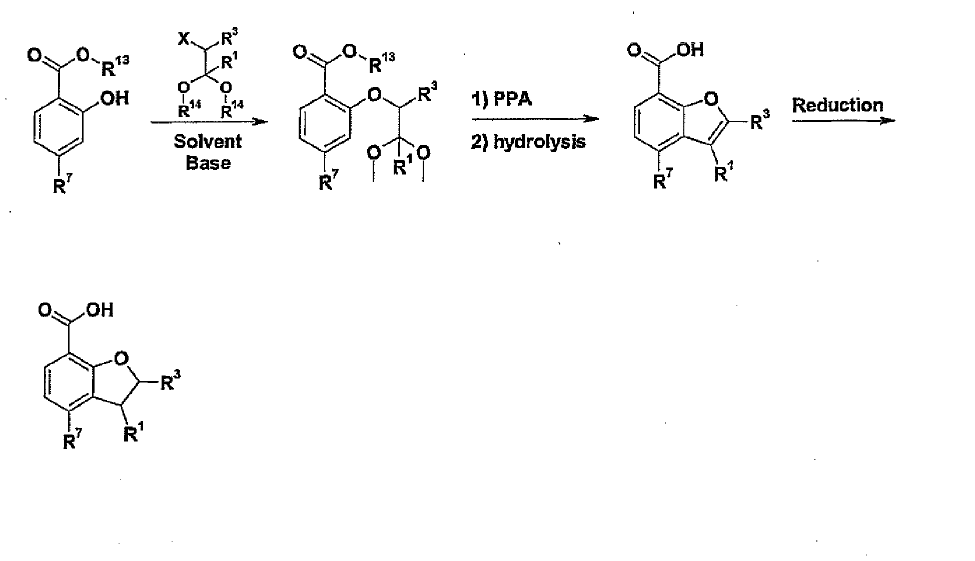

wherein X is halo, R¹³ and R¹⁴ are independently C₁-C₆alkyl or arylC₁-C₆alkyl and R¹, R³, and R⁷ are defined as above.
Cherif, M.; Cotelle, P.; Catteau, J.-P.; Heterocycles(1992),34,1749-1758. Barker, P.; Finke, P.; Thompson, K.; Synth Commun (1989), (19), 257.

### General method D:

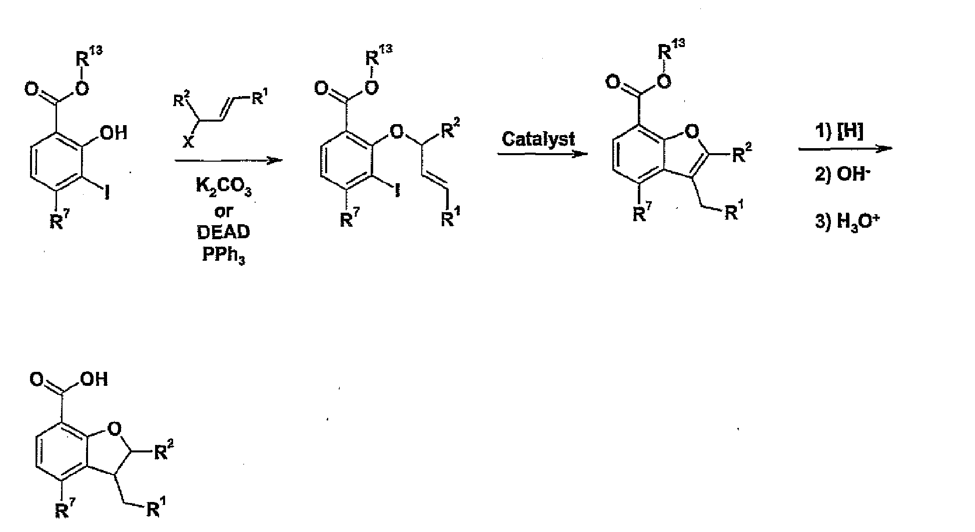

wherein X is OH or halo; R¹³ is C₁-C₆alkyl or arylC₁-C₆alkyl and R¹, R³, and R⁷ are defined as above.
Catalyst: AIBN: Graham, S. R.; Murphy, J. A.; Coates, D.; Tetrahedron Lett., 40, (1999), 2415-2416.
Catalyst: Pd(OAc)₂: Larock, R. C.; Stinn, D. E.; Tetrahedron Lett.; 29; (1988), 4687 - 4690.

### General method E:

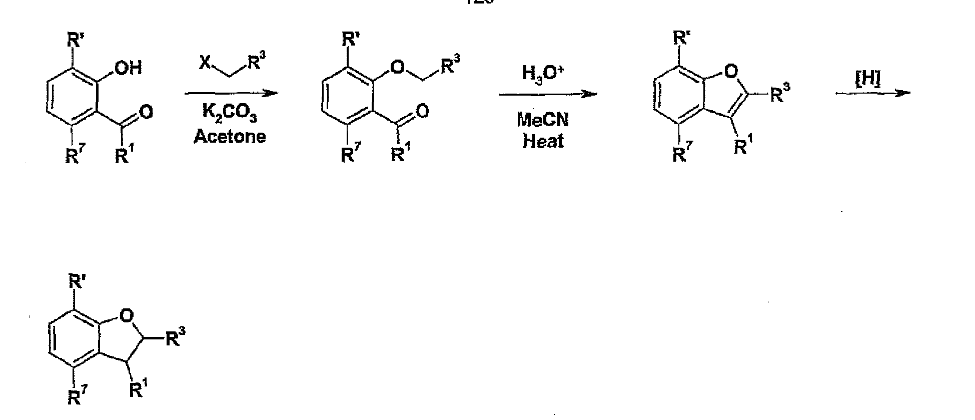

wherein R' is CN or COOR"; R¹ is hydrogen, C₁-C₆alkyl, aryl, heteroaryl or carylC₁-C₆alkyl; R³ is aryl, heteroaryl, cyano, COR¹³, nitro or COOR", wherein R" and R¹³ independently are C₁-C₆alkyl, aryl, heteroaryl, or arylC₁-C₆alkyl;
Lau, C. K. et al.; J. Med. Chem. 32, (1989) 1190-1197.
For conversion of R' = CN to COOH see e.g. Cagniant; Bull. Soc. Chim. Fr,; (1957), 827 - 834.

### General method F:

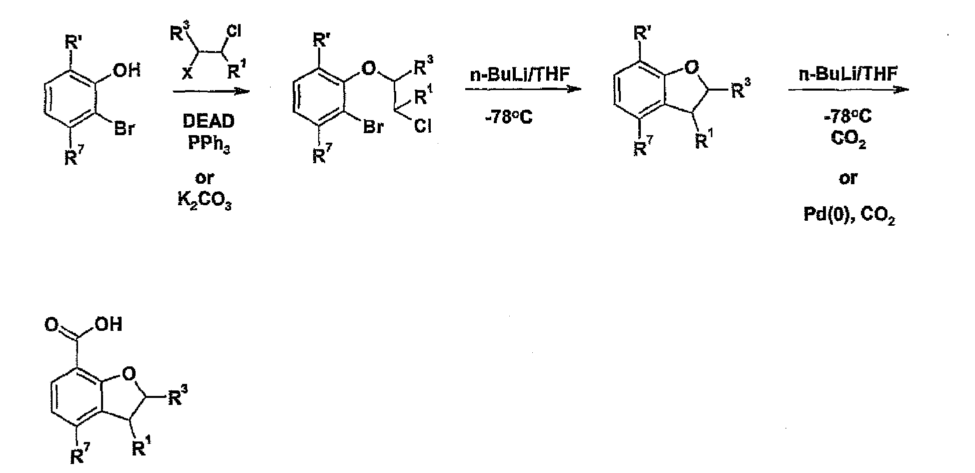

wherein R' is hydrogen or bromo; R¹, R³ and R⁷ are defined as above;
Al-bojuk, N. R.; El-Abadelah, M. M.; Sabri, S. S.; Michel, A.; Voelter, W.; M.-Moessmer, C.; Al-Abed, Y.; Heterocycles; 55, (2001), 1789-1804.
Stanetty, P.; Koller, H.; Puerstinger, G.; Monatsh.Chem., 121, (1990), 883-891.

### General method H:

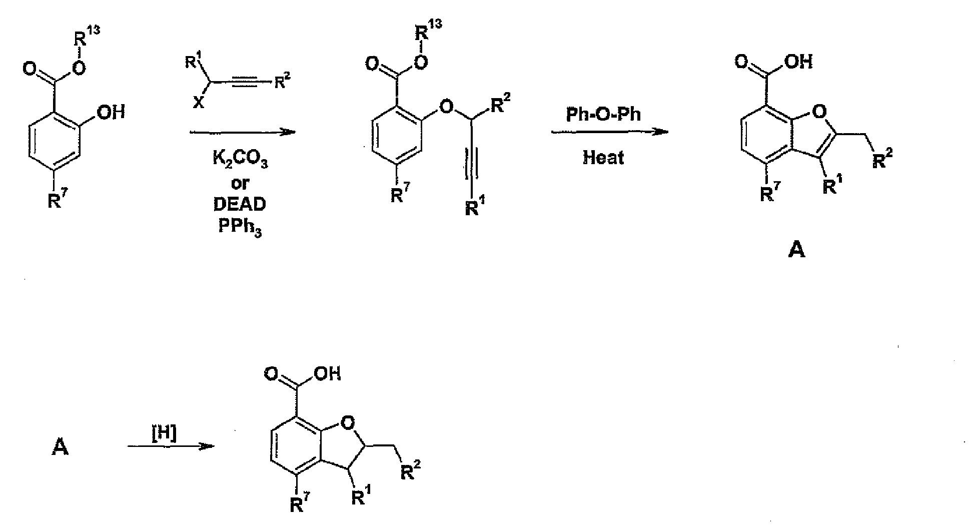

wherein R¹³ is C₁₋C₆alkyl, aryl, heteroaryl, or arylC₁-C₆alkyl and R¹, R² and R⁷ are defined as above;
Kakigami, T.; Baba, K.; Usui, T.; Heterocycles; 48, (1998), 2611 - 2620.

### Example 1 (General method B)

### 2,3-Dimethyl-2.3-dihydro-benzofuran-7-carboxylic acid cyclohexyl-methyl-amide

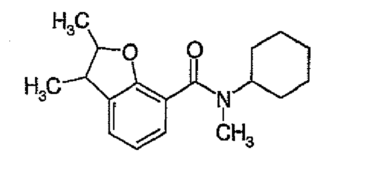

To a solution of 2,3-dimethyl-2,3-dihydro-benzofuran-7-carboxylic acid (0.5 g, 2.60 mmol), HOBT (0.39 g, 2.86 mmol) in dry THF (25 ml) was added EDAC (0.55 g, 2.86 mmol). The mixture was stirred for 10 min. followed by addition of di-isopropyl ethyl amine (DIPEA) (0.5 ml, 2.86 mmol) and cyclohexyl-methyl-amine (0.37 ml, 2.86 mmol). The resulting mixture was stirred for 16 hrs. at room temperature, the volatiles were evaporated *in vacuo* and to the residue was added water (25 ml) and diethyl ether (75 ml). The organic phase was separated and dried (Na₂SO₄), filtered and the solvent evaporated *in vacuo.* The residue was dissolved in a mixture ofAcOEt/Heptane (1:1) and filtered through a 2.5 cm silicagel plug. The solvent was evaporated *in vacuo* affording 0.7 g (93%) of the title compounds as an oil. ¹H NMR (300 MHz, CDCl₃) δ 1.06 (m, 3H), 1.29 (m, 4H), 1.48 (m, 5H), 1.67-1.8 (m, 4H), 2.77 and 2.97 (d and s, 3H, rotamer), 3.05 and 4.37 (2 x m, 1H), 3.35 and 4.55 (2 x m, 1H), 3.39 and 4.89 (2 x m, 1H), 6.86 (t, 1H), 7.12 (t, 2H).
Calculated for C₁₈H₂₅NO₂, 0.15 H₂O C, 74.52 %; H, 8.79 %; N, 4.83 %. Found:
C, 74.54 %; H, 8.98 %; N, 4.98 %.

The following compounds were synthesised in a similar way as described in Example 1 (general method B).

| **No.** | **Structure** | **MW** | **lupac Name** |
|---|---|---|---|
| B-1 | 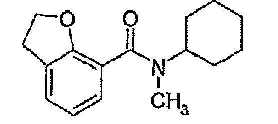 | 259.35 | 2,3-Dihydro-benzofuran-7-carboxylic acid cyclo-hexyl-methyl-amide |
| B-2 | 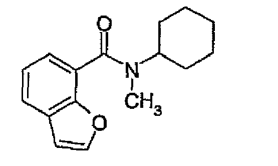 | 257.34 | Benzofuran-7-carboxylic acid cyclohexyl-methyl-amide |
| B-3 | 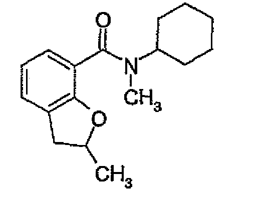 | 273.38 | 2-Methyl-2,-dihydro-benzofuran-7-carboxylic acid cyclohexyl-methyl-amide |
| B-4 | 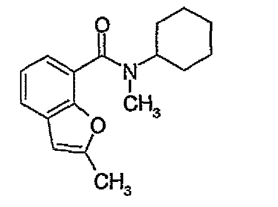 | 271.36 | 2.Methyl-benzofuran-7-carboxylic acid cyclohexyl-methyl-amide |
| B-5 | 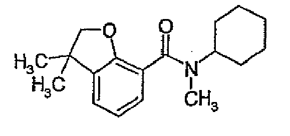 | 287.41 | 3,3-Dimethyl-2,3-dihydro-benzofuran-7-carboxylic acid cyclohexyl-methyl-amide |
| B-6 | 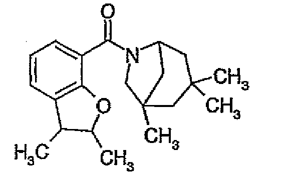 | 327.47 | (2,3-Dimethyl-2,3-dihydro-benzofuran-7-yl)-(2,4,4-trimethyl-6-aza-bicyclo[3.2.1]oct-6-yl)-methanone |
| B-7 | 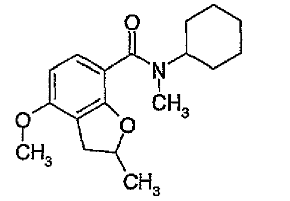 | 303.40 | 4-Methoxy-2-methyl-2,3-dihydro-benzofuran-7-carboxylic acid cyclohexyl-methyl-amide |
| B-8 | 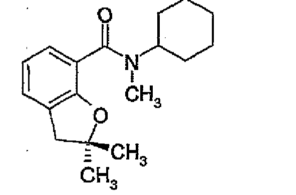 | 287.41 | 2,2-Dimethyl-2,3-dihydro-benzofuran-7-carboxylic acid cyclohexyl-methyl-amide |
| B-9 | 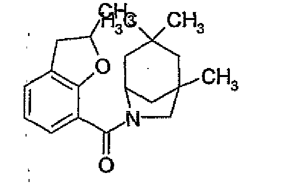 | 313.44 | (2-Methyl-2,3-dihydro-benzofuran-7-yl)-(1,3,3-trimethyl-6-aza-bicydo[3.2.1]oct-6-yl)-methanone |
| B-10 | 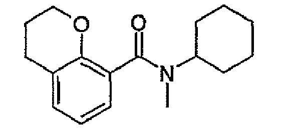 | 273.37 | Chroman-8-carboxylic acid cyclohexyl-methyl-amide |

### EXAMPLES, COMPOUNDS OF GENERAL FORMULA (VII)

The following examples and general procedures refer to intermediate compounds and final products for general formula (VII) identified in the specification and in the synthesis schemes. The preparation of the compounds of general formula (VII) of the present invention is described in detail using the following examples. Occasionally, the reaction may not be applicable as described to each compound included within the disclosed scope of the invention. The compounds for which this occurs will be readily recognised by those skilled in the art. In these cases the reactions can be successfully performed by conventional modifications known to those skilled in the art, that is, by appropriate protection of interfering groups, by changing to other conventional reagents, or by routine modification of reaction conditions. Alternatively, other reactions disclosed herein or otherwise conventional will be applicable to the preparation of the corresponding compounds of the invention. In all preparative methods, all starting materials are known or may easily be prepared from known starting materials. The structures of the compounds are confirmed by nuclear magnetic resonance (NMR), where peaks assigned to characteristic protons in the title compounds are presented where appropriate. ¹H NMR shifts (δ_{H}) are given in parts per million (ppm) down field from tetramethylsilane as internal reference standard. M.p.: is melting point and is given in °C and is not corrected. Column chromatography was carried out using the technique described by W.C. Still et al., J. Org. Chem. 43: 2923 (1978) on Merck silica gel 60 (Art. 9385). LC-MS analyses are performed using Waters XTerra MS C-3 x 18 mm RP-C18 column eluted with various mixtures of water and acetonitrile, flow = 1 mL/min, with UV detection at 210 nm and MS scanning (ES+) from 100-1000 amu. An injection volume of 1µL was used.

**Microwave oven synthesis:** The reaction was heated by microwave irradiation in sealed microwave vessels in a single mode Emrys Optimizer EXP from PersonalChemistry^{®}.

**Solid-phase synthesis:** All reactions were performed in Teflon apparatus suitable for solid-phase synthesis or on an ACT 496 robot employing the standard procedures described.

**Preparative HPLC:** Column: 1.9 x 15 cm Waters XTerra RP-18. Buffer: linear gradient 5 - 95 % in 15 min, MeCN, 0.1% TFA, flow rate of 15 mL/min. The pooled fractions are either evaporated to dryness *in vacuo,* or evaporated *in vacuo* until the MeCN is removed, and then frozen and freeze dried.

The abbreviations as used in the examples have the following meaning:
- TLC:: Thin layer chromatography
- CDCl₃:: Deuterio chloroform
- CD₃OD: Tetradeuterio methanol
- DMSO-d₆:: Hexadeuterio dimethylsulfoxide
- DMSO:: Dimethylsulfoxide
- THF:: Tetrahydrofuran
- DMF:: N,N-dimethylformamide
- HOBT:: 1-Hydroxy-benzotriazole
- EDAC:: 1-(3-Ddimethylaminopropyl)-3-ethylcarbodiimide, hydrochloride
- min:: minutes
- hrs:: hours

The compounds of the invention are prepared as illustrated in the following reaction schemes:

### General method A

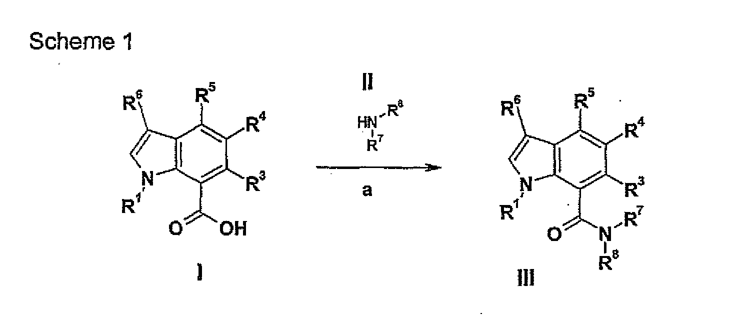

Indole-7-carboxylic acids (1) wherein R¹, R³, R⁴, R⁵ and R⁶ are as defined above are generally prepared as described in the following literature references;
Clark, R. D. et al.; J. Heterocycl. Chem. 22, (1985), 121-125.
Kasahara, A. ef al.; J. Heterocycl. Chem. 26, (1989), 1405-1413.
Kamiya, S. et al.; Chem. Pharm. Bull, 43, (1995), 1692-1695.
Somei, M. et a/.; Chem. Pharm. Bull. 34, (1986), 4116-4125.
Wiedenau, P. et al.; Synth. Commun. 27, (1997), 2033-2040.
Soederberg, B. C. et al.; J. Org. Chem. 62, (1997), 5838-5845.

By allowing an acid (I) wherein R¹, R³, R⁴, R⁵ and R⁶ are as defined above to be coupled with an amine (II) wherein R⁷ and R⁸ are defined as above under standard amide forming conditions using a coupling reagent (a) (e.g. HOBT, EDAC and DIPEA in dry DMF) affording amide (III) wherein R¹, R³, R⁴, R⁵, R⁶, R⁷ and R⁸ are as defined above.

### General method B

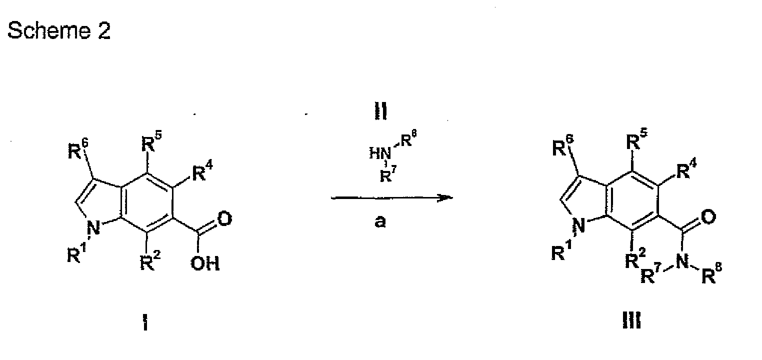

Indole-6-carboxylic acids (I) wherein R¹, R², R⁴, R⁵ and R⁶ are as defined above are generally prepared as described in the following literature references;
Kermack; J. Chem. Soc. 125, (1924), 2288.
Tischler, A. N.; Lanza, T. J.; Tetrahedron Lett. 27, (1986), 1653-1656.
Gharagozloo, P. et al.; Tetrahedron, 52, (1996), 10185-10192.
Zhang, H.-C. et al.; Tetrahedron Lett. 39, (1998), 4449-4452.
Kasahara, A. et al.; J. Heterocycl. Chem. 24, (1987), 1555-1556.
Brown, F. J. et al.; J. Med. Chem. 35, (1992), 2419-2439.
Izumi, T. et al.; J. Heterocycl. Chem. 29, (1992), 1625-1629.
Soederberg, B. C and Shriver, J. A.; J. Org. Chem. 62, (1997), 5838-5845.
Kitano, M. et al.; Chem. Pharm. Bull. 47, (1999), 1538-1548.
Snyder et al.; J. Am. Chem, Soc. 80, (1958), 4622-4624.

By allowing an acid (I) wherein R¹, R², R⁴, R⁵ and R⁶ are as defined above to be coupled with an amine (II) wherein R⁷ and R⁸ are defined as above under standard amide forming conditions using a coupling reagent (a) (e.g. HOBT, EDAC and DIPEA in dry DMF) affording amide (III) wherein R¹, R², R⁴, R⁵, R⁶, R⁷ and R⁸ are as defined above.

### General method C

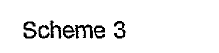

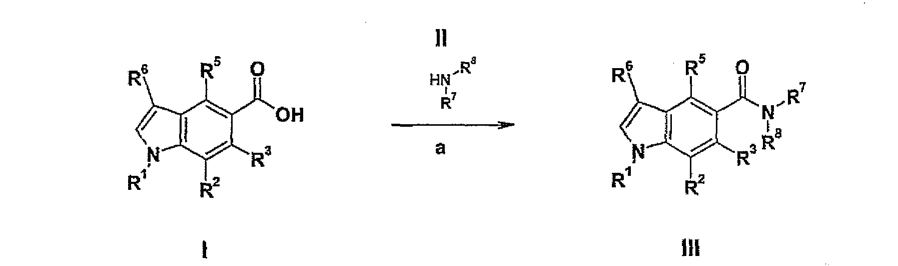

Indole-5-carboxylic acids **(I)** wherein R¹, R², R³, R⁵ and R⁶ are as defined above are generally prepared as described in the following literature references;
Singer, S., J. Org. Chem. 20, (1955), 1458.
Noland, W. E. and Reich, C.; J. Org. Chem. 32, (1967), 828-832.
Street, L. J. et al.; J. Med. Chem. 36, (1993), 1529-1538.
Bosch, J. et al.; Tetrahedron, 57, (2001), 1041-1048.
Agarwal, A. et al.; J. Med. Chem. 36, (1993), 4006-4014.
Kasahara, A. et al.; J. Heterocycl. Chem. 26, (1989); 1405-1413.
Boettcher, H. and Gericke, R.; Liebigs Ann. Chem. (1988), 749-752.
Somel, M. et al.; Chem. Pharm. Bull. 34, (1986),4116-4125.
Kamiya, S. et al.; Chem. Pharm. Bull. 43, (1995), 1692-1695.
Odle, R. et a/.; J. Org. Chem. 45, (1980), 2709-2710.

By allowing an acid **(I)** wherein R¹, R², R³, R⁵ and R⁶ are as defined above to be coupled with an amine **(II)** wherein R⁷ and R⁸ are defined as above under standard amide forming conditions using a coupling reagent (a) (e.g. HOBT, EDAC and DIPEA in dry DMF) affording amide **(III)** wherein R¹. R², R³, R⁵, R⁶, R⁷ and R⁸ are as defined above.

### General method D

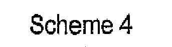

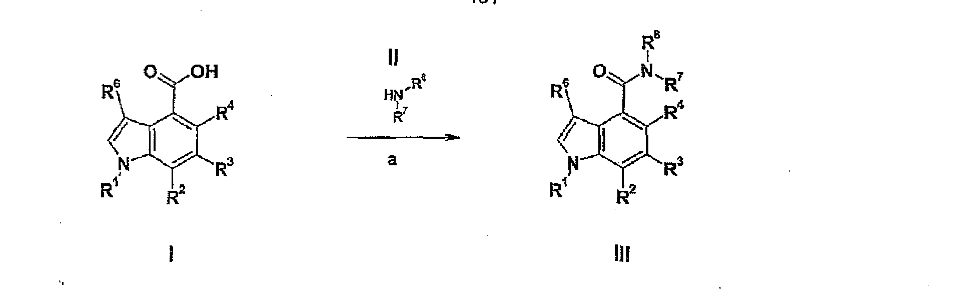

Indole-4-carboxylic acids (I) wherein R¹, R², R³, R⁴ and R⁶ are as defined above are generally prepared as described in the following literature references;
Beswick, P. J. et al.; Tetrahedron 44, (1988), 7325.
Muchowski, J. M. et al.; Tetrahedron Lett. 28, (1987), 3453.
Kasahara, A. et al.; J. Heterocycl. Chem. 26, (1989); 1405-1413.
Kasahara, A. et al.; J. Heterocycl. Chem. 24, (1987), 1555-1556.
Clark, R. D. et al.; J. Heterocycl. Chem. 22, (1985), 121-125.
Kruse, L. I. Heterocycles, 16, (1981),1119-1124.
Soederberg, B. C. Org. Synth. 80, (2002), 75.

By allowing an acid (I) wherein R¹, R², R³, R⁴ and R⁶ are as defined above to be coupled with an amine (II) wherein R⁷ and R⁸ are defined as above under standard amide forming conditions using a coupling reagent (a) (e.g. HOBT, EDAC and DIPEA in dry DMF) affording amide (III) wherein R¹, R², R³, R⁴, R⁶, R⁷ and R⁸ are as defined' above.

### General method E

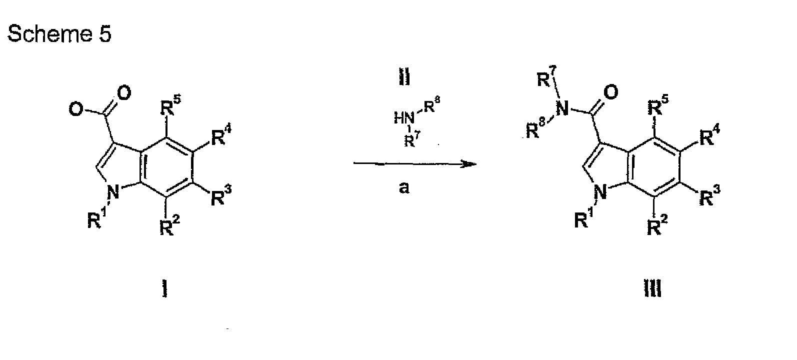

lndole-3-carboxylic acids (I) wherein R¹, R², R³, R⁴ and R⁵ are as defined above are generally prepared as described in the following literature references;
Weissgerber; Chem. Ber. 43, (1910), 3526.
Whalley; J. Chem. Soc. (1954), 1651.
Bravo, P. et al.; Tetrahedron Lett. 10, (1969),679.
Sus, O. et al.; Justus Liebigs Ann. Chem. (1953), 583, 150.
Melzer, M. S. et al.; J. Org. Chem. 27, (1962),496.
Amat, M. et al.; J. Org. Chem. 59, (1994), 10-11.

By allowing an acid **(I)** wherein R¹, R², R³, R⁴ and R⁶ are as defined above to be coupled with an amine **(II)** wherein R⁷ and R⁸ are defined as above under standard amide forming conditions using a coupling reagent (a) (e.g. HOBT, EDAC and DIPEA in dry DMF) affording amide **(III)** wherein R¹, R², R³, R⁴, R⁵, R⁷ and R⁸ are as defined above.

### Example 1

### (1H-Indol-5-yl)-(1,3,3-trimethyl-6-aza-bicyclo[3.2.1]oct-6-yl)-methanone

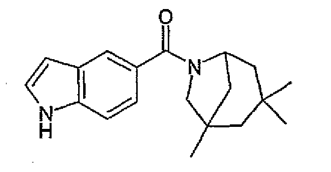

A solution of 1*H*-indole-5-carbooylic acid (1.0 g, 6.21 mmol), HOBT (0.9 g, 6.83 mmol) in dry THF (50 mL) and EDAC(1.31 g, 6.83 mmol) was stirred for 10 mins. Di-isopropyl ethyl amine (DIPEA) (1.2 mL, 6.83 mmol) and 6-aza-bicyclo[3.2.1]octyl)-amine (1.16 mL, 6.83 mmol) was added and the resulting mixture was stirred for 16 hrs. at room temperature. The volatiles were evaporated *in vacuo* and to the residue was added water (25 mL) followed by extraction with diethyl ether (2 x 25 mL). The combined organic phases were dried (Na₂SO₄), filtered and evaporated *in vacuo.* The residue was purified by silicagel chromatography using a mixture of ethyl acetate and heptane (1:2) as eluent. Pure fractions were collected and the solvent evaporated *in vacuo.* To the residue was added diethyl ether (10 mL) and the resulting mixture was stirred for 1 h. The precipitate was filtered off and dried *in vacuo* at 50 °C affording 1.3 g (71 %) of the title compounds as a solid.

MS-ESI *m*/*z* 297; ¹H NMR (300 MHz, CDCl₃) δ 0.93 - 1.44 (m, 13H), 1.62 (m, 1H), 1.76 (m, 1H), 2.29 and 3.28 (2x m, 1H), 3.31 and 3.62 (2x m, 1H), 4.10 and 4.65 (2x m, 1H), 6.59 (s, 1H), 7.22-7.35 (m, 3H), 7.74 (d, 1H), 8.69 (bs, 1H, N*H*).

### Example 2

### 1H-Indole-6-carboxylic acid cyclohexyl-methyl-amide

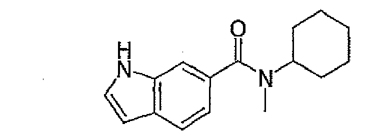

A solution of 1*H*-indole-6-carboxylic acid (1.0 g, 6.21 mmol), HOBT (0.92 g, 6.83 mmol) in dry THF (50 mL) and EDAC (1.31 g, 6.83 mmol) was stirred for 20 mins. Diisopropyl ethyl amine (DIPEA) (1.2 mL, 6.83 mmol) and cydohexyl-methyl-amine (0.9 mL, 6.83 mmol) was added and the resulting mixture was stirred for 16 hrs. at room temperature. To the precipitate was added water (50 mL), the solid filtered off, washed with water followed by diethyl ether and dried *in vacuo* at 50°C which afforded 0.6 g (38%) of the title compounds as a solid.
¹H NMR(300 MHz, CDCl₃) δ 1.05 (bm, 2H), 1.15-1.85 (m, 8H), 2.96 (bs, 3H), 3.65 and 4.55 (2x m, 1H), 6.54 (s, 1H), 7.09 (d, 1H), 7.24 (m, 1H), 7.50 (s, 1H), 7.60 (d, 1H), 8.89 (bs, 1H, N*H*).
Calculated for C₁₆H₂₀N₂O;
C, 74.97 %; H, 7.86 %; N, 10.93 %. Found:C, 74.90 %; H, 8.01 %; N, 10.88 %.

### Example 3

### (1H-Indol-7-yl)-(1.3.3-trimethyl-6-aza-bicvclo[3.2.1]oct-6-yl-methanone

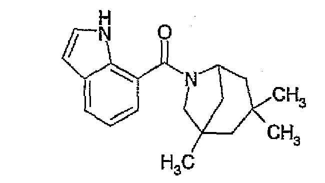

A solution of 1*H*-indole-7-carboxylic acid (323 mg, 2 mmol), HOAt (300 mg, 2.2 mmol) in dry DMF (10 mL) and EDAC (500 mg, 2.6 mmol) was stirred for 10 mins. Triethyl-amine (TEA) (0.84 mL, 6 mmol) and 6-aza-bicyclo[3.2.1]octyl)-amine (338 mg, 2.2 mmol) was added and the resulting mixture was stirred for 16 hrs. at room temperature. The volatiles were evaporated *in vacuo* and to the residue was added water (10 mL) followed by extraction with dichloromethane (DCM, 2 x 25 mL). The combined organic phases were dried (MgSO₄), filtered and evaporated *in vacuo.* The residue was purified by preparative HPLC, dried *in vacuo* at 50°C affording 283 mg (47%) of the title compound as a solid.

MS-ESI *m*/*z* 297;¹H NMR (300 MHz, DMSO) δ 0.85-1.40 (m, 13H), 1.50-1.54 (m, 1H), 1.80-1.86 (m, 1H), 2.19-2.24 and 2.43-2.57 (2x m, 1H), 3.13 and 3.32 (m, 1H), 3.84-3.87 and 4.47-4.50 (2x m, 1H), 6.46-6.49 (m, 1H), 7.00 - 7.19 (m, 2H), 7.30-7.34 (m, 1H), 7.60-7.63 (m, 1H), 10.97 (d, 1H, N*H*).

### Example 4

### (1H-indot-6-yl)-(1.3,3-trirnethyl-6-aza-bicyclo[3.2.1]oct-6-yl)-methanone

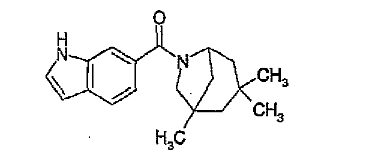

A solution of 1*H*-indole-6-carboxylic acid (1 g, 6.2 mmol), HOAt (929 mg, 6.82 mmol) in dry DMF (20 mL) and EDAC (1.54 g, 8.06 mmol) was stirred for 10 mins. TEA (2.59 mL, 18.6 mmol) and 6-aza-bicyclo[3.2.1]octyl)-amine (1.04 g, 6.82 mmol) was added and the resulting mixture was stirred for 5 hrs. at room temperature. The volatiles were evaporated *in vacuo* and to the residue was added water (10 mL) followed by extraction with dichloromethane (DCM, 2 x 50 mL). The combined organic phases were dried (MgSO₄), filtered and evaporated *in vacuo.* The residue was purified by silicagel chromatography using a mixture of ethyl acetate and heptane (3:7) as eluent. Pure fractions were collected, the solvent evaporated *in vacuo* and dried *in vacuo* at 50 °C affording 1.6 g (87%) of the title compounds as a solid.

MS-ESI *m*/*z* 297; ¹H NMR (300 MHz, CDCl₃) δ 0.93 (d, 3H), 1.02 (d, 3H), 1.11-1.15 (m, 4H), 1.26-1.39 (m, 2H), 1.56-1.66 (m, 1H), 1.73-1.79 and 2.27-2.33 (2x m, 2H), 3.22-3.35 and 3.61-3.65 (m, 2H), 4.07-4.10 and 4.62-4.65 (2x m, 1H), 6.52-6.54 (m, 1H), 7.17 - 7.25 (m, 2H), 7.53-7.63 (m, 2H), 9.03 (bs, 1H, N*H*).

The following compounds were synthesised employing a similar method to the ones described in examples 1, 2, 3 and 4 above:

| **No** | **Molecule** | **MW** | **IUPAC Name** | **MS-ESI *mlz*** |
|---|---|---|---|---|
| 4-1 | 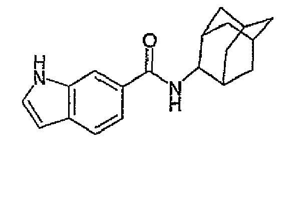 | 294.40 | 1*H*-Indole-6-carboxylic acid adamentan-2-ylamide | 295 |
| 4-2 | 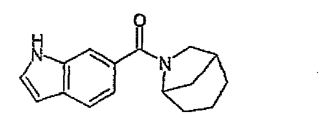 | 254,33 | (6-Aza-bicyclo[3.2.1]oct-6-yl)-(1*H*-indol-6-yl)-methanone | 255 |
| 4-3 | 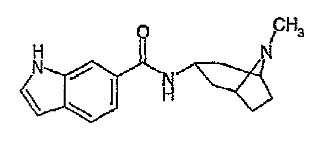 | 283,37 | 1*H*-Indole-6-carboxylic acid (8-methyl-8-aza-bicyclo[3.2,1]oct-3-yl)-amide | 284 |
| 4-4 | 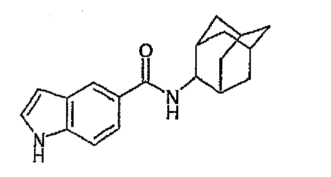 | 294.40 | 1*H*-indole-5-carboxylic acid adamantan-2-ylamide | 295 |
| 4-5 | 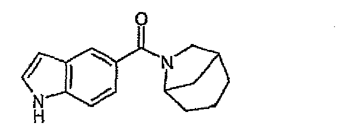 | 254.33 | (6-Aza-bicyclo[3.2.1]oct-6-yl)-(1*H*-indol-5-yl)-methanone | 255 |
| 4-6 | 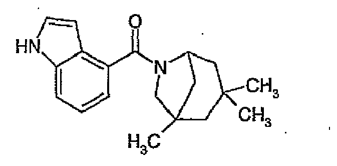 | 296.40 | (1*H*-Indol-4-yl)-(1,3,3-trimethyl-6-aza-bicyclo[3.2.1]oct-6-yl)-methanone | 297 |
| 4-7 | 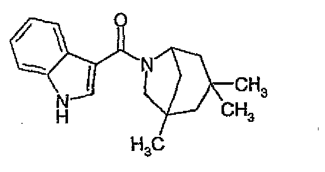 | 296.40 | (1*H*-Indol-3-yl)-(1,3,3-trimethyl-6-aza-bicyclo[3.2.1]oct-6-yl)-methanone | 297 |
| 4-8 | 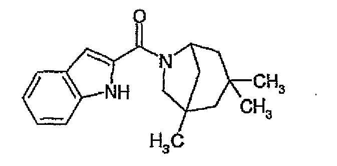 | 296.40 | (1*H*-indol-2-yl)-(1,3,3-trimethyl-6-aza-bicyclo[3.2.1]oct-6-yl)-methanone | 297 |
| 4-9 | 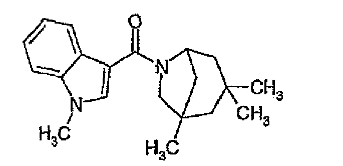 | 310.44 | (1-Methyl-1*H*-indol-3-yl)-(1,3,3-trimethyl-6-aza-bicyclo[3.2.1]oct-6-yl)-methanone | 311 |
| 4-10 | 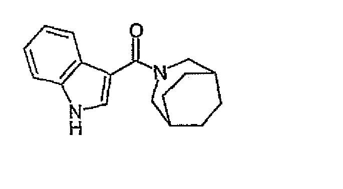 | 268.36 | (3-Aza-bicyclo[3.2.2]non-3-yl)-(1*H*-indol-3-yl)-methanone | 269 |
| 4-11 | 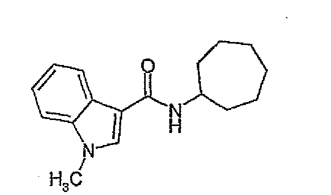 | 270.38 | 1-Methyl-1*H*-indole-3-carboxylic acid cycloheptyla-mide | 271 |
| 4-12 | 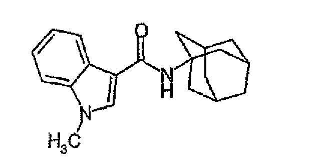 | 308.42 | 1-Methyl-1*H*-indole-3-carboxylic acid adamantan-1-ylamide | 309 |
| 4-13 | 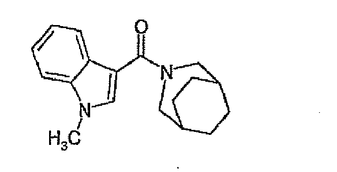 | 282.39 | (3-Aza-bicyclo[3.2.2]non-3-yl)-(1-methyl-1*H*-indol-3-yl)-methanone | 283 |
| 4-14 | 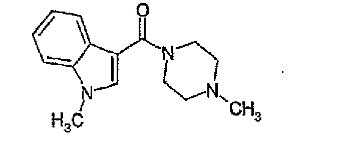 | 257.34 | (1-Methyl-1*H*-indol-3-yl)-(4-methyl-piperazin-1-yl)-methanone | 258 |
| 4-15 | 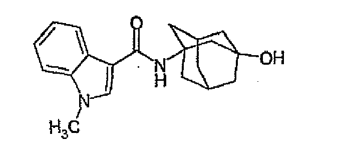 | 324.43 | 1-Methyl-1*H*-indole-3-carboxylic acid (3-hydroxy-adamantan-1-yl)-amide | 325 |
| 4-16 | 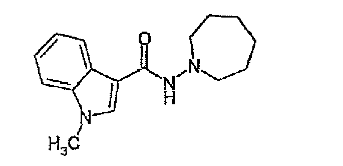 | 324.42 | 1-Methyl-1*H*-indole-3-carboxylic acid azepan-1-ylamide | 325 |
| 4-17 | 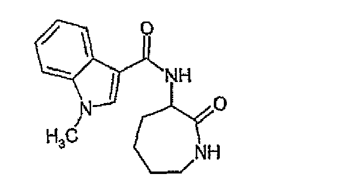 | 285.35 | 1-Methyl-1*H*-indole-3-carboxylic acid (2-oxo-azepan-3-yl)-amide | 286 |
| 4-18 | 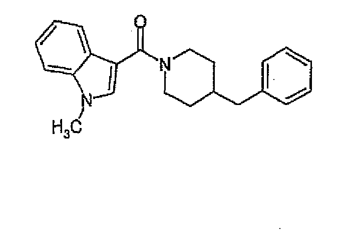 | 332.45 | (4-Benzyl-piperldine-1-yl)-(1-methyl-1*H*-indol-3-yl)-methanone | 333 |
| 4-19 | 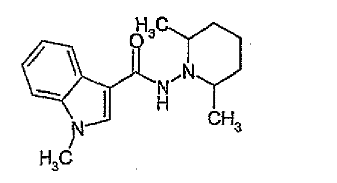 | 285.39 | 1-Methyl-1*H*-indole-3-carboxylic acid (2,6-dimethyl-piperidin-1-yl)-amide | 286 |
| 4-20 | 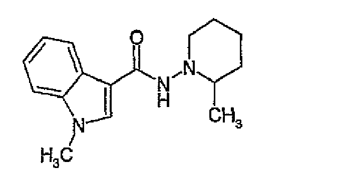 | 256.35 | 1-Methyl-1*H*-indole-3-carboxylic acid (2-methyl-piperidin-1-yl)-amide | 257 |
| 4-21 | 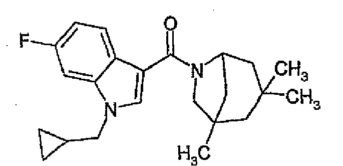 | 368.50 | (1-Cyclopropylmethyl-6-fluoro-1*H*-indo)-3-yl)-(1,3,3-trimethyl-6-aza-bicyclo[3.2.1]oct-6-yl)-methanone | 369 |
| 4-22 | 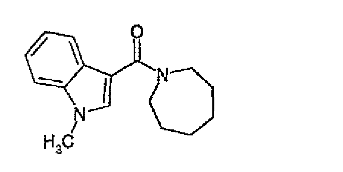 | 256.35 | Azepan-1-yl-(1-methyl-1*H*-indol-3-yl)-methanone | 257 |
| 4-23 | 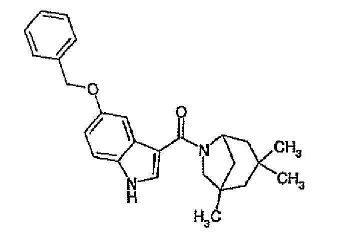 | 402.54 | (5-Benzyloxy-1*H*-indol-3-yl)-(1,3,3-trimethyl-6-aza-bicyclo[3.2.1]oct-6-yl)-methanone | 403 |
| 4-24 | 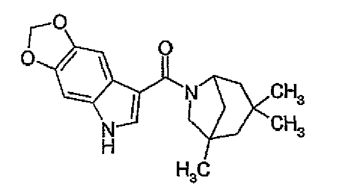 | 340.42 | (5*H*-[1,3]Dioxolo[4,5]indol-7-yl)-(1,3.3-trimethyl-6-aza-bicycto[3.2.1]oct-6-yl)-methanone | 341 |
| 4-25 | 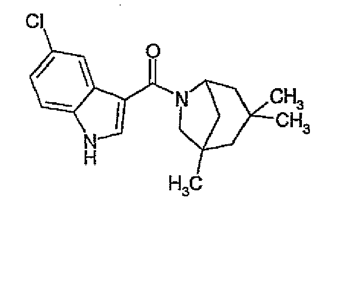 | 330.86 | (5-Chloro-1*H*-indol-3-yl)-(1,3,3-trimethyl-6-aza-bicyclo[3.2.1]oct-6-yl)-methanone | 332 |
| 4-26 | 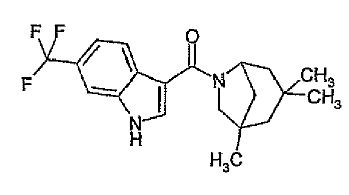 | 364.41 | (6-Trifluoromethyl-1*H*-indol-3-yl)-(1,3,3-trimethyl-6-aza-bicyclo[3.2.1]oct-6-yl)-methanone | 365 |
| 4-27 | 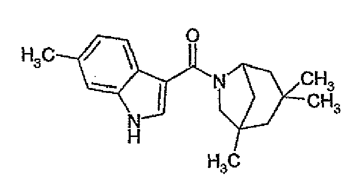 | 310.44 | (6-Methyl-1*H*-indol-3-yl)-(1,3,3-trimethyl-6-aza-bicyclo[3.2.1]oct-6-yl)-methanone | 311 |
| 4-28 | 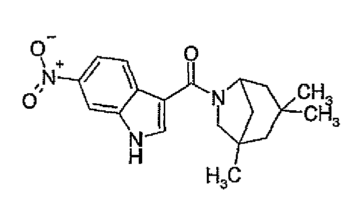 | 341.41 | (6-Nitro-1*H*-indol-3-yl)-(1,3,3-trimethyl-6-aza-bicyclo[3.2.1]oct-6-yl)-methanone | 342 |
| 4-29 | 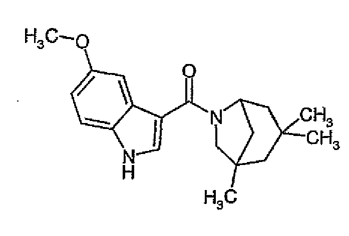 | 326.44 | (5-Methoxy-1*H*-indol-3-yl)-(1,3,3-trimethyl-6-aza-bicyclo[3.2.1]oct-6-yl)-methanone | 327 |
| 4-30 | 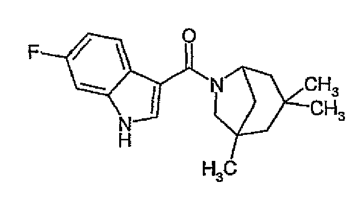 | 314.40 | (6-Fluoro-1*H*-indol-3-yl)-(1,3,3-trimethyl-6-aza-bicyclo[3.2.1]oct-6-yl)-methadone | 315 |
| 4-31 | 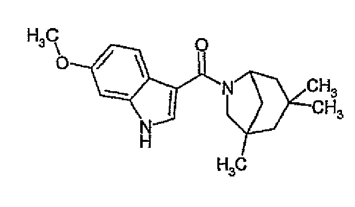 | 326.44 | (6-Methoxy-1*H*-indol-3-yl)-(1,3,3-trimethyl-6-aza-bicyclo[3.2,1]oct-6-yl)-methanone | 327 |
| 4-32 | 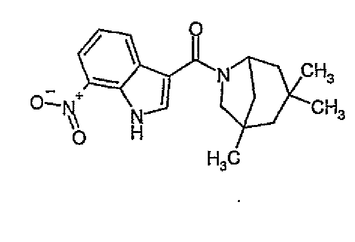 | 341.41 | (7-Nitro-1*H*-indol-3-yl)-(1,3,3-trimethyl-6-aza-bicyclo[3.2.1]oct-6-yl-methanone | 342 |
| 4-33 | 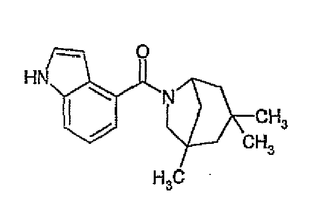 | 296.40 | (1*H*-indol-4-yl)-(1,3,3-trimethyl-6-aza-bicyclo[3.2.1]oct-6-yl)-methadone | 297 |
| 4-34 | 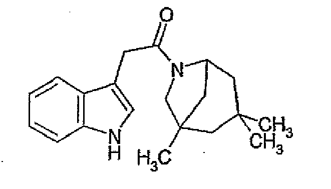 | 310.44 | 2-(1*H*-Indol-3-yl)-1-(1,3,3-trimethyl-6-aza-bicyclo[3.2.1]oct-6-yl)-ethanone | 311 |
| 4-35 | 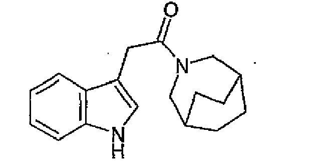 | 282.39 | 1-(3-Aza-bicyclo[3.2.2]non-3-yl)-2-(1*H*-indol-3-yl)-ethanone | 283 |
| 4-36 | 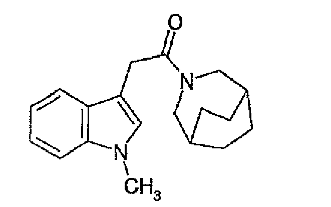 | 296.42 | 1-(3-Aza-bicyclo[3.2.2]non-3-yl)-2-(1-methyl-1*H*-indol-3-yl)-ethanone | 297 |
| 4-37 | 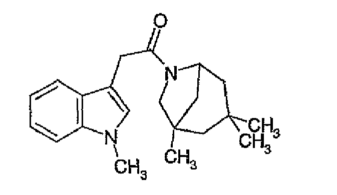 | 324.47 | 2-(1-Methyl-1*H*-indol-3-yl)-1-(1,3,3-trimethyl-6-aza-bicyclo[3.2.1]oct-6-yl)-ethanone | 325 |
| 4-38 | 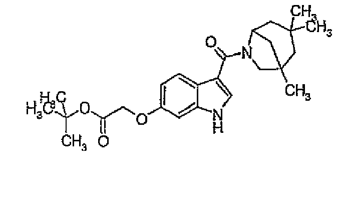 | 426.55 | [3-(1,3,3-Trimethyl-6-aza-bicyclo[3.2.1]octane-6-carbonyl)-1*H*-indol-6-yloxy]-acetic acid *tert*-butyl ester | 427 |

### Example 5

### 6-(1,3,3-Trimethyl-6-aza-bicyclo[3.2.1]octane-6-carbonyl)-1H-indole-3-carboxylic acid

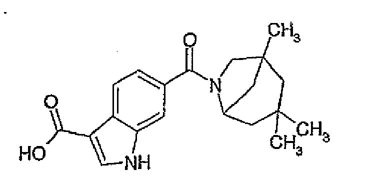

To a solution of (1*H*-Indol-6-yl)-(1,3,3-trimethyl-6-aza-bicyclo[3.2.1]oct-6-yl)-methanone (1.49 g, 5.02 mmol) in pyridine (0.81 mL, 5.02 mmol) and DCM (25 mL) at 0°C was added trichloroacetyl chloride after which cooling was stopped and the resulting mixture was stirred for 16 hrs. at room temperature. The volatiles were evaporated *in vacuo* and to the residue was added water (20 mL) followed by extraction with dichloromethane (DCM, 2 x 50 mL). The combined organic phases were dried (MgSO₄), filtered and evaporated *in vacuo* to afford 2.48 g (100%) of 2,2,2-Trichloro-1-[6-(1,3,3-trimethyl-6-aza-bicyclo[3.2.1]octane-6-carbonyl)-1*H*-indol-3-yl]-ethanone. To 2,2,2-Trichloro-1-[6-(1,3,3-trimethyl-6-aza-bicyclo-[3.2.1]octane-6-carbonyl)-1*H*-indol-3-yl]-ethanone (100 mg, 0.22 mmol) was added a solution of ethanol:THF:1M NaOH solution (20 mL, 1:2:1, v/v/v) and the mixture was stirred at room temperature for 5 hrs. The organic solvents were evapourated *in vacuo* and the aqueous collections were acidified to pH 1 with concentrated hydrochloric acid followed by extraction with ethyl acetate (2x 20 mL). The combined organic phases were dried (MgSO₄), filtered, evapourated and the residue after trituration with diethyl ether was filtered and dried *in vacuo* at 50°C affording 56 mg (72%) of the title compound as a solid.

MS-ESI *mlz* 341; ¹H NMR (300 MHz, DMSO) δ 0.91 (d, 3H), 0.97 (d, 3H), 1.09 (s, 3H), 1.13-1.54 (m, 4H), 1.73-1.78 (m, 1H), 3.13-3.18 (m, 1H), 3.30-3.47 (m, 2H), 3.98-4.02 and 4.38-4.42 (2x m, 1H), 7.19-7.28 (m, 1H), 7.53 (d, 1H), 7.99-8.04 (m, 1H), 8.09 (d, 1H), 11.92 (bs, 1H, N*H*), 12.05 (bs, 1H, O*H*).

### Example 6

### 6-(1,3,3-Trimethyl-6-aza-bicyclo[3.2.1]octane-6-carbonyl)-1H-indole-3-carboxylic acid ethyl ester

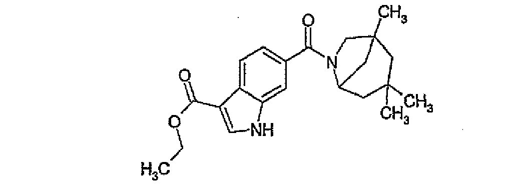

To a solution of 2,2,2-Trichloro-1-[6-(1,3,3-trimethyl-6-aza-bicyclo[3.2.1]octane-6-carbonyl)-1*H*-indol-3-yl]-ethanone (100 mg, 0.22 mmol) in ethanol (2 mL) was added sodium ethoxide (77 mg, 1.13 mmol) and the resulting mixture was stirred for 16 hrs. at room temperature. The volatiles were evaporated *in vacua* and the residue was purified by preparative HPLC, dried *in vacuo* at 50 °C affording 33 mg (43%) of the title compound as a solid.

MS-ESI *m*/*z* 369; ¹H NMR (300 MHz, CDCl₃) δ 0.91-1.01 (m, 3H), 1.12 (d, 3H), 1.17-1.36 (m, 1H), 1.40-1.44 (m, 4H), 1.56-1.61 (m, 1H), 1.73-1.78 (m, 1H), 3.20-3.33 and 3.62-3.66 (m, 2H), 4.05-4.08 and 4.58-4.61 (m, 1H), 4.38 (q, 2H), 7.29 - 7.33 (m, 1H), 7.55 (d, 1H), 7.97-7.99 (m, 1H), 8.15-8.19 (m, 1H), 10.01 (d, 1H, N*H*).

The following compounds were synthesised employing a similar method to the ones described in examples 5 and 6.

| **No** | **Molecule** | **MW** | **IUPAC Name** | **MS-ESI m/z** |
|---|---|---|---|---|
| 6-1 | 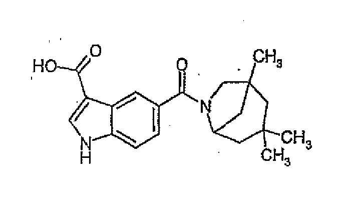 | 340.42 | 5-(1,3,3-Trimethyl-6-aza-blcyclo[3.2.1]octane-6-carbonyl)-1*H*-indole-3-carboxylic acid | 341 |
| 6-2 | 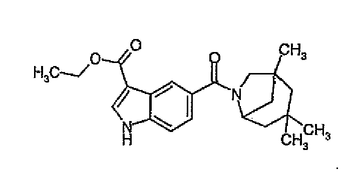 | 368.47 | 5-(1,3,3-Trimethyl-6-aza-bicycio[3.2.1]octane-6-carbonyl)-1*H*-indole-3-carboxylic acid ethyl ester | 369 |
| 6-3 | 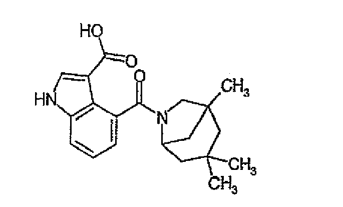 | 340.42 | 4-(1,3,3-Trimethyl-6-aza-bicyclo[3.2.1]octane-6-carbonyl)-1*H*-indole-3-carboxylic acid | 341 |
| 6-4 | 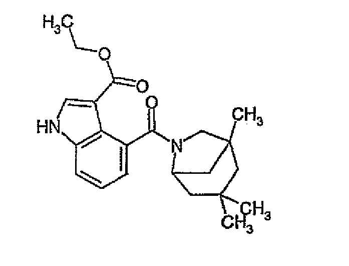 | 368.47 | 4-(1,3,3-Trimethyl-6-aza-bicyclo[3.2.1]octane-6-carbonyl)-1*H*-indole-3-carboxylic acid ethyl ester | 369 |

### Example 7

### [3-(Piperidine-1-carbonyl)-1H-indol-5-yl]-(1,3,3-trimethyl-6-aza-bicyclo[3.2.1]oct-6-yl)-methanone

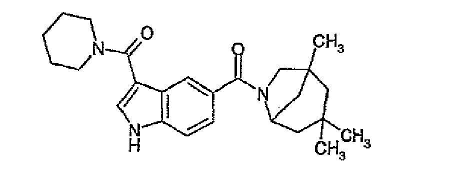

To 4-hydroxy-2,3,5,6-tetrafluorobenzamidomethyl polystyrene (PS-TFP resin, 100 mg, 1 mmol/g, 100-200 mesh, polystyrene-divinylbenzene 1%, Argonaut technologies, USA) pre-swollen in DCM was added a solution of 5-(1,3,3-Trimethyl-6-aza-bicyclo[3.2.1]octane-6-carbonyl)-1*H*-indole-3-carboxylic acid (51 mg, 0.15 mmol) in DMF (0.25 mL) followed by a solution of DMAP (7.3 mg, 0.06 mmol) in DCM (0.75 mL). The mixture was shaken for 10 min before a solution of *N*,*N*'-diisopropylcarbodiimide (DIC, 56 mg, 0.44 mmol) in DCM (0.25 mL) was added and the resulting mixture shaken for 16 hrs. at room temperature. The excess solvents were removed by filtration and the resin was washed with DMF (3 x 1 mL) and DCM (10 x1 mL). To the resin was added a solution of piperidine (7.3 mg, 0.085 mmol) in 1,2-dichloroethane (1.2 mL) and DIPEA (0.03 mL, 0.17 mmol). The resulting mixture was shaken for 16 hrs. at room temperature. The product was removed by filtration and the resin washed with DCM:MeOH (1 mL, 3:1, v/v). The volatiles were evaporated in *vacuo* and the residue was purified by preparative HPLC, dried *in vacuo* at 50 °C affording 22 mg (54%) of the title compound as a solid.

MS-ESI *m*/*z* 408; ¹H NMR (300 MHz, CDCl₃) δ 0.94 (d, 3H), 1.02 (d, 3H), 1.14 (s, 3H), 1.15-1.19 (m, 1H), 1.34-1.48 (m, 3H), 1.58-1.69 (m, 7H), 1.78-1.83 (m, 1H), 2.25-2.31 and 3.27-3.29 (2 x m, 1H), 3.33-3.34 (m, 1H), 3.64-3.67 (m, 4H), 4.08-4.11 and 4.62-4.64 (2 x m, 1H), 7,25 - 7.28 (m, 1H), 7.32-7.37 (m, 1H), 7.47-7.49 (m, 1H), 7.69-7.71 (m, 1H), 9.65 (bd, 1H, N*H*).

The following compounds were synthesised employing a similar method to the ones described in example 7.

| **No** | **Molecule** | **MW** | **IUPAC Name** | **MS-ESI *m*/*z*** |
|---|---|---|---|---|
| 7-1 | 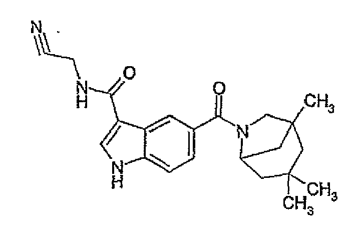 | 378.48 | 5-(1,3,3-Trimethyl-6-aza-bicyclo[3.2.1]octane-6-carbonyl)-1*H*-indole-3-carboxylic acid cyanomethyl-amide | 379 |
| 7-2 | 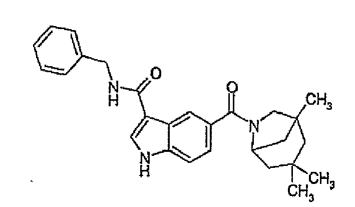 | 429.57 | 5-(1,3,3-Trimethyl-6-aza-bicyclo[3.2.1]octane-6-carbonyl)-1*H*-indole-3-carboxylic acid benzylamide | 430 |
| 7-3 | 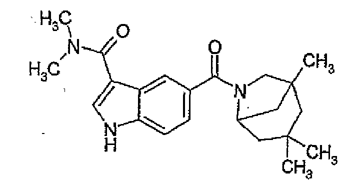 | 367.50 | 5-(1,3,3-Trimethyl-6-aza-bicyclo[3.2.1]octane-6-carbonyl)-1*H*-indole-3-carboxylic acid dimethyla-mide | 368 |
| 7-4 | 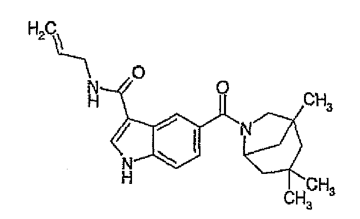 | 379.51 | 5-(1,3,3-Trimethyl-6-aza-bicyclo[3.2.1]octane-6-carbonyl)-1*H*-indole-3-carboxylic acid allylamide | 380 |
| 7-5 | 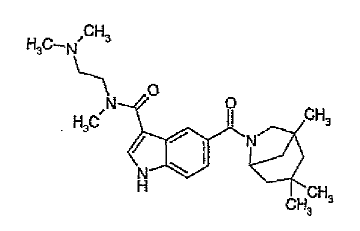 | 424.59 | 5-(1,3,3-Trimethyl-6-aza-bicyclo[3.2.1]octane-6-carbonyl)-1*H*-indole-3-carboxylic acid (2-dimethylamino-ethyl)-methyl-amide | 425 |
| 7-6 | 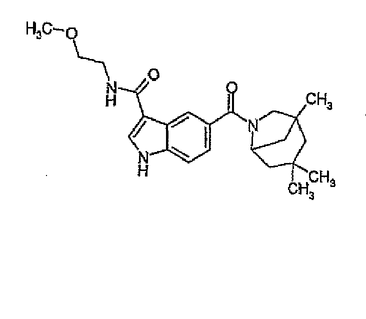 | 397.52 | 5-(1,3,3-Trimethyl-6-aza-bicyclo[3.2.1]octane-6-carbonyl)-1*H*-indole-3-carboxylic acid (2-methoxy-ethyl)-amide | 398 |
| 7-7 | 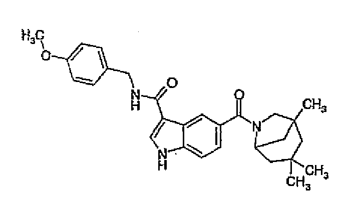 | 459.59 | 5-(1,3,3-Trimethyl-6-aza-bicyclo[3.2.1]octane-6-carbonyl)-1*H*-indole-3-carboxylic acid 4-methoxy-benzylamide | 460 |
| 7-8 | 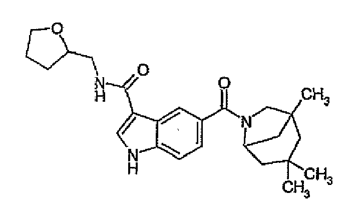 | 423.56 | 5-(1,3,3-Trimethyl-5-aza-bicycto[3.2.1]octane-6-carbonyl)-1*H*-indole-3-carboxylic acid (tetrahydro-furan-2-ylmethyl)-amide | 424 |
| 7-9 | 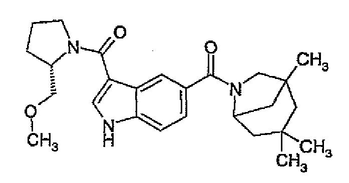 | 437.59 | [3-(2-Methoxymethyl-pyrrolidine-1-carbonyl)-1*H*-indol-5-yl]-(1,3,3-trimethyl-6-aza-bicyclo[3.2.1]oct-6-yl)-methanone | 438 |
| 7-10 | 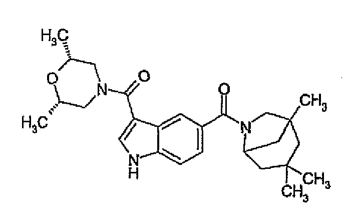 | 437.59 | [3-(2,6-Dimethyl-morpholine-4-carbonyl)-1*H*-indol-5-yl]-(1,3,3-trimethyl-6-aza-bicyclo[3.2.1]oct-6-yl)-methanone | 438 |
| 7-11 | 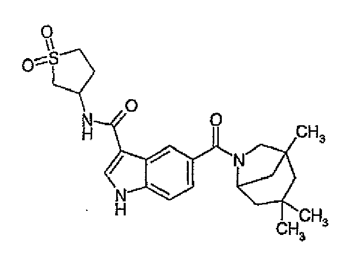 | 457.60 | 5-(1,3,3-Trimethyl-6-aza-bicyclo[3.2.1]octane-6-carbonyl)-1*H*-indole-3-carboxylic acid (1,1-dioxo-tetrahydro-thiophen-3-yl)-amide | 458 |
| 7-12 | 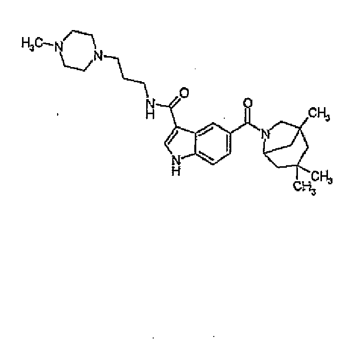 | 479.67 | 5-(1,3,3-Trimethyl-6-aza-bicyclo[3.2.1]octane-6-carbonyl)-1*H*-indole-3-carboxylic acid [3-(4-methyl-piperazin-1-yl)-propyl]-amide | 481 |
| 7-13 | 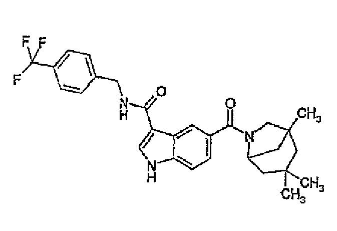 | 497.57 | 5-(1,3,3-Trimethyl-6-aza-bicyclo[3.2.1]octane-6-carbonyl)-1*H*-indole-3-carboxylic acid 4-trifluoromethyl-benzylamide | 498 |
| 7-14 | 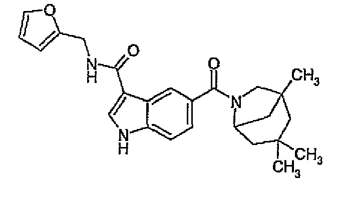 | 419.53 | 5-(1,3,3-Trimethyl-6-aza-bicyclo[3.2.1]octane-5-carbonyl)-1*H*-indole-3-carboxylic acid (furan-2-ylmethyl)-amide [3-(2,3,5,6-Tetrahydro- | 420 |
| 7-15 | 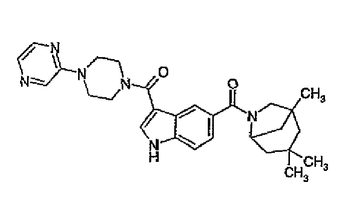 | 486.62 | [1,2']blpyrazinyl-4-carbonyl)-1H-indol-5-yl]-(1,3,3-trimethyl-6-aza-bicyclo[3.2.1]oct-6-yl)-methanone | 487 |
| 7-16 | 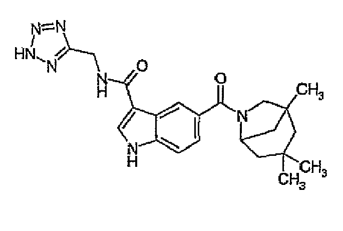 | 421.51 | 5-(1,3,3-Trimethyl-6-aza-bicyclo[3.2.1]octane-8-carbonyl)-1*H*-indole-3-carboxylic acid (2H-tetrazol-5-ylmethyl)-amide | 422 |
| 7-17 | 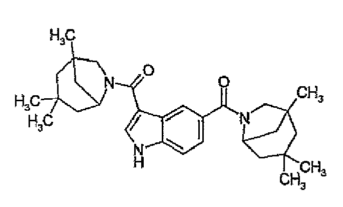 | 475.68 | [3-(1,3,3-Trimethyl-6-aza-bicyclo[3.2.1]octane-6-carbonyl)-1*H*-indol-5-yl]-(1,3,3-trimethyl-6-aza-bicyclo[3.2.1]oct-6-yl)-methanone | 476 |
| 7-18 | 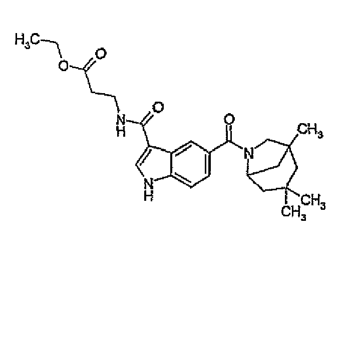 | 439.56 | 3-{[5-(1,3,3-Trimethyl-6-aza-bicyclo[3.2.1]octane-6-carbonyl)-1*H*-indole-3-carbonyl]-amino}-propionic acid ethyl ester | 440 |
| 7-19 | 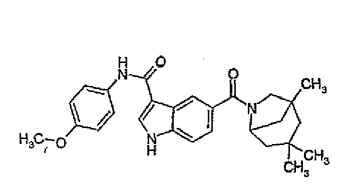 | 445.57 | 5-(1,3,3-Trimethyl-6-aza-bicyclo[3.2.1]octane-6-carbonyl)-1*H*-indole-3-carboxylic acid (4-methoxy-phenyl)-amide | 446 |
| 7-20 | 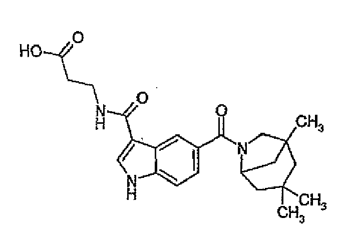 | 411.51 | 3-{[5-(1,3,3-Trimethyl-6-aza-bicyclo[3.2.1]octane-6-carbonyl)-1*H*-indole-3-carbonyl]-amino}-propionic acid | 412 |
| 7-21 | 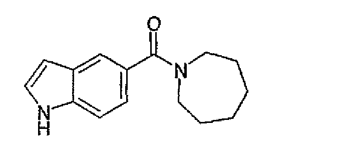 | 242.32 | Azepan-1-yl-(1*H*-indol-5-yl)-methanone | 243 |
| 7-22 | 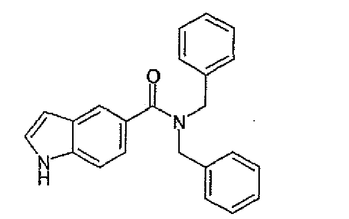 | 340.43 | 1*H*-Indole-5-carboxylic acid dibenzylamide | 341 |
| 7-23 | 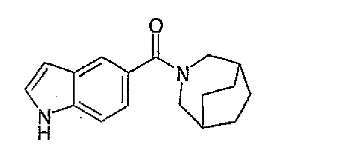 | 268.36 | (3-Aza-bicyclo[3.2.2]non-3-yl)-(1*H*-indol-5-yl)-methanone | 269 |
| 7-24 | 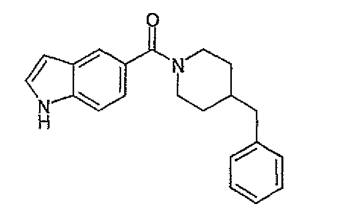 | 318.42 | (4-Benzyl-piperidin-1-yl)-(1*H*-indol-5-yl)-methanone | 319 |
| 7-25 | 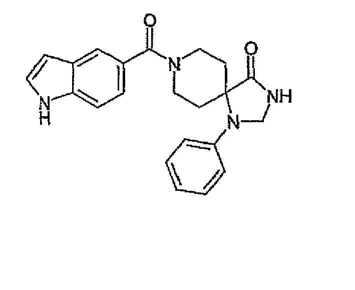 | 374.45 | 8-(1*H*-indole-5-carbonyl)-1-phenyl-1,3,8-triaza-spiro[4.5]decan-4-one | 375 |
| 7-26 | 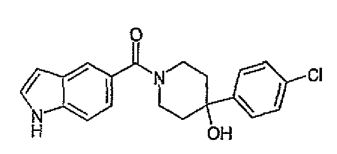 | 354.84 | [4-(4-Chloro-phenyl)-4-hydroxy-piperidin-1-yl]-(1*H*-indol-5-yl)-methanone | 356 |
| 7-27 | 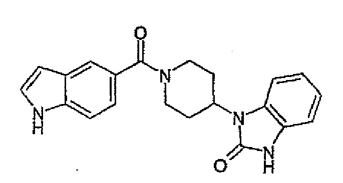 | 360.42 | 1-[1-(1*H*-Indole-5-carbonyl)-piperidin-4-yl]-1,3-dihydro-benzolmidazol-2-one | 361 |
| 7-28 | 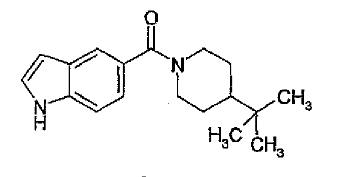 | 284.40 | (4-*tert*-Butyl-piperidin-1-yl)-(1*H*-indol-5-yl)-methanone | 285 |
| 7-29 | 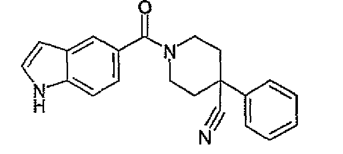 | 329.41 | 1-(1*H*-Indole-5-carbonyl)-4-phenyl-piperidine-4-carbonitrile | 330 |
| 7-30 | 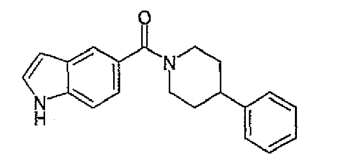 | 304.40 | (1*H*-Indol-5-yl)-(4-phenyl-piperidin-1-yl)-methanone | 305 |
| 7-31 | 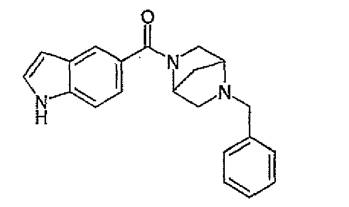 | 331.42 | (5-Benzyl-2,5-diaza-bicyclo[2.2.1]hept-2-yl)-(1*H*-indol-5-yl)-methanone | 332 |
| 7-32 | 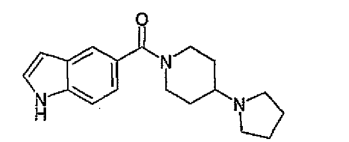 | 297.40 | (1*H*-Indol-5-yl)-(4-pyrrolidin-1-yl-piperidin-1-yl)-methanone | 298 |
| 7-33 | 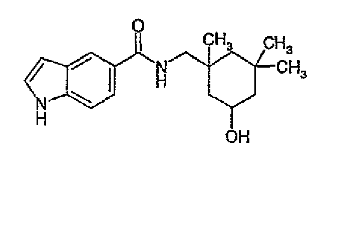 | 314.43 | 1*H*-Indole-5-carboxylic acid (5-hydroxy-1,3,3-trimethyl-cyclohexylmethyl)-amide | 315 |
| 7-34 | 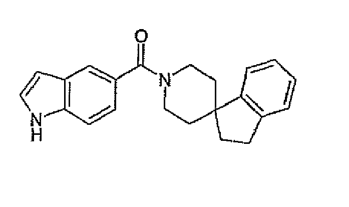 | 330.43 | 1*H*-Indole-5carboxylic acid (3,4-dihydrospiro(1*H*-indene-1,4-piperidine)-amide | 331 |

### Example 8

### (3-Methanesulfonylmethyl-1H-indol-5-yl)-(1,3,3-trimethyl-6-aza-bicyclo[3.2.1]oct-6-yl)-methanone

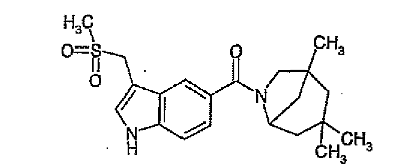

A solution of (1*H*-Indol-5-yl)-(1,3,3-trimethyl-6-aza-bicyclo[3.2.1]oct-6-yl)-methanone (300 mg, 1.01 mmol), paraformaldehyde (35 mg, 1.16 mmol), sodium methylsulfinate (103 mg, 1.01 mmol) and acetic acid (0.1 mL) in DMF (2 mL) was heated at 90°C for 3 hrs. After cooling, the mixture was poured onto water (100 mL) followed by extraction with DCM (3 x 50 mL). The combined organic phases were dried (MgSO₄), filtered and evaporated *in vacuo.* The residue was purified by preparative HPLC, dried *in vacuo* at 50 °C affording 167 mg (43%) of the title compound as a solid.

MS-ESI *mlz* 389; ¹H NMR (300 MHz, CDCl₃) δ 0.94 (d, 3H), 1.03 (d, 3H), 1.14-1.17 (m, 4H), 1.29-1.40 (m, 2H), 1.57-1.62 (m, 1H), 1.79-1.84 (m, 1H), 2.67 (d, 3H), 3.25-3.34 and 3.64-3.67 (2 x m, 2H), 4.08-4.11 and 4.61-4.64 (2 x m, 1H), 4.39 (s, 2H), 7.22-7.32 (m, 3H), 7.78 (d, 1H), 9.42 (s, 1H, N*H*).

### Example 9

### (3-Dimethylaminomethyl-1H-indol-6-yl)-(1,3,3-trimethyl-6-aza-bicyclo[3.2.1]oct-6-yl)-methanone

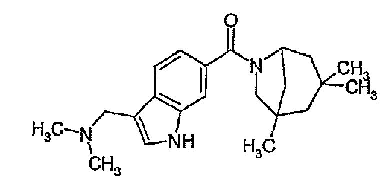

To a solution of (1*H*-indol-6-yl)-(1,3,3-trimethyl-6-aza-bicyclo[3.2.1]oct-6-yl)-methanone (200 mg, 0.67 mmol) in DCM (10 mL) was added *N*,*N*-dimethylammonium iodide and the resulting mixture stirred at room temperature for 16 hrs. The volatiles were evaporated *in vacuo* and the resulting residue was purified by preparative HPLC, dried *in vacuo* at 50 °C affording 54 mg (23%) of the title compound isolated as the trifluoroacetate salt.

MS-ESI *m*/*z* 354; ¹H NMR (400 MHz, DMSO) δ 0.90 (d, 3H), 0.97 (d, 3H), 1.08-1.17 (m, 4H), 1.28-1.53 (m, 4H), 1.72-1.79 (m, 1H), 2.76 (s, 6H), 3.14-3.16 (m, 1H), 3.33-3.36 and 3.44-3.47 (2 x m, 1H), 4.08-4.15 and 4.39-4.42 (2 x m, 1H), 4.45 (s, 2H), 7.19 (dd, 1H), 7.54 (d, 1H), 7.69 (s, 1H), 7.81 (t, 1H), 9.90 (s, 1H), 11.74 (s, 1H).

### Example 10

### 1-{3-Acetyl-2-[5-(1,3,3-trimethyl-6-aza-bicyclo[3.2.1]octane-6-carbonyl)-1H-indol-3-yl]-2,3-dihydro-imidazol-1-yl}-ethanone

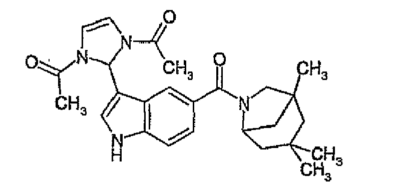

To a solution of imidazole (23 mg, 0.34 mmol) in acetic anhydride at 125°C was added dropwise over 40 min a solution of (1*H*-indol-5-yl)-(1,3,3-trimethyl-6-aza-bicyclo[3.2.1]oct-6-yl)-methanone (100 mg, 0.34 mmol) in acetic anhydride (13 mL). The resulting mixture was heated at 125°C for 30 min then cooled and solvents evaporated in *vacuo.* The resulting residue was purified by preparative HPLC, dried *in vacuo* at 50 °C to afford 5.4 mg (4%) of the title compound as a solid.

MS-ESI *m*/*z* 449; ¹H NMR (400 MHz, DMSO) δ 0.94 (d, 3H), 0.97 (d, 3H), 1.08-1.17 (m, 4H), 1.28-1.62 (m, 4H), 1.72-1.79 (m, 1H), 2.05-2.08 (m, 6H), 3.14-3.16 (m, 1H), 3.26-3.28 (m, 1H), 3.33-3.36 and 3.63-3.67 (2 x m, 1H), 4.04-4.06 and 4.61-4.63 (2 x m, 1H), 6.33-6.40 (m, 2H), 6.96-7.00 (m, 1H), 7.17-7.49 (m, 2H), 7.67-7.82 (m, 1H), 8.94 (s, 1H).

### Example 11

### 1-Ethyl-3-[5-(1,3,3-trimethyl-6-aza-bipyclo[3.2.1]octane-6-carbonyl)-1H-indol-3-yl]-pyrrolidine-2-5-dione

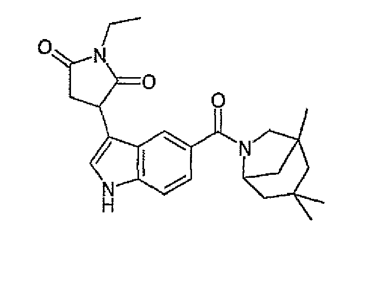

A solution of (1*H*-Indol-5-yl)-(1,3,3-trimethyl-6-aza-bicyclo[3.2.1]oct-6-yl)-methanone (100 mg, 0.34 mmol) and *N*-ethylmaleimide (127 mg, 1.01 mmol) in acetic acid (2 mL) was heated at 160°C employing microwave irradiation for 1 hr. The solvents were evaporated in *vacuo* and the resulting residue purified by preparative HPLC, dried *in vacuo* at 50 °C to afford 25 mg (18%) of the title compound as a solid.

MS-ESI *m*/*z* 422; ¹H NMR (400 MHz, CDCl₃) δ 0.93-0.96 (m, 3H), 1.01-1.06 (m, 3H), 1.13-1.17 (m, 3H), 1.21-1.25 (m, 3H), 1.28-1.44 (m, 3H), 1.55-1.64 (m, 1H), 1.73-1.81 (m, 1H), 2.05-2.28 (m, 1H), 2.78-2.87 (m, 1H), 3.17-3.34 (m, 1H), 3.57-3.68 (m, 3H), 4.01-4.07 and 4.62-4.64 (2 x m, 1H), 4.24-4.28 (m, 1H), 7.11-7.12 (m, 1H), 7.22-7.31 (m, 3H), 7.53-7.60 (m, 1H), 8.83 (s, 1H).

### Example 12

### (3-Thiazol-2-yl-1H-indol-5-yl)-(1,3,3-trimethyl-6-aza-bicyclo[3.2.1]oct-6-yl)-methanone

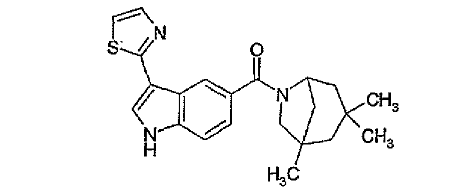

To a slurry of (1*H*-Indol-5-yl)-(1,3,3-trimethyl-6-aza-bicyclo[3.2.1]oct-6-yl)-methanone (300 mg, 1.01 mmol) in benzene (8 mL) under an inert atmosphere of nitrogen was added methylmagnesium iodide (0.34 mL, 1.01 mmol), after stirring for 10 mins 2-bromothiazole was added where upon the mixture was heated at 90°C for 16 hrs. Water (20 mL) was added and the organics were extracted with DCM (3 x 20 mL). The combined organic phases were dried (MgSO₄), filtered and evaporated *in vacuo.* The residue was purified by preparative HPLC, dried *in vacuo* at 50 °C affording 48 mg (25%) of the title compound as a solid.

MS-ESI *mlz* 380; ¹H NMR (300 MHz, CDCl₃) δ 0.93-1.08 (m, 6H), 1.16-1.20 (m, 5H), 1.39-1.47 (m, 2H), 1.59-1.64 (m, 1H), 1.80-1.85 (m, 1H), 3.21-3.24 (m, 1H), 3.34-3.38 and 3.65-3.69 (2 x m, 1H), 4.01-4.03 and 4.85-4.63 (2 x m, 1H), 7.31-7.35 (m, 2H), 7.48-7.52 (m, 1H), 7.82 (d, 1H), 7.91 (d, 1H), 8.34 (s, 1H), 9.77 (s, 1H).

### Example 13

### (3-Iodo-1H-indol-5-yl)-(1,3,3-trimethyl-6-aza-bicyclo[3.2.1]oct-6-yl)-methanone

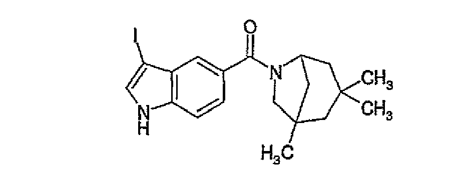

To a solution of (1*H*-indol-5-yl)-(1,3,3-trimethyl-6-aza-bicyclo[3.2.1]oct-6-yl)-methanone (1 g, 3.37 mmol) and potassium hydroxide (364 mg, 6.75 mmol) in DMF (40 m) was added iodine (0.86 g, 3.41 mmol). The reaction mixture was stirred for 1 hr at room temperature then poured onto water (100 mL), extracted with DCM (3 x 20 mL). The combined organic phases were washed with water and brine then dried (MgSO₄), filtered and evaporated *in vacuo.* The resulting solid was dried *in vacuo* at 50 °C affording 1.18 g (83%) of the title compound.

MS-ESI *mlz* 423; ¹H NMR (300 MHz, DMSO) δ 0.90 (d, 3H), 0.98 (d, 3H), 1.10 (d, 3H), 1.15-1.45 (m, 4H), 1.50-1.54 (m, 1H), 1.75-1.78 (m, 1H), 3.15-3.18 (m, 1H), 3.30-3.33 and 3.41-3.45 (2 x m, 1H), 3.98-4.00 and 4.40-4.42 (2 x m, 1H), 7.22-7.42 (m, 2H), 7.44-7.47 (m, 1H), 7.64 (s, 1H), 11.73 (s, 1H).

### Example 14

### 6-(1,3,3-Trimethyl-6-aza-bicyclo[3.2.1]octane-6-carbonyl)-1H-indole-3-carbonitrile

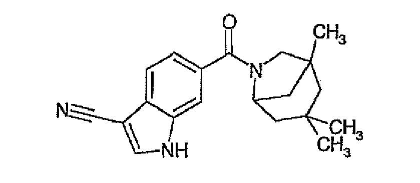

To an ice-cooled slurry of (1*H*-Indol-6-yl)-(1,3,3-trimethyl-6-aza-bicyclo[3.2.1]oct-6-yl)-methanone (1 g, 3.37 mmol) in acetonitrile (25 mL) was added dropwise a solution of chlorosulfunylisocyanate (0.48 g, 3.37 mmol). The reaction mixture was stirred for 1 hr at 0°C then triethylamine (0.33 g, 3.3 mmol) was added dropwise maintaining an internal temperature of 0°C. The reaction mixture was allowed to warm to room temperature over 2 hrs. The solvents were evaporated *in vacuo* and the residue treated with DCM (10 mL) and an ice cold solution of sodium bicarbonate (5%, 10 mL) and the organics were extracted with DCM (3 x 20 mL). The combined organic phases were dried (MgSO₄), filtered and evaporated *in vacuo.* The residue was purified by preparative HPLC, dried *in vacuo* at 50 °C affording 182 mg (17%) of the title compound as a solid.

MS-ESI *m*/*z* 322; ¹H NMR (400 MHz, DMSO) δ 0.90 (d, 3H), 0.98 (d, 3H), 1.09 (s, 3H), 1.15-1.54 (m, 5H), 1.74-1.78 (m, 1H), 3.13-3.16 (m, 1H), 3.29-3.32 and 3.44-3.47 (2 x m, 1H), 3.94-3.98 and 4.40-4.42 (2 x m, 1H), 7.27-7.35 (m, 1H), 7.60 (d, 1H), 7.66-7.70 (m, 1H), 8.35-8.37 (m, 1H), 12.32 (s, 1H).

The following compound was synthesised employing a similar method to the one described in example 14.

| **No** | **Molecule** | **MW** | **IUPAG Name** | **MS-ESI m/z** |
|---|---|---|---|---|
| 14-1 | 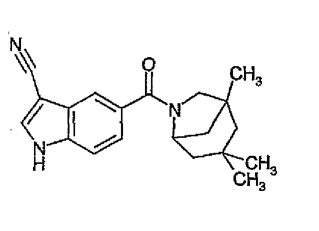 | 321.42 | 5-(1,3,3-Trimethtyl-6-aza-bicyclo[3.2.1]octane-6-carbonyl)-1*H*-indole-3-carbonitrile | 322 |

### Example 15

### 6-(1,3,3-Trimethyl-6-aza-bicyclo[3.2.1]octane-6-carbonyl)-1H-indole-3-carboxylic acid amide

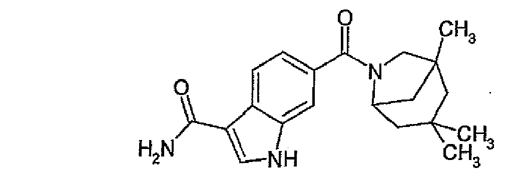

To a solution of 6-(1,3,3-Trimethyl-6-aza-bicyclo[3.2,1]octane-6-carbonyl)-1*H*-indole-3-carbonitrile (65 mg, 0.2 mmol) in 1,4-dioxane (2 mL) was added sodium hydroxide solution (2 mL, 1M) and hydrogen peroxide (2 mL) and the resulting solution was heated at 50°C for 16 hrs. The solvents were evaporated *in vacuo* and the residue was purified by preparative HPLC, dried *in vacuo* at 50 °C affording 15 mg (21%) of the title compound as a solid.

MS-ESI *mlz* 340; ¹H NMR (400 MHz, CDCl₃) δ 0.92 (d, 3H), 0.98 (d, 3H), 1.10 (d, 3H), 1.15-1.54 (m, 5H), 1.74-1.79 (m, 1H), 3.18-3.29 (m, 1H), 3.25-3.28 and 3.61-3.64 (2 x m, 1H), 4.01-4.03 and 4.55-4.58 (2 x m, 1H), 6.11 (bs, 2H), 7.19-7.23 (m, 1H), 7.34-7.38 (m, 1H), 7.77-7.80 (m, 1H), 7.95-7.99 (m, 1H), 10.80 (s, 1H).

### Example 16

### [3-(2H-Tetrazol-5-yl)-1H-indol-6-yl]-(1,3,3-trimethyl-6-aza-bicyclo[3.2.1]oct-6-yl)-methanone

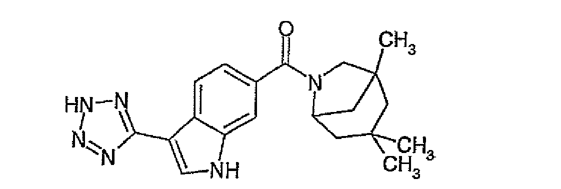

A slurry of 6-(1,3,3-Trimethyl-6-aza-bicydo[3.2.1]octane-6-carbonyl)-1*H*-indole-3-carbonitrile (100 mg, 0.31 mmol), zinc bromide (70 mg, 0.31 mmol) and sodium azide (22 mg, 0.34 mmol) in water (0.65 mL) was heated at 200°C employing microwave irradiation for 6 min. A solution of sodium hydroxide (3 mL, 0.25 M) was added and the mixture stirred for 45 min. Filtration of the inorganics followed by acidification of the filtrate with concentrated HCl to pH 1 yielded after filtration crude product which was purified by preparative HPLC, dried *in vacuo* at 50 °C affording 6 mg (5%) of the title compound as a solid.

MS-ESI *mlz* 365; ¹H NMR (400 MHz, DMSO) δ 0.92 (d, 3H), 0.99 (d, 3H), 1.10 (d. 3H), 1.15-1.65 (m, 5H), 1.74-1.79 (m, 1H), 3.15-3.20 (m, ¹H). 3.25-3.32 and 3.45-3.48 (2 x m, 1H), 4.01-4.03 and 4.41-4.43 (2 x m, 1H), 7.26-7.35 (m, 1H), 7.61 (d, 1H), 8.15-8.18 (m, 1H), 8.21-8.25 (m, 1H), 11.98 (s, 1H).

### Example 17

### N-[3-(1,3,3-Trimethyl-6-aza-bipyclo[3.2.1]octane-6-carbonyl)-1H-indol-7-yl]-acetamide

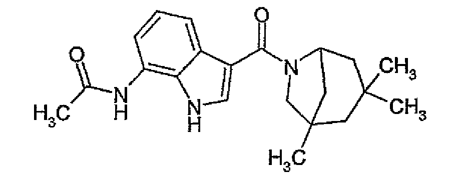

To a solution of (7-nitro-1*H*-indol-3-yl)-(1,3,3-trimethyl-6-aza-bicyclo[3.2.1]oct-6-yl)-methanone (730 mg, 2.14 mmol) in MeOH (40 mL) was added palladium on activated charcoal (10% Pd, 50% H₂O, 0.2 g). The reaction mixture was stirred for 16 hrs under an atmosphere of hydrogen. The catalyst was removed by filtration and the solvents were evaporated *in vacuo* and the solid dried *in vacuo* at 50 °C affording 481 mg (72%) of (7-amino-1*H*-indol-3-yl)-(1,3,3-trimethyl-6-aza-bicyclo[3.2.1]oct-6-yl)-methanone. To a solution of (7-amino-1*H-*indol-3-yl)-(1,3,3-trimethyl-6-aza-bicyclo[3.2.1]oct-6-yl)-methanone (100 mg, 0.32 mmol) in DCM (1 mL) was added DIPEA (829 mg, 6.4 mmol) and acetic anhydride (327 mg, 3.21 mmol), the solution was stirred at room temperature for 1 hr. Solvents were evaporated *in vacuo* and the residue was purified by preparative HPLC, dried *in vacuo* at 50 °C affording 47 mg (42%) of the title compound as a solid.

MS-ESI *mlz* 354; ¹H NMR (300 MHz, CDCl₃) δ 0.82-1.13 (m, 10H), 1.22-1.54 (m, 6H), 1.75-1.78 (m, 1H), 3.10-3.25 (m, 1H), 3.27-3.37 and 3.59-3.66 (2 x m, 1H), 4.20-4.22 and 4.49-4.52 (2 x m, 1H), 6.88-7.01 (m, 1H), 7.02-7.08 (m, 1H), 7.40-7.61 (m, 3H), 8.77 (s, 1H), 11.21 (bd, 1H).

### Example 18

### (1-Benzenesulfonyl-1H-indol-5-yl)-(1,3,3-trimethyl-6-aza-bicyclo[3.2.1]oct-6-yl)-methanone

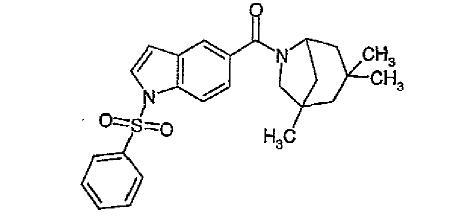

To a solution of (1*H*-Indol-5-yl)-(1,3,3-trimethyl-6-aza-bicyclo[3.2.1]oct-6-yl)-methanone (1 g, 3.37 mmol) in dry THF (2 mL) at -78°C under an inert atmosphere of nitrogen was added a solution of *n*-butyl lithium (2.14 mL, 1.65 M in hexane). Cooling was removed and the solution was allowed to warm to room temperature with stirring. The solution was cooled to -78°C where upon benzenesulphonyl chloride (655 mg, 3.71 mmol) was added and the reaction mixture was stirred for 16 hrs whilst warming to room temperature. The reaction was quenched by the addition of sodium bicarbonate solution (5%, 200 mL) and extracted with DCM (3 x 50 mL). The combined organic phases were washed with water and brine then dried (MgSO₄), filtered and evaporated *in vacuo.* The resulting solid was purified by flash column chromatography (mobile phase ethylacetate: heptane, 1:2). Product fractions were combined, evaporated *in vacuo,* dried *in vacuo* at 50 °C to afford 1.46 g (99%) of the title compound.

MS-ESI *m*/*z* 437; ¹H NMR (300 MHz, CDCl₃) δ 0.94 (d, 3H), 1.02 (d, 3H), 1.12-1.17 (m, 3H), 1.24-1.40 (m, 2H), 1.45 (s, 1H), 1.50-1.61 (m, 2H), 1.73-1.78 (m, 1H), 3.15-3.25 (m, 1H), 3.26-3.31 and 3.58-3.63 (2 x m, 1H), 3.96-3.99 and 4.60-4.62 (2 x m, 1H), 6.68-6.70 (m, 1H), 7.37-7.47 (m, 3H), 7.53-7.63 (m, 3H), 7.84-7.87 (m, 2H), 7.99-8.03 (m, 1H).

### Example 19

### (1-Benzenesulfonyl-2-methyl-1H-indol-5-yl)-(1,3,3-trimethyl-6-aza-bicyclo[3.2.1]oct-6-yl)-methanone

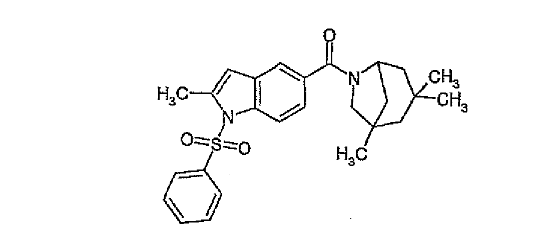

To a solution of (1-Benzenesulfonyl-1*H*-indol-5-yl)-(1,3,3-trimethyl-6-aza-bicyclo-[3.2.1]oct-6-yl)-methanone (100 mg, 0.23 mmol) in dry THF (2 mL) at-78°C under an inert atmosphere of nitrogen was added a solution of lithium *N*,*N*-diisopropylamide (0.165 mL, 1.5 M in cyclohexane). The mixture was stirred for 1 hr at -78°C then cooling was removed and the solution was allowed to warm to room temperature with stirring. The solution was cooled to -78°C where upon methyl iodide (48 mg, 0.343 mmol) was added and the reaction mixture was stirred for 16 hrs whilst warming to room temperature. The reaction was quenched by the addition of saturated ammonium chloride solution (10 mL) and extracted with DCM (3 x 10 mL). The combined organic phases were dried (MgSO₄), filtered and evaporated *in vacuo.* The residue was purified by preparative HPLC, dried *in vacuo* at 50°C affording 12 mg
(11%) of the title compound as a solid.

MS-ESI *m*/*z* 451; ¹H NMR (300 MHz, CDCl₃) δ 0.94 (d, 3H), 1.04 (d, 3H), 1.13-1.15 (m, 3H), 1.25-1.40 (m, 2H), 1.45 (s, 1H), 1.55-1.61 (m, 2H), 1.74-1.80 (m, 1H), 2.60 (s, 3H), 3.17-3.25 (m, 1H), 3.27-3.31 and 3.59-3.64 (2 x m, 1H), 4.00-4.02 and 4.61-4.63 (2 x m, 1H), 6.38 (s, 1H), 7.32-7.58 (m, 5H), 7.74-7.77 (m, 2H), 8.16-8.20 (m, 1H).

### Example 20

### (1-methyl-1H-indol-5-yl)-(1,3,3-trimethyl-6-aza-bicyclo[3.2.1]oct-6-yl)-methanone

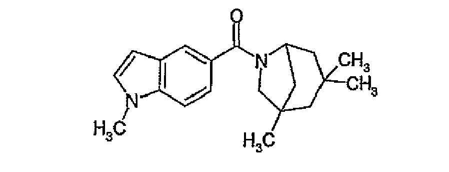

To a solution of (1*H*-indol-5-yl)-(1,3,3-trimethyl-6-aza-bicyclo[3.2.1]oct-6-yl)-methanone (500 mg, 1.69 mmol) in dry DMF (10 mL) at room temperature under an inert atmosphere of nitrogen was added sodium hydride (53 mg, 2.19 mmol, 60% dispersion in oil), after stirring for 30 min methyl iodide (263 mg, 1.85 mmol) was added and the reaction mixture was stirred for 16 hrs at 60°C. The reaction was quenched by the addition of water (20 mL) followed by extraction with DCM (3 x 50 mL). The combined organic phases were dried (MgSO₄), filtered and evaporated *in vacuo.* The resulting solid was purified by preparative HPLC, dried *in vacuo* at 50 °C affording 261 mg (50%) of the title compound as a solid.

MS-ESI *mlz* 311; ¹H NMR (300 MHz, DMSO) δ 0.89 (d, 3H), 0.96 (d, 3H), 1.07-1.21 (m, 4H), 1.23-1.53 (m, 4H), 1.72-1.78 (m, 1H), 3.10-3.95 (m, 1H), 3.34-3.47 (m, 1H), 3.80 (d, 3H), 4.01-4.04 and 4.38-4.40 (2 x m, 1H), 6.48-6.50 (m, 1H), 7.18-7.28 (m, 1H), 7.39 (d, 1H), 7.43-7.48 (m, 1H), 7.64 (d, 1H).

The following compounds were synthesised employing a similar method to the ones described in example 20.

| **No** | **Molecule** | **MW** | **IUPAC Name** | **MS- ESI *m*/*z*** |
|---|---|---|---|---|
| 20-1 | 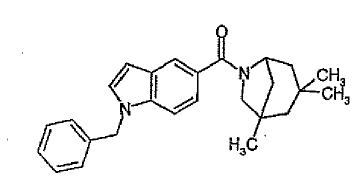 | 386.54 | (1-Benzyl-1*H*-indol-5-yl)-(1,3,3-trimethyl-6-aza-bicyclo[3.2.1]oct-6-yl)-methanone | 387 |
| 20-2 | 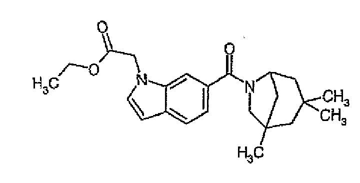 | 382.50 | [6-(1,3,3-Trimethyl-6-aza-bicyclo[3.2.1]octane-6-carbonyl)-indol-1-yl]-acetic acid ethyl ester | 383 |
| 20-3 | 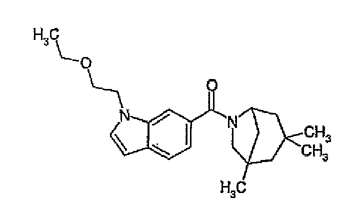 | 368.52 | [1-(2-Ethoxy-ethyl)-1H-indol-6-yl]-(1,3,3-tirimethyl-6-aza-bicyclo[3.2.1]oct-6-yl)-methanone | 369 |
| 20-4 | 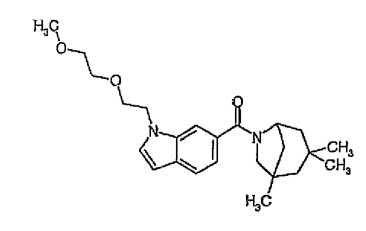 | 398.54 | {1-[2-(2-Methoxy-ethoxy)-ethyl]-1*H*-indol-6-yl}-(1,3,3-trimethyl-6-aza-bicyclo[3.2.1]oct-6-yl)-methanone | 399 |
| 20-5 | 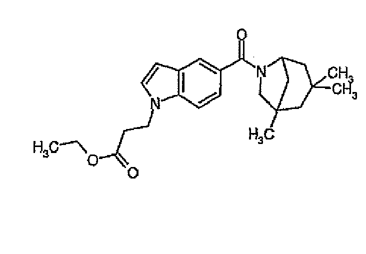 | 396.53 | 3-[5-(1,3,3-Trimethyl-6-aza-bicyclo[3.2.1]octane-6-carbonyl)-indol-1-yl]-propionic acid ethyl ester | 397 |
| 20-6 | 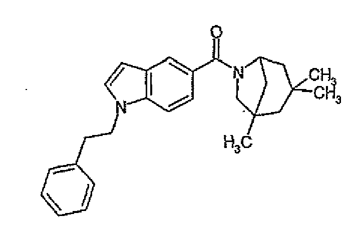 | 400.56 | (1-Phenethyl-1*H*-indol-5-yl)-(1,3,3-trimethyl-6-aza-bicyclo[3.2.1]oct-6-yl)-methanone | 401 |
| 20-7 | 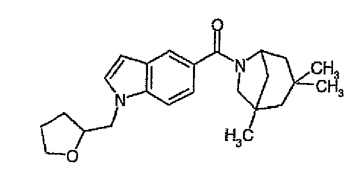 | 380.53 | [1-(Tetrahydro-furan-2-ylmethyl)-1*H*-indol-5-yl]-(1,3,3-trimethyl-6-aza-bicyclo[3.2.1]oct-6-yl)-methanone | 381 |
| 20-8 | 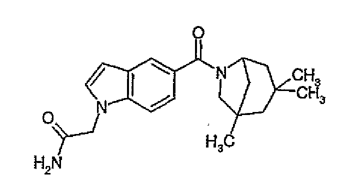 | 353.46 | 2-[5-(1,3,3-Trimethyl-6-aza-bicyclo[3.2.1]octane-6-carbonyl)-indol-1-yl]-acetamide | 354 |
| 20-9 | 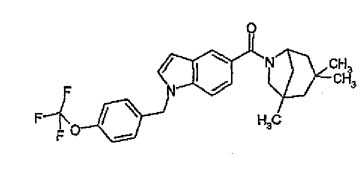 | 470.53 | [1-(4-Trifluoromethoxy-benzyl)-1*H*-indol-5-yl]-(1,3,3-trimethyl-6-aza-blcyclo[3.2.1]oct-6-yl)-methanone | 471 |
| 20-10 | 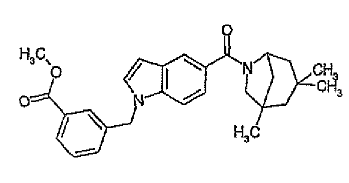 | 444.57 | 3-[5-(1,3,3-Trimsthyl-6-aza-bicyclo[3.2.1]octane-6-carbonyl)-indol-9-ylmethyl]-benzoic acid methyl ester | 445 |
| 20-11 | 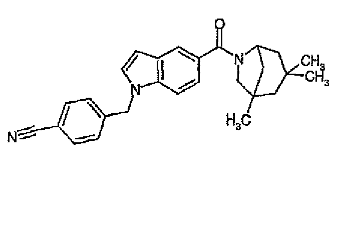 | 4.11.85 | 4-[5-(1,3,3-Trimethyl-6-aza-bicyclo[3.2.1]octane-6-carbonyl)-indol-1-ylmethyl]-benzonitrile | 412 |

### EXAMPLES, COMPOUNDS OF GENERAL FORMULA (VIII)

The following examples and general procedures refer to intermediate compounds and final products for general formula (VIII) identified in the specification and in the synthesis schemes. The preparation of the compounds of general formula (VIII) of the present invention is described in detail using the following examples, Occasionally, the reaction may not be applicable as described to each compound included within the disclosed scope of the invention. The compounds for which this occurs will be readily recognised by those skilled in the art. In these cases the reactions can be successfully performed by conventional modifications known to those skilled in the art, that is, by appropriate protection of interfering groups, by changing to other conventional reagents, or by routine modification of reaction conditions. Alternatively, other reactions disclosed herein or otherwise conventional will be applicable to the preparation of the corresponding compounds of the invention. In all preparative methods, all starting materials are known or may easily be prepared from known starting materials. The structures of the compounds are confirmed by either elemental analysis or nuclear magnetic resonance (NMR), where peaks assigned to characteristic protons in the title compounds are presented where appropriate. ¹H NMR shifts (δ_{H}) are given in parts per million (ppm) down field from tetramethylsilane as internal reference standard. M.p.: is melting point and is given in °C and is not corrected. Column chromatography was carried out using the technique described by W.C. Still et al., J. Org. Chem. 43: 2923 (1978) on Merck silica gel 60 (Art. 9385). HPLC analyses are performed using 5µm C18 4 x 250 mm column eluted with various mixtures of water and acetonitrile, flow = 1 ml/min, as described in the experimental section.

The abbreviations as used in the examples have the following meaning:
- TLC:: thin layer chromatography
- CDCl₃:: deuterio chloroform
- CD₃OD:: tetradeuterio methanol
- DMSO-d₆:: hexadeuterio dimethylsulfoxide
- DMSO:: dimethylsulfoxide
- THF:: tetrahydrofuran
- DMF:: N,N-dimethylformamide
- HOBT:: 1-hydroxy-benzotriazole
- EDAC:: 1-(3-dimethylaminopropyl)-3-ethylcarbodiimide, hydrochloride
- min:: minutes
- hrs:: hours

The compounds of the invention are prepared as illustrated in the following reaction scheme 1:

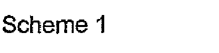

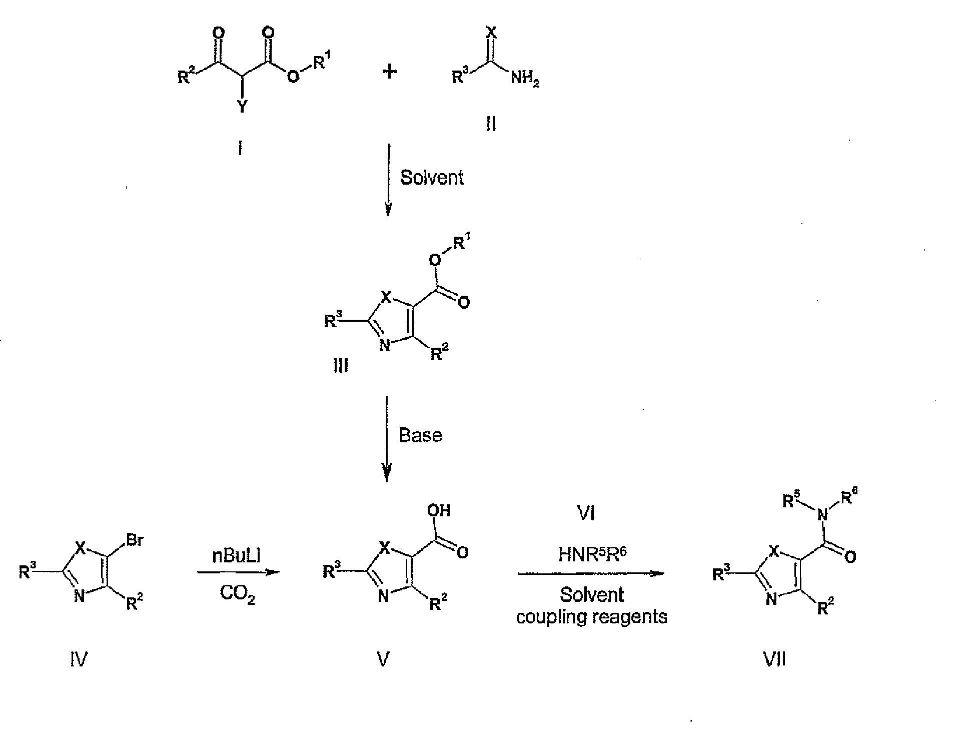

### General method:

By allowing a 2-halo-3-oxo-propionic acid ester (I) wherein Y is halo, R¹ is C₁-C₆aklkyl, aryl, arylC₁-C₆alkyl and R² is as defined above to react with an amide (II), wherein R³ is as defined above and X is O or S, in a solvent such as ethanol and the like affording an thiazole, oxazol or imidazol carboxylic acid ester (III), wherein R² and R³ is as defined above. The thiazole, oxazol or imidazol carboxylic acid ester (III) is hydrolysed with base affording thiazole, oxazol or imidazol carboxylic acid (V), wherein R² and R³ are as defined above. The thiazole, oxazol or imidazol carboxylic acid (V) can also be obtained from the corresponding bromo or iodo substituted thiazole, oxazol or imidazol (IV) via halogen lithium exchange followed by reaction with carbon dioxide in a solvent such as THF. The thiazole, oxazol or imidazol carboxylic acid (V) is coupled with an amine (VI), wherein R⁵ and R⁶ are as defined above, under standard amide forming conditions (e.g. HOBT, EDAC and DIPEA in dry THF) affording thiazole, oxazol or imidazol (VII), wherein R², R³, R⁵ and R⁶ are as defined above.

### Example 1

### 4-Methyl-2-phenyl-thiazol-5-yl)-(1,3,3-trimethyl-6-aza-bicyclo[3.2.1]oct-6-yl)-methanone

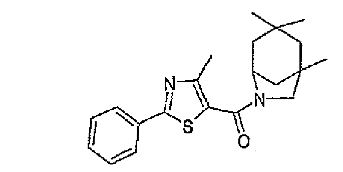

A solution of 4-methyl-2-phenyl-1,3-thiazole-5-carboxylic acid (0.44 g, 2.00 mmol), HOBT (0.297 g, 2.2 mmol), EDAC (0.42 g, 2.2 mmol) and di-isopropyl ethyl amine (DIPEA) (383 µl, 2.2 mmol) in dry THF (30 ml) was stirred for 30 minutes and 1,3,3-trimethyl-6-azabicyclo[3.2.1]octane (0.34 g, 2.2 mmol) was added. The mixture was stirred for 16 hrs. at room temperature. The mixture was added water (30 ml) and extracted with ETOAc (2 x 50 ml), dried (MgSO₄), filtered and evaporated *in vacuo* affording 0.67 g (94%) of the title compounds as an oil.
¹H-NMR (400 MHz, CDCl₃) δ 0.94 - 11.4 (m, 9 H), 1.26 - 1.8 (m, 5 H), 2.25 (m, 1H), 2.55 (d, 3H), 3.26 (m, 1H), 3.44 and 3.66 (2xd, 1H), 4.18 and 4.60 (2xt, 1H), 7.44 (m, 3H), 7.92 (m, 2H).

### Example 2

### (2,4-Dimethyl-thiazol-5-yl)-(1,3,3-trimethyl-6-aza-bicyclo[3.2.1]oct-6-yl)-methanone

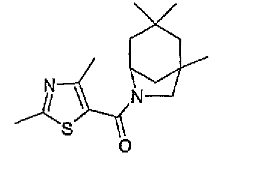

A solution of 2,4-dimethyl-1,3-thiazole-5-carboxylic acid (0.47 g, 3.00 mmol), HOBT (0.46 g, 3.3 mmol), EDAC (0.63 g, 3.3 mmol) and di-isopropyl ethyl amine (DIPEA) (575 µl, 3.3 mmol) in dry THF (50 ml) was stirred for 40 minutes and 1,3,3-trimethyl-6-azabicyclo-[3.2.1]octane (0.51 g, 3.3 mmol) was added. The mixture was stirred for 16 hrs. at room temperature. The mixture was added water (30 ml) and extracted with ETOAc (2 x 50 ml), dried (MgSO₄), filtered and evaporated *in vacuo* affording 0.145 g (17%) of the title compounds as an oil.
¹H-NMR (400 MHz, CDCl₃) δ 0.97 - 1.15 (m, 9,5 H), 1.35 - 1.63 (m, 4.5 H), 2.97 (dd, 1H), 2.53 (d, 3H), 2.89 (d, 3H), 3.24 (m, 1H), 3.4 and 3.67 (2xd, 1H), 4.10 and 4.58 (2xt, 1H).

### Example 3

### (4-Methyl-2-pyrazin-2-yl-thiazol-5-yl)-(1,3,3-trimethyl-6-aza-bicyclo[3.2.1]oct-6-yl)-methanone

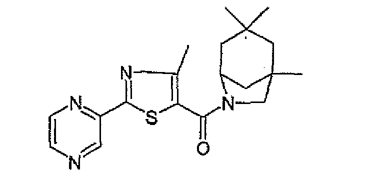

A solution of 4-methyl-2-(2-pyrazinyl)-1,3-thiazole-5-5-carboxylic acid (0.22 g, 1.00 mmol), HOBT (0.17 g, 1.1 mmol), EDAC (0.21 g, 1.1 mmol) and di-isopropyl ethyl amine (DIPEA) (192 µl, 1.1 mmol) in dry THF (10 ml) was stirred for 30 minutes and 1.3,3-trimethyl-6-azabicyclo[3.2.1]octane (0.17 g, 1.1 mmol) was added. The mixture was stirred for 16 hrs. at room temperature. The mixture was added water (30 ml) and extracted with ETOAc (2 x 50 ml), dried (MgSO₄), filtered and evaporated *in vacuo.* affording 0.345 g (97%) of the title compounds as an oil.

The following compounds were prepared in a similar way as described in Example 3 above.

### Example 4

### [4-Methyl-2-(4-trifluoromethyl-phenyl)-thiazol-5-yl]-(1,3,3-trimethyl-6-aza-bicyclo[3.2.1]oct-6-yl)-methanone

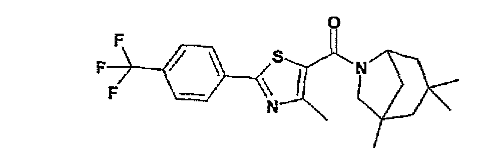

¹H-NMR (400 MHz, CDCl₃) δ 0.94 (m, 3H), 1.07 (t, 3H), 1.17 (t, 3H), 1.32 - 1.84 (m, 4.5 H), 2.25 (m, 0.5H), 2.58 (d, 3H), 2.86 (d, 0.5H), 3.23 (q, 1H), 3.4 (d, 0.5H), 3.66 (d, 0.5H), 3.80 (m, 0.5H), 4.18 (m, 0.5H), 4.60 (m, 0.5H), 7.68 (t, 2H), 8.04 (d, 2H).

### Example 5

### (1H-Imidazal-4-yl)-(1,3,3-trimethyl-6-aza-bicyrclo[3.2.1]oct-6-yl)-methanone

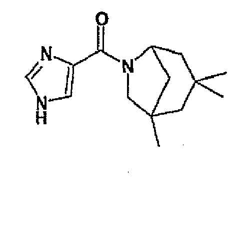

¹H-NMR (400 MHz, CDCl₃) δ 0.94 - 0.99 (m, 6H), 1.15 (d, 3H), 1.37 (d, 1H), 1.44 - 1.52 (m, 2H), 1.60 (d, 1H), 1.74 (m, 0.5H), 1.83 (m, 0.5H), 2.09 (t, 1H), 3.29 (d, 0.5H), 3.45 (d, 0.5H), 3.61 (d, 0.5H), 3.77 (d, 0.5H), 4.68 (m, 0.5H), 4.80 (bs, 0.5H), 7.49 (d, 1H), 7.69 (d, 1H), 11.45 (bs, 1H).

### Example 6

### (3-Aza-bicyclo[3.2.2]non-3-yl)-(4-methyl-2-phenyl-thiazol-5-yl)-methanone

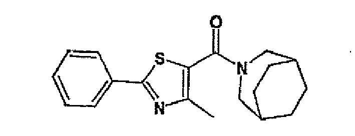

¹H-NMR (400 MHz, CDCl₃) δ 1.70 (bs, 10H), 2.48 (s, 3H), 3.75 (bs, 4H), 7.43 (t, 3H), 7.91 (m, 2H).

### Example 7

### (1-Methyl-1H-imidazol-4-yl)-(1.3.3-trimethyl-6-aza-bicyclo[3.2.1]oct-6-yl)-methanone

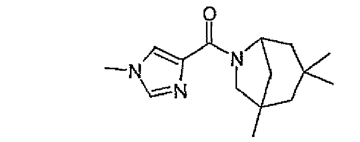

¹H-NMR (400 MHz, CDCl₃) δ 0.93 (s, 3H), 0.98 (d, 3H), 1.11 (s, 3H), 1.28 - 1.46 (m, 3H), 1.55 (d, 1H), 1.69 (m, 0.5H), 1.80 (m, 0.5H), 2.05 - 2.16 (m, 1H), 3.22 (dd, 0.5H), 3.62 (dq, 1H), 3.71 (s, 3H), 4.12 (d, 0.5H), 4.64 (m, 0.5H), 5.36 (m, 0.5H), 7.39 (d, 1H), 7.58 (d, 1H).

### Example 8

### [1-(6-Methyl-pyridin-2-yl)-1H-imidazol-4-yl]-(1,3,3-trimethyl-6-aza-bicyclo[3.2.1]oct-6-yl)-methanone

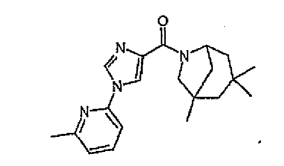

¹H-NMR (400 MHz, CDCl₃) δ 0.94 (s, 3H), 1.00 (d, 3H), 1.13 (d, 3H), 1.25 - 1.44 (m, 3H), 1.56 (m, 1H), 1.72 (m, 0.5H), 1.83 (m, 0.5H), 2.08 - 2.18 (m, 1H), 2.57 (s, 3H), 3.26 (d, 0.5H), 3.65 (q, 1H), 4.16 (d, 0.5H), 4.68 (m, 0.5H), 5.42 (m, 0.5H), 7.12 (d, 1H), 7.18 (d, 1H), 7.72 (t, 1H), 8.24 (s, 1H), 8.35 (s, 1H).

### Example 9

### [1-(4-Chloro-benzyl)-5-methyl-1H-imidazol-4-yl]-(1,3,3-trimethyl-6-aza-bicyclo[3.2.1]oct-6-yl)-methanone

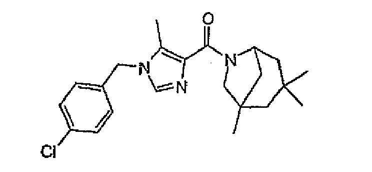

¹H-NMR (300 MHz, CDCl₃) δ 0.93 (s, 3H), 1.01 (d, 3H), 1.10 (d, 3H), 1.26 - 1.46 (m, 3H), 1.55 (d, 1H), 1.69 (m, 0.5H), 1.79 (m, 0.5H), 2.0 - 2.18 (m, 1H), 2.41 (d, 3H), 3.22 (d, 0.5H), 3.59 (d, 1H), 4.06 (d, 0.5H), 4.64 (m, 0.5H), 5.05 (s, 2H), 5.15 (m, 0.5H), 7.00 (d, 2H), 7.32 (d, 2H), 7.43 (d, 1H).

### PHARMACOLOGIGAL METHODS

### 11βHSD1 enzyme assay

### Materials

³H-cortisone and anti-rabbit Ig coated scintillation proximity assay (SPA) beads were purchased from Amersham Pharmacia Biotech, β-NADPH was from Sigma and rabbit anti-cortisol antibodies were from Fitzgerald. An extract of yeast transformed with h-11βHSD1 (Hult et al., FEBS Lett., 441, 25 (1998)) was used as the source of enzyme. The test compounds were dissolved in DMSO (10 mM). All dilutions were performed in a buffer containing 50 mM TRIS-HCl (Sigma Chemical Co), 4 mM EDTA (Sigma Chemical Co), 0.1% BSA (Sigma Chemical Co), 0.01% Tween-20 (Sigma Chemical Co) and 0.005% bacitracin (Novo Nordisk A/S), pH=7.4. Optiplate 96 wells plates were supplied by Packard. The amount of ³H-cortisol bound to the SPA beads was measured on TopCount NXT, Packard.

### Methods

h-11βHSD1, 120 nM ³H-cortisone, 4 mM β-NADPH, antibody (1:200), serial dilutions of test compound and SPA particles (2 mg/well) were added to the wells. The reaction was initiated by mixing the different components and was allowed to proceed under shaking for 60 min at 30°C. The reaction was stopped be the addition of 10 fold excess of a stopping buffer containing 500 µM carbenoxolone and 1 µM cortisone. Data was analysed using GraphPad Prism software.

**Table 1**

| Inhibition of 11βHSD1 by compounds of the invention | | |
|---|---|---|
| Formula | Example No. | 11βHSD1 IC₅₀ values |
| (I) | 1 | 0.56 µM |
| (I) | 2 | 120 µM |
| (III) | 1 | 0.04 µm |
| (V) | 1 | 45 nM |
| (VI) | 1 | 6nM |
| (VII) | 2 | 118 nM |

## Claims

1. A compound of general formula (II) 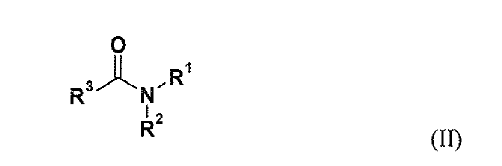 wherein
R¹ is C₃-C₁₀cycloalkyl or C₃-C₁₀hetcycloalkyl, wherein the cycloalkyl and hetcycloalkyl groups independently are optionally substituted with one or more of R⁴;
R² is hydrogen, C₁-C₈alkyl, arylC₁-C₆alkyl, wherein the alkyl and aryl groups independently are optionally substituted with one or more of R⁵; or
R¹ and R² together with the nitrogen to which they are attached, are forming a saturated or partially saturated cyclic, bicyclic or tricyclic ring system containing from 4 to 10 carbon atoms and from 0 to 2 additional heteroatoms selected from nitrogen, oxygen or sulphur, the ring system optionally being substituted with at least one of C₁-C₈alkyl, aryl, hetaryl, arylC₁-C₆alkyl, hetarylC₁-C₆alkyl, hydroxy, oxo, cyano, C₁-C₆alkyloxy, arylC₁-C₆alkyloxy, hetarylC₁-C₆alkyloxy, C₁-C₆alkyloxyC₁-C₆alkyl, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy or arylC₁-C₆alkylcarboxy wherein the alkyl and aryl groups independently are optionally substituted with one or more of R¹⁴;
R³ is C₃-C₁₀cycloalkyl, C₃-C₁₀hetcycloalkyl, or aryl, wherein the cycloalkyl, hetcycloalkyl, and aryl groups independently are optionally substituted with one or more of R⁷;
R⁴ and R⁵ independently are hydrogen, hydroxy, oxo, cyano, halo, methylendioxo, NR⁸R⁹, C₁-C₈alkyl, C₁-C₆alkyloxy, trihalomethyl, trihalomethyloxy, C₃-C₁₀cycloalkyl, C₃-C₁₀hetcycloalkyl, C₃-C₁₀cycloalkenyl, aryl, hetaryl, hetarylSOₙ, arylC₁-C₆alkyloxy, hetarylC₁-C₆alkyloxy, C₁-C₆alkyl-R⁶-C₁-C₆alkyl, arylC₁-C₆alkyl-R⁶-C₁-C₆alkyl, C₁-C₆alkylcarbonyl, arylcarbonyl, arylC₁-C₆alkylcarbonyl, hetarylcarbonyl, hetarylC₁-C₆alkylcarbonyl, C₁-C₆alkylSOₙ, C₁-C₆-alkylcarboxy, arylcarboxy, hetarylcarboxy, arylC₁-C₆alkylcarboxy or hetarylC₁-C₆alkylcarboxy wherein the alkyl, cycloalkyl, hetcycloalkyl, aryl and hetaryl groups independently are optionally substituted with one ore more of R¹⁵;
R⁶ is oxygen, sulphur, SOₙ or NR¹⁶;
R⁷ is hydrogen, halo, hydroxy, cyano, nitro, COOR¹⁷, C₁-C₈alkyl, C₃-C₁₀cycloalkyl, C₃-C₁₀hetcycloalkyl, methylendioxo, trihalomethyl, trihalomethyloxy, aryl, arylC₁-C₆alkyl, C₁-C₆alkyloxy, C₁-C₆alkyloxyC₁-C₆alkyl, aryloxy, arylC₁-C₆alkyloxy, aryloxyC₁-C₆alkyl, arylC₁-C₆alkyloxyC₁-C₆alkyl, hetaryl, hetarylC₁-C₆alkyl, hetaryloxy, hetarylC₁-C₆alkyloxy, hetaryloxyC₁-C₆alkyl, hetarylC₁-C₆alkyl-oxyC₁-C₆alkyl, NR^{s}R⁹, SOₘNR⁸R⁹, NR⁴R⁵carbonylC₁-C₆alkyl, arylthio, hetarylthio, R¹⁸carbonylNR⁸, arylSOₙ, hetarylSOₙ, R¹⁹SOₘNR⁸, arylthioC₁-C₆alkyl, hetarylthioC₁-C₆alkyl or arylC₁-C₆alkylR⁶C₁-C₆alkyl; wherein the aryl and hetaryl groups independently are optionally substituted with one or more R¹⁰;
R⁸ and R⁹ independently are hydrogen, C₁-C₈alkyl, aryl, hetaryl, C₁-C₆alkylSOₘ, arylSOₘ, arylC₁-C₆alkylSOₘ, arylC₁-C₆alkyl or hetarylC₁-C₆alkyl wherein the alkyl, aryl and hetaryl groups independently are optionally substituted with one or more of R¹¹; or
R⁸ and R⁹ together with the nitrogen to which they are attached, are forming a saturated or partially saturated cyclic, bicyclic or tricyclic ring system containing from 4 to 10 carbon atoms and from 0 to 2 additional heteroatoms selected from nitrogen, oxygen or sulfur, the ring system optionally being substituted with at least one halo, cyano, C₁-C₈alkyl, aryl, hetaryl, arylC₁-C₆alkyl, hetarylC₁-C₆alkyl, hydroxy, oxo, C₁-C₆alkyloxy, arylC₁-C₆alkyloxy, hetarylC₁-C₆alkyloxy, C₁-C₆alkyloxyC₁-C₆alkyl, C₁-C₆alkylcarbonyl, arylcarbonyl, hetarylearbonyl, arylC₁-C₆alkylcarbonyl, hetarylC₁-C₆alkylcarbonyl, C₁-C₆alkylcarboxy, arylcarboxy, hetarylcarboxy, arylC₁-C₆alkylcarboxy or hetarylC₁-C₆alkylcarboxy;
R¹⁰ and R¹¹ independently are hydrogen, hydroxy, oxo, halo, cyano, nitro, C₁-C₈alkyl, C₁-C₆alkyl-oxy, NR¹²R¹³, methylendioxo, trihalomethyl or trihalomethyloxy;
R¹² and R¹³ independently are hydrogen, C₁-C₈alkyl or arylC₁-C₆alkyl;
R¹⁴ is hydrogen, halo, hydroxy, oxo, nitro, cyano, C₁-C₈alkyl, C₁-C₆alkyloxy or aryloxy;
R¹⁵ is hydrogen, halo, hydroxy, oxo, nitro, cyano, CONR⁸R⁹ or COOR¹⁷;
R¹⁶ is hydrogen, C₁-C₈alkyl, C₃-C₁₀cycloalkyl, C₃-C₁₀hetcycloalkyl, aryl, arylC₁-C₆alkyl, hetaryl, hetarylC₁-C₆alkyl, alkylcarbonyl, arylcarbonyl, arylC₁-C₆alkylcarbonyl, aryloxyC₁-C₆alkyl, hetaryloxyC₁-C₆alkyl, arylthioC₁-C₆alkyl or hetarylthioC₁-C₆alkyl; wherein the alkyl, cycloalkyl, hetcycloalkyl, aryl and hetaryl groups independently are optionally substituted with one or more of R¹⁰;
R¹⁷ is hydrogen, C₁-C₈alkyl, aryl or arylC₁-C₆alkyl;
R¹⁸ is C₁-C₆alkyl, C₂-C₆alkenyl, aryl, arylC₁-C₆alkyl, hetaryl, hetarylC₁-C₆alkyl, C₃-C₁₀cycloalkyl, C₃-C₁₀hetcycloalkyl, C₁-C₆alkyloxy, aryloxy, arylC₁-C₆alkyloxy, arylC₁-C₆alkyloxyC₁-C₆alkyl, hetarylC₁-C₆alkyloxyC₁-C₆alkyl, hetaryloxy, hetarylC₁-C₆alkyloxy or R⁸R⁹NC₁-C₆alkyl wherein the alkyl, alkenyl, cycloalkyl, hetcycloalkyl, aryl and hetaryl groups are optionally substituted with R¹⁵;
R¹⁹ is C₁-C₆alkyl, C₃-C₁₀cycloalkyl, C₃-C₁₀hetcycloalkyl, aryl, arylC₁-C₆alkyl, hetaryl, hetarylC₁-C₆alkyl;
m is 1 or 2;
n is 0, 1 or 2; or
a salt thereof with a pharmaceutically acceptable acid or base, or any optical isomer or mixture of optical isomers, including a racemic mixture, or any tautomeric forms.

2. A compound according to claim 1, wherein R¹ is C₃-C₁₀cycloalkyl or C₃-C₁₀hetcycloalkyl, wherein the cycloalkyl and hetcycloalkyl groups independently are optionally substituted with one or more of R⁴.

3. A compound according to claim 1 or 2, wherein R² is hydrogen or C₁-C₈alkyl, wherein the alkyl group is optionally substituted with one or more of R⁵.

4. A compound according to claim 1 wherein R¹ and R² together with the nitrogen to which they are attached, are forming a saturated or partially saturated cyclic, bicyclic or tricyclic ring system containing from 4 to 10 carbon atoms and from 0 to 2 additional heteroatoms selected from nitrogen, oxygen or sulphur, the ring system optionally being substituted with at least one of C₁-C₈alkyl, aryl, hetaryl, arylC₁-C₆alkyl, hetarylC₁-C₆alkyl, hydroxy, oxo, cyano, C₁-C₆alkyloxy, arylC₁-C₆alkyloxy, hetarylC₁-C₆alkyloxy, C₁-C₆alkyloxyC₁-C₆alkyl, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy or arylC₁-C₆alkylcarboxy wherein the alkyl and aryl groups independently are optionally substituted with one or more of R¹⁴.

5. A compound according to any one of claims 1 to 4, wherein R³ is aryl, wherein the aryl group independently is optionally substituted with one or more of R⁷.

6. A compound according to any of the claims 1 to 5 wherein R⁴ and R⁵ independently are hydrogen, hydroxy, oxo, halo, C₁-C₈alkyl, wherein the alkyl group is optionally substituted with one or more of R¹⁵.

7. A compound according to any of the claims 1 to 6 wherein R⁷ is hydrogen, halo, hydroxy, cyano, C₁-C₈alkyl, C₃-C₁₀cycloalkyl, C₃-C₁₀het-cycloalkyl, trihalomethyl, aryl, arylC₁-C₆alkyl, C₁-C₆alkyloxy, C₁-C₆alkyloxyC₁-C₆alkyl, aryloxy, arylC₁-C₆alkyloxy, aryloxyC₁-C₆alkyl, arylC₁-C₆alkyloxyC₁-C₆alkyl, hetaryl, hetarylC₁-C₆alkyl, hetaryloxy, hetarylC₁-C₆alkyloxy, hetaryloxyC₁-C₆alkyl, hetarylC₁-C₆alkyl-oxyC₁-C₆alkyl, NR⁸R⁹, R¹⁸carbonyl0NR⁸, R¹⁹SOₘNR⁸; wherein the aryl and hetaryl groups independently are optionally substituted with one or more R¹⁰, preferably wherein R⁷ is halo, cyano, C₁-C₈alkyl, C₃-C₁₀hetcycloalkyl, trihalomethyl, aryl, C₁-C₆alkyloxy, aryloxy, arylC₁-C₆alkyloxy, aryloxyC₁-C₆alkyl, arylC₁-C₆alkyloxyC₁-C₆alkyl, hetaryl, hetaryloxy, hetarylC₁-C₆alkyloxy, hetaryloxyC₁-C₆alkyl, hetarylC₁-C₆alkykloxyC₁-C₆alkyl, NR⁸R⁹, R¹⁸carbonyNR⁸, or R¹⁹SOₘNR⁸, wherein the aryl and hetaryl groups are independently optionally substituted with one or more R¹⁰.

8. A compound according to any of the claims 1 to 7 wherein R⁸ and R⁹ together with the nitrogen to which they are attached, are forming a saturated or partially saturated cyclic, bicyclic or tricyclic ring system containing from 4 to 10 carbon atoms and from 0 to 2 additional heteroatoms selected from nitrogen, oxygen or sulfur, the ring system optionally being substituted with at least one halo, cyano, C₁-C₈alkyl, aryl, hetaryl, arylC₁-C₆alkyl, hetarylC₁-C₆alkyl, hydroxy, oxo, C₁-C₆alkyloxy, arylC₁-C₆alkyloxy, hetarylC₁-C₆alkyloxy, C₁-C₆alkyloxyC₁-C₆alkyl, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy, hetarylcarboxy, arylC₁-C₆alkylcarboxy or hetarylC₁-C₆alkylcarboxy.

9. A compound according to any of the claims 1 to 8 wherein R¹⁵ is CONR⁸R⁹.

10. A compound according to any of the claims 1 to 9 wherein R¹⁸ is C₁-C₆alkyl.

11. A compound according to any of the claims 1 to 10 which is:
(4-Tetrazol-1-yl-phenyl)-(1,3,3-trimethyl-6-aza-bicyclo[3.2.1]oct-6-yl)-methanone;
N-Cyclohexyl-N-methyl-2-phenoxymethyl-benzamide;
4-Amino-N-cyclohexyl-N-methyl-benzamide;
N-Cycloheptyl-N-methyl-2-phenoxymethyl-benzamide;
N-Cyclohexyl-N-methyl-benzamide;
2-Chloro-N-cyclohexyl-6-fluoro-N-methyl-benzamide;
N-Cyclohexyl-N-methyl-4-trifluoromethoxy-benzamide;
N-Cyclohexyl-2,3,N-trimethyl-benzamide;
3,5-Dichloro-N-cyclohexyl-N-methyl-benzamide;
N-Cyclohexyl-N-methyl-2-phenoxy-benzamide;
2,4-Bis-benzyloxy-N-cyclohexyl-N-methyl-benzamide;
2-Benzyloxy-N-cyclohexyl-N-methyl-benzamide;
N-Cyelohexyl-N-methyl-4-phenoxy-benzamide;
4-Benzyloxy-N-cyclohexyl-N-methyl-benzamide;
N-Cyclohexyl-N-methyl-4-phenoxymethyl-benzamide;
2-Chloro-N-cyclohexyl-N-ethyl-4-nitro-benzamide;
4-Chloro-N-cyclohexyl-N-ethyl-3-nitro-benzamide;
Azepan-1-yl-(2-chloro-phenyl)-methanone;
Azepan-1-yl-(3-chloro-phenyl)-methanone;
Azepan-1-yl-phenyl-methanone;
2-(Biphenyl-4-yloxy)-N-cyclohexyl-N-methyl-benzamide;
N-Cyclohexyl-2-(3,5-dimethoxy-phenoxy)-N-methyl-benzamide;
N-Cyclohexyl-2-(2,3-dimethoxy-phenoxy)-N-methyl-benzamide;
2,4-Dichloro-N-(3,3-dimethyl-1,5-dioxa-spiro[5.5]undec-9-yl)-N-methyl-benzamide;
2,4-Dichloro-N-methyl-N-(4-oxo-cyclohexyl)-benzamide;
N-Cyclohexyl-2-hydroxy-N-methyl-benzamide;
N-Cyclohexyl-3-methoxy-N-methyl-benzamide;
3-Benzyloxy-N-cyelohexyl-N-methyl-benzamide;
N-Cyclohexyl-3-hydroxy-N-methyl-benzamide;
[4-(Morpholine-4-sulfonyl)-phenyl]-(1,3,3-trimethyl-6-aza-bicyclo[3.2.1]oct-6-yl)-methanone;
N-Benzyl-3-(1,3,3-trimethyl-6-aza-bicyclo[3.2.1]octane-6-carbonyl)-benzenesulfonamide;
[4-Fluoro-3-(morpholine-4-sulfonyl)-phenyl]-(1,3,3-trimethyl-6-aza-bicyclo[3.2.1]oct-6-yl)-methanone;
Thiophene-2-sulfonic acid [4-(1,3,3-trimethyl-6-aza-bicyclo[3.2.1]octane-6-carbonyl)-phenyl]-amide;
N-Phenyl-4-(1,3,3-trimethyl-6-aza-bicyclo[3.2.1]octane-6-carbonyl)-benzenesulfonamide;
(4-Phenoxy-phenyl)-(1,3,3-trimethyl-6-aza-bicyclo[3.2,1]oct-6-yl)-methanone;
N-(2,4-Dimethyl-phenyl)-3-(1,3,3-trimethyl-6-aza-bicyclo[3.2.1]octane-6-carbonyl)-benzenesulfonamide;
(2-Phenoxymethyl-phenyl)-(1,3,3-trimethyl-6-aza-bicydo[3.2.1]oct-6-yl)-methanone;
4-(3-Aza-bicyclo[3.2.2]nonane-3-carbonyl)-N,N-dipropyl-benzenesulfonamide;
2-Bromo-N-cyclohexyl-N-methyl-benzamide;
N-[4-(1,3,3-Trimethyl-6-aza-bicyclo[3.2.1]octane-6-carbonyl)-phenyl]-acetamide;
(4-Dimethylamino-phenyl)-(1,3,3-trimethyl-6-aza-bicyclo[3.2.1]oct-6-yl)-methanone;
(4-Pyrrol-1-yl-phenyl)-(1,3,3-trimethyl-6-aza-bicyclo[3.2.1]oct-6-yl)-methanone;
(4-Imidazol-1-yl-phenyl)-(1,3,3-trimethyl-6-aza-bicyclo[3.2.1]oct-6-yl)-methanone;
(4-Amino-2-methoxy-phenyl)-(trimethyl-6-aza-bicyclo[3.2.1]oct-6-yl)-methanone;
(4-Methanesulfonyl-phenyl)-(1,3,3-trimethyl-6-aza-bicyclo[3.2.1]oct-6-yl)-methanone;
(3-Methanesulfonyl-phenyl)-(1,3,3-trimethyl-6-aza-bicyclo[3.2.1]oct-6-yl)-methanone;
(4-Benzenesulfonyl-phenyl)-(1,3,3-trimethyl-6-aza-blcyclo[3.2.1]oct-6-yl)-methanone;
Azepan-1-yl-[4-(3,4-dihydro-1H-isoquinolin-2-ylmethyl)-phenyl]-methanone;
Azepan-1-yl-(4-morpholin-4-ylmethyl-phenyl)-methanone;
[4-(3-Trifluoromethyl-pyrazol-1-yl)-phenyl]-(1,3,3-trimethyl-6-aza-bicycto[3.2.1]oct-6-yl)-methanone;
(4-[1,2,4]Triazol-1-yl-phenyl)-(1,3,3-trimethyl-6-aza-bicyclo[3.2.1]oct-6-yl)-methanone;
(4-Pyrazol-1-yl-phenyl)-(1,3,3-trimethyl-6-aza-bicyclo[3.2.1]oct-6-yl)-methanone;
2-Benzyloxymethyl-N-cyclohexyl-N-methyl-benzamide;
N-Cyctohexyl-N-methyl-4-(3-methyl-5-oxo-4,5-dihydro-pyrazol-1-yl)-benzamide;
5-Methyl-2-[4-(1,3,3-trimethyl-6-aza-bicyclo[3.2.1]octane-6-carbonyl)-phenyl]-2,4-dihydro-pyrazol-3-one;
[4-(3,5-Dimethyl-pyrazol-1-yl)-phenyl]-(1,3,3-trimethyl-6-aza-bicyclo[3.2.1]oct-6-yl)-methanone;
Phenyl-(1,3,3-trimethyl-6-aza-bicyclo[3.2.1]oct-6-yl)-methanone;
Azepan-1-yl-(2-bromo-phenyl)-methanone;
(3-Aza-bicyclo[3.2.2]non-3-yl)-(2-bromo-phenyl)-methanone;
1-[4-(1,3,3-Trimethyl-6-aza-bicyclo[3.2.1]octane-6-carbonyl)-phenyl]-pyrrolidine-2,5-dione;
4-(1,3,3-Trimethyl-6-aza-bicyclo[3.2.1]octane-6-carbonyl)-benzoicacid;
8-(4-Dimethylamino-benzoyl)-8-aza-bicyclo[3.2.1]octan-3-one;
(4-Dimethylamino-phenyl)-(3-hydroxy-8-aza-bicyclo[3.2.1]oct-8-yl)-methanone;
(4-Dimethylamino-phenyl)-(3-hydroxy-3-methyl-8-aza-bicyclo[3.2.1]oct-8-yl)-methanone;
Trifluoro-acetic acid 8-(4-dimethylamino-benzoyl)-8-aza-bicyclo[3.2.1]oct-3-yl ester;
or a salt thereof with a pharmaceutically acceptable acid or base, or any optical isomer or mixture of optical isomers, including a racemic mixture, or any tautomeric forms.

12. A combination of a compound as defined in any one of claims I to 11 and one or more further active substances.

13. A pharmaceutical composition comprising, as an active ingredient, at least one compound of any one of claims I to 11, or a combination as defined in claim 12, together with one or more pharmaceutically acceptable carriers or diluents, which composition preferably comprises from about 0.05 mg to about 2000 mg, or from about 1 mg to about 500 mg, of the compound.

14. A compound as defined in any one of claims 1 to 11, a combination as defined in claim 12, or a composition as defined in claim 13, for use as a medicament.

15. A compound, combination or composition according to claim 14 for use in the treatment, prevention, or prophylaxis of a disorder or disease wherein modulation or inhibition of the activity of 11βHSD1 is desirable, including metabolic syndrome, insulin resistance, dyslipidemia, hypertension, obesity, type 2 diabetes, impaired glucose tolerance (IGT), impaired fasting glucose (IFG), the delaying or prevention of the progression from IGT into type 2 diabetes, the delaying or prevention of the progression of the metabolic syndrome into type 2 diabetes, Latent Autoimmune Diabetes in the Adult (LADA), type 1 diabetes, diabetic late complications such as cardiovascular diseases, cardiovascular disorders, disorders of lipid metabolism, neurodegenerative and psychiatric disorders, dysregulation of intraocular pressure including glaucoma, immune disorders, inappropriate immune responses, musculo-skeletal disorders, gastrointestinal disorders, polycystic ovarie syndrome (PCOS), reduced hair growth or other diseases, disorders or conditions that are influenced by intracellular glucocorticoid levels, adverse effects of increased blood levels of active endogenous or exogenous glucocorticoid, adverse effects of increased plasma levels of endogenous active glucocorticoid, Cushing's disease, Cushing's syndrome, adverse effects of glucocorticoid receptor agonist treatment of autoimmune diseases, adverse effects of glucocorticoid receptor agonist treatment of inflammatory diseases, adverse effects of glucocorticoid receptor agonist treatment of diseases with an inflammatory component, adverse effects of glucocorticoid receptor agonist treatment as a part of cancer chemotherapy, adverse effects of glucocorticoid receptor agonist treatment for surgical/post-surgical or other trauma, adverse effects of glucocorticoid receptor agonist therapy in the context of organ or tissue transplantation or adverse effects of glucocorticoid receptor agonist treatment in other diseases, and disorders or conditions where glucocorticoid receptor agonists provide clinically beneficial effects.
